# EUROPEAN PATENT APPLICATION

(11) **EP 4 552 496 A1**
(43) Date of publication of application: **14.05.2025**
(21) Application number: 23819699.2
(22) Date of filing: 29.05.2023
(51) Int. Cl.: A01N 37/18

(54) **HERBICIDAL COMPOSITION CONTAINING DIFLUOROBUTENE ACID AMIDE COMPOUND**

(30) Priority: 10.06.2022 JP 2022094501
(71) Applicant: Ishihara Sangyo Kaisha, Ltd., Osaka-shi, Osaka 550-0002 (JP)
(72) Inventor: UEKI Toshihiko, Osaka-shi, Osaka 550-0002 (JP); NAKAMOTO Kenichi, Osaka-shi, Osaka 550-0002 (JP); TSUDA Kazuomi, Osaka-shi, Osaka 550-0002 (JP); TANAKA Hisaki, Osaka-shi, Osaka 550-0002 (JP); NAKAMURA Masayuki, Osaka-shi, Osaka 550-0002 (JP); HARA Yoshichika, Osaka-shi, Osaka 550-0002 (JP); NAITO Yu, Osaka-shi, Osaka 550-0002 (JP)
(74) Representative: Wächtershäuser & Hartz Patentanwaltspartnerschaft mbB
(86) International application number: PCT/JP2023/019879
(87) International publication number: WO 2023/238718

(57) **Abstract**

To provide a difluorobutenoic acid amide compound having excellent controlling effects on harmful weeds, and a herbicidal composition containing it.

A herbicidal composition comprising as an active ingredient a difluorobutenoic acid amide compound represented by the formula (1) or a salt thereof: 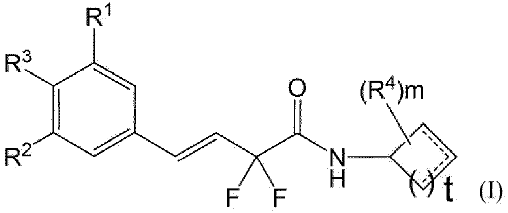 wherein the symbols are as defined in the specification, has excellent controlling effects on harmful weeds.

## Description

### TECHNICAL FIELD

The present invention relates to a novel difluorobutenoic acid amide compound or a salt thereof, a herbicidal composition containing it as an active ingredient.

### BACKGROUND ART

Patent Document 1 discloses cinnamamide derivatives having herbicidal activity. However, Patent Document 1 does not disclose difluorobutenoic acid amide compounds represented by the following formula (I), having substituents on phenyl and C₄-C₅ alicyclic hydrocarbon (hereinafter sometimes referred to as specific difluorobutenoic acid amide compounds).

Patent Document 2 discloses aromatic-aliphatic carboxylic acid derivatives as a safener. However, Patent Document 2 does not disclose specific difluorobutenoic acid amide compounds represented by the following formula (I).

On the other hand, 1-[{4-(3-fluorophenyl)-1-oxo-3-buten-1-yl}amino]cyclopropanecarboxylic acid (CAS Registry Number: 1608641-02-6), cis-3-[{(3E)-4-(4-fluorophenyl)-1-oxo-3-buten-1-yl}amino]cyclobutanecarboxylic acid (CAS Registry Number: 2026935-61-3), 3-[{4-(4-fluorophenyl)-1-oxo-3-buten-1-yl}amino]cyclopentanecarboxylic acid (CAS Registry Number: 1608649-05-3) and **N-**[(1S,4R)-4-(hydroxymethyl)-2-cyclopenten-1-yl]-4-(4-methylphenyl)-3-butenamide (CAS Registry Number: 1808292-36-5) are known, however, their application is unknown. Needless to say, herbicidal activity of these compounds has not been known.

Further, Patent Document 3 and Non-Patent Document 1 disclose a method for producing N-cyclohexyl-4-phenyl-2,2-difluoro-3-butenamide compound.

Non-Patent Document 2 discloses a method for preparing bicyclic amidines. Non-Patent Document 2 describes, in Supporting Information, physical property data of N-cyclohexyl-4-phenyl-2,2-difluoro-3-butenamide analogues as by-product of cyclization reaction of Entry 9 in Table 1.

However, Patent Document 3, Non-Patent Document 1 and Non-Patent Document 2 do not disclose controlling effects of the described compounds on undesired plants or inhibitory effects on their growth. Further, they do not disclose at all the specific difluorobutenoic acid amide compounds represented by the following formula (I).

### PRIOR ART DOCUMENTS

### PATENT DOCUMENTS

Patent Document 1: Chinese Patent Publication No. 109896973
Patent Document 2: WO2005/016001
Patent Document 3: Chinese Patent Publication No. 113511972

### NON-PATENT DOCUMENTS

Non-Patent Document 1: European Journal Organic Chemistry 2021, 48, 6700-6704
Non-Patent Document 2: European Journal Organic Chemistry 2011, 36, 7317-7323

### DISCLOSURE OF INVENTION

### TECHNICAL PROBLEM

For labor-saving in operation to control undesired plants (hereinafter sometimes referred to simply as harmful weeds) and improvement in productivity of agricultural and horticultural crop plants, herbicides are desired to have excellent herbicidal effects on harmful weeds at a low dose when applied to soil, stems and leaves, etc. Further, herbicides which have less environmental burden (influence over surrounding plants and ecosystem) and are thereby highly safe for useful plants, are desired. The object of the present invention is to provide a difluorobutenoic acid amide compound and a herbicidal composition containing it, having excellent controlling effects on harmful weeds and having excellent safety for useful plants.

### SOLUTION TO PROBLEM

The present inventors have synthesized specific difluorobutenoic acid amide compounds represented by the following formula (I), the herbicidal activity of which has not been known, and conducted extensive studies on their herbicidal activity and usefulness. As a result, they have found that a composition comprising as an active ingredient the specific difluorobutenoic acid amide compound represented by the following formula (I) or a salt thereof has excellent controlling effects on weeds. They have further found that the compounds of the present invention have high safety for useful plants. The present invention has been accomplished based on these discoveries.

That is, the present invention is as follows.

A herbicidal composition (hereinafter also referred to as the present composition) comprising as an active ingredient a difluorobutenoic acid amide compound represented by the formula (I) or a salt thereof (hereinafter also referred to as compound (I) or the Invention Compound): 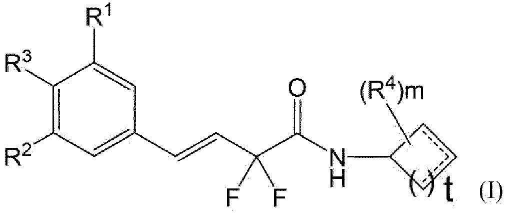
wherein R¹ and R² are each a halogen, a (C₁-C₆)-chain hydrocarbon which may be substituted by at least one Y¹, a (C₁-C₆)-alkoxy which may be substituted by at least one halogen, cyano or H,
R³ is H or a fluorine atom (excluding a case where all of R¹, R² and R³ are H),
Y¹ is a halogen, a (C₁-C₆)-alkoxy or cyano,
t is 1 or 2,
R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, -C(=O)H, -N(R¹⁰)C(=O)R¹¹, - SR⁶, -S(=O)R⁶, -S(=O)₂R⁶, =CHC(=O)R¹¹, cyano, OH, 1,3-dioxolan-2-yl, 2-thiazolyl, 2-imidazolyl which may be substituted by at least one Z⁴, =CHCH=CHC(=O)R¹¹, - (C=O)₂R¹¹, -C(=NR¹¹)-C(=O)R¹¹ or -CH(OH)-C(=O)R¹¹,
R⁵ is a (C₁-C₆)-alkyl which may be substituted by at least one Z¹, H, a (C₃-C₆)-cycloalkyl, a (C₂-C₆)-alkenyl, a (C₂-C₆)-alkynyl or a 4- to 6-membered oxygen-containing saturated heterocyclic ring,
Z¹ is cyano, a halogen, a (C₁-C₆)-alkylthio, a (C₁-C₆)-alkylsulfonyl, a (C₁-C₆)-alkoxy, phenyl, pyridyl, a (C₁-C₆)-alkoxycarbonyl, a (C₃-C₆)-cycloalkyl, tert-butyldimethylsilyloxy, OH, amino or 1,3-dioxolan-4-yl,
R⁶ is a (C₁-C₆)-alkyl which may be substituted by at least one halogen, a (C₃-C₆)-cycloalkyl or benzyl,
R⁷ is a (C₁-C₆)-alkyl which may be substituted by at least one Y², H, a (C₁-C₆)-alkoxy, a (C₂-C₆)-alkenyl, a (C₂-C₆)-alkynyl, -C(=O)R⁶, 3-pyridazinyl, a (C₂-C₆)-alkenyloxy, phenoxy, benzyl, a (C₁-C₆)-alkylsulfonyl or OH,
R⁸ is H, a (C₁-C₆)-alkyl or a (C₁-C₆)-alkoxy,
R ⁷ and R⁸ together may form a completely saturated 4- to 6-membered ring with N to which they are bonded, the ring may be substituted by at least one halogen, and the carbon atom constituting the ring may be replaced with a hetero atom,
Y² is a (C₁-C₆)-alkoxy, a (C₁-C₆)-alkylthio, a (C₁-C₆)-alkoxycarbonyl, -(C=O)NH-(CH₂)-C(=O)R¹¹, a halogen or cyano,
R⁹ is a (C₁-C₆)-alkyl which may be substituted by at least one Z², or 1-imidazolyl,
Z² is a (C₁-C₆)-alkoxycarbonyl,
Z³ is a halogen, a (C₁-C₆)-alkoxy, cyano, OH, a (C₁-C₆)-alkoxycarbonyl, a (C₁-C₆)-alkylcarbonyloxy, a (C₁-C₆)-alkylsulfonyloxy or -C(=O)OH,
Z⁴ is a (C₁-C₆)-alkyl, a (C₁-C₆)-alkoxymethyl or a (C₁-C₆)-alkoxycarbonyl,
R¹⁰ is H,
R¹¹ is a (C₁-C₆)-alkoxy,
m is 1, 2, 3 or 4, and
the broken line moiety of carbon-carbon bond is a single bond or a double bond (provided that two broken line moieties of carbon-carbon bond are not simultaneously double bonds); a method for controlling undesired plants or inhibiting their growth, which comprises applying an effective amount of the compound (I) to harmful weeds, useful plants or soil.

The present invention further relates to difluorobutenoic acid amide compounds represented by the formula (I) or a salt thereof, which are a group of compounds which have not been known specifically and which have particularly excellent controlling effects on undesired plants.

### ADVANTAGEOUS EFFECTS OF INVENTION

The present composition has excellent controlling effects on undesired plants and is useful as a herbicide.

### DESCRIPTION OF EMBODIMENTS

In the compound (I), in a case where the number of R⁴ as a substituent is two or more, the respective R⁴ may be identical or different from each other, and in a case where the number of R¹¹ as a substituent is two or more, the respective R¹¹ may be identical or different from each other.

In the compound (I), the halogen or halogen as a substituent may be each atom of fluorine, chlorine, bromine or iodine. The number of the halogen as a substituent may be one or more, and in the case of two or more, the respective halogen atoms may be identical or different from each other. Further, the substitution position of the halogen as a substituent may be any position.

The expression "C_{P}-C_{T}" means that the number of carbon atoms is from P to T. For example, the expression "C₁-C₃" means that the number of carbon atoms is from 1 to 3. Further, the expression "which may be substituted" means having a substituent or being unsubstituted.

In the compound (I), in a case where t is 1, an alicyclic hydrocarbon having 4 carbon atoms (C₄) is formed, and in a case where t is 2, an alicyclic hydrocarbon having 5 carbon atoms (Cs) is formed. The C₄ or C₅ alicyclic hydrocarbon means cyclobutyl, cyclopentyl, 2-cyclobutenyl, 3-cyclopentenyl or 4-cyclopentenyl.

The (C₁-C₆)-chain hydrocarbon means a (C₁-C₆)-alkyl, a (C₂-C₆)-alkenyl or a (C₂-C₆)-alkynyl.

The (C₁-C₆)-alkyl or the (C₁-C₆)-alkyl moiety may be a linear or branched alkyl group having 1 to 6 carbon atoms such as methyl, ethyl, n-propyl, isopropyl, n-butyl, isobutyl, sec-butyl, tert-butyl, n-pentyl, neopentyl or n-hexyl.

The (C₂-C₆)-alkenyl or (C₂-C₆)-alkenyl moiety may, for example, be a linear or branched alkenyl group having 2 to 6 carbon atoms, having at least one double bond at an optional position, such as vinyl, 1-propenyl, 2-propenyl, isopropenyl, 2-methyl-1-propenyl, 1-methyl-1-propenyl, 2-methyl-2-propenyl, 1-methyl-2-propenyl, 1-butenyl, 2-butenyl, 3-butenyl, 1-pentenyl, 2-pentenyl, 2-methyl-2-butenyl, 3-methyl-2-butenyl, 1-hexenyl or 2,3-dimethyl-2-butenyl. Further, in a case where such a group has geometrical isomers, the group may be one of E-isomer and Z-isomer or may be a mixture of E-isomer and Z-isomer in an optional ratio and is not particularly limited so long as it has carbon atoms within a specified range.

The (C₂-C₆)-alkynyl or the (C₂-C₆)-alkynyl moiety may, for example, be a linear or branched alkynyl group having 2 to 6 carbon atoms, having at least one triple bond at an optional position, such as ethynyl, 1-propynyl, 2-propynyl, 1-butynyl, 2-butynyl, 3-butynyl, 1-methyl-2-propynyl, 2-methyl-3-butynyl, 1-hexynyl, 2-hexynyl, 3-hexynyl, 4-hexynyl or 5-hexynyl.

The substitution position of Y¹ as a substituent on the (C₁-C₆)-chain hydrocarbon which may be substituted by at least one Y¹, may be any position of the (C₁-C₆)-chain hydrocarbon substituted by Y¹. In a case where the number of Y¹ is two or more, the respective Y¹ may be identical or different from each other.

The (C₁-C₆)-chain hydrocarbon which may be substituted by at least one Y¹, when substituted by at least one Y¹, means a linear or branched C₁-C₆ hydrocarbon of which one or more hydrogen atoms are replaced with Y¹. Specifically, it may, for example, be a linear or branched alkyl group having 1 to 6 carbon atoms, partially or completely substituted by one or more identical or different Y¹, such as fluoromethyl, chloromethyl, bromomethyl, iodomethyl, difluoromethyl, dichloromethyl, trifluoromethyl, chlorodifluoromethyl, trichloromethyl, bromodifluoromethyl, 1-fluoroethyl, 1-chloroethyl, 1-bromoethyl, 1,1-difluoroethyl, 1,1-dichloroethyl, 2-fluoroethyl, 2-chloroethyl, 2-bromoethyl, 2,2-difluoroethyl, 2,2,2-trifluoroethyl, 2-chloro-2,2-difluoroethyl, 2,2,2-trichloroethyl, 1,1,2,2-tetrafluoroethyl, 2-chloro-1,1,2-trifluoroethyl, pentafluoroethyl, 3,3,3-trifluoropropyl, 2,2,3,3,3-pentafluoropropyl, 1,1,2,3,3,3-hexafluoropropyl, heptafluoropropyl, 2,2,2-trifluoro-1-(trifluoromethyl)ethyl, 1,2,2,2-tetrafluoro-1-(trifluoromethyl)ethyl, 2,2,3,3,4,4,4-heptafluorobutyl, nonafluorobutyl, methoxymethyl, 1-methoxyethyl, 2-methoxyethyl, 1-methoxypropyl, 2-methoxypropyl, 3-methoxypropyl, 1-methoxybutyl, 2-methoxybutyl, 3-methoxybutyl, 4-methoxybutyl, 3-methoxy-1-methylpropyl, 3-methoxy-2-methylpropyl, 2-methoxy-1,1-dimethylethyl, 5-methoxypentyl, 4-methoxy-1-methylbutyl, 6-methoxyhexyl, ethoxymethyl, 2-ethoxyethyl, 3-ethoxypropyl, 4-ethoxybutyl, 5-ethoxypentyl, 6-ethoxyhexyl, n-propyloxymethyl, 2-n-propyloxyethyl, 3-n-propyloxypropyl, 4-n-propyloxybutyl, 5-n-propyloxypentyl, 6-n-propyloxyhexyl, n-butyloxymethyl, 2-n-butyloxyethyl, 3-n-butyloxypropyl, 4-n-butyloxybutyl, 5-n-butyloxypentyl, 6-n-butyloxyhexyl, n-pentyloxymethyl, 2-n-pentyloxyethyl, 3-n-pentyloxypropyl, 4-n-pentyloxybutyl, 5-n-pentyloxypentyl, 6-n-pentyloxyhexyl, n-hexyloxymethyl, 2-n-hexyloxyethyl, 3-n-hexyloxypropyl, 4-n-hexyloxybutyl, 5-n-hexyloxypentyl, 6-n-hexyloxyhexyl, cyanomethyl, dicyanomethyl, 1-cyanoethyl, 2-cyanoethyl, 3-cyanopropyl, 4-cyanobutyl, 5-cyanopentyl or 6-cyanohexyl.

In addition, a linear or branched alkenyl group having 2 to 6 carbon atoms, partially or completely substituted by one or more identical or different Y¹ may, for example, be mentioned, such as 1-chloroethenyl, 2-chloroethenyl, 2-fluoroethenyl, 2,2-dichloroethenyl, 2,2-difluoroethenyl, 3-chloro-2-propenyl, 3-fluoro-2-propenyl, 2-chloro-2-propenyl or 4-chloro-3-butenyl. Further, a linear or branched alkynyl group having 2 to 6 carbon atoms, partially or completely substituted by one or more identical or different Y¹ may, for example, be mentioned such as 2-chloroethynyl, 2-bromoethynyl, 2-iodoethynyl, 3-chloro-2-propynyl or 4-chloro-2-butynyl.

The substitution position of Z³ as a substituent on the (C₁-C₆)-chain hydrocarbon which may be substituted by at least one Z³, may be any position on the (C₁-C₆)-chain hydrocarbon substituted by Z³. In a case where the number of Z³ is two or more, the respective Z³ may be identical or different from each other.

The substitution position of Z¹ as a substituent on the (C₁-C₆)-alkyl which may be substituted by at least one Z¹, may be any position on the (C₁-C₆)-alkyl substituted by Z¹. In a case where the number of Z¹ is two or more, the respective Z¹ may be identical or different from each other.

The substitution position of Z² as a substituent on the (C₁-C₆)-alkyl which may be substituted by at least one Z², may be any position on the (C₁-C₆)-alkyl substituted by Z². In a case where the number of Z² is two or more, the respective Z² may be identical or different from each other.

The (C₁-C₆)-alkyl which may be substituted by at least one halogen, when substituted, means a linear or branched C₁-C₆ hydrocarbon of which one or more hydrogen atoms are replaced with a halogen atom. Specifically, it may, for example, be a linear or branched alkyl group having 1 to 6 carbon atoms, partially or completely substituted by one or more identical or different halogen, such as fluoromethyl, chloromethyl, bromomethyl, iodomethyl, difluoromethyl, dichloromethyl, trifluoromethyl, chlorodifluoromethyl, trichloromethyl, bromodifluoromethyl, 1-fluoroethyl, 1-chloroethyl, 1-bromoethyl, 1,1-difluoroethyl, 1,1-dichloroethyl, 2-fluoroethyl, 2-chloroethyl, 2-bromoethyl, 2,2-difluoroethyl, 2,2,2-trifluoroethyl, 2-chloro-2,2-difluoroethyl, 2,2,2-trichloroethyl, 1,1,2,2-tetrafluoroethyl, 2-chloro-1,1,2-trifluoroethyl, pentafluoroethyl, 3,3,3-trifluoropropyl, 2,2,3,3,3-pentafluoropropyl, 1,1,2,3,3,3-hexafluoropropyl, heptafluoropropyl, 2,2,2-trifluoro-1-(trifluoromethyl)ethyl, 1,2,2,2-tetrafluoro-1-(trifluoromethyl)ethyl, 2,2,3,3,4,4,4-heptafluorobutyl or nonafluorobutyl.

The substitution position of Y² as a substituent on the (C₁-C₆)-alkyl which may be substituted by at least one Y², may be any position on the (C₁-C₆)-alkyl substituted by Y². In a case where the number of Y² is two or more, the respective Y² may be identical or different from each other.

The (C₁-C₆)-alkoxy or (C₁-C₆)-alkoxy moiety may, for example, be a linear or branched alkoxy group having 1 to 6 carbon atoms, such as methoxy, ethoxy, n-propyloxy, isopropyloxy, n-butyloxy, sec-butyloxy, tert-butyloxy, n-pentyloxy, isopentyloxy or n-hexyloxy.

The (C₁-C₆)-alkoxy which may be substituted by at least one halogen, which is substituted, means a linear or branched C₁-C₆ alkoxy of which one or more hydrogen atoms are replaced with a halogen atom. It may, for example, be a linear or branched alkoxy group having 1 to 6 carbon atoms, partially or completely substituted by one or more identical or different halogen atoms, such as fluoromethoxy, difluoromethoxy, trifluoromethoxy, chlorodifluoromethoxy, bromodifluoromethoxy, dichlorofluoromethoxy, chloromethoxy, dichloromethoxy, trichloromethoxy, bromomethoxy, 1-fluoroethoxy, 2-fluoroethoxy, 2-chloroethoxy, 2-bromoethoxy, 2,2-difluoroethoxy, 2,2,2-trifluoroethoxy, 1,1,2,2-tetrafluoroethoxy, pentafluoroethoxy, 2,2,2-trichloroethoxy, 1-fluoropropyloxy, 2-fluoropropyloxy, 3-fluoropropyloxy, 3-chloropropyloxy, 3-bromopropyloxy, 1-fluorobutyloxy, 2-fluorobutyloxy, 3-fluorobutyloxy, 4-fluorobutyloxy or 4-chlorobutyloxy.

The (C₂-C₆)-alkenyloxy moiety may, for example, be a linear or branched alkenyloxy group having 2 to 6 carbon atoms, such as vinyloxy, 1-propenyloxy, 2-propenyloxy, isopropenyloxy, 2-methyl-1-propenyloxy, 1-methyl-1-propenyloxy, 2-methyl-2-propenyloxy, 1-methyl-2-propenyloxy, 1-butenyloxy, 2-butenyloxy, 3-butenyloxy, 1-pentenyloxy, 2-pentenyloxy, 2-methyl-2-butenyloxy, 3-methyl-2-butenyloxy, 1-hexenyloxy or 2,3-dimethyl-2-butenyloxy. Further, in a case where such a group has geometrical isomers, the group may be one of E-isomer and Z-isomer or may be a mixture of E-isomer and Z-isomer in an optional ratio and is not particularly limited so long as it has carbon atoms within a specified range.

The (C₁-C₆)-alkylthio moiety may, for example, be a linear or branched alkylthio group having 1 to 6 carbon atoms, such as methylthio, ethylthio, n-propylthio, isopropylthio, n-butylthio, sec-butylthio, tert-butylthio, n-pentylthio, isopentylthio or n-hexylthio.

The (C₁-C₆)-alkylsulfonyl moiety may, for example, be a linear or branched alkylsulfonyl group having 1 to 6 carbon atoms, such as methylsulfonyl, ethylsulfonyl, n-propylsulfonyl, isopropylsulfonyl, n-butylsulfonyl, sec-butylsulfonyl, n-pentylsulfonyl, isopentylsulfonyl or n-hexylsulfonyl.

The (C₁-C₆)-alkylsulfonyloxy moiety may, for example, be a linear or branched (C₁-C₆)-alkylsulfonyloxy group having 1 to 6 carbon atoms, such as methylsulfonyloxy, ethylsulfonyloxy, n-propylsulfonyloxy, isopropylsulfonyloxy, n-butylsulfonyloxy, sec-butylsulfonyloxy, n-pentylsulfonyloxy, isopentylsulfonyloxy or n-hexylsulfonyloxy.

The (C₁-C₆)-alkoxycarbonyl moiety may, for example, be a linear or branched alkoxycarbonyl group having 1 to 6 carbon atoms, such as methoxycarbonyl, ethoxycarbonyl, n-propyloxycarbonyl, isopropyloxycarbonyl, n-butyloxycarbonyl, sec-butyloxycarbonyl, n-pentyloxycarbonyl, isopentyloxycarbonyl or n-hexyloxycarbonyl.

The (C₁-C₆)-alkylcarbonyloxy moiety may, for example, be a linear or branched alkylcarbonyloxy group having 1 to 6 carbon atoms, such as methylcarbonyloxy, ethylcarbonyloxy, n-propylcarbonyloxy, isopropylcarbonyloxy, n-butylcarbonyloxy, sec-butylcarbonyloxy, tert-butylcarbonyloxy, n-pentylcarbonyloxy, isopentylcarbonyloxy or n-hexylcarbonyloxy.

The (C₃-C₆)-cycloalkyl moiety may, for example, be a cycloalkyl group having 3 to 6 carbon atoms, such as cyclopropyl, cyclobutyl, cyclopentyl or cyclohexyl.

2-imidazolyl which may be substituted by at least one Z⁴, means 2-imidazolyl substituted by one, two or three Z⁴ or unsubstituted 2-imidazolyl. The substitution position of Z⁴ as a substituent may be any position on 2-imidazolyl substituted by Z⁴. In a case where the number of Z⁴ is 2 or 3, the respective Z⁴ may be identical or different from each other.

The 4- to 6-membered oxygen-containing saturated heterocyclic ring means a 4-to 6-membered saturated heterocyclic ring containing one or two oxygen atoms as hetero atoms. Specifically, it may, for example, be 3-oxetanyl, 2-tetrahydrofuranyl, 3-tetrahydrofuranyl, 1,3-dioxolan-4-yl, 2-tetrahydropyranyl, 3-tetrahydropyranyl, 4-tetrahydropyranyl, 1,3-dioxan-4-yl, 1,3-dioxan-5-yl.

The pyridyl means 2-pyridyl, 3-pyridyl or 4-pyridyl.

In the compound (I), in a case where R⁴ is -C(=O)NR⁷R⁸, R⁷ and R⁸ together may form a completely saturated 4- to 6-membered ring with N to which they are bonded, the ring may be substituted by at least one halogen, and the carbon atom constituting the ring may be replaced with a hetero atom. The hetero atom may be O, S, S(=O), S(=O)₂ or N, and the number of the hetero atom in the ring if formed is usually 1. Specific examples of the ring formed by -C(=O)NR⁷R⁸ include the formulae (a-1) to (a-14). # represents the bonding position.

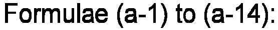

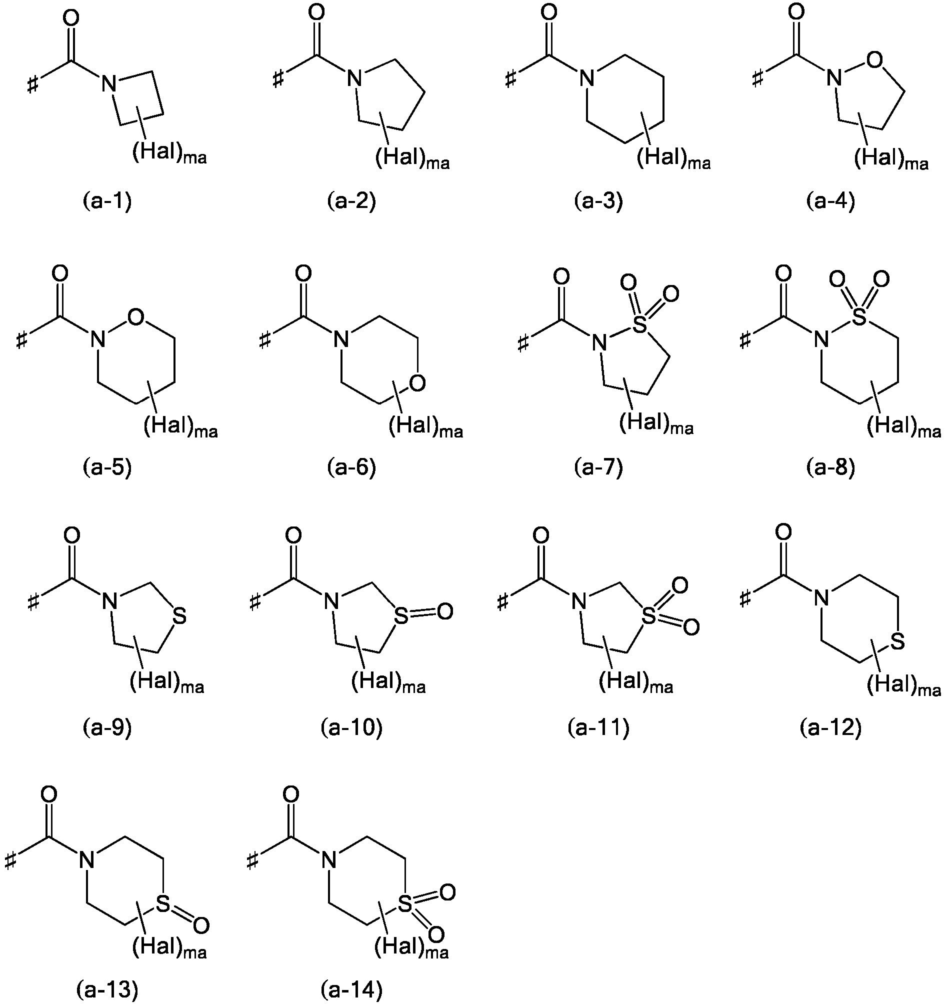

In the formulae, ma is 0, 1, 2 or 3, and Hal is a halogen.

In the formula (I), the broken line moiety of carbon-carbon bond (hereinafter also referred to simply as broken line moiety) described below is a single bond or a double bond. However, the two broken line moieties are not simultaneously double bonds.

The formula (I) in which t is 1, and the two broken line moieties are both single bonds, represents a compound represented by the formula (IAa) or a salt thereof (hereinafter also referred to as compound (IAa)): 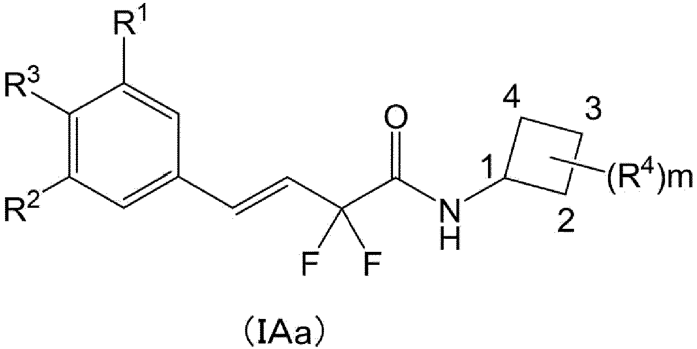 wherein R¹, R², R³, R⁴ and m are as defined for the formula (I). In the compound (IAa), in a case where the number of R⁴ as a substituent is 2 or more, the respective R⁴ may be identical or different from each other.

The formula (I) in which t is 1, and one of the two broken line moieties is a double bond and the other is a single bond, represents a compound represented by the formula (IAb) or a salt thereof (hereinafter also referred to as compound (IAb)): 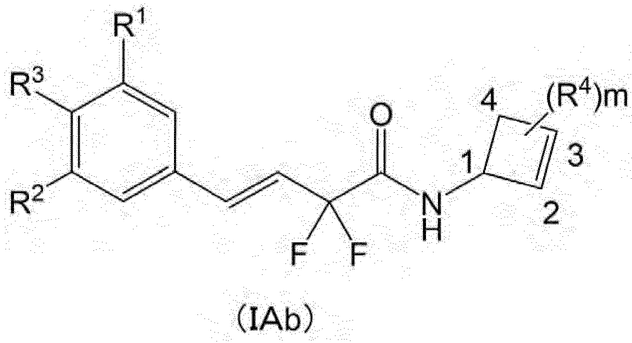 wherein R¹, R², R³, R⁴ and m are as defined for the formula (I). In the compound (IAb), in a case where the number of R⁴ as a substituent is 2 or more, the respective R⁴ may be identical or different from each other.

The formula (I) in which t is 2, and the two broken line moieties are both single bonds, represents a compound represented by the formula (IBa) or a salt thereof (hereinafter also referred to as compound (IBa)): 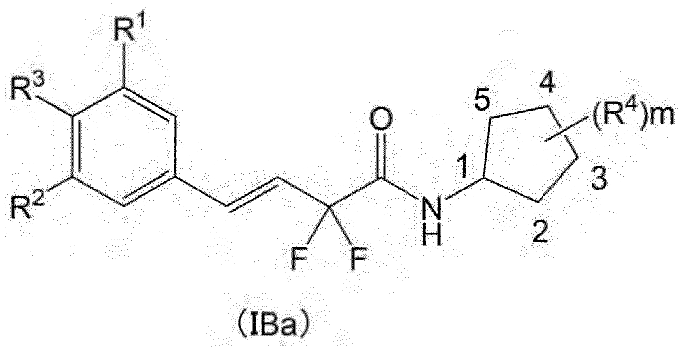 wherein R¹, R², R³, R⁴ and m are as defined for the formula (I). In the compound (IBa), in a case where the number of R⁴ as a substituent is 2 or more, the respective R⁴ may be identical or different from each other.

The formula (I) in which t is 2, and one of the two broken line moieties is a double bond and the other is a single bond, represents a compound represented by the formula (IBb) or formula (IBc) or a salt thereof (hereinafter, a compound represented by the formula (IBb) will also be referred to as compound (IBb), and a compound represented by the formula (IBc) will also be referred to as compound (IBc)): 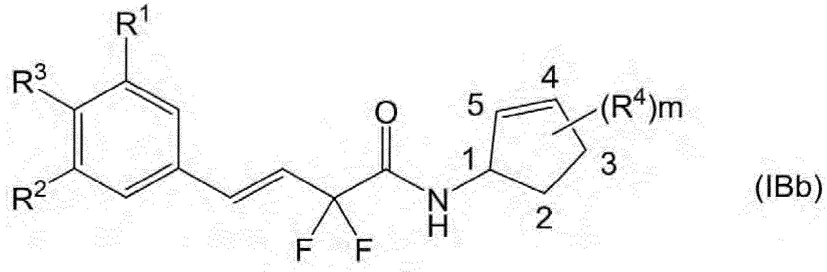 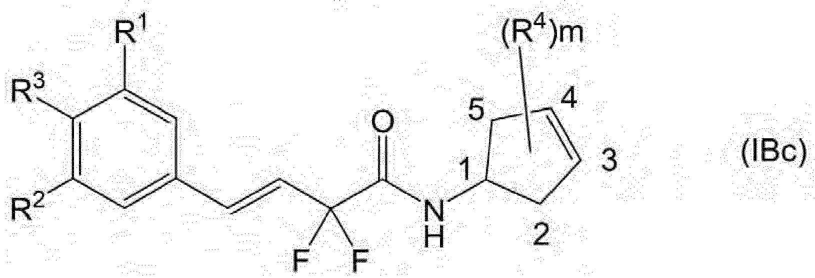 wherein R¹, R², R³, R⁴ and m are as defined for the formula (I). In the compound (IBb) or (IBc), in a case where the number of R⁴ as a substituent is 2 or more, the respective R⁴ may be identical or different from each other.

Accordingly, the compound (I) includes the above compounds (IAa), (IAb), (IBa), (IBb) and (IBc). Further, the description regarding the compound (I) in this specification includes the descriptions for the compounds (IAa), (IAb), (IBa), (IBb) and (IBc). The respective symbols in the compounds (IAa), (IAb), (IBa), (IBb) and (IBc) have the same meanings in the respective symbols in the compound (I).

In the present invention, preferred is a compound of the formula (I) wherein t is 1 or 2, and the two broken line moieties are both single bonds, and preferred is a compound of the formula (I) wherein t is 2, and one of the two broken line moieties is a single bond and the other is a double bond. That is, in the present invention, the compounds (IAa), (IBa), (IBb) and (IBc) are preferred.

The salts of the compound (I) include all kinds of salts so long as they are agriculturally acceptable, and, for example, alkali metal salts (such as a sodium salt and a potassium salt), alkaline earth metal salts (such as a calcium salt and a magnesium salt), amine salts (such as a dimethylamine salt and a triethylamine salt), inorganic acid salts (such as a hydrochloride, a perchlorate, a sulfate and a nitrate), and organic acid salts (such as an acetate and a methanesulfonate) may be mentioned.

As the compound (I), isomers such as optical isomers and geometrical isomers may sometimes be present, and the present invention includes the respective isomers and isomer mixtures. In the compound (I), various isomers other than the above-mentioned isomers are included within a range of common knowledge in this technical field. Further, an individual isomer may be synthesized by common knowledge and general experimental means in this technical field.

Further, depending upon the type of an isomer, it may have a chemical structure different from the described structural formula, but for those skilled in the art, it is sufficiently recognizable that such a chemical structure is in a relation of an isomer, and such an isomer is evidently within the scope of the present invention.

Now, the method for producing the compound (I) will be described.

The compound (I) may be produced in accordance with the following Production Process A (Reactions A to Z and AA to AC) and a conventional method for producing a salt, however, the method to obtain the compound is not limited to such a method. For example, the compound (I) of the present invention may be produced by applying a substituent transformation (such as alkylation, haloalkylation, cross coupling reaction such as Suzuki coupling, Sandmeyer reaction, halogenation, oxidation or reduction) known in this technical field, on the substituent on phenyl. Further, in the production of the Invention Compounds, protection and deprotection reaction commonly employed in this technical field may be applied as the case requires. The reaction may be carried out in an atmosphere of an inert gas such as nitrogen or argon as the case requires, and a salt reagent may be used.

### Production Process A

### [Reaction A]

Reaction A is a method of reacting a compound of the formula (II) with a compound of the formula (X-1) to obtain a compound of the formula (I).

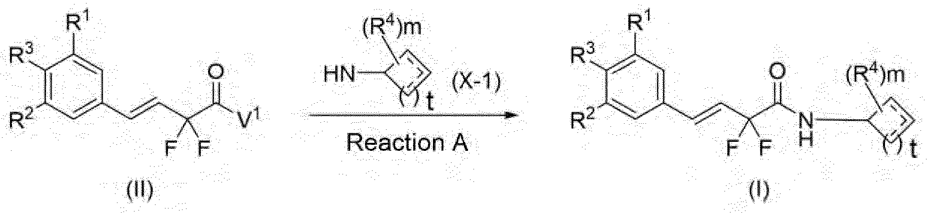

In the formulae, V¹ is OH, a chlorine atom or a bromine atom, and the other symbols are as define above.

Reaction A may be carried out usually in the presence of a base and a solvent, and optionally with the addition of a dehydration condensation agent. In a case where a specific dehydration condensation agent such as a carbodiimide condensation agent, an imidazole condensation agent or a triazine condensation agent is used, the reaction will proceed even in the absence of a base. The compound of the formula (X-1) may be used in an amount of 0.5 to 10 equivalents, preferably 1 to 5 equivalents to 1 equivalent of the compound of the formula (II) (equivalents means molar equivalents, the same applies hereinafter).

As the base, one or more may properly be selected and mixed from carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (II).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, **N-**methylpyrrolidone, pyridine, acetonitrile and propionitrile; ketones such as acetone and methyl ethyl ketone; protic polar solvents such as methanol and ethanol; water, and the like.

As the dehydration condensation agent, one or more may properly be selected and mixed from carbodiimide condensation agents such as N,N'-dicyclohexylcarbodiimide (DCC), 1-[3-(dimethylamino)propyl]-3-ethylcarbodiimide (EDC) or its hydrochloride; imidazole condensation agents such as 1,1'-carbonyldiimidazole (CDI); triazine condensation agents such as 4-(4,6-dimethoxy-1,3,5-triazin-2-yl)-4-methylmorpholinium chloride (DMT-MM); phosphonium condensation agents such as 1H-benzotriazol-1-yloxy tripyrrolidinophosphonium hexafluorophosphate (PyBOP); uronium condensation agents such as 1-[bis(dimethylamino)methylene]-1H-1,2,3-triazolo[4,5-b]pyridinium 3-oxide hexafluorophosphate (HATU); acid anhydrides such as 2-methyl-6-nitrobenzoic anhydride (MNBA); 2-halopyridinium salts such as 2-chloro-1-methylpyridinium p-toluenesulfonate; propylphosphonic acid anhydride (cyclic trimer) (T3P); diphenylphosphoryl azide (DPPA); and the like. Optionally, a conventional additive used in combination with a dehydration condensation agent, such as 1-hydroxybenzotriazole (HOBt) or 4-N,N-dimethylaminopyridine (DMAP), may be added. In a case where an additive is used in Reaction A, the additive may be used alone or as a mixture of two or more. The dehydration condensation agent may be used in an amount of 0.5 to 10 equivalents, preferably 1 to 5 equivalents to 1 equivalent of the compound of the formula (II), and the additive may be used in an amount of 0.1 to 10 equivalents, preferably 1 to 5 equivalents to 1 equivalent of the compound of the formula (II).

In Reaction A, the reaction temperature is about -30°C to 80°C, preferably about - 10°C to 50°C, and the reaction time is usually about 10 minutes to 30 hours, preferably about 30 minutes to 15 hours. In a case where the dehydration condensation agent is used in Reaction A, the reaction temperature is about -30°C to 120°C, preferably about 0°C to 80°C, and the reaction time is usually about 10 minutes to 100 hours, preferably about 30 minutes to 30 hours.

The compound of the formula (II) used in Reaction A may be produced in accordance with the following Reaction 1-5, Reaction 1-6 or a known method. The compound of the formula (X-1) may be commercially available.

### [Reaction B] and [Reaction C]

Reaction B is a method of hydrolyzing a compound of the formula (I-1) to obtain a compound of the formula (I-2). Reaction C is a method of reacting the compound of the formula (I-2) with a compound of the formula (X-2) to obtain a compound of the formula (I-3).

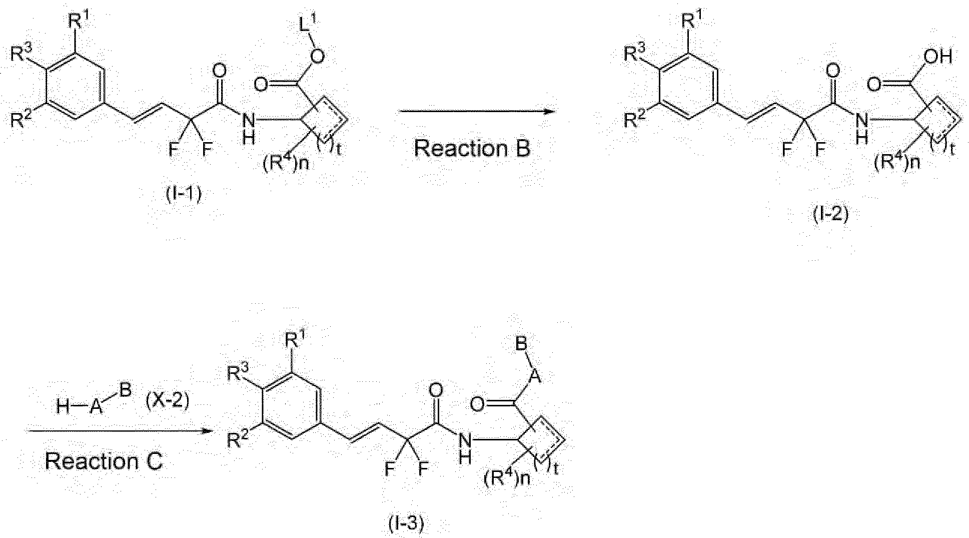

In the formulae, A is -O-, -S- or -NR⁸-, L¹ is a (C₁-C₆)-chain hydrocarbon, and n is 0, 1, 2 or 3 (n=0 means there is no R⁴ as a substituent). In a case where A is -O-, B is a (C₁-C₆)-alkyl which may be substituted by at least one Z¹, H, a (C₃-C₆)-cycloalkyl, a (C₂-C₆)-alkenyl, a (C₂-C₆)-alkynyl, or a 4- to 6-membered oxygen-containing saturated heterocyclic ring. In a case where A is -S-, B is a (C₁-C₆)-alkyl which may be substituted by at least one halogen, a (C₃-C₆)-cycloalkyl, or benzyl. In a case where A is -NR⁸-, B is a (C₁-C₆)-alkyl which may be substituted by at least one Y², H, a (C₁-C₆)-alkoxy, a (C₂-C₆)-alkenyl, a (C₂-C₆)-alkynyl, -C(=O)R⁶, 3-pyridazinyl, a (C₂-C₆)-alkenyloxy, phenoxy, benzyl, a (C₁-C₆)-alkylsulfonyl or OH. Further, in a case where A is -NR⁸-, B and R^{B} together may form a completely saturated 4- to 6-membered ring with **N** to which they are bonded, the ring may be substituted by at least one halogen, and the carbon atom constituting the ring may be replaced with a hetero atom. The other symbols are as defined above.

Reaction B may be carried out usually by mixing the compound of the formula (I-1) with a base or an acid in the presence of a solvent.

The base may, for example, be an alkali metal hydroxide such as lithium hydroxide, sodium hydroxide or potassium hydroxide; an alkali metal carbonate such as sodium carbonate, potassium carbonate or cesium carbonate; an alkali metal hydrogencarbonate such as sodium hydrogencarbonate or potassium hydrogencarbonate; an alkaline earth metal carbonate such as calcium carbonate or barium carbonate; or an alkaline earth metal hydroxide such as calcium hydroxide or barium hydroxide, but is not limited thereto. As the base, one or more may properly be selected and mixed from them. The base may be used in an amount of 1 to 10 equivalents, preferably 1 to 5 equivalents to 1 equivalent of the compound of the formula (I-1).

The acid may, for example, be an organic acid such as formic acid, acetic acid, methanesulfonic acid, p-toluenesulfonic acid, trifluoroacetic acid or trifluoromethanesulfonic acid; or an inorganic acid such as hydrochloric acid, sulfuric acid or hydrogen bromide, but is not limited thereto. As the acid, one or more may properly be selected and mixed from them. The acid may be used in an amount of 0.1 to 30 equivalents, preferably 1 to 10 equivalents to 1 equivalent of the compound of the formula (I-1).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; aprotic polar solvents such as acetonitrile, N,N-dimethylformamide, dimethyl sulfoxide, N,N-dimethylacetamide, hexamethylphosphoric triamide and sulfolane; protic polar solvents such as methanol, ethanol and isopropanol; water; and the like.

In a case where the base is used in Reaction B, the reaction temperature is about -30°C to 120°C, preferably about -10°C to 80°C, and the reaction time is usually about 10 minutes to 30 hours, preferably about 30 minutes to 15 hours. In a case where the acid is used in Reaction B, the reaction temperature is about -30°C to 200°C, preferably about 0°C to 100°C, and the reaction time is usually about 10 minutes to 30 hours, preferably about 30 minutes to 15 hours.

The compound of the formula (I-1) used in Reaction B may be produced in accordance with the above Reaction A or a known method.

Reaction C may be carried out in accordance with the above Reaction A. The compound of the formula (X-2) may be used in an amount of 0.5 to 10 equivalents, preferably 1 to 5 equivalents to 1 equivalent of the compound of the formula (I-2). The base may be used in an amount of 0.5 to 10 equivalents, preferably 1 to 5 equivalents to 1 equivalent of the compound of the formula (I-2). The dehydration condensation agent may be used in an amount of 0.5 to 10 equivalents, preferably 1 to 5 equivalents to 1 equivalent of the compound of the formula (I-2), and the additive may be used in an amount of 0.1 to 10 equivalents, preferably 1 to 5 equivalents to 1 equivalent of the compound of the formula (I-2).

The compound of the formula (X-2) used in Reaction C may be commercially available.

### [Reaction D]

Reaction D is a method of reacting the compound of the formula (I-1) with a compound of the formula (X-3) to obtain a compound of the formula (I-4).

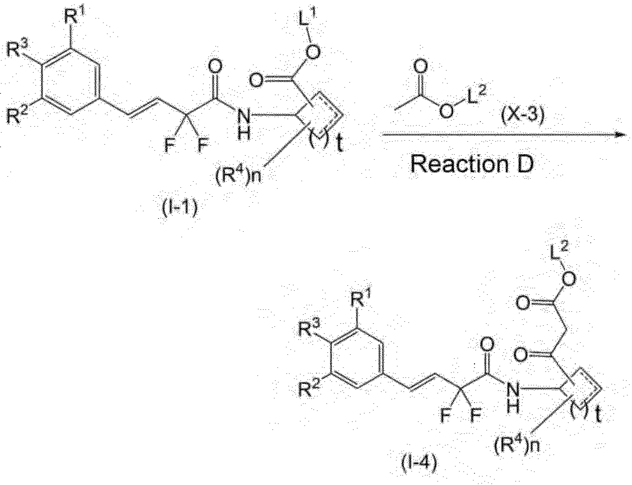

In the formulae, L² is (C₁-C₆)-alkyl. The other symbols are as defined above.

Reaction D may be carried out by mixing the compound of the formula (I-1) with the compound of the formula (X-3) usually in the presence of a solvent and a base. The compound of the formula (X-3) may be used in an amount of 0.5 to 10 equivalents, preferably 1 to 5 equivalents to 1 equivalent of the compound of the formula (I-1).

As the base, one or more may properly be selected and mixed from metal amine salts such as lithium diisopropylamide and lithium bis(trimethylsilyl)amide; metal hydrides such as sodium hydride and potassium hydride; metal alkoxides such as potassium tert-butoxide and lithium tert-butoxide; and the like. The base may be used in an amount of 1 to 10 equivalents, preferably 1 to 5 equivalents to 1 equivalent of the compound of the formula (I-1).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; and the like.

In Reaction D, the reaction temperature is about -100°C to 100°C, preferably about -80°C to 50°C, and the reaction time is usually about 10 minutes to 30 hours, preferably about 1 hour to 15 hours.

The compound of the formula (I-1) used in Reaction D may be produced in accordance with the above Reaction A or a known method. The compound of the formula (X-3) may be commercially available.

### [Reaction E]

Reaction E is a method of reacting a compound of the formula (I-3-1) with an alkylating agent to obtain a compound of the formula (I-5).

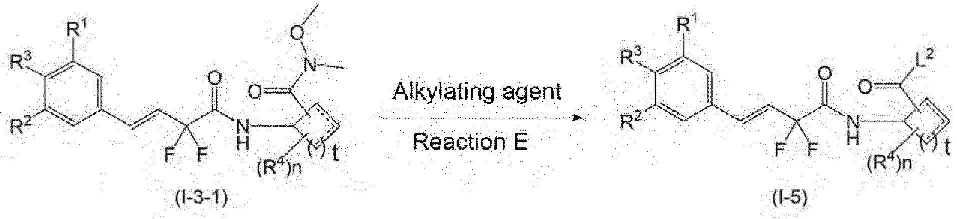

In the formulae, the symbols are as defined above.

Reaction E may be carried out by mixing the compound of the formula (I-3-I) with an alkylating agent usually in the presence of a solvent.

The alkylating agent may, for example, be a metal alkyl salt such as methyl lithium or n-butyl lithium, but is not limited thereto. The alkylating agent may be used in an amount of 0.5 to 10 equivalents, preferably 1 to 5 equivalents to 1 equivalent of the compound of the formula (I-3-1).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; and the like.

In Reaction E, the reaction temperature is about -100°C to 100°C, preferably about -80°C to 50°C, and the reaction time is usually about 10 minutes to 30 hours, preferably about 1 hour to 15 hours.

The compound of the formula (I-3-1) used in Reaction E may be produced in accordance with the above Reaction C or a known method.

### [Reaction F]

Reaction F is a method of reacting the compound of the formula (I-2), diphenylphosphoryl azide (DPPA) and a compound of the formula (X-4) to obtain a compound of the formula (I-6).

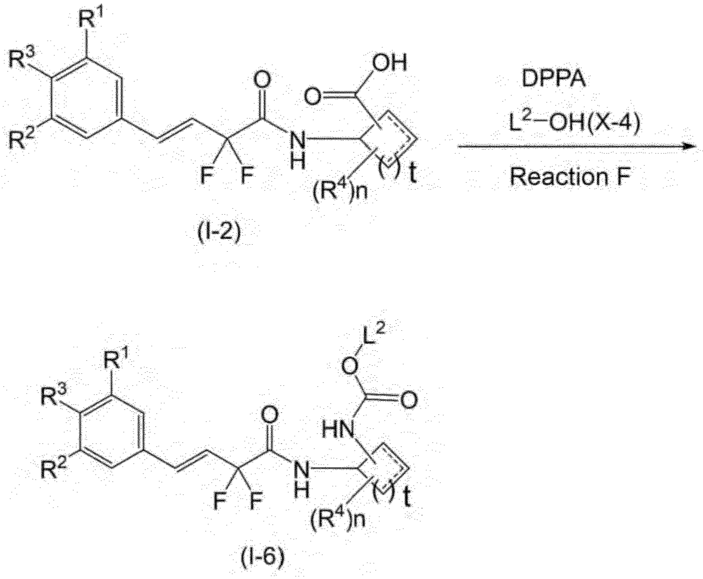

The symbols in the formula are as defined above.

Reaction F may be carried out by mixing the compound of the formula (I-2), DPPA and the compound of the formula (X-4) usually in the presence of a solvent and a base. DPPA may be used in an amount of 0.5 to 10 equivalents, preferably 1 to 5 equivalents to 1 equivalent of the compound of the formula (I-2). The compound of the formula (X-4) may be used in an amount of 1 to 100 equivalents, preferably 1 to 30 equivalents to 1 equivalent of the compound of the formula (I-2).

As the base, one or more may properly be selected and mixed from carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 1 to 30 equivalents, preferably 1 to 10 equivalents to 1 equivalent of the compound of the formula (I-2).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; and the like.

In Reaction F, the reaction temperature is about -30°C to 180°C, preferably about 30°C to 130°C, and the reaction time is usually about 10 minutes to 30 hours, preferably about 30 minutes to 15 hours.

The compound of the formula (I-2) used in Reaction F may be produced in accordance with the above Reaction B or a known method. The compound of the formula (X-4) may be commercially available.

### [Reaction G]

Reaction G is a method of reacting the compound of the formula (1-1) with a reducing agent to obtain a compound of the formula (I-7).

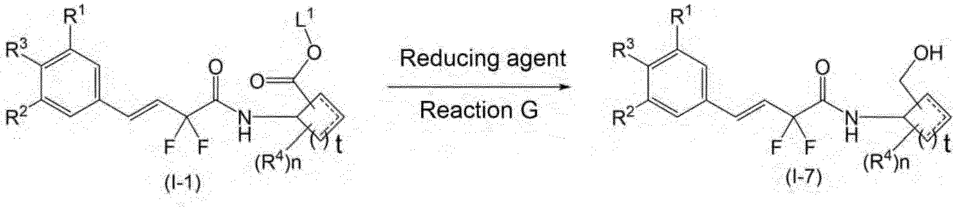

The symbols in the formulae are as defined above.

Reaction G may be carried out by mixing the compound of the formula (I-1) with a reducing agent usually in the presence of a solvent.

The reducing agent may, for example, be a metal hydride such as lithium borohydride, sodium borohydride, lithium aluminum hydride or diisobutyl aluminum hydride but is not limited thereto. The reducing agent may be used in an amount of 0.5 to 10 equivalents, preferably 1 to 5 equivalents to 1 equivalent of the compound of the formula (I-1).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; protic polar solvents such as methanol and ethanol; water; and the like.

In Reaction G, the reaction temperature is about -100°C to 100°C, preferably about -80°C to 50°C, and the reaction time is usually about 10 minutes to 30 hours, preferably about 1 hour to 15 hours.

The compound of the formula (I-1) used in Reaction G may be produced in accordance with the above Reaction A or a known method.

### [Reaction H], [Reaction I] and [Reaction J]

Reaction H is a method of reacting the compound of the formula (I-7) with a compound of the formula (X-5) to obtain a compound of the formula (I-8). Reaction I is a method of reacting the compound of the formula (I-7) with a halogenating agent to obtain a compound of the formula (I-9). Reaction J is a method of reacting the compound of the formula (I-9) with a cyanating agent to obtain a compound of the formula (I-10).

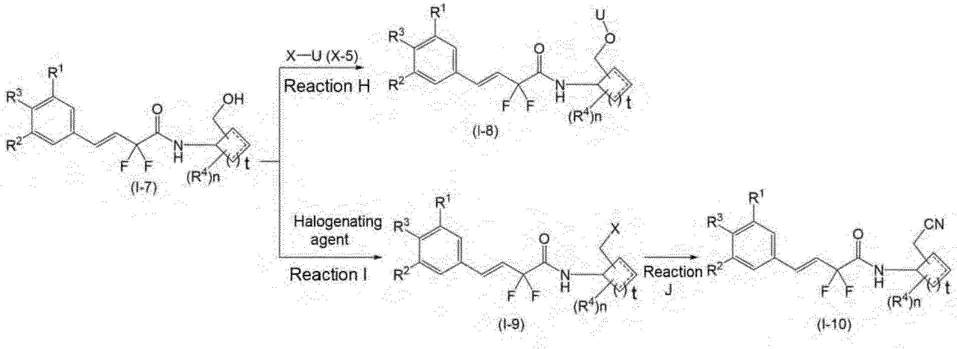

In the formulae, U is a (C₁-C₆)-alkylcarbonyl or a (C₁-C₆)-alkylsulfonyl, X is each atom of fluorine, chlorine, bromine or iodine. The other symbols are as defined above.

Reaction H may be carried out by reacting the compound of the formula (I-7) with the compound of the formula (X-5) usually in the presence of a solvent and a base. The compound of the formula (X-5) may be used in an amount of 0.5 to 10 equivalents, preferably 1 to 5 equivalents to 1 equivalent of formula (I-7).

As the base, one or more may properly be selected and mixed from carbonates such as sodium carbonate and potassium carbonate; hydrogencarbonates such as sodium hydrogencarbonate and potassium hydrogen carbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (I-7).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; protic polar solvents such as methanol and ethanol; water; and the like.

In Reaction H, the reaction temperature is about -30°C to 120°C, preferably about 0°C to 80°C, and the reaction time is usually about 10 minutes to 30 hours, preferably about 30 minutes to 15 hours.

Reaction I may be carried out by mixing the compound of the formula (I-7) with a halogenating agent usually in the base of a solvent and optionally a base.

As the halogenating agent, one or more may properly be selected and mixed from metal fluorides such as potassium fluoride and cesium fluoride; phosphorus halides such as phosphorus trichloride and phosphorus tribromide; halogen molecules such as bromine and iodine; carbon tetrahalides such as carbon tetrachloride and carbon tetrabromide; and the like. The halogenating agent may be used in an amount of 0.5 to 10 equivalents, preferably 1 to 5 equivalents to 1 equivalent of the compound of the formula (I-7). Further, in a case where a carbon tetrahalide is used as the halogenating agent, triphenylphosphine is used as the additive (Appel Reaction). In a case where triphenylphosphine is used, triphenylphosphine may be used in an amount of 0.5 to 10 equivalents, preferably 1 to 5 equivalents to 1 equivalent of the compound of the formula (I-7).

As the base, one or more may properly be selected and mixed from carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.1 to 10 equivalents, preferably 1 to 5 equivalents to 1 equivalent of the compound of the formula (I-7).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; and the like.

In Reaction I, the reaction temperature is about -30°C to 150°C, preferably about 0°C to 100°C, and the reaction time is usually about 10 minutes to 30 hours, preferably about 30 minutes to 15 hours.

The compound of the formula (I-7) used in Reaction H and Reaction I may be produced in accordance with the above Reaction G or a known method. The compound of the formula (X-5) used in Reaction H may be commercially available.

Reaction J may be carried out by mixing the compound of the formula (I-9) and a cyanating agent usually in the presence of a solvent.

As the cyanating agent, one or more may properly be selected and mixed from metal cyanides such as sodium cyanide and potassium cyanide; and the like. The cyanating agent may be used in an amount of 0.5 to 10 equivalents, preferably 1 to 5 equivalents to 1 equivalent of the compound of the formula (I-9).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; aprotic polar solvents such as dimethyl sulfoxide, sulfolane, N,N-dimethylacetamide, N,N-dimethylformamide, N-methylpyrolidone, pyridine, acetonitrile and propionitrile; and the like.

In Reaction J, the reaction temperature is about -30°C to 120°C, preferably about 0°C to 80°C, and the reaction time is usually about 10 minutes to 30 hours, preferably about 30 minutes to 15 hours.

### [Reaction K] and [Reaction L]

Reaction K is a method of reacting the compound of the formula (I-7) with an oxidizing agent to obtain a compound of the formula (I-11). Reaction L is Horner-Wadsworth-Emmons Reaction, and is a method of reacting the compound of the formula (I-11) with a compound of the formula (X-6) to obtain a compound of the formula (I-12).

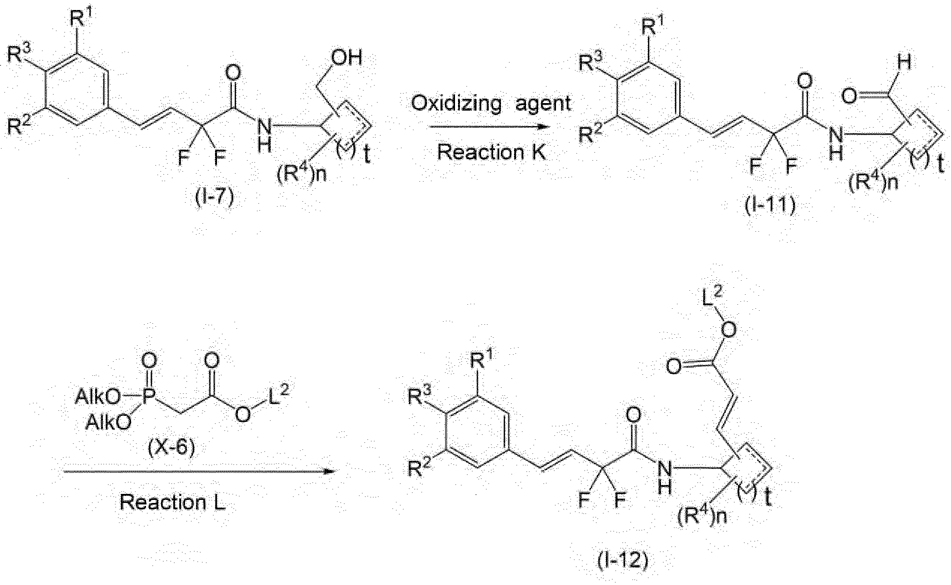

In the formulae, Alk is a (C₁-C₆)-alkyl. The other symbols are as defined above.

Reaction K may be carried out by mixing the compound of the formula (I-7) with an oxidizing agent usually in the presence of a solvent.

The oxidizing agent is not particularly limited so long as the reaction proceeds, and may, for example, be a hypervalent iodine compound such as Dess-Martin Periodinane used in Dess-Martin oxidation; a metal oxide such as manganese dioxide; a sulfur oxide such as sulfur trioxide pyridine complex; or dimethyl sulfoxide (DMSO) used in Swern oxidation reaction. The oxidizing agent may be used in an amount of 0.5 to 30 equivalents, preferably 1 to 10 equivalents to 1 equivalent of the compound of the formula (I-7). In a case where Swern oxidation reaction is employed, oxalyl chloride or acetic anhydride as an activating agent is added. Each of such activating agents may be used in an amount of 0.5 to 10 equivalents, preferably 1 to 5 equivalents to 1 equivalent of the compound of the formula (I-7).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; and the like.

In Reaction K, the reaction temperature is about -80°C to 100°C, preferably about 0°C to 50°C, and the reaction time is usually about 10 minutes to 30 hours, preferably about 1 hour to 15 hours. In a case where Swern oxidation reaction is employed in Reaction K, the reaction temperature is preferably about -80°C to -60°C.

The compound of the formula (I-7) used in Reaction K may be produced in accordance with the above Reaction G or a known method.

Reaction L is Horner-Wadsworth-Emmons Reaction. That is, Reaction L may be carried out by mixing the compound of the formula (I-11) with the compound of the formula (X-6) usually in the presence of a solvent and a base. The compound of the formula (X-6) may be used in an amount of 0.5 to 10 equivalents, preferably 1 to 5 equivalents to 1 equivalent of the compound of the formula (I-11).

As the base, one or more may properly be selected and mixed from metal amine salts such as lithium diisopropylamide and lithium bis(trimethylsilyl)amide; metal hydrides such as sodium hydride and potassium hydride; metal alkoxides such as potassium tert-butoxide and lithium tert-butoxide; and the like. The base may be used in an amount of 1 to 10 equivalents, preferably 1 to 5 equivalents to 1 equivalent of the compound of the formula (I-11).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, hexane and cyclohexane; ethers such as dioxane, tetrahydrofuran, diethyl ether and dimethoxyethane; and the like.

In Reaction L, the reaction temperature is about -100°C to 100°C, preferably about -80°C to 50°C, and the reaction time is usually about 10 minutes to 30 hours, preferably about 1 hour to 15 hours.

The compound of the formula (X-6) used in Reaction L may be commercially available.

### [Reaction M]

Reaction M is a method of reacting a compound of the formula (II-1) with a compound of the formula (X-7) to obtain a compound of the formula (III).

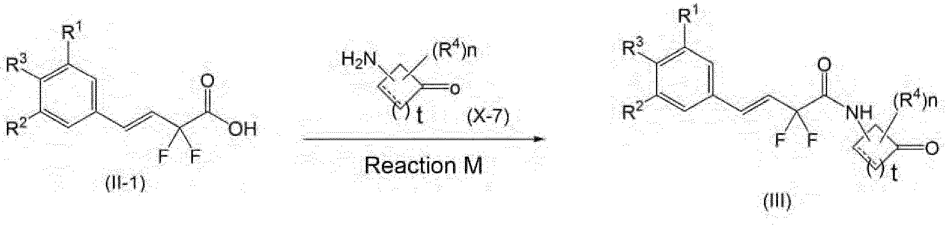

The symbols in the formulae are as defined above.

Reaction M may be carried out in accordance with the above Reaction A. The compound of the formula (X-7) may be used in an amount of 0.5 to 10 equivalents, preferably 1 to 5 equivalents to 1 equivalent of the compound of the formula (II-1). The base may be used in an amount of 0.5 to 10 equivalents, preferably 1 to 5 equivalents to 1 equivalent of the compound of the formula (II-1). The dehydration condensation agent may be used in an amount of 0.5 to 10 equivalents, preferably 1 to 5 equivalents to 1 equivalent of the compound of the formula (II-1), and the additive may be used in an amount of 0.1 to 10 equivalents, preferably 1 to 5 equivalents to 1 equivalent of the compound of the formula (II-1).

The compound of the formula (II-1) used in Reaction M may be produced in accordance with the following Reaction 1-5 or a known method. The compound of the formula (X-7) used in Reaction M may be commercially available.

### [Reaction N]

Reaction N is a method of reducing a compound of the formula (III-1), a compound of the formula (III-2), a compound of the formula (III-3) or a compound of the formula (III-4) to obtain a compound of the formula (I-13).

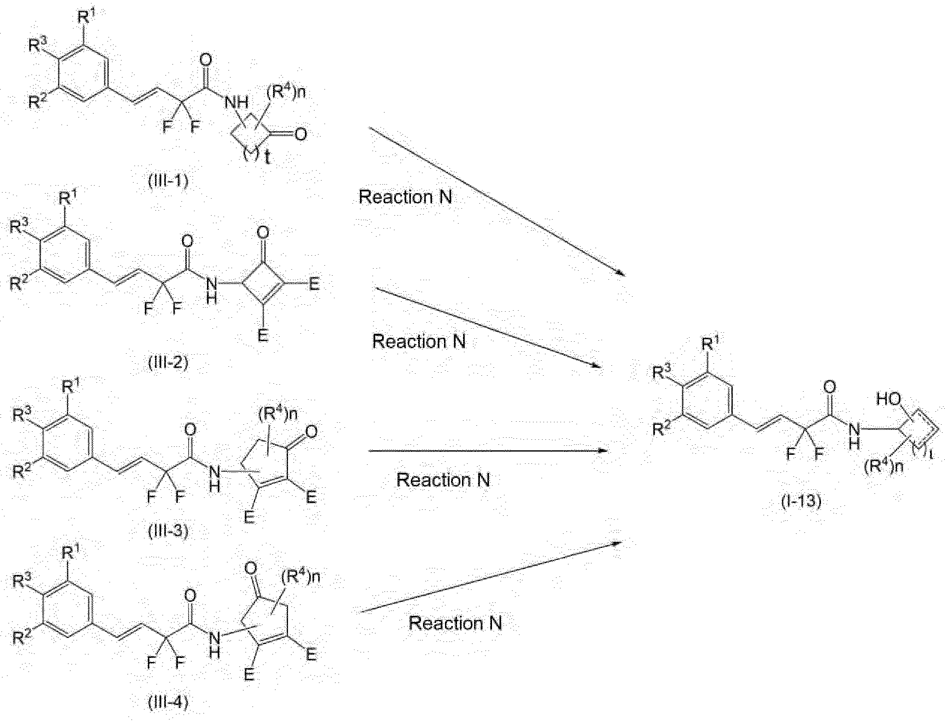

In the formulae, E is H, a C₁-C₆ alkyl or a halogen, and the other symbols are as defined above.

Reaction N may be carried out in accordance with the above Reaction G. The reducing agent may be used in an amount of 0.5 to 10 equivalents, preferably 1 to 5 equivalents to 1 equivalent of each of the compounds of the formula (III-1) to the compound of the formula (III-4).

The compound of the formula (III-1) to the compound of the formula (III-4) used in Reaction N may be produced in accordance with the above Reaction M or a known method.

### [Reaction O]

Reaction O is Horner-Wadsworth-Emmons Reaction, and is a method of reacting the compound of the formula (III-1) with the compound of the formula (X-6) to obtain a compound of the formula (I-14). It is also a method of reacting the compound of the formula (III-2) with the compound of the formula (X-6) to obtain a compound of the formula (I-15). It is also a method of reacting the compound of the formula (III-3) with the compound of the formula (X-6) to obtain a compound of the formula (I-16). It is also a method of reacting the compound of the formula (III-4) with the compound of the formula (X-6) to obtain a compound of the formula (I-17).

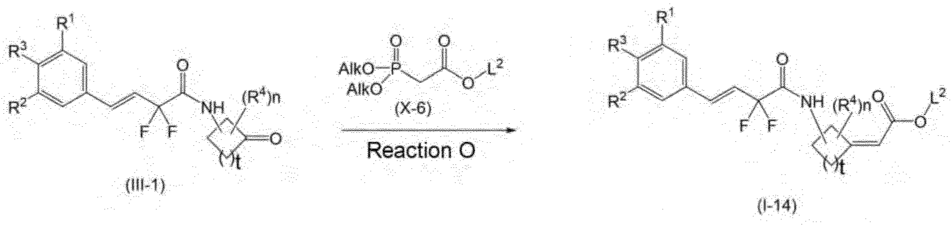

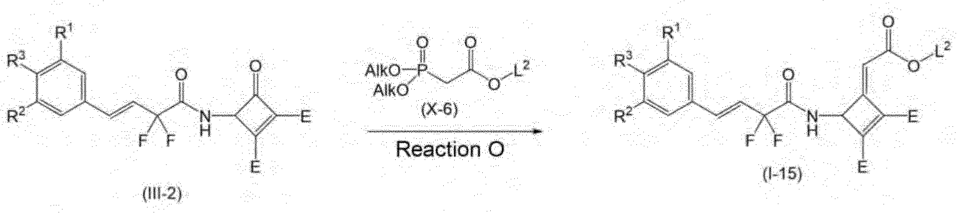

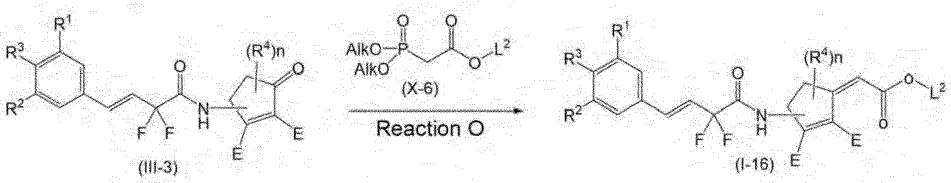

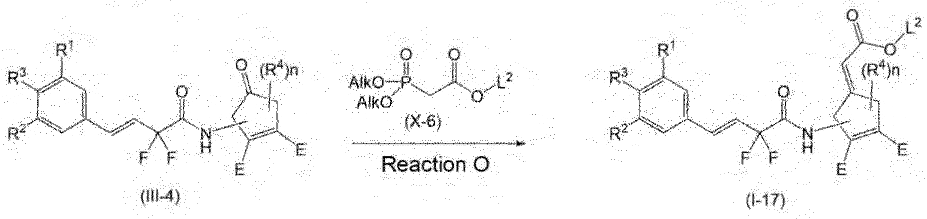

The symbols in the formulae are as defined above.

Reaction O may be carried out in accordance with the above Reaction L. The compound of the formula (X-6) may be used in an amount of 0.5 to 10 equivalents, preferably 1 to 5 equivalents to 1 equivalent of the compound of the formula (III-1) as the raw material. The base may be used in an amount of 1 to 10 equivalents, preferably 1 to 5 equivalents to 1 equivalent of the compound of the formula (III-1). Also in a case where each of the compound of the formula (III-2) to the compound of the formula (III-4) is used as the raw material, the compound of the formula (X-6) and the base may be used in the same amount as above.

The compound of the formula (III-1) to the compound of the formula (III-4) used in Reaction O may be produced in accordance with the above Reaction M or a known method. The compound of the formula (X-6) used in Reaction O may be commercially available.

### [Reaction P]

Reaction P is a method of hydrolyzing the compound of the formula (I-12) to obtain a compound of the formula (I-18).

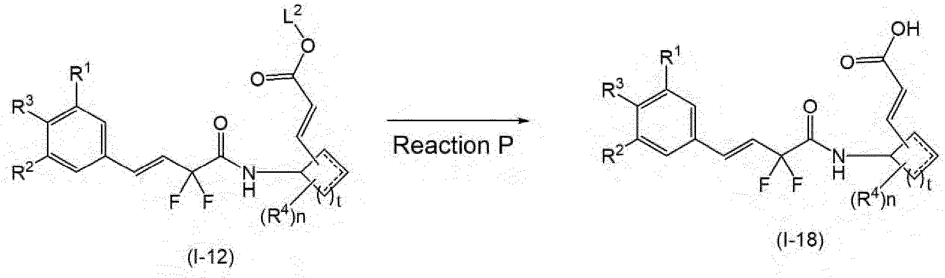

The symbols in the formulae are as defined above.

Reaction P may be carried out in accordance with the above Reaction B. In a case where a base is used in Reaction P, the base may be used in an amount of 1 to 10 equivalents, preferably 1 to 5 equivalents to 1 equivalent of the compound of the formula (I-12). In a case where an acid is used in Reaction P, the acid may be used in an amount of 0.1 to 30 equivalents, preferably 1 to 10 equivalents to 1 equivalent of the compound of the formula (I-12).

### [Reaction Q] and [Reaction R]

Reaction Q is a method of hydrogenating the compound of the formula (I-12) to obtain a compound of the formula (I-19). Reaction R is a method of hydrolyzing the compound of the formula (I-19) to obtain a compound of the formula (I-20).

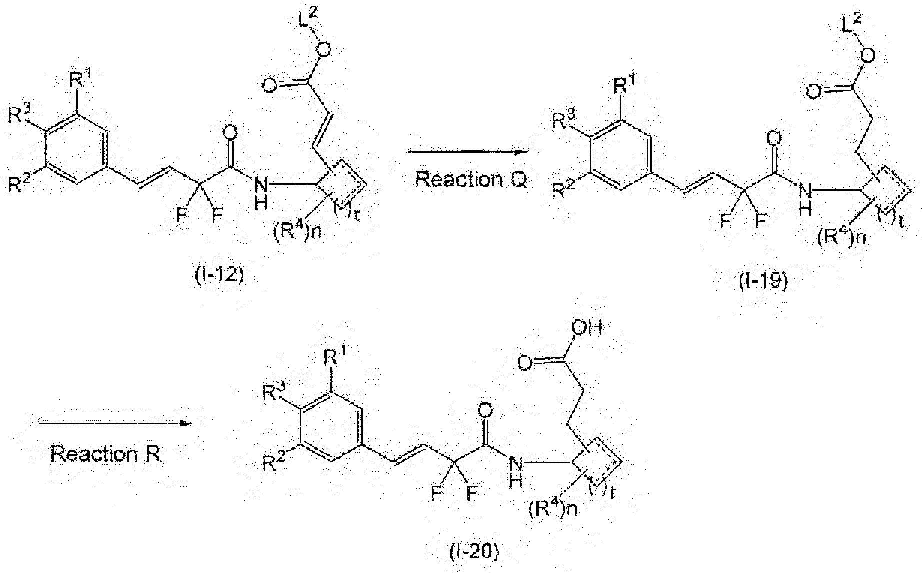

The symbols in the formulae are as defined above.

Reaction Q may be carried out by hydrogenating the compound of the formula (I-12) using palladium on carbon as a catalyst in a hydrogen atmosphere usually in the presence of a solvent. The palladium on carbon may be used in an amount of 0.001 to 1 equivalents, preferably 0.01 to 0.5 equivalents to 1 equivalent of the compound of the formula (I-12).

The solvent may be any solvent so long as it is inert to the reaction, and one or more may properly be selected from ethers such as dioxane, tetrahydrofuran, diethyl ether and dimethoxyethane; esters such as methyl acetate and ethyl acetate; alcohols such as methanol, ethanol and isopropanol; water; and the like.

In Reaction Q, the reaction temperature is about -20°C to 150°C, preferably about 0°C to 100°C, and the reaction time is usually about 0.5 to 48 hours. Further, Reaction Q may be carried out under elevated pressure as the case requires.

Reaction R may be carried out in accordance with the above Reaction B. In a case where a base is used in Reaction R, the base may be used in an amount of 1 to 10 equivalents, preferably 1 to 5 equivalents to 1 equivalent of the compound of the formula (I-19). In a case where an acid is used in Reaction R, the acid may be used in an amount of 0.1 to 30 equivalents, preferably 1 to 10 equivalents to 1 equivalent of the compound of the formula (I-19).

### [Reaction S] and [Reaction T]

Reaction S is a method of reacting the compound of the formula (I-11) with Ohira-Bestmann reagent to obtain a compound of the formula (I-21). Reaction T is a method of reacting the compound of the formula (I-21) with a compound of the formula (X-9) to obtain a compound of the formula (I-22).

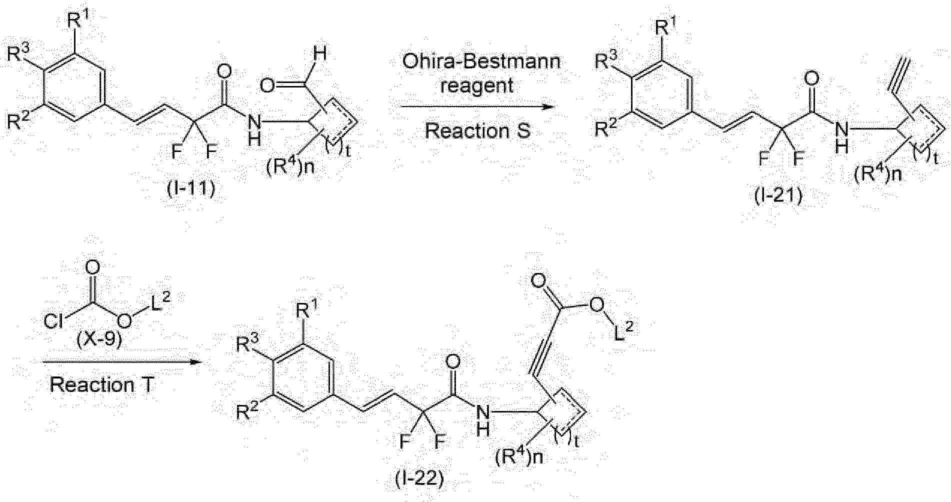

The symbols in the formulae are as defined above.

Reaction S may be carried out by mixing the compound of the formula (I-11) with Ohira-Bestmann reagent usually in the presence of a solvent and a base.

Ohira-Bestmann reagent in Reaction S means dimethyl (1-diazo-2-oxopropyl)phosphonate. The Ohira-Bestmann reagent may be used in an amount of 0.5 to 10 equivalents, preferably 1 to 5 equivalents to 1 equivalent of the compound of the formula (I-11).

As the base, one or more may properly be selected and mixed from carbonates such as sodium carbonate and potassium carbonate; hydrogencarbonates such as sodium hydrogencarbonate and potassium hydrogencarbonate; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (I-11).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; protic polar solvents such as methanol and ethanol; and the like.

In Reaction S, the reaction temperature is about -30°C to 120°C, preferably about 0°C to 80°C, and the reaction time is usually about 10 minutes to 30 hours, preferably about 30 minutes to 15 hours.

The Ohira-Bestmann reagent used in Reaction S may be commercially available.

Reaction T may be carried out by mixing the compound of the formula (I-21) with the compound of the formula (X-9) usually in the presence of a solvent and a base. The compound of the formula (X-9) may be used in an amount of 0.5 to 10 equivalents, preferably 1 to 5 equivalents to 1 equivalent of the compound of the formula (I-21).

As the base, one or more may properly be selected and mixed from metal amine salts such as lithium diisopropylamide and lithium bis(trimethylsilyl)amide; metal hydrides such as sodium hydride and potassium hydride; metal alkoxides such as potassium tert-butoxide and lithium tert-butoxide; and the like. The base may be used in an amount of 1 to 10 equivalents, preferably 1 to 5 equivalents to 1 equivalent of the compound of the formula (I-21).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; and the like.

In Reaction T, the reaction temperature is about -100°C to 100°C, preferably about -80°C to 50°C, and the reaction time is usually about 10 minutes to 30 hours, preferably about 1 hour to 15 hours.

The compound of the formula (X-9) used in Reaction T may be commercially available.

### [Reaction U], [Reaction V], [Reaction W] and [Reaction X]

Reaction U is a method of reacting the compound of the formula (I-2) with a compound of the formula (X-10) to obtain a compound of the formula (I-23). Reaction V is a method of reacting the compound of the formula (I-23) with an oxidizing agent to obtain a compound of the formula (I-24). Reaction W is a method of reacting the compound of the formula (I-24) with a compound of the formula (X-11) to obtain a compound of the formula (I-25). Reaction X is a method of reacting the compound of the formula (I-24) with a reducing agent to obtain a compound of the formula (I-26).

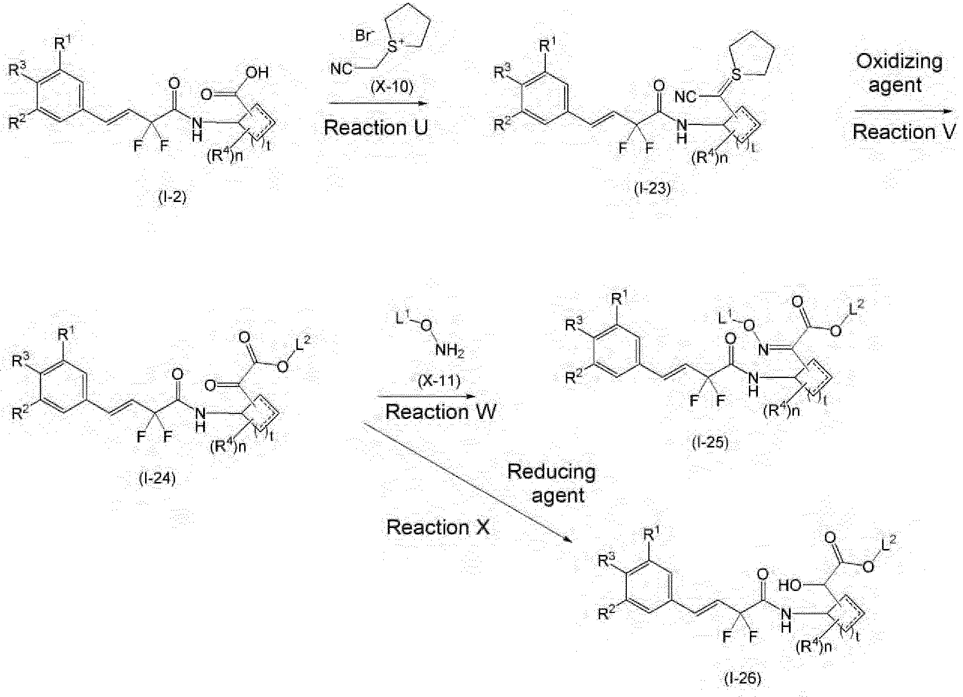

The symbols in the formulae are as defined above.

Reaction U may be carried out by mixing the compound of the formula (I-2) with the compound of the formula (X-10) usually in the presence of a solvent, a dehydration condensation agent and a base. The compound of the formula (X-10) may be used in an amount of 0.5 to 10 equivalents, preferably 1 to 5 equivalents to 1 equivalent of the compound of the formula (I-2).

As the base, one or more may properly be selected and mixed from carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (I-2).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; and the like.

As the dehydration condensation agent, one or more may properly be selected and mixed from carbodiimide condensation agents such as N,N'-dicyclohexylcarbodiimide (DCC), 1-[3-(dimethylamino)propyl]-3-ethylcarbodiimide (EDC) or its hydrochloride; imidazole condensation agents such as 1,1'-carbonyldiimidazole (CDI); triazine condensation agents such as 4-(4,6-dimethoxy-1,3,5-triazin-2-yl)-4-methylmorpholinium chloride (DMT-MM); phosphonium condensation agents such as 1H-benzotriazol-1-yloxy tripyrrolidinophosphonium hexafluorophosphate (PyBOP); uronium condensation agents such as 1-[bis(dimethylamino)methylene]-1H-1,2,3-triazolo[4,5-b]pyridinium 3-oxide hexafluorophosphate (HATU); acid anhydrides such as 2-methyl-6-nitrobenzoic anhydride (MNBA); 2-halopyridinium salts such as 2-chloro-1-methylpyridinium p-toluenesulfonate; propylphosphonic acid anhydride (cyclic trimer) (T3P); diphenylphosphoryl azide (DPPA); and the like. Optionally, a conventional additive used in combination with a dehydration condensation agent, such as 1-hydroxybenzotriazole (HOBt) or 4-N,N-dimethylaminopyridine (DMAP), may be added. In a case where an additive is used in Reaction U, the additive may be used alone or as a mixture of two or more. The dehydration condensation agent may be used in an amount of 0.5 to 10 equivalents, preferably 1 to 5 equivalents to 1 equivalent of the compound of the formula (I-2). The additive may be used in an amount of 0.1 to 10 equivalents, preferably 1 to 5 equivalents to 1 equivalent of the compound of the formula (I-2).

In Reaction U, the reaction temperature is about -30°C to 120°C, preferably about 0°C to 80°C, and the reaction time is usually about 10 minutes to 30 hours, preferably about 30 minutes to 15 hours.

The compound of the formula (X-10) used in Reaction U may be produced by the method described in Journal of The American Chemical Society 2008, 130, 4253-4255, Supporting Information, or a method in accordance therewith.

Reaction V may be carried out by mixing the compound of the formula (I-23) with an oxidizing agent usually in the presence of a solvent.

The oxidizing agent is not particularly limited so long as the reaction proceeds, and may, for example, be a potassium persulfate such as potassium peroxymonosulfate (OXONE). The oxidizing agent may be used in an amount of 0.5 to 30 equivalents, preferably 1 to 10 equivalents to 1 equivalent of the compound of the formula (I-23).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; protic polar solvents such as methanol and ethanol; water; and the like.

In Reaction V, the reaction temperature is about -80°C to 100°C, preferably about 0°C to 50°C, and the reaction time is usually about 10 minutes to 30 hours, preferably about 1 hour to 15 hours.

Reaction W may be carried out by mixing the compound of the formula (I-24) with the compound of the formula (X-11) usually in the presence of a solvent and a base. The compound of the formula (X-11) may be used in an amount of 0.5 to 10 equivalents, preferably 1 to 5 equivalents to 1 equivalent of the compound of the formula (I-24).

As the base, one or more may properly be selected and mixed from carbonates such as sodium carbonate and potassium carbonate; hydrogencarbonates such as sodium hydrogencarbonate and potassium hydrogencarbonate; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (I-24).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; protic polar solvents such as methanol and ethanol; and the like.

In Reaction W, the reaction temperature is about -30°C to 120°C, preferably about 0°C to 80°C, and the reaction time is usually about 10 minutes to 30 hours, preferably about 30 minutes to 15 hours.

Reaction X may be carried out in accordance with the above Reaction G. The reducing agent may be used in an amount of 0.5 to 10 equivalents, preferably 1 to 5 equivalents to 1 equivalent of the compound of the formula (I-24).

### [Reaction Y]

Reaction Y may be carried out by mixing the compound of the formula (I-11) with an alcohol and an acid, usually in the presence of a solvent.

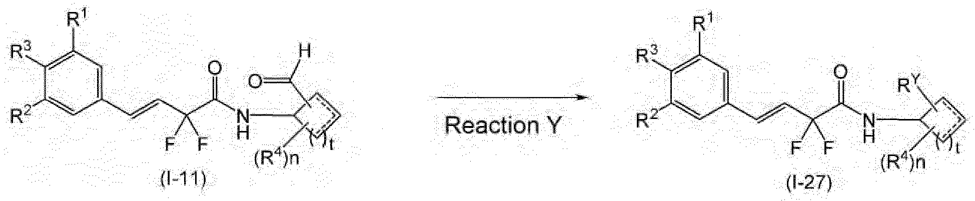

In the formulae, R^{Y} is 1,3-dioxolan-2-yl or -CH(OL²)₂ (L² is as defined above), and the other symbols are as defined above.

The alcohol may, for example, be methanol, ethanol or ethylene glycol. The alcohol may be used in an amount of 1 equivalent or more or in large excess to 1 equivalent of the compound of the formula (I-11).

The acid may, for example, be an organic acid such as formic acid, acetic acid, methanesulfonic acid, p-toluenesulfonic acid, trifluoroacetic acid or trifluoromethanesulfonic acid; or an inorganic acid such as hydrochloric acid, sulfuric acid or hydrogen bromide, but is not limited thereto. As the acid, one or more may properly be selected and mixed from them. The acid may be used in an amount of 0.01 to 30 equivalents, preferably 0.1 to 10 equivalents to 1 equivalent of the compound of the formula (I-11).

The solvent may be any solvent so long as it is inert to the reaction, and one or more may properly be selected from aromatic hydrocarbons such as benzene and toluene; and the like.

In Reaction Y, the reaction temperature is about -30°C to 150°C, preferably about 30°C to 100°C, and the reaction time is usually about 10 minutes to 30 hours, preferably about 30 minutes to 15 hours.

### [Reaction Z] and [Reaction AA]

Reaction Z is Horner-Wadsworth-Emmons Reaction and is a method of reacting the compound of the formula (III) with a compound of the formula (X-12) to obtain a compound of the formula (I-29). Reaction AA is a method of hydrogenating the compound of the formula (I-29) to obtain a compound of the formula (I-30).

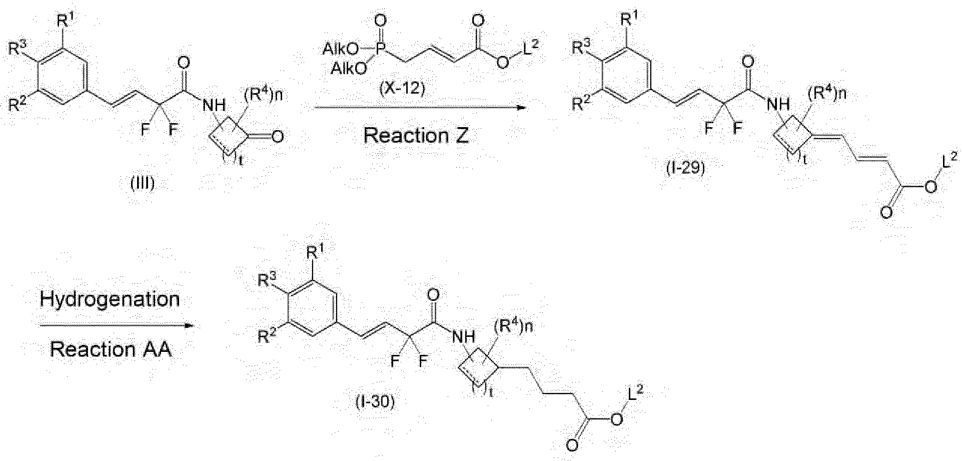

The symbols in the formulae are as defined above.

Reaction Z may be carried out in accordance with the above Reaction L. The compound of the formula (X-12) may be used in an amount of 0.5 to 10 equivalents, preferably 1 to 5 equivalents to 1 equivalent of the compound of the formula (III). The base may be used in an amount of 1 to 10 equivalents, preferably 1 to 5 equivalents to 1 equivalent of the compound of the formula (III).

Reaction AA may be carried out in accordance with the above Reaction Q. The palladium on carbon may be used in an amount of 0.001 to 1 equivalents, preferably 0.01 to 0.5 equivalents to 1 equivalent of the compound of the formula (I-29).

### [Reaction AB] and [Reaction AC]

Reaction AB is a method of reacting the compound of the formula (I-13) with a halogenating agent to obtain a compound of the formula (1-31). Reaction AC is a method of coupling a compound of the formula (I-31) with a compound of the formula (X-13) to obtain a compound of the formula (I-32).

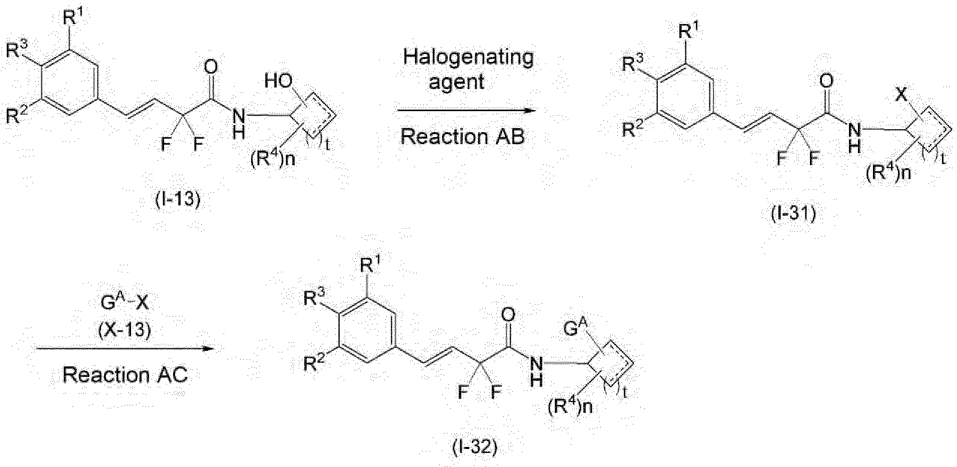

In the formulae, G^{A} is 2-thiazolyl or 2-imidazolyl which may be substituted by at least one Z⁴, and the other symbols are as defined above.

Reaction AB may be carried out in accordance with the above Reaction I. The halogenating agent may be used in an amount of 0.5 to 10 equivalents, preferably 1 to 5 equivalents to 1 equivalent of the compound of the formula (I-13). Further, in a case where triphenylphosphine is used in Reaction AB, triphenylphosphine may be used in an amount of 0.5 to 10 equivalents, preferably 1 to 5 equivalents to 1 equivalent of the compound of the formula (I-13). Further, the base may be used in an amount of 0.1 to 10 equivalents, preferably 1 to 5 equivalents to 1 equivalent of the compound of the formula (I-13).

Reaction AC may be carried out by mixing the compound of the formula (I-31) with the compound of the formula (X-13) usually in the presence of a solvent, a reducing agent and a catalyst. Reaction AC may be carried out optionally with the addition of a ligand. The compound of the formula (X-13) may be used in an amount of 0.5 to 10 equivalents, preferably 1 to 3 equivalents to 1 equivalent of the compound of the formula (I-31).

The reducing agent may, for example, be a metal such as zinc or manganese, and one or more may properly be selected and mixed. The reducing agent may be used in an amount of 1 to 10 equivalents, preferably 1 to 5 equivalents to 1 equivalent of the compound of the formula (I-31).

As the catalyst, one or more may properly be selected and mixed from nickel(II) chloride ethylene glycol dimethyl ether complex, nickel iodide and the like. The catalyst may be used in an amount of 0.002 to 1 equivalents, preferably 0.02 to 0.4 equivalents to 1 equivalent of the compound of the formula (I-31).

As the ligand, one or more may properly be selected and mixed from 2,2'-bipyridine, 4,4'-dimethyl-2,2'-dipyridyl, 4,4'-di-tert-butyl-2,2'-dipyridyl, 1,10-phenanthroline, and the like. The ligand may be used in an amount of 0.002 to 1 equivalents, preferably 0.02 to 0.4 equivalents to 1 equivalent of the compound of the formula (I-31).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; and the like.

In Reaction AC, the reaction temperature is about -30°C to 150°C, preferably about 0°C to 100°C, and the reaction time is usually about 2 hours to 80 hours, preferably about 10 hours to 50 hours.

The compound of the formula (X-13) used in Reaction AC may be commercially available. The compound of the formula (I-32) may be produced by the method described in The Journal of Organic Chemistry 2014, 79, 4793 or a method in accordance therewith, or may be produced in accordance with a known method.

The compounds used in Production Process A (Reactions A to Z and AA to AC) may be produced in accordance with the following Production Process B (Reactions 1-1 to 1-16), however, their production is not limited thereto, and such compounds may be produced in accordance with a known method of may be commercially available.

### Production Process B

### [Reaction 1-1] to [Reaction 1-3]

Reaction 1-1 is Horner-Wadsworth-Emmons Reaction and is a method of reacting a compound of the formula (VI) with the compound of the formula (X-6) to obtain a compound of the formula (V). Reaction 1-2 is a method of hydrolyzing the compound of the formula (V) to obtain a compound of the formula (IV). Reaction 1-3 is a method of reacting the compound of the formula (VI) with malonic acid to obtain the compound of the formula (IV).

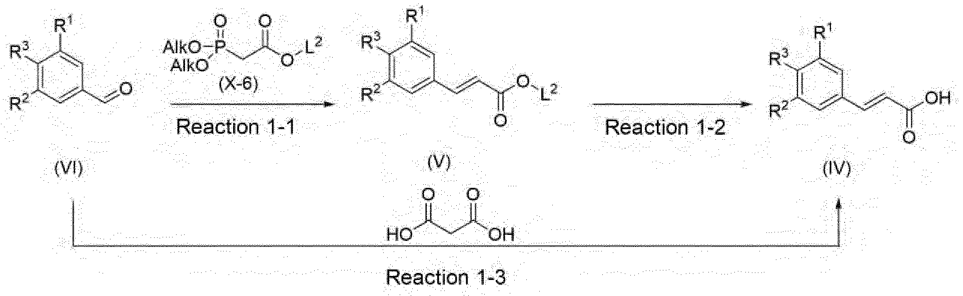

The symbols in the formulae are as defined above.

Reaction 1-1 may be carried out in accordance with the above Reaction L. The compound of the formula (X-6) may be used in an amount of 0.5 to 5 equivalents, preferably 1 to 3 equivalents to 1 equivalent of the compound of the formula (VI). The base may be used in an amount of 1 to 10 equivalents, preferably 1 to 5 equivalents to 1 equivalent of the compound of the formula (VI).

Reaction 1-2 may be carried out in accordance with the above Reaction B. In a case where a base is used in Reaction 1-2, the base may be used in an amount of 1 to 10 equivalents, preferably 1 to 5 equivalents to 1 equivalent of the compound of the formula (V). In a case where an acid is used in Reaction 1-2, the acid may be used in an amount of 0.1 to 30 equivalents, preferably 1 to 10 equivalents to 1 equivalent of the compound of the formula (V).

Reaction 1-3 may be carried out by mixing the compound of the formula (VI) with malonic acid usually in the presence of a solvent, and optionally with the addition of a base. Malonic acid may be used in an amount of 0.5 to 10 equivalents, preferably 1 to 5 equivalents to 1 equivalent of the compound of the formula (VI).

As the base, one or more may properly be selected and mixed from carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.01 to 10 equivalents, preferably 0.1 to 5 equivalents to 1 equivalent of the compound of the formula (VI).

The solvent may be any solvent so long as it is inert to the reaction, and one or more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; aprotic polar solvents such as dimethyl sulfoxide, sulfolane, N,N-dimethylacetamide, N,N-dimethylformamide, N-methylpyrolidone, pyridine, acetonitrile and propionitrile; protic polar solvents such as methanol and ethanol; and the like.

In Reaction 1-3, the reaction temperature is about 0°C to 180°C, preferably about 30°C to 130°C, and the reaction time is usually about 10 minutes to 30 hours, preferably about 1 hour to 15 hours.

The compound of the formula (VI) used in the above Reaction 1-1 and Reaction 1-3 may be produced by the method described in The Journal of Organic Chemistry 2000, 65, 9120-9124 or a method in accordance therewith, or may be commercially available. The compound of the formula (X-6) used in Reaction 1-1 may be commercially available.

### [Reaction 1-4]

Reaction 1-4 is a method of reacting the compound of the formula (IV) with a compound of the formula (X-8) to obtain a compound of the formula (VII).

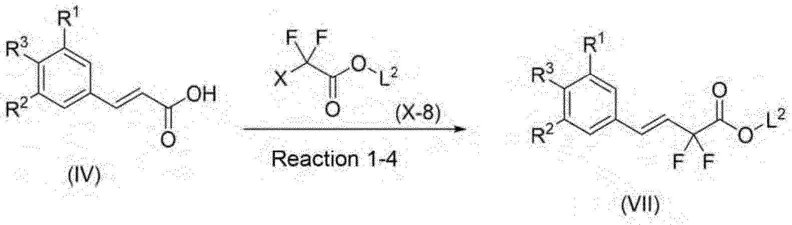

The symbols in the formulae are as defined above.

Reaction 1-4 may be carried out by mixing the compound of the formula (IV) with the compound of the formula (X-8) usually in the presence of a solvent, an additive and a catalyst. Reaction 1-4 may be carried out optionally by the addition of a ligand. The compound of the formula (X-8) may be used in an amount of 0.5 to 10 equivalents, preferably 1 to 3 equivalents to 1 equivalent of the compound of the formula (IV).

As the additive, one or more may properly be selected and mixed from dimethyl phosphite, diethyl phosphite, triethylsilane, sodium dithionite, bis(pinacolato)diboron, and the like. The additive may be used in an amount of 1 to 10 equivalents, preferably 1 to 5 equivalents to 1 equivalent of the compound of the formula (IV).

As the catalyst, one or more may properly be selected and mixed from copper oxide, copper iodide, copper thiocyanate, copper trifluoromethanesulfonate, and the like. The catalyst may be used in an amount of 0.002 to 1 equivalents, preferably 0.02 to 0.4 equivalents to 1 equivalent of the compound of the formula (IV).

As the ligand, one or more may properly be selected and mixed from 2,2'-bipyridine, 4,4'-dimethyl-2,2'-dipyridyl, 4,4'-di-tert-butyl-2,2'-dipyridyl, 1,10-phenanthroline, and the like. The ligand may be used in an amount of 0.002 to 1 equivalents, preferably 0.02 to 0.4 equivalents to 1 equivalent of the compound of the formula (IV).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; and the like.

In Reaction 1-4, the reaction temperature is about -30°C to 150°C, preferably about 0°C to 100°C, and the reaction time is usually about 2 hours to 80 hours, preferably about 10 hours to 50 hours.

The compound of the formula (IV) used in Reaction 1-4 may be produce in accordance with the above Reaction 1-2 or a known method. The compound of the formula (X-8) may be commercially available.

### [Reaction 1-5] to [Reaction 1-6]

Reaction 1-5 is a method of hydrolyzing the compound of the formula (VII) to obtain the compound of the formula (II-1). Reaction 1-6 is a method of reacting the compound of the formula (II-1) with a halogenating agent to obtain a compound of the formula (II-2).

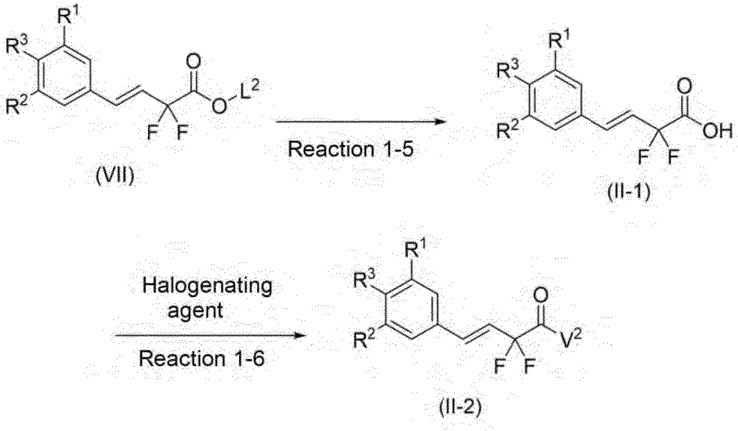

In the formulae, V² is a chlorine atom or a bromine atom. The other symbols are as defined above.

Reaction 1-5 may be carried out in accordance with the above Reaction B. In a case where a base is used in Reaction 1-5, the base may be used in an amount of 1 to 10 equivalents, preferably 1 to 5 equivalents to 1 equivalent of the compound of the formula (VII). In a case where an acid is used in Reaction 1-5, the acid may be used in an amount of 0.1 to 30 equivalents, preferably 1 to 10 equivalents to 1 equivalent of the compound of the formula (VII).

The compound of the formula (VII) used in Reaction 1-5 may be produced in accordance with the above Reaction 1-4 or a known method.

Reaction 1-6 may be carried out by mixing the compound of the formula (II-1) with a halogenating agent usually in the presence of a solvent.

As the halogenating agent, one or more may properly be selected and mixed from sulfur halides such as thionyl chloride; phosphorus halides such as phosphorus trichloride and phosphorus pentachloride; a reactant having carbon tetrachloride, carbon tetrabromide and triphenylphosphine combined (Appel Reaction); acid halides such as oxalyl chloride and benzoyl chloride; and the like. Optionally, a conventional additive used together with a halogenating agent such as N,N-dimethylformamide may be added.

The halogenating agent may be used in an amount of 0.5 to 100 equivalents, preferably 1 to 10 equivalents to 1 equivalent of the compound of the formula (II-1), and the additive may be used in an amount of 0.001 to 50 equivalents, preferably 0.01 to 15 equivalents to 1 equivalent of the compound of the formula (II-1).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; and the like.

In Reaction 1-6, the reaction temperature is about -30°C to 150°C, preferably about -10°C to 80°C, and the reaction time is usually about 10 minutes to 30 hours, preferably about 30 minutes to 15 hours.

The compound of the formula (II-1) used in Reaction 1-6 may be produced in accordance with the above Reaction 1-5 or a known method.

### [Reaction 1-7]

Reaction 1-7 is a method of reacting a compound of the formula (VIII) with the compound of the formula (X-8) to obtain the compound of the formula (VII).

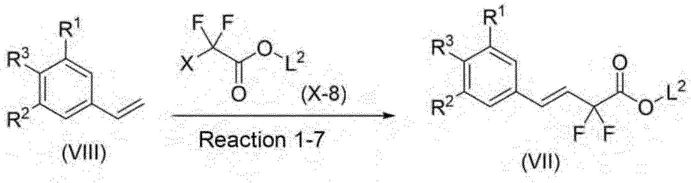

The symbols in the formulae are as defined above.

Reaction 1-7 may be carried out by mixing the compound of the formula (VIII) with the compound of the formula (X-8) usually in the presence of a base, a catalyst and a solvent. The compound of the formula (X-8) may be used in an amount of 0.5 to 10 equivalents, preferably 1 to 3 equivalents to 1 equivalent of the compound of the formula (VIII).

As the base, one or more may properly be selected and mixed from carbonates such as sodium carbonate and potassium carbonate; hydrogencarbonates such as sodium hydrogencarbonate and potassium hydrogen carbonate; metal hydroxides such as sodium hydroxide and potassium hydroxide; metal hydrides such as sodium hydride and potassium hydride; amines such as triethylamine, N,N-diisopropylethylamine, N,N,N',N'-tetramethylethylenediamine, N,N,N',N'-tetramethyl-1,3-propanediamine and pentamethyldiethylenetriamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (VIII).

As the catalyst, one or more may properly be selected and mixed from copper(I) chloride, copper(I) bromide, copper(II) bromide, copper iodide, copper(II) trifluoromethanesulfonate and the like. The catalyst may be used in an amount of 0.002 to 1 equivalents, preferably 0.02 to 0.2 equivalents to 1 equivalent of the compound of the formula (VIII).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; and the like.

In Reaction 1-7, the reaction temperature is about -30°C to 150°C, preferably about 0°C to 100°C, and the reaction time is usually about 10 minutes to 30 hours, preferably about 2 hours to 24 hours.

The compound of the formula (X-8) used in Reaction 1-7 may be commercially available. The compound of the formula (VIII) may be produced by the method described in Chemical Science 2019,10,8331-8337 or a method in accordance therewith, may be commercially available, or may be produced in accordance with a known method.

### [Reaction 1-8] and [Reaction 1-9]

Reaction 1-8 is a method of reacting a compound of the formula (VII-2) with a formylating agent to obtain a compound of the formula (IX). Reaction 1-9 is a method of reacting the compound of the formula (IX) with a Wittig reagent or Ohira-Bestmann reagent to obtain a compound (IV-3).

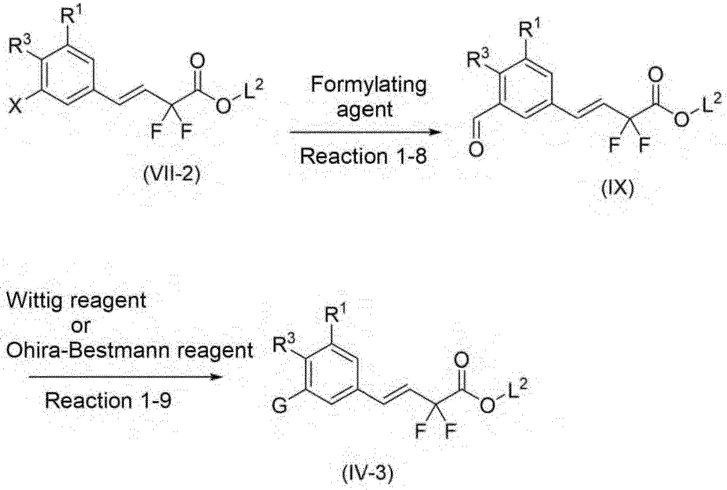

In the formulae, G is a (C₂-C₆)-alkenyl or a (C₂-C₆)-alkynyl. The other symbols are as defined above.

Reaction 1-8 may be carried out by mixing the compound of the formula (VII-2) with a formylating agent usually in the presence of a solvent and a base.

As the formylating agent, one on more may properly be selected and mixed from N,N-di-substituted formamides such as N,N-dimethylformamide and N-formylmorpholine; orthoformate esters such as ethyl orthoformate; N-ethoxymethyleneaniline; and the like. The formylating agent may be used in an amount of 0.5 to 5 equivalents, preferably 1 to 3 equivalents to 1 equivalent of the compound of the formula (VII-2).

As the base, one or more may properly be selected and mixed from metal amine salts such as lithium diisopropylamide and lithium bis(trimethylsilyl)amide; metal hydrides such as sodium hydride and potassium hydride; metal alkoxides such as potassium tert-butoxide and lithium tert-butoxide; metal alkyl salts such as methyl lithium and n-butyl lithium; and the like. The base may be used in an amount of 0.5 to 5 equivalents, preferably 1 to 3 equivalents to 1 equivalent of the compound of the formula (VII-2).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, hexane and cyclohexane; ethers such as dioxane, tetrahydrofuran, diethyl ether and dimethoxyethane; and the like.

In Reaction 1-8, the reaction temperature is about -100°C to 50°C, preferably about -80°C to 30°C, and the reaction time is usually about 10 minutes to 30 hours, preferably about 1 hour to 15 hours.

The compound of the formula (VII-2) used in Reaction 1-8 may be produced in accordance with the above Reaction 1-4 or a known method.

Reaction 1-9 may be carried out by mixing the compound of the formula (IX) with a Wittig reagent or Ohira-Bestmann reagent usually in the presence of a solvent and a base.

In a case where a Wittig reagent is used in Reaction 1-9, the Wittig reagent may, for example, be methyltriphenylphosphine bromide or ethyltriphenylphosphine bromide, but is not limited thereto. The Wittig reagent may be used in an amount of 0.5 to 10 equivalents, preferably 1 to 5 equivalents to 1 equivalent of the compound of the formula (IX).

The Ohira-Bestmann reagent in Reaction 1-9 means dimethyl (1-diazo-2-oxopropyl)phosphonate. In a case where the Ohira-Bestmann reagent is used, it may be used in an amount of 0.5 to 10 equivalents, preferably 1 to 5 equivalents to 1 equivalent of the compound of the formula (IX).

As the base, one or more may properly be selected and mixed from carbonates such as sodium carbonate and potassium carbonate; hydrogencarbonates such as sodium hydrogencarbonate and potassium hydrogencarbonate; metal hydrides such as sodium hydride and potassium hydride; amines such as triethylamine and N,N-diisopropylethylamine; pyridines such as pyridine, 4-dimethylaminopyridine and 2,6-lutidine; alkali metal carboxylates such as sodium acetate and potassium acetate; and the like. The base may be used in an amount of 0.5 to 10 equivalents, preferably 1 to 5 equivalents to 1 equivalent of the compound of the formula (IX).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrolidone, pyridine, acetonitrile and propionitrile; ketones such as acetone and methyl ethyl ketone; protic polar solvents such as methanol and ethanol; and the like.

In Reaction 1-9, the reaction temperature is about -30°C to 120°C, preferably about 0°C to 80°C, and the reaction time is usually about 10 minutes to 30 hours, preferably about 30 minutes to 15 hours.

The Wittig reagent and the Ohira-Bestmann reagent used in Reaction 1-9 may be commercially available.

### [Reaction 1-10] to [Reaction 1-12]

Reaction 1-10 is a method of reacting the compound of the formula (IX) with a Wittig reagent to obtain a compound of the formula (XI-1). Reaction 1-11 is a method of reacting the compound of the formula (XI-1) with an acid to obtain a compound of the formula (XI-2). Reaction 1-12 is a method of reacting the compound of the formula (XI-2) with a fluorinating agent to obtain a compound of the formula (XI-3).

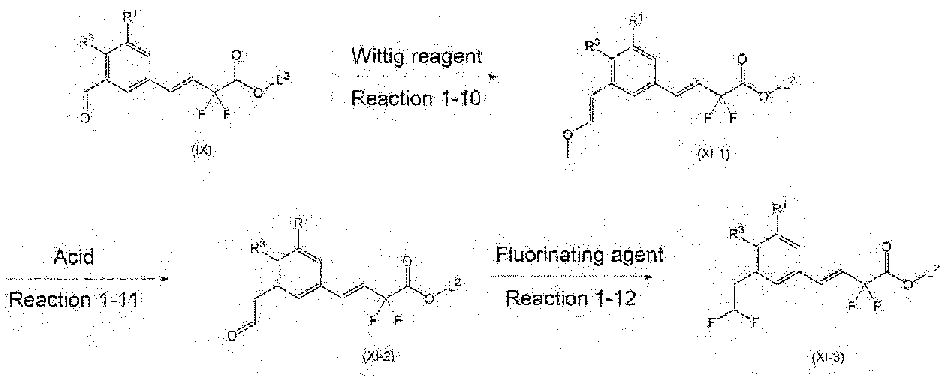

The symbols in the formulae are as defined above.

Reaction 1-10 may be carried out in accordance with the above Reaction 1-9.

The Wittig reagent may, for example, be (methoxymethyl)triphenylphosphonium chloride, but is not limited thereto. The Wittig reagent may be used in an amount of 0.5 to 10 equivalents, preferably 1 to 5 equivalents to 1 equivalent of the compound of the formula (IX). The base may be used in an amount of 0.5 to 5 equivalents, preferably 1 to 3 equivalents to 1 equivalent of the compound of the formula (IX).

The compound of the formula (IX) used in Reaction 1-10 may be produced in accordance with the above Reaction 1-8 or a known method.

Reaction 1-11 may be carried out by mixing the compound of the formula (XI-1) with an acid usually in the presence of a solvent.

The acid may, for example, be an organic acid such as formic acid, acetic acid, methanesulfonic acid, p-toluenesulfonic acid, trifluoroacetic acid or trifluoromethanesulfonic acid; or an inorganic acid such as hydrochloric acid, sulfuric acid or hydrogen bromide, but is not limited thereto. As the acid, one or more may properly be selected and mixed from them. The acid may be used in an amount of 0.1 to 30 equivalents, preferably 1 to 10 equivalents to 1 equivalent of the compound of the formula (XI-1).

The solvent may be any solvent so long as it is inert to the reaction and one ore more may properly be selected from aromatic hydrocarbons such as benzene, toluene, xylene and chlorobenzene; aliphatic hydrocarbons such as hexane and cyclohexane; ethers such as dioxane, tetrahydrofuran, diethyl ether and dimethoxyethane; aprotic polar solvents such as acetonitrile, N,N-dimethylformamide, dimethyl sulfoxide, N,N-dimethylacetamide, hexamethylphosphoric triamide and sulfolane; protic polar solvents such as methanol, ethanol and isopropanol; water; and the like.

In Reaction 1-11, the reaction temperature is about -30°C to 150°C, preferably about 30°C to 100°C, and the reaction time is usually about 10 minutes to 30 hours, preferably about 30 minutes to 15 hours.

Reaction 1-12 may be carried out by mixing the compound of the formula (XI-2) with a fluorinating agent usually in the presence of a solvent.

The fluorinating agent may, for exmaple, be N,N-dimethylaminosulfur trifluoride (DAST), bis(2-methoxyethyl)aminosulfur trifluoride, 1,1,2,2-tetrafluoro-N,N-dimethylethylamine (TFEDMA), (diethylamino)difluorosulfonium tetrafluoroborate (XtalFluor), 4-tert-butyl-2,6-dimethylphenylsulfur trifluoride (FluoLead), 2-chloro-1,3-bis(2,6-diisopropylphenyl)-1H-imidazolium chloride (PhenoFluor), but is not limited thereto. As the fluorinating agent, one or more may properly be selected and mixed from them. The fluorinating agent may be used in an amount of 0.1 to 30 equivalents, preferably 1 to 10 equivalents to 1 equivalent of the compound of the formula (XI-2).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; and the like.

In Reaction 1-12, the reaction temperature is about -30°C to 150°C, preferably about 0°C to 80°C, and the reaction time is usually about 10 minutes to 30 hours, preferably about 30 minutes to 15 hours.

### [Reaction 1-13] to [Reaction 1-16]

Reaction 1-13 is a method of reacting the compound of the formula (IX) with a reducing agent to obtain a compound of the formula (XI-4). Reaction 1-14 is a method of reacting the compound of the formula (XI-4) with a halogenating agent to obtain a compound of the formula (XI-5). Reaction 1-15 is a method of reacting the compound of the formula (XI-5) with a nucleophile to obtain a compound of the formula (XI-6). Reaction 1-16 is a method of reacting the compound of the formula (XI-5) with a cyanizing agent to obtain a compound of the formula (XI-7).

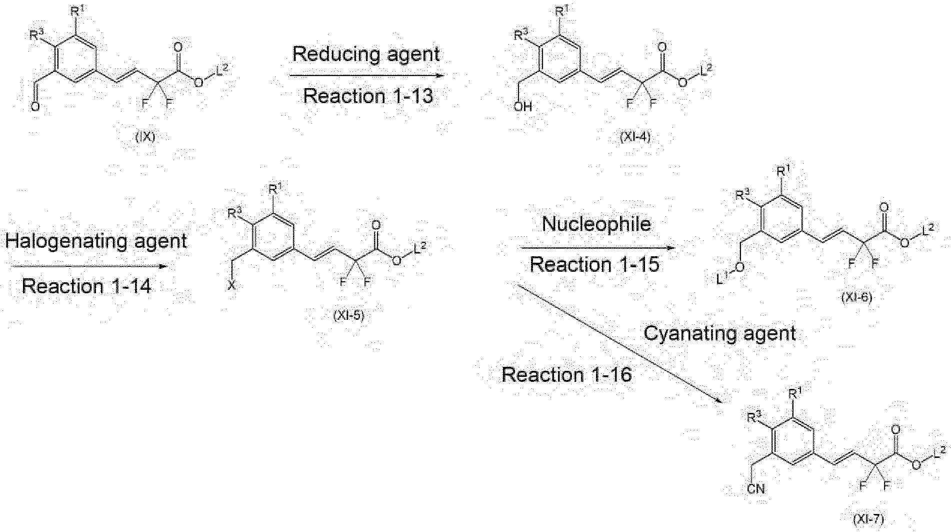

The symbols in the formulae are as defined above.

Reaction 1-13 may be carried out in accordance with the above Reaction G. The reducing agent may be used in an amount of 0.5 to 10 equivalents, preferably 1 to 5 equivalents to 1 equivalent of the compound of the formula (IX).

Reaction 1-14 may be carried out in accordance with the above Reaction I. The halogenating agent may be used in an amount of 0.5 to 10 equivalents, preferably 1 to 5 equivalents to 1 equivalent of the compound of the formula (XI-4). Further, in a case where triphenylphosphine is used in Reaction 1-14, triphenylphosphine may be used in an amount of 0.5 to 10 equivalents, preferably 1 to 5 equivalents to 1 equivalent of the compound of the formula (XI-4). The base may be used in an amount of 0.1 to 10 equivalents, preferably 1 to 5 equivalents to 1 equivalent of the compound of the formula (XI-4).

Reaction 1-15 may be carried out by mixing the compound of the formula (XI-5) with a nucleophile usually in the presence of a solvent.

As the nucleophile, one or more may properly be selected and mixed from alkali metal alkoxides such as sodium methoxide and sodium ethoxide; and the like. The nucleophile may be used in an amount of 0.1 to 30 equivalents, preferably 1 to 10 equivalents to 1 equivalent of the compound of the formula (XI-5).

The solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; and the like.

In Reaction 1-15, the reaction temperature is about -30°C to 150°C, preferably about 0°C to 80°C, and the reaction time is usually about 10 minutes to 30 hours, preferably about 30 minutes to 15 hours.

Reaction 1-16 may be carried out in accordance with the above Reaction J. The cyanating agent may be used in an amount of 0.5 to 10 equivalents, preferably 1 to 5 equivalents to 1 equivalent of the compound of the formula (XI-5).

The compound (I) as the active ingredient of the present composition is useful as an active ingredient of a herbicide which can control wide ranges of undesired plants such as annua weeds and perennial weeds. Further, the compound (I) can be used as a herbicide in a wide period from before budding to after budding of the undesired plants. And, the present composition is applicable to treatment methods assuming practical use of herbicides, such as soil treatment, foliar treatment and water treatment, and thus the present composition has excellent effectiveness as a herbicide in practical use.

Specific examples of the undesired plants may be as follows. That is, the present composition can control various harmful weeds including Gramineae weeds such as barnyardgrass (Echinochloa crus-galli), Rice barnyardgrass (Echinochloa oryzicola), crabgrass (Digitaria sanguinalis, D. ischaemum, D. adscendens, D. microbachne, D. horizontalis), green foxtail (Setaria viridis), giant foxtail (Setaria faberi), yellow foxtail (Setaria lutescens), goosegrass (Eleusine indica), johnsongrass (Sorghum halepense), quackgrass (Agropyron repens), alexandergrass (Brachiaria plantaginea), guineagrass (Panicum maximum), paragrass (Brachiaria mutica), smooth witchgrass (Panicum dichotomiflorum), sprangletop (Leptochloa chinensis), red sprangletop (Leptochloa panicea), annual bluegrass (Poa annua), roughstalk bluegrass (Poa trivialis), blackgrass (Alopecurus myosuroides), shortawn foxtail (Alopecurus aequalis), cholorado bluestem (Agroqyron tsukushiense), broadleaf signalgrass (Brachiaria platyphylla), southern sandbur (Cenchrus echinatus), Italian ryegrass (Lolium multiflorum), rigid ryegrass (Lolium rigidum), perennial ryegrass (Lolium perenne), wild oat (Avena fatua), black oat (Avena strigosa), field brome (Bromus arvensis), rescuegrass (Bromus catharticus Vahl), hairy chess (Bromus commutatus), smooth brome (Bromus inermis), japanese brome (Bromus japonicus), compact brome (Bromus madritensis), soft chess (Bromus mollis), ripgut brome (Bromus rigidus), red brome (Bromus rubens), cheat (Bromus secalinus), poverty brome (Bromus sterilis), downy brome (Bromus tectorum), windgrass (Apera spica-venti), littleseed canarygrass (Phalaris minor), rattail fescue (Festuca myuros), cheat grass (Bromus tectorum) and bermuda grass (Cynodon dactylon); Cyperaceae weeds such as rice flatsedge (Cyperus iria), purple nutsedge (Cyperus rotundus), yellow nutsedge (Cyperus esculentus), rock bulrush (Schoenoplectus juncoides), tidalmarsh flatsedge (Cyperus serotinus), small-flower umbrellaplant (Cyperus difformis), needle spikerush (Eleocharis acicularis) and water chestnut (Eleocharis kuroguwai); Alismataceae weeds such as dwarf arrowhead (Sagittaria pygmaea), threeleaf arrowhead (Sagittaria trifolia) and channelled water plantain (Alisma canaliculatum); Pontederiaceae weeds such as monochoria (Monochoria vaginalis) and heartshape false pickerelweed (Monochoria korsakowii); Linderniaceae weeds such as false pimpernel (Lindernia pyxidaria); Plantaginaceae weeds such as dopatrium (Dopatrium junceum) and common field speedwell (Veronica persica); Lythraceae weeds such as indian toothcup (Rotala India) and manyflower ammannia (Ammannia multiflora); Elatinaceae weeds such as Long stem waterwort (Elatine triandra); Malvaceae weeds such as velvetleaf (Abutilon theophrasti) and prickly sida (Sida spinosa); Compositae weeds such as common cocklebur (Xanthium strumarium), common ragweed (Ambrosia elatior), Canada thistle (Cirsium arvense), hairy galinsoga (Galinsoga ciliata), wild chamomile (Matricaria chamomilla), henbit (Lamium amplexicaule), common dandelion (Taraxacum officinale) and horseweed (Conyza canadensis); Solanaceae weeds such as black nightshade (Solanum nigrum) and jimsonweed (Datura stramonium); Amaranthaceae weeds such as slender amaranth (Amaranthus viridis), redroot pigweed (Amaranthus retroflexus), common lambsquarters (Chenopodium album) and mexican burningbush (Kochia scoparia); Polygonaceeae weeds such as pale smartweed (Polygonum lapathifolium), ladysthumb (Polygonum persicaria), wild buckwheat (Polygonum convolvulus) and knotweed (Polygonum aviculare); Cruciferae weeds such as flexuous bittercress (Cardamine flexuosa), shepherd's-purse (Capsella bursa-pastoris), wild mustard (Sinapis arvensis) and indian mustard(Brassica juncea); Convolvulaceae weeds such as tall morningglory (Ipomoea purpurea), field bindweed (Convolvulus arvensis) and ivyleaf morningglory (Ipomoea hederacea); Portulacaceae weeds such as common purslane (Portulaca oleracea); Fabaceae weeds such as sicklepod (Cassia obtusifolia); Caryophyllaceae weeds such as common chickweed (Stellaria media); Rubiaceae weeds such as catchweed (Galium spurium); Euphorbiaceae weeds such as threeseeded copperleaf (Acalypha australis); Commelinaceae weeds such as common dayflower (Commelina communis); and Lamiaceae weeds such as red deadnettle (Lamium purpureum).

Since the compound (I) can control the above various harmful weeds, the present composition is effective in cultivation of useful plants when the harmful weeds are controlled selectively or non-selectively.

Specific examples of the useful plants include the following. Corn (Zea mays), soybean (Glycine max), cotton (Gossypium spp.), wheat (Triticum aestivum), rice (Oryza sativa), barley (Hordeum vulgare), rye (Secale cereale), oat (Avena sativa), sorgo (Sorghum bicolor), rape (Brassica napus), sunflower (Helianthus annuus), suger beet (Beta Vulgaris), suger cane (Saccharum officinarum), Japanese Lawngrass (Zoysia japonica steud.), peanut (Arachis hypogaea), flax (Linum usitatissimum), tobacco (Nicotiana tabacum), coffee(Coffea spp.), apple (Malus pumila), Japanese pear (Pyrus pyrifolia), pear (Pyrus communis), Japanese medlar (Eriobotrya japonica), peach (Prunus persica), nectarine (Prunus persica, var. nectarina), Japanese apricot (Prunus mume), cherry (Prunus avium), apricot (Prunus armeniaca), plum (Prunus domestica), Satsuma mandarin (Citrus unshiu), orange (Citrus sinensis), lemon (Citrus limon), lime (Citrus aurantiifolia), grapefruit (Citrus paradisi), blueberry (Vaccinium spp.), blackberrie (Rubus fruticosus), raspberrie (Rubus idaeus), grape (Vitis vinifera), Japanese chestnut (Castanea crenata), walnut (Juglans spp.), almond (Amygdalus dulcis), pistachio (Pistacia vera), cashew (Anacardium occidentale), macadamia nut (Macadamia integrifolia), persimmon (Diospyros kaki Thunb.), olive (Olea europaea), banana (Musa spp.), date palm (Phoenix dactylifera) and oil palm (Elaeis spp.).

The present composition can be applied to harmful weeds, useful plants or soil, to control various undesired plants. Soil include soil where plants grow and soil where plants can grow. For example, the present composition can be used to control harmful weeds in e.g. agricultural fields such as paddy fields and crop plant fields and non-agricultural fields such as lawn and roadsides.

The useful plants include plants having, imparted by classical breeding methods, resistance to a herbicide, for example, a HPPD inhibitor such as isoxaflutole; an ALS inhibitor such as imazethapyr or thifensulfuron-methyl; an EPSP synthase inhibitor such as glyphosate; a glutamine synthase inhibitor such as glufosinate; an acetyl CoA carboxylase inhibitor such as sethoxydim; a PPO inhibitor such as flumioxazin, bromoxynil; dicamba; or 2,4-D. The useful crops may, for example, be specifically imidazolinone herbicide resistant corn, rapeseed, wheat, sunflower and rice (tradename: Clearfield^{™}); STS soybean resistant to sulfonylurea ALS inhibitor type herbicide; SR corn resistant to acetyl CoA carboxylase inhibitors such as trione oxime and aryl oxyphenoxypropionic acid herbicides.

The useful plants include transgenic plants generated by gene recombination or gene editing. For example, plants having imparted resistance to environmental stress, resistance to herbicides, resistance to pests and resistance to phytopathogen, and plants having growth, fertility, quality or yield of crops modified, are included. Further, the useful plants also include stacked cultivars in which these multiple useful traits are combined.

The herbicide-resistant transgenic plants include glyphosate-resistant corn, soybean, cotton, rapeseed and sugar beet (tradename: RoundupReady^{™}, Agrisure^{™}, Gly-Tol^{™}); glufosinate-resistant corn, soybean, cotton and rapeseed (tradename: LibertyLink^{™}); and bromoxynil-resistant cotton (tradename: BXN).

The noxious insect-resistant transgenic plants include, for example, plants which have acquired the ability to synthesize e.g. toxins known to be synthesized by the genus Bacillus. Toxins synthesized by the plants generated by gene recombination or gene editing impart to the plants resistance particularly to Coleoptera insects, Hemiptera insects, Diptera insects, Lepidoptera insects and nematodes.

The above toxins include insecticidal proteins derived from Bacillus cereus and Bacillus popilliae; δ-endotoxins derived from Bacillus thuringiensis, such as Cry1Ab, Cry1Ac, Cry1F, Cry1Fa2, Cry2Ab, Cry3A, Cry3Bb1 and Cry9C; insecticidal proteins such as VIP1, VIP2, VIP3 and VIP3A; insecticidal proteins derived from nematodes; toxins produced by animals, such as scorpion toxin, spider toxin, bee toxin and insect-specific neurotoxin; filamentous fungi toxin; plant lectin; agglutinin; protease inhibitors such as trypsin inhibitor, serine protease inhibitor, patatin, cystatin and papain inhibitor; ribosome-inactivating proteins (RIP) such as ricin, abrin, luffin, saporin and bryodin; steroid metabolizing enzymes such as 3-hydroxysteroid oxidase, ecdysteroid-UDP-glucosyltransferase and cholesterol oxidase; ecdysone inhibitor; HMG-CoA reductase; ion channel blockers such as sodium channel blocker and calcium channel blocker; juvenile hormone esterase; diuretic hormone receptor; stilbene synthase; bibenzyl synthase; chitinase; and glucanase. Further, such toxins include hybrid toxins of δ-endotoxin proteins such as Cry1Ab, Cry1Ac, Cry1F, Cry1Fa2, Cry2Ab, Cry3A, Cry3Bb1, Cry9C, Cry34Ab and Cry35Ab, and insecticidal proteins such as VIP1, VIP2, VIP3 and VIP3A, toxins lacking part thereof, and modified toxins.

The transgenic plants relating to plant components include plants having imparted oil component modified character or amino acid content increased character. Specifically, low-linolenic soybean (tradename: VISTIVE^{™}) having a linolenic content decreased; corn having a lysine or oil content increased, may, for example, be mentioned.

The phytopathogen-resistant transgenic plants include plants having imparted the ability to produce anti-pathogen substances with selective action.

The present composition may be obtained by mixing the compound (I) with a solid carrier or a liquid carrier and as the case requires, a surfactant and other additives for formulation, and applied in the form of various formulations such as dusts, granules, micro granules, water dispersible granules, wettable powders, water-based suspensions, oil-based suspensions, water soluble powders, emulsifiable concentrates, liquid, pastes, aerosols, ultra low-volume formulations and microcapsules. It may be formed into any usual formulation used in this field, so long as the object of the present invention is thereby met.

The solid carrier may, for example, be specifically diatomaceous earth, slaked lime, a carbonate, talc, white carbon, kaoline, bentonite, kaolinite, sericite, clay, sodium bicarbonate, salt cake, zeolite, starch, a polycarboxylate, an anhydrous sulfate or dodecylbenzenesulfonate.

The liquid carrier may, for example, be specifically water, toluene, xylene, solvent naphtha, dioxane, acetone, isophorone, methyl isobutyl ketone, chlorobenzene, cyclohexane, dimethyl sulfoxide, dimethylformamide, dimethylacetamide, N-methyl-2-pyrrolidone, N,N-dimethylacetamide, diethylene glycol monoethyl ether or alcohols (such as ethylene glycol).

The surfactant may, for example, be specifically an anionic surfactant or spreader such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkyl ether phosphate ester, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester, a salt of polyoxyethylene arylether phosphoric acid ester, a polyoxyethylene styryl phenyl ether phosphate, a salt of naphthalene sulfonate condensed with formaldehyde, a salt of alkylnaphthalene sulfonate condensed with formaldehyde, an alkylnaphthalene sulfonate, a lignin sulfonate or a polyoxyethylene styrylphenyl ether sulfate; or a nonionic surfactant or spreader such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, polyethylene glycol, a polyoxyethylene fatty acid ester, a polyoxyethylene sorbitan fatty acid ester, a polyoxyethylene glycerin fatty acid ester, a polyoxyethylene hydrogenated castor oil, a polyoxypropylene fatty acid ester or polyoxyethylene styrylphenyl ether.

Other additives for formulation may, for example, be a vegetable oil or mineral oil such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil, liquid paraffins, silicone oil or xanthan gum.

These additive components may suitably be selected for use alone or in combination as a mixture of two or more of them, so long as the object of the present invention is met. Further, in addition to the above, various additives known in this technical field, such as a filler, a thickener, an anti-settling agent, an anti-freezing agent, a dispersion stabilizer, a safener and an anti-mold agent, may be used. The mixing ratio of the compound (I) to such various additives is usually 0.1:99.9 to 95:5, preferably 0.2:99.8 to 85:15, by weight ratio. In the actual application of such a formulation, it may be used as it is, or may be diluted to a predetermined concentration with a diluent such as water, and may be mixed with various spreaders (e.g. surfactants, vegetable oils or mineral oils), as the case requires.

The application of the present composition cannot generally be defined, as it varies depending upon the weather conditions, the type of the formulation, the plants to be treated, the application season, the application site, the type or degree of outbreak of harmful weeds, etc. However, the present composition or a diluted product thereof may be applied by a conventional application method, that is soil treatment, foliar treatment, water treatment, etc. Usually, in the case of soil treatment, the amount of the present composition applied is about 0.1 g to 5,000 g per hectare, and the amount of the compound (I) as the active ingredient of the present composition is about 0.001 to 4,500 g per hectare. Usually, in the case of foliar treatment, the amount of the present composition applied is about 0.1 g to 5,000 g per hectare, and the amount of the compound (I) as the active ingredient of the present composition is about 0.001 to 4,500 g per hectare. Usually, in the case of water treatment, the amount of the present composition applied is about 0.1 g to 5,000 g per hectare, and the amount of the compound (I) as the active ingredient of the present composition is about 0.001 to 4,500 g per hectare.

The soil treatment is a method of controlling undesired plants by applying the compound (I) to soil where the useful plants grow or where they can grow. Specifically, it may, for example, be a treatment method of applying a liquid concentrate such as an emulsifiable concentrate, a liquid or a wettable powder, or a water-diluted solution thereof, granules, dust, micro granules or the like to the soil surface, or a treatment method of applying a liquid concentrate such as an emulsifiable concentrate, a liquid or a wettable powder, or a water-diluted solution thereof, granules, dust, particles, or the like to the soil surface, and mixing the soil by e.g. a machine.

The foliar treatment may, for example, be a method of controlling harmful weeds by applying the compound (I) to the whole or a part of the useful plants or the harmful weeds. A part of the useful plants or the harmful weeds means all portions and a combination thereof constituting the useful plants or the harmful weeds, such as leaves, stems, branches, buds and flowers of the useful plants or the harmful weeds.

The water treatment is a method of controlling harmful weeds growing or harmful weeds before budding, in a flooded paddy field where the useful plants grow or where the useful plants are to be grown, and specifically, a method of spraying wettable powder, granules, dust or micro granules to peripheries or ridges of a paddy field, into a paddy field, a water inlet, etc.

The present composition may be mixed with or may be used in combination with other components selected from other agricultural and horticultural chemicals, fertilizers and safeners, whereby more excellent effects or activities may sometimes be obtained.

The mixing with or use in combination with other components means that the present composition and other components are used simultaneously, separately or with an interval.

Such other agricultural and horticultural chemicals include, for example, a herbicide, an insecticide, a miticide, a nematicide, a soil pesticide, a herbicide, an antivirus agent, an attractant, an antibiotic, a plant hormone, a plant growth regulator, and so on. Especially, a mixed herbicidal composition having the present composition mixed with or used in combination with one or more active ingredient compounds of other herbicide, may improve the application range, the application time, the controlling activities, etc. to preferred directions. The present composition and the active ingredient compounds of other herbicide may separately be formulated so that they are mixed for use at the time of application, or they may be formulated together. The present invention includes such a mixed herbicidal composition.

The mixing ratio of the compound (I) to the active ingredient compounds of other herbicide can not generally be defined as it varies depending upon the weather conditions, the types of formulations, the crop plants to be treated, the application time, the application site, the types or degree of outbreak of harmful weeds, etc., but it is usually within a range of 1:0.001 to 1:10,000, preferably 1:0.01 to 1:1,000, by weight ratio. Further, the dose for the application is such that the total amount of the active ingredient compounds is 0.1 to 10,000 g, preferably 0.2 to 5,000 g, per hectare. The present invention includes a method for controlling harmful weeds by application of such a mixed herbicidal composition.

When the herbicidal composition containing the compound (I) is applied, it may be used in combination with other agricultural and horticultural chemicals, such as a herbicide, an insecticide, a miticide, a nematicide, a soil pesticide, an antivirus agent, an attractant, a herbicide and a plant growth regulator.

The active ingredient compounds of a herbicide in the above-mentioned other agricultural and horticultural chemicals may properly be selected, for example, from the following group of compounds (by common names). In a case where these compounds have their salts, alkyl esters, various structural isomers such as optical isomers, etc., all of them are included, even if no specific disclosure thereof is made.

(1) Compounds which are believed to exhibit herbicidal effects by disturbing hormone activities of plants, including phenoxy compounds such as mecoprop, mecoprop-butotyl, mecoprop-sodium, mecoprop-P, mecoprop-P-butotyl, mecoprop-P-dimethylammonium, mecoprop-P-2-ethylhexyl, mecoprop-P-potassium, dichlorprop, naproanilide, clomeprop, erbon, disul, disul-sodium, fenoprop and HIA-1; aromatic carboxylic acid compounds such as 2,3,6-TBA, dicamba, dicamba-butotyl, dicamba-diglycolamine, dicamba-dimethylammonium, dicamba-diolamine, dicamba-isopropylammonium, dicamba-potassium, dicamba-sodium, chloramben, picloram, picloram-dimethylammonium, picloram-isoctyl, picloram-potassium, picloram-triisopropanolammonium, picloram-triisopropylammonium, picloram-trolamine, triclopyr, triclopyr-butotyl, triclopyr-triethylammonium, clopyralid, clopyralid-olamine, clopyralid-potassium, clopyralid-triisopropanolammonium, aminopyralid, aminocyclopyrachlor, halauxifen, halauxifen-methyl, florpyrauxifen, florpyrauxifen-benzyl, tricamba and DAS-534; and others such as cliodinate, naptalam, naptalam-sodium, benazolin, benazolin-ethyl, quinclorac, quinmerac, diflufenzopyr, diflufenzopyr-sodium, fluroxypyr, fluroxypyr-2-butoxy-1-methylethyl, fluroxypyr-meptyl, chlorflurenol, chlorflurenol-methyl, clacyfos, aminocyclopyrachlor-methyl, aminocyclopyrachlor-potassium, fluchloraminopyr and indolauxipyr.
(2) Compounds which are believed to exhibit herbicidal effects by inhibiting photosynthesis of plants, including urea compounds such as chlorotoluron, diuron, fluometuron, linuron, metobromuron, monuron, isoproturon, metobenzuron, tebuthiuron, dimefuron, isouron, karbutilate, methabenzthiazuron, metoxuron, buthiuron, monolinuron, neburon, buturon, chlorbromuron, chloreturon, chloroxuron, difenoxuron, ethidimuron, fenuron, fluothiuron, isonoruron, monuron-TCA and siduron; triazine compounds such as terbumeton, trietazine, simazine, aziprotryne, chlorazine, cyprazine, desmetryn, dipropetryn, methoprotryne, proglinazine, proglinazine-ethyl, sebuthylazine, secbumeton, atrazine, atraton, simetryn, prometryn, dimethametryn, terbuthylazine, cyanazine, ametryn, cybutryne, terbutryn, propazine, cyanatryn and prometon; triazinone compounds such as hexazinone, ametridione, amibuzin, ethiozin, isomethiozin, metribuzin and metamitron; uracil compounds such as bromacil, bromacyl-lithium, lenacil, isocil and terbacil; anilide compounds such as propanil, chloranocryl, cypromid and pentanochlor; carbamate compounds such as desmedipham, chlorprocarb, phenisopham and phenmedipham; hydroxybenzonitrile compounds such as bromoxynil, bromoxynil-octanoate, bromoxynil-heptanoate, chloroxynil, ioxynil, ioxynil-octanoate, ioxynil-potassium and ioxynil-sodium; and others such as pyridate, bentazone, bentazone-sodium, amicarbazone, methazole, iodobonil, methiuron, pyridafol, tioclorim and chloridazon.
(3) Quaternary ammonium salt compounds such as paraquat or diquat, which are believed to be converted to free radicals by themselves to form active oxygen in the plant and show rapid herbicidal efficacy.
(4) Compounds which are believed to exhibit herbicidal effects by inhibiting chlorophyll biosynthesis of plants and abnormally accumulating a photosensitizing peroxide substance in the plant, including diphenyl ether compounds such as chlornitrofen, fluorodifen, fluoronitrofen, furyloxyfen, halosafen, nitrofen, chlomethoxyfen, bifenox, acifluorfen, acifluorfen-sodium, fomesafen, fomesafen-sodium, oxyfluorfen, lactofen, aclonifen, ethoxyfen-ethyl, fluoroglycofen-ethyl and fluoroglycofen; cyclic imide compounds such as chlorphthalim, flumioxazin, flumiclorac, flumiclorac-pentyl, flumipropyn, cinidon-ethyl, fluthiacet-methyl and EK-5385; and others such as flupropacil, oxadiargyl, oxadiazon, sulfentrazone, carfentrazone-ethyl, thidiazimin, pentoxazone, azafenidin, pyraflufen-ethyl, benzfendizone, butafenacil, saflufenacil, fluazolate, profluazol, flufenpyr-ethyl, bencarbazone, tiafenacil, trifludimoxazin, epyrifenacil, pyraclonil, cyclopyranil, flufenoximacil, compound I described in WO2007/101587, compound 1 described in WO2000/050407, and compound 11 described in WO2002/019825.
(5) Compounds which are believed to exhibit herbicidal effects characterized by bleaching activities by inhibiting chromogenesis of plants such as carotenoids, including pyridazinone compounds such as norflurazon and metflurazon; pyrazole compounds such as pyrazolynate, pyrazoxyfen, benzofenap, topramezone, tripyrasulfone, fenpyrazone, cypyrafluone, pyrasulfotole, tolpyralate and pyraquinate; and others such as bicyclopyrone, amitrole, flurochloridone, fluridone, flurtamone, diflufenican, methoxyphenone, clomazone, bixlozone, broclozone, mesotrione, tefuryltrione, fenquinotrione, cyclopyrimorate, difenzoquat, difenzoquat-metilsulfate, isoxachlortole, benzobicyclon, picolinafen, beflubutamid, beflubutamid-M, benquitrione, rimisoxafen, dioxopyritrione, quintrione, isoxaflutole, lancotrione, lancotrione-sodium, sulcotrione, tembotrione, ketospiradox, ketospiradox-potassium, iptriazopyrid and flusulfinam.
(6) Compounds which are believed to exhibit herbicidal effects by inhibiting a fatty acid biosynthesis of plants, including aryloxyphenoxypropionic acid compounds such as diclofop-methyl, diclofop, pyriphenop-sodium, fluazifop-butyl, fluazifop, fluazifop-P, fluazifop-P-butyl, fenthiaprop, fenthiaprop-ethyl, haloxyfop-methyl, haloxyfop, haloxyfop-etotyl, haloxyfop-P, haloxyfop-P-methyl, quizalofop-ethyl, quizalofop-P, quizalofop-P-ethyl, quizalofop-P-tefuryl, cyhalofop-butyl, fenoxaprop-ethyl, fenoxaprop-P, fenoxaprop-P-ethyl, metamifop-propyl, metamifop, clodinafop-propargyl, clodinafop, propaquizafop, isoxapyrifop, trifop, trifop-methyl, trifopsime, HNPC-A8169 and SYP-1924; cyclohexanedione compounds such as alloxydim-sodium, alloxydim, clethodim, sethoxydim, tralkoxydim, butroxydim, tepraloxydim, profoxydim, cycloxydim and cloproxydiml; and phenylpyrazoline compounds such as pinoxaden.
(7) Compounds which are believed to exhibit herbicidal effects by inhibiting an amino acid biosynthesis of plants, including sulfonylurea compounds such as chlorimuron-ethyl, chlorimuron, sulfometuron-methyl, sulfometuron, primisulfuron-methyl, primisulfuron, bensulfuron-methyl, bensulfuron, chlorsulfuron, metsulfuron-methyl, metsulfuron, cinosulfuron, pyrazosulfuron-ethyl, pyrazosulfuron, flazasulfuron, rimsulfuron, nicosulfuron, imazosulfuron, flucetosulfuron, cyclosulfamuron, prosulfuron, flupyrsulfuron-methyl-sodium, flupyrsulfuron, triflusulfuron-methyl, triflusulfuron, halosulfuron-methyl, halosulfuron, thifensulfuron-methyl, thifensulfuron, ethoxysulfuron, oxasulfuron, ethametsulfuron, ethametsulfuron-methyl, iodosulfuron, iodosulfuron-methyl-sodium, sulfosulfuron, triasulfuron, tribenuron-methyl, tribenuron, tritosulfuron, foramsulfuron, trifloxysulfuron, trifloxysulfuron-sodium, mesosulfuron-methyl, mesosulfuron, orthosulfamuron, amidosulfuron, azimsulfuron, propyrisulfuron, metazosulfuron, methiopyrsulfuron, monosulfuron-methyl, iofensulfuron and iofensulfuron-sodium; triazolopyrimidinesulfonamide compounds such as flumetsulam, metosulam, diclosulam, cloransulam-methyl, florasulam, penoxsulam and pyroxsulam; imidazolinone compounds such as imazapyr, imazapyr-isopropylammonium, imazethapyr, imazethapyr-ammonium, imazaquin, imazaquin-ammonium, imazamox, imazamox-ammonium, imazamethabenz, imazamethabenz-methyl and imazapic; pyrimidinylsalicylic acid compounds such as pyrithiobac-sodium, bispyribac-sodium, pyriminobac-methyl, pyribenzoxim and pyriftalid; sulfonylaminocarbonyltriazolinone compounds such as flucarbazone, flucarbazone-sodium, propoxycarbazone-sodium, propoxycarbazone and thiencarbazone-methyl; sulfonanilide compounds such as pyrimisulfan and triafamone; and others such as glyphosate, glyphosate-sodium, glyphosate-potassium, glyphosate-ammonium, glyphosate-diammonium, glyphosate-isopropylammonium, glyphosate-trimesium, glyphosate-sesquisodium, glufosinate, glufosinate-ammonium, glufosinate-P, glufosinate-P-ammonium, glufosinate-P-sodium, bilanafos, bilanafos-sodium, cinmethylin and pyriflubenzoxim.
(8) Compounds which are believed to exhibit herbicidal effects by inhibiting cell mitoses of plants, including dinitroaniline compounds such as trifluralin, oryzalin, nitralin, pendimethalin, ethalfluralin, benfluralin, prodiamine, profluralin, butralin, dinitramine, isopropalin and fluchloralin; amide compounds such as bensulide, napropamide, napropamide-M, propyzamide and pronamide; organic phosphorus compounds such as amiprofos-methyl, butamifos, DMPA, anilofos and piperophos; phenylcarbamate compounds such as propham, chlorpropham, barban, carbetamide and swep; cumylamine compounds such as daimuron, cumyluron, bromobutide and methyldymron; and others such as chlorbufam, dithiopyr, thiazopyr, chlorthal-dimethyl, chlorthal, diphenamid, flamprop-M-methyl, flamprop-M and flamprop-M-isopropyl.
(9) Compounds which are believed to exhibit herbicidal effects by inhibiting protein biosynthesis or lipid biosynthesis of plants, including chloroacetamide compounds such as alachlor, metazachlor, butachlor, pretilachlor, metolachlor, S-metolachlor, thenylchlor, pethoxamid, acetochlor, propachlor, dimethenamid, dimethenamid-P, propisochlor, dimethachlor, delachlor, diethatyl, diethatyl-ethyl, prynachlor and terbuchlor; thiocarbamate compounds such as molinate, dimepiperate, pyributicarb, EPTC, butylate, pebulate, cycloate, prosulfocarb, esprocarb, thiobencarb, diallate, triallate, vernolate, tiocarbazil and orbencarb; and others such as etobenzanid, mefenacet, flufenacet, tridiphane, indanofan, fentrazamide, cafenstrole, ipfencarbazone, benfuresate, pyroxasulfone, fenoxasulfone, methiozolin, dalapon, dalapon-sodium, TCA-sodium, ethofumesate and trichloroacetic acid.
(10) Compounds which are believed to exhibit herbicidal effects by inhibiting a cellulose biosynthesis of plants, such as dichlobenil, triaziflam, indaziflam, flupoxam, isoxaben and chlorthiamide.
(11) MSMA, DSMA, CMA, endothall, endothall-dipotassium, endothall-sodium, endothall-mono(N,N-dimethylalkylammonium), asulam, asulam-sodium oxaziclomefone, tetflupyrolimet, sodium chlorate, pelargonic acid, nonanoic acid, fosamine, fosamine-ammonium, aclolein, ammonium sulfamate, borax, chloroacetic acid, sodium chloroacete, cyanamide, methylarsonic acid, dimethylarsinic acid, sodium dimethylarsinate, dinoterb, dinoterb-ammonium, dinoterb-diolamine, dinoterb-acetate, DNOC, dinoseb, ferrous sulfate, flupropanate, flupropanate-sodium, mefluidide, mefluidide-diolamine, metam, metam-ammonium, metam-potassium, metam-sodium, methyl isothiocyanate, pentachlorophenol, sodium pentachlorophenoxide, pentachlorophenol laurate, quinoclamine, sulfuric acid, urea sulfate, xanthinosin, herbimycin, unguinol, metatyrosine, sarmentine, thaxtominA, mevalocidin, alpha-limonene, pyribambenz-propyl, pyribambenz-isopropyl, allidochlor, alorac, amidochlor, anisuron, benzadox, benzadoxammonium, benzipram, benzofluor, benzoylprop, benzoylprop-ethyl, bromobonil, bromofenoxim, butenachlor, buthidazole, cambendichlor, carbasulam, carboxazole, chlorazifop, chlorazifop-propargyl, chlorfenac, chlorfenac-salts, chlorfenprop, chlorfenprop-methyl, cisanilide, clofop, clofop-isobutyl, cloprop, credazine, cycluron, cyperquat, cyperquat-chloride, dazomet, dazomet-sodium, dichlormate, o-dichlorobenzene, diethamquat, difenopenten, difenopenten-ethyl, dimesulfazet, dimexano, dimidazon, dimethyl disulfide, dinofenate, dinosam, dipropalin, eglinazine, eglinazine-ethyl, ethachlor, etinofen, fenasulam, flumezin, fluoromidine, halosafen, haloxydine, hexachloroacetone, isocarbamid, isopolinate, medinoterb, medinoterb actate, monalide, morfamquat, morfamquat dichloride, noruron, oleic acid, oxapyrazon, perfluidone, pydanon, pyriclor, quinonamid, sulfallate, tebutam, tetrafluron, thiazafluron, thidiazuron, xylachlor, bipyrazone, Aspterric acid, icafolin, compound A1 described in JP-A-2022/42267, compound 43 described in WO2016/095768, and compound I-1-1 described in WO2012/065573.
(12) Those which are believed to exhibit herbicidal effects by being parasitic on plants, such as Xanthomonas campestris, Epicoccosirus nematosorus, Epicoccosirus nematosperus, Exserohilum monoseras, or Drechsrela monoceras.

One or more compounds may properly be selected from among the above compounds as the active ingredient of other herbicides. The active ingredients of other herbicides are not limited to the above-exemplified compounds.

Preferred embodiments of the present invention will be described below. However, it should be understood that the present invention is by no means restricted thereto.
[1] A herbicidal composition comprising as an active ingredient a difluorobutenoic acid amide compound represented by the formula (I) or a salt thereof.
[2] A method for controlling undesired plants or inhibiting their growth, which comprises applying an effective amount of a difluorobutenoic acid amide compound represented by the formula (I) or a salt thereof to harmful weeds, useful plants or soil.
[3] A difluorobutenoic acid amide compound represented by the formula (I) or a salt thereof.
[4] A difluorobutenoic acid amide compound represented by the formula (IAa), (IAb), (IBa), (IBb) or (IBc), or a salt thereof.
[5] A difluorobutenoic acid amide compound represented by the formula (IAa), (IBa), (IBb) or (IBc), or a salt thereof.
[6] A difluorobutenoic acid amide compound represented by the formula (IAa), (IBa) or (IBb), or a salt thereof.
[7] A difluorobutenoic acid amide compound represented by the formula (IAa), (IBa) or (IBc), or a salt thereof.
[8] A difluorobutenoic acid amide compound represented by the formula (IAa), (IBb) or (IBc), or a salt thereof.
[9] A difluorobutenoic acid amide compound represented by the formula (IBa), (IBb) or (IBc), or a salt thereof.
[10] A difluorobutenoic acid amide compound represented by the formula (IAa) or (IBa), or a salt thereof.
[11] A difluorobutenoic acid amide compound represented by the formula (IAa) or (IBb), or a salt thereof.
[12] A difluorobutenoic acid amide compound represented by the formula (IAa) or (IBc), or a salt thereof.
[13] A difluorobutenoic acid amide compound represented by the formula (IBa) or (IBb), or a salt thereof.
[14] A difluorobutenoic acid amide compound represented by the formula (IBa) or (IBc), or a salt thereof.
[15] A difluorobutenoic acid amide compound represented by the formula (IBb) or (IBc), or a salt thereof.
[16] A difluorobutenoic acid amide compound represented by the formula (IAa), or a salt thereof.
[17] A difluorobutenoic acid amide compound represented by the formula (IBa), or a salt thereof.
[18] A difluorobutenoic acid amide compound represented by the formula (IBb), or a salt thereof.
[19] A difluorobutenoic acid amide compound represented by the formula (IBc), or a salt thereof.
[20] The difluorobutenoic acid amide compound or a salt thereof according to [3], wherein the compound represented by the formula (I) is a compound represented by the formula (IAa) or (IBa).
[21] The difluorobutenoic acid amide compound or a salt thereof according to [3], wherein the compound represented by the formula (I) is a compound represented by the formula (IAa) or (IBb).
[22] The difluorobutenoic acid amide compound or a salt thereof according to [3], wherein the compound represented by the formula (I) is a compound represented by the formula (IAa) or (IBc).
[23] The difluorobutenoic acid amide compound or a salt thereof according to [3], wherein the compound represented by the formula (I) is a compound represented by the formula (IBa) or (IBb).
[24] The difluorobutenoic acid amide compound or a salt thereof according to [3], wherein the compound represented by the formula (I) is a compound represented by the formula (IBa) or (IBc).
[25] The difluorobutenoic acid amide compound or a salt thereof according to [3], wherein the compound represented by the formula (I) is a compound represented by the formula (IBb)or (IBc).
[26] The difluorobutenoic acid amide compound or a salt thereof according to [3], wherein the compound represented by the formula (I) is a compound represented by the formula (IAa).
[27] The difluorobutenoic acid amide compound or a salt thereof according to [3], wherein the compound represented by the formula (I) is a compound represented by the formula (IBa).
[28] The difluorobutenoic acid amide compound or a salt thereof according to [3], wherein the compound represented by the formula (I) is a compound represented by the formula (IBb).
[29] The difluorobutenoic acid amide compound or a salt thereof according to [3], wherein the compound represented by the formula (I) is a compound represented by the formula (IBc).
[30] The difluorobutenoic acid amide compound or a salt thereof according to [3], wherein in a case where t is 1, the broken line moiety of carbon-carbon bond is a single bond.
[31] The difluorobutenoic acid amide compound or a salt thereof according to [3], wherein t is 1, and the broken line moiety of carbon-carbon bond is a single bond.
[32] The difluorobutenoic acid amide compound or a salt thereof according to [3], wherein t is 2, and the broken line moiety of carbon-carbon bond is a single bond.
[33] The difluorobutenoic acid amide compound or a salt thereof according to [3], wherein t is 2, and one of the two broken line moieties of carbon-carbon bond is a single bond and the other is a double bond.
[34] The difluorobutenoic acid amide compound or a salt thereof according to [3], wherein t is 1, the broken line moiety of carbon-carbon bond is a single bond, and R⁴ substitutes at the 3-position on cyclobutyl.
[35] The difluorobutenoic acid amide compound or a salt thereof according to [3], wherein t is 2, the broken line moiety of carbon-carbon bond is a single bond, and R⁴ substitutes at the 3-position on cyclopentyl.
[36] The difluorobutenoic acid amide compound or a salt thereof according to [10], wherein in the compound (IAa), R⁴ substitutes at the 3-position on cyclobutyl, or in the compound (IBa), R⁴ substitutes at the 3-position on cyclopentyl.
[37] The difluorobutenoic acid amide compound or a salt thereof according to [11], wherein in the compound (IAa), R⁴ substitutes at the 3-position on cyclobutyl, or in the compound (IBb), R⁴ substitutes at the 3-position on 4-cyclopentenyl.
[38] The difluorobutenoic acid amide compound or a salt thereof according to [12], wherein in the compound (IAa), R⁴ substitutes at the 3-position on cyclobutyl, or in the compound (IBc), R⁴ substitutes at the 3-position on 3-cyclopentenyl.
[39] The difluorobutenoic acid amide compound or a salt thereof according to [13], wherein in the compound (IBa), R⁴ substitutes at the 3-position on cyclopentyl, or in the compound (IBb), R⁴ substitutes at the 3-position on 4-cyclopentenyl.
[40] The difluorobutenoic acid amide compound or a salt thereof according to [14], wherein in the compound (IBa), R⁴ substitutes at the 3-position on cyclopentyl, or in the compound (IBc), R⁴ substitutes at the 3-position on 3-cyclopentenyl.
[41] The difluorobutenoic acid amide compound or a salt thereof according to [15], wherein in the compound (IBb), R⁴ substitutes at the 3-position on 4-cyclopentenyl, or in the compound (IBc), R⁴ substitutes at the 3-position on 3-cyclopentenyl.
[42] The difluorobutenoic acid amide compound or a salt thereof according to [16], wherein in the compound (IAa), R⁴ substitutes at the 3-position on cyclobutyl.
[43] The difluorobutenoic acid amide compound or a salt thereof according to [17], wherein in the compound (IBa), R⁴ substitutes at the 3-position on cyclopentyl.
[44] The difluorobutenoic acid amide compound or a salt thereof according to [18], wherein in the compound (IBb), R⁴ substitutes at the 3-position on 4-cyclopentenyl.
[45] The difluorobutenoic acid amide compound or a salt thereof according to [19], wherein in the compound (IBc), R⁴ substitutes at the 3-position on 3-cyclopentenyl.
[46] The difluorobutenoic acid amide compound or a salt thereof according to any one of [3] to [45], wherein R¹ and R² are each a halogen, a (C₁-C₆)-chain hydrocarbon which may be substituted by at least one Y¹, a (C₁-C₆)-alkoxy which may be substituted by at least one halogen, cyano, or H, and Y¹ is a halogen.
[47] The difluorobutenoic acid amide compound or a salt thereof according to any one of [3] to [45], wherein R¹ and R² are each a halogen, a (C₁-C₆)-alkyl which may be substituted by at least one Y¹, a (C₁-C₆)-alkoxy which may be substituted by at least one halogen, cyano, or H, and Y¹ is a halogen.
[48] The difluorobutenoic acid amide compound or a salt thereof according to any one of [3] to [45], wherein R¹ and R² are each a halogen, a (C₁-C₆)-alkyl which may be substituted by at least one Y¹, a (C₁-C₆)-alkoxy which may be substituted by at least one halogen, cyano, or H and Y' is a fluorine atom.
[49] The difluorobutenoic acid amide compound or a salt thereof according to any one of [3] to [45], wherein R¹ and R² are each a fluorine atom, a (C₁-C₆)-alkyl which may be substituted by at least one Y¹, a (C₁-C₆)-alkoxy which may be substituted by at least one halogen, cyano, or H, and Y¹ is a fluorine atom.
[50] The difluorobutenoic acid amide compound or a salt thereof according to any one of [3] to [45], wherein R¹ and R² are each a fluorine atom, a chlorine atom, a (C₁-C₆)-alkyl which may be substituted by at least one Y¹, cyano or H, and Y¹ is a fluorine atom.
[51] The difluorobutenoic acid amide compound or a salt thereof according to any one of [3] to [45], wherein R¹ and R² are each a halogen, methyl, ethyl, n-propyl, isopropyl, monofluoromethyl, difluoromethyl, trifluoromethyl, 2,2,2-trifluoroethyl, 2,2-difluoroethyl, 1,1-difluoroethyl, methoxy, ethoxy, isopropyloxy, difluoromethoxy, trifluoromethoxy, 2,2-difluoroethoxy, cyano or H.
[52] The difluorobutenoic acid amide compound or a salt thereof according to any one of [3] to [45], wherein R¹ and R² are each a halogen, methyl, difluoromethyl, trifluoromethyl, methoxy, trifluoromethoxy, cyano or H.
[53] The difluorobutenoic acid amide compound or a salt thereof according to any one of [3] to [45], wherein R¹ and R² are each a fluorine atom, methyl, 2,2-difluoroethyl, methoxy, trifluoromethoxy, cyano or H.
[54] The difluorobutenoic acid amide compound or a salt thereof according to any one of [3] to [45], wherein R¹ and R² are each a fluorine atom, methyl, 2,2-difluoroethyl, methoxy or H.
[55] The difluorobutenoic acid amide compound or a salt thereof according to any one of [3] to [45], wherein R¹ and R² are each a fluorine atom, a chlorine atom, difluoromethyl, trifluoromethyl, cyano or H.
[56] The difluorobutenoic acid amide compound or a salt thereof according to any one of [3] to [45], wherein R¹ and R² are each a fluorine atom, a chlorine atom, methyl or H.
[57] The difluorobutenoic acid amide compound or a salt thereof according to any one of [3] to [56], wherein R³ is H.
[58] The difluorobutenoic acid amide compound or a salt thereof according to any one of [3] to [56], wherein R³ is a fluorine atom.
[59] The difluorobutenoic acid amide compound or a salt thereof according to any one of [3] to [58], wherein R⁴ is -C(=O)OR⁵, -C(=O)SR⁶, -C(=O)NR⁷R⁸, -C(=O)R⁹, -SR⁶, - S(=O)R⁶, a (C₁-C₆)-chain hydrocarbon which may be substituted by at least one Z³, or OH.
[60] The difluorobutenoic acid amide compound or a salt thereof according to any one of [3] to [58], wherein R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, or -CH(OH)-C(=O)R¹¹.
[61] The difluorobutenoic acid amide compound or a salt thereof according to any one of [3] to [58], wherein R⁴ is -C(=O)OR⁵, -C(=O)NR⁷R⁸, -C(=O)R⁹ or a (C₁-C₆)-chain hydrocarbon which may be substituted by at least one Z³.
[62] The difluorobutenoic acid amide compound or a salt thereof according to any one of [3] to [58], wherein R⁴ is -C(=O)OR⁵, -C(=O)SR⁶, -C(=O)NR⁷R⁸, a (C₁-C₆)-alkyl which may be substituted by at least one Z³, or -CH(OH)-C(=O)R¹¹, and Z³ is a halogen or a (C₁-C₆)-alkylsulfonyloxy.
[63] The difluorobutenoic acid amide compound or a salt thereof according to any one of [3] to [58], wherein R⁴ is -C(=O)OR⁵, a (C₁-C₆)-alkyl which may be substituted by at least one Z³, or -CH(OH)-C(=O)R¹¹, and Z³ is a halogen or a (C₁-C₆)-alkylsulfonyloxy.
[64] The difluorobutenoic acid amide compound or a salt thereof according to any one of [3] to [63], wherein R⁵ is a (C₁-C₆)-alkyl, H, a (C₃-C₆)-cycloalkyl, a (C₂-C₆)-alkenyl, a (C₂-C₆)-alkynyl or a 4- to 6-membered oxygen-containing saturated heterocyclic ring.
[65] The difluorobutenoic acid amide compound or a salt thereof according to any one of [3] to [63], wherein R³ is a (C₁-C₆)-alkyl.
[66] The difluorobutenoic acid amide compound or a salt thereof according to any one of [3] to [63], wherein R⁵ is methyl, ethyl, n-propyl, isopropyl, n-butyl, tert-butyl, 3,3-dimethylbutyl, n-pentyl, cyclopropyl, cyclopentyl, 2-propenyl, 2-butenyl, 3-butenyl, 2-propynyl, 2-butynyl, 3-butynyl, 3-oxetanyl, 3-tetrahydrofuranyl or 4-tetrahydropyranyl.
[67] The difluorobutenoic acid amide compound or a salt thereof according to any one of [3] to [63], wherein R⁵ is methyl.
[68] The difluorobutenoic acid amide compound or a salt thereof according to any one of [3] to [63], wherein Z¹ is cyano, a halogen, a (C₁-C₆)-alkylthio, a (C₁-C₆)-alkylsulfonyl, a (C₁-C₆)-alkoxy, phenyl, pyridyl, a (C₁-C₆)-alkoxycarbonyl or 1,3-dioxolan-4-yl.
[69] The difluorobutenoic acid amide compound or a salt thereof according to any one of [3] to [63], wherein Z¹ is cyano, a halogen, a (C₁-C₆)-alkylthio, a (C₁-C₆)-alkylsulfonyl, a (C₁-C₆)-alkoxy or 1,3-dioxolan-4-yl.
[70] The difluorobutenoic acid amide compound or a salt thereof according to any one of [3] to [63], wherein Z¹ is cyano, a fluorine atom, a chlorine atom, a bromine atom, methylthio, methylsulfonyl, methoxy, phenyl, 2-pyridyl, 3-pyridyl, 4-pyridyl, methoxycarbonyl or 1,3-dioxan-4-yl.
[71] The difluorobutenoic acid amide compound or a salt thereof according to any one of [3] to [60], [62] and [63] to [69], wherein R⁶ is a (C₁-C₆)-alkyl which may be substituted by at least one halogen.
[72] The difluorobutenoic acid amide compound or a salt thereof according to any one of [3] to [60], [62] and [63] to [69], wherein R⁶ is methyl, ethyl, trifluoromethyl, cyclopentyl or benzyl.
[73] The difluorobutenoic acid amide compound or a salt thereof according to any one of [3] to [62] and [64] to [72], wherein R⁷ is a (C₁-C₆)-alkyl which may be substituted by at least one Y², H or a (C₁-C₆)-alkoxy.
[74] The difluorobutenoic acid amide compound or a salt thereof according to any one of [3] to [62] and [64] to [72], wherein R⁷ is a (C₁-C₆)-alkyl which may be substituted by at least one Y², H or a (C₁-C₆)-alkoxy, and Y² is a (C₁-C₆)-alkoxy, a halogen or cyano.
[75] The difluorobutenoic acid amide compound or a salt thereof according to any one of [3] to [62] and [64] to [72], wherein R⁷ is a (C₁-C₆)-alkyl, H or a (C₁-C₆)-alkoxy.
[76] The difluorobutenoic acid amide compound or a salt thereof according to any one of [3] to [62] and [64] to [72], wherein R⁷ is H, methyl, ethyl, n-propyl, n-pentyl, 2-propenyl, 2-propynyl, 2-chloroethyl, methylsulfonyl, OH, 2-methoxyethyl, 2-methylthioethyl, cyanomethyl, methoxycarbonylmethyl, ethoxycarbonylmethyl, tert-butyloxycarbonylmethyl, 1-(methoxycarbonyl)ethyl, benzyl, methoxy, ethoxy, n-propyloxy, isopropyloxy, 2-propenyloxy, phenoxy, 3-pyridazinyl or -(CH₂)-(C=O)NH-(CH₂)-C(=O)OCH₃, and in a case where R⁷ and R⁸ together form a completely saturated 4- to 6-membered ring with N to which they are bonded, the 4- to 6-membered ring is 2-oxazolidinyl, 1,2- oxazinan-1-yl, 4-morpholino, 1-piperidinyl, 4-chloropiperidin-1-yl, 1-pyrrolidinyl or 1- azetidinyl.
[77] The difluorobutenoic acid amide compound or a salt thereof according to any one of [3] to [62] and [64] to [76], wherein R⁸ is H or a (C₁-C₆)-alkyl.
[78] The difluorobutenoic acid amide compound or a salt thereof according to any one of [3] to [62] and [64] to [76], wherein R⁸ is H, methyl, ethyl, n-propyl, isopropyl, n-butyl or methoxy.
[79] The difluorobutenoic acid amide compound or a salt thereof according to any one of [3] to [61] and [64] to [78], wherein R⁹ is methyl, ethoxycarbonylmethyl or 1-imidazolyl.
[80] The difluorobutenoic acid amide compound or a salt thereof according to any one of [3] to [61] and [64] to [79], wherein Z³ is a (C₁-C₆)-alkoxycarbonyl or -C(=O)OH.
[81] The difluorobutenoic acid amide compound or a salt thereof according to any one of [3] to [61] and [64] to [79], wherein Z³ is OH, a fluorine atom, a chlorine atom, a bromine atom, an iodine atom, cyano, methoxy, methylsulfonyloxy, ethylsulfonyloxy, n-propylsulfonyloxy, isopropylsulfonyloxy, n-butylsulfonyloxy, acetoxy, ethylcarbonyloxy, isopropylcarbonyloxy, methoxycarbonyl, ethoxycarbonyl, tert-butyloxycarbonyl or - C(=O)OH.
[82] The difluorobutenoic acid amide compound or a salt thereof according to any one of [3] to [58], [60] and [62] to [81], wherein R¹¹ is methyl or ethyl.
[83] The difluorobutenoic acid amide compound or a salt thereof according to any one of [3] to [82], wherein m is 1 or 2.
[84] The difluorobutenoic acid amide compound or a salt thereof according to any one of [3] to [82], wherein m is 1.
[85] The difluorobutenoic acid amide compound or a salt thereof according to [3], wherein R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, or -CH(OH)-C(=O)R¹¹, R⁷ is a (C₁-C₆)-alkyl which may be substituted by at least one Y², H, or a (C₁-C₆)-alkoxy, and Z³ is a (C₁-C₆)-alkoxycarbonyl or -C(=O)OH.
[86] The difluorobutenoic acid amide compound or a salt thereof according to [3], wherein R¹ and R² are each a halogen, a (C₁-C₆)-alkyl which may be substituted by at least one Y¹, a (C₁-C₆)-alkoxy which may be substituted by at least one halogen, cyano, or H, Y¹ is a halogen, R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, or -CH(OH)-C(=O)R¹¹, R⁷ is a (C₁-C₆)-alkyl which may be substituted by at least one Y², H or a (C₁-C₆)-alkoxy, and Z³ is a (C₁-C₆)-alkoxycarbonyl or -C(=O)OH.
[87] The difluorobutenoic acid amide compound or a salt thereof according to [3], wherein R¹ and R² are each a halogen, a (C₁-C₆)-alkyl which may be substituted by at least one Y¹, a (C₁-C₆)-alkoxy which may be substituted by at least one halogen, cyano or H, Y¹ is a halogen, R⁴ is -C(=O)OR⁵, -C(=O)SR⁶, -C(=O)NR⁷R⁸ or -CH(OH)-C(=O)R¹¹, R⁷ is a (C₁-C₆)-alkyl which may be substituted by at least one Y², H or a (C₁-C₆)-alkoxy, Z¹ is cyano, a halogen, a (C₁-C₆)-alkylthio, a (C₁-C₆)-alkylsulfonyl, a (C₁-C₆)-alkoxy or 1,3-dioxolan-4-yl, R⁶ is a (C₁-C₆)-alkyl which may be substituted by at least one halogen, R⁷ is a (C₁-C₆)-alkyl which may be substituted by at least one Y², H or a (C₁-C₆)-alkoxy, R³ is H or a (C₁-C₆)-alkyl, and Y² is a (C₁-C₆)-alkoxy, a halogen or cyano.
[88] The difluorobutenoic acid amide compound or a salt thereof according to any one of [85] to [87], wherein m is 1 or 2.
[89] The difluorobutenoic acid amide compound or a salt thereof according to any one of [85] to [88], wherein t is 1, and the broken line moiety of carbon-carbon bond is a single bond.
[90] The difluorobutenoic acid amide compound or a salt thereof according to any one of [85] to [88], wherein t is 2, and the broken line moiety of carbon-carbon bond is a single bond.
[91] The difluorobutenoic acid amide compound or a salt thereof according to any one of [85] to [88], wherein t is 2, and one of the broken line moieties of carbon-carbon bond is a single bond, and the other is a double bond.
[92] A herbicidal composition comprising as an active ingredient the difluorobutenoic acid amide compound or a salt thereof as defined in any one of [4] to [91].
[93] A method for controlling undesired plants or inhibiting their growth, which comprises applying an effective amount of the difluorobutenoic acid amide compound or a salt thereof as defined in any one of [4] to [92] to harmful weeds, useful plants or soil.

### EXAMPLES

Now, examples of the present invention will be described, however, the present invention is by no means restricted thereto. First, Synthesis Examples of the Invention Compounds will be described. ¹H-NMR spectrum data of the Invention Compounds were obtained in a measurement solvent by means of JEOL JNM-ECX (500 MHz) or Bruker AVANCE III HD (300 MHz) (¹H-nuclear magnetic resonance spectroscopy). The measurement solvent may sometimes contain tetramethylsilane (TMS) as an internal standard.

In this specification, the room temperature indicates about 10 to 35°C.

### Synthesis Example 1

Synthesis of methyl (1RS,4SR)-4-[(E)-2,2-difluoro-4-(3-fluorophenyl)but-3-enamide]cyclopent-2-ene-1-carboxylate (the after-described Compound No. Bb-3-1)

### (1) Synthesis of ethyl (E)-2,2-difluoro-4-(3-fluorophenyl)but-3-enoate

In a nitrogen atmosphere, to a mixed solution of 1-fluoro-3-vinylbenzene (5.12 g), ethyl 2-bromo-2,2-difluoroacetate (12.76 g), copper(I) iodide (0.08 g) and acetonitrile (90 mL), N1-[2-(dimethylamino)ethyl]-N1,N2,N2-trimethylethane-1,2-diamine (10.9 g) was added and reacted at 80°C for 14 hours to obtain a reaction mixture. The reaction mixture was cooled, water was added, and the mixture was extracted with ethyl acetate. The resulting organic layer was washed with brine and dried over anhydrous sodium sulfate, the solvent was distilled off under reduced pressure, and the obtained residue was purified by column chromatography (eluent: heptane/ethyl acetate) to obtain a yellow transparent liquid ethyl (E)-2,2-difluoro-4-(3-fluorophenyl)but-3-enoate (7.02 g, yield: 69%).

¹H NMR (500 MHz, CDCl₃): δ ppm = 7.34 (td, 1H), 7.21 (d, 1H), 7.15 (dt, 1H), 7.08-7.00 (m, 2H), 6.30 (dt, 1H), 4.35 (q, 2H), 1.36 (t, 3H).

### (2) Synthesis of (E)-2,2-difluoro-4-(3-fluorophenyl)but-3-enoic acid

A mixed solution of ethyl (E)-2,2-difluoro-4-(3-fluorophenyl)but-3-enoate (7.02 g), tetrahydrofuran (40 mL) and water (40 mL) was cooled with water, and lithium hydroxide (4.13 g) was added. The mixed solution was reacted at room temperature for 16.5 hours to obtain a reaction mixture. Tetrahydrofuran was distilled off under reduced pressure from the reaction mixture to obtain a mixture. The obtained mixture was acidified with water and 1M hydrochloric acid in this order and extracted with ethyl acetate. The resulting organic layer was washed with brine and dried over anhydrous sodium sulfate, and the solvent was distilled off under reduced pressure. The obtained residue was left to stand at room temperature for 20 hours, whereby crystals formed, which were collected by filtration and washed with washed with n-heptane. Further, the solvent was distilled off under reduced pressure from the filtrate, and the obtained crystals were washed with n-heptane. The obtained crystals and the crystals obtained above were put together to obtain a white solid (E)-2,2-difluoro-4-(3-fluorophenyl)but-3-enoic acid (4.85 g, yield: 78%).

¹H NMR (500 MHz, CDCl₃): δ ppm =7.46 (br, 1H), 7.35 (td, 1H), 7.22 (d, 1H), 7.15 (dt, 1H), 7.12-7.00 (m, 2H), 6.31 (dt, 1H).

### (3) Synthesis of methyl (1RS,4SR)-4-[(E)-2,2-difluoro-4-(3-fluorophenyl)but-3-enamide]cyclopent-2-ene-1-carboxylate (the after-described Compound No. Bb-3-1)

To a mixed solution of (E)-2,2-difluoro-4-(3-fluorophenyl)but-3-enoic acid (1.5 g), methyl (1RS,4SR)-4-aminocyclopent-2-ene-1-carboxylate hydrochloride (1.36 g), 1-[bis(dimethylamino)methylene]-1H-1,2,3-triazolo[4,5-b]pyridinium 3-oxide hexafluorophosphate (3.17 g) and N,N-dimethylformamide (15 mL), N,N-diisopropylethylamine (2.69 g) was added and reacted at room temperature for 15 hours to obtain a mixed solution. Water was added to the mixed solution, and the mixture was extracted with ethyl acetate. The resulting organic layer was washed with brine, and the solvent was distilled off under reduced pressure. The obtained residue was purified by column chromatography (eluent: heptane/ethyl acetate) to obtain a pale yellow solid desired product (2.16 g, yield: 92%).

¹H NMR (300 MHz, CDCl₃): 7.32 (dt, 1H), 7.22 (d, 1H), 7.16-7.14 (m, 2H), 7.06-7.01 (m, 2H), 6.37 (dt, 1H), 5.99-5.97 (m, 1H), 5.95-5.93 (m, 1H), 5.09 (t, 1H), 3.73 (s, 3H), 3.55 (d, 1H), 2.45 (dt, 1H), 1.98 (dt, 1H).

### Synthesis Example 2

### Synthesis of methyl cis-3-[(E)-2,2-difluoro-4-(3-fluorophenyl)but-3-enamide]cyclobuane-1-carboxylate (the after-described Compound No. Aa-3-1)

To a mixed solution of (E)-2,2-difluoro-4-(3-fluorophenyl)but-3-enoic acid (0.1 g), methyl cis-3-aminocyclobutane-1-carboxylate hydrochloride (0.1 g), 1-[bis(dimethylamino)methylene]-1H-1,2,3-triazolo[4,5-b]pyridinium 3-oxide hexafluorophosphate (0.21 g) and N,N-dimethylformamide (2 mL), N,N-diisopropylethylamine (0.24 g) was added and reacted at room temperature for 62.5 hours to obtain a mixed solution. Ethyl acetate was added to the mixed solution, the resulting organic layer was washed with brine, and the solvent was distilled off under reduced pressure. The obtained residue was purified by column chromatography (eluent: heptane/ethyl acetate) to obtain a white solid desired product (0.04 g, yield: 26%).

¹H NMR (300 MHz, CDCl₃): 7.37-7.30 (m, 1H), 7.22 (d, 1H), 7.15 (d, 1H), 7.08-6.99 (m, 2H), 6.68 (br, 1H), 6.35 (dt, 1H), 4.45 (sext, 1H), 3.71 (s, 3H), 2.90 (quin, 1H), 2.77-2.65 (m, 2H), 2.29-2.17 (m, 2H).

### Synthesis Example 3

### Synthesis of cis-3-[(E)-2,2-difluoro-4-(3,5-difluorophenyl)but-3-enamide]-N-methoxy-N-methylcyclobutane-1-carboxylic acid amide (the after-described Compound No. Aa-50-1)

### (1) Synthesis of cis-3-[(E)-2,2-difluoro-4-(3,5-difluorophenyl)but-3-enamide]cyclobutane-1-carboxylic acid

A mixed solution of methyl cis-3-[(E)-2,2-difluoro-4-(3,5-difluorophenyl)but-3-enamide]cyclobutane-1-carboxylate (0.40 g) and tetrahydrofuran (5 mL) was cooled with ice. Under cooling with ice bath, an aqueous solution (5 mL) of lithium hydroxide (0.07 g) was added to the mixed solution, and reacted under cooling with ice bath for 1 hour to obtain a reaction mixture. The reaction mixture was acidified with 1M hydrochloric acid, water was added, and tetrahydrofuran in the reaction mixture was distilled off under reduced pressure. The obtained residue was filtrated to obtain crystals, which were dissolved in ethyl acetate, sodium sulfate was added, and the mixture was left at rest. Sodium sulfate was removed by filtration, and the filtrate was distilled under reduced pressure to obtain a white solid cis-3-[(E)-2,2-difluoro-4-(3,5-difluorophenyl)but-3-enamide]cyclobutane-1-carboxylic acid (0.35 g, yield: 91%).

¹H NMR (300 MHz, acetone-d₆): 10.64 (br, 0.44H), 8.30 (br, 1H), 7.42-7.28 (m, 2H), 7.18-7.00 (m, 2H), 6.69 (dt, 1H), 4.40 (sext, 1H), 2.99-2.85 (m, 1H), 2.65-2.52 (m, 2H), 2.42-2.25 (m, 2H). The proton peak of the carboxy group was detected at 10.64 ppm.

### (2) Synthesis of cis-3-[(E)-2,2-difluoro-4-(3,5-difluorophenyl)but-3-enamide]-N-methoxy-N-methylcyclobutane-1-carboxylic acid amide (the after-described Compound No. Aa-50-1)

To a mixed solution of cis-3-[(E)-2,2-difluoro-4-(3,5-difluorophenyl)but-3-enamide]cyclobutane-1-carboxylic acid (0.15 g), N,O-dimethylhydroxyamine hydrochloride (0.05 g), triethylamine (0.11 g) and dichloromethane (5 mL), a 1.7M ethyl acetate solution (0.36 mL) of propylphosphonic anhydride was added and reacted at room temperature for 1.5 hours to obtain a mixed solution. Water was added to the mixed solution and the mixture was extracted with ethyl acetate. The resulting organic layer was washed with brine, and the solvent was distilled off under reduced pressure. The obtained residue was purified by column chromatography (eluent: heptane/ethyl acetate) to obtain a white solid desired product (0.16 g, yield: 92%).

¹H NMR (300 MHz, CDCl₃): 7.16-6.88 (m, 4H), 6.78 (tt, 1H), 6.36 (dt, 1H), 4.47 (sext, 1H), 3.66 (s, 3H), 3.31 (t, 1H), 3.20 (s, 3H), 2.72-2.58 (m, 2H), 2.32-2.17 (m, 2H).

### Synthesis Example 4

### Synthesis of ethyl 2-[3-{(E)-2,2-difluoro-4-(3,5-difluorophenyl)but-3-enamide}cyclobutylidene] acetate (the after-described Compound No. Aa-59)

### (1) Synthesis of (E)-[2,2-difluoro-4-(3,5-difluorophenyl)]-N-(3-oxocyclobutyl)but-3-enamide

To a mixed solution of (E)-2,2-difluoro-4-(3,5-difluorophenyl)but-3-enoic acid (0.4 g), N,N-dimethylformamide (3 drops added by a Pasteur Pipette) and tetrahydrofuran (4 mL), oxalyl chloride (0.26 g) was added and reacted at room temperature for 30 minutes to obtain reaction liquid A. To a mixed solution of 3-aminocyclobutan-1-one hydrochloride (0.23 g) and tetrahydrofuran (6 mL) in another reactor, triethylamine (0.52 g) was added and cooled with ice bath to obtain reaction liquid B. The reaction liquid A was added to the reaction liquid B under cooling with ice bath, and reacted at room temperature for 1 hour to obtain a mixed solution. Water was added to the mixed solution, and the mixture was extracted with ethyl acetate. The resulting organic layer was washed with brine, the solvent was distilled off under reduced pressure. The obtained residue was purified by column chromatography (eluent: heptane/ethyl acetate) to obtain a white solid (E)-[2,2-difluoro-4-(3,5-difluorophenyl)]-N-(3-oxocyclobutyl)but-3-enamide (0.46 g, yield: 90%).

¹H NMR (300 MHz, CDCl₃): δ ppm = 7.05-6.93 (m, 3H), 6.81 (tt, 1H), 6.77 (br, 1H), 6.38 (dt, 1H), 4.61-4.49 (m, 1H), 3.61-3.48 (m, 2H), 3.25-3.13 (m, 2H).

### (2) Synthesis of ethyl 2-[3-{(E)-2,2-difluoro-4-(3,5-difluorophenyl)but-3-enamide}cyclobutylidene] acetate (the after-described Compound No. Aa-59)

A mixed solution of ethyl 2-diethoxyphosphinoyl acetate (0.16 g) and tetrahydrofuran (5 mL) was cooled with ice bath. Sodium hydride (0.03 g, purity: 60%) was added to the mixed solution under cooing with ice bath, followed by stirring under cooling with ice bath for 30 minutes. Under cooling with ice bath, (E)-[2,2-difluoro-4-(3,5-difluorophenyl)]-N-(3-oxocyclobutyl)but-3-enamide (0.20 g) was added, and reacted at room temperature 1 hour to obtain a mixed solution. Ice water was added to the mixed solution, and the mixture was extracted with ethyl acetate. The resulting organic layer was washed with brine, and the solvent was distilled off under reduced pressure. The obtained residue was purified by column chromatography (eluent: heptane/ethyl acetate) to obtain a white solid desired product (0.21 g, yield: 85%).

¹H NMR (300 MHz, CDCl₃): 7.07-6.91 (m, 3H), 6.80 (tt, 1H), 6.63 (br, 1H), 6.37 (dt, 1H), 5.74 (quin, 1H), 4.54 (sext, 1H), 4.16 (q, 2H), 3.75-3.61 (m, 1H), 3.38-3.25 (m, 1H), 3.13-3.01 (m, 1H), 2.92-2.80 (m, 1H), 1.28 (s, 3H).

Compounds listed below can be synthesized in accordance with the above Production Method, the above Synthesis Example, and a method known in this technical field.

In Table 1, No. represents No. of combination of substituents. In Table 1, abbreviations used in the columns R¹, R², R³ and (R⁴)ₘ represent the following substituents and number of substituents. Me is a methyl group, Et is an ethyl group, CN is a cyano group, n-Pr is a n-propyl group, i-Pr is an isopropyl group, c-Pr is a cyclopropyl group, n-Bu is a n-butyl group, i-Bu is an isobutyl group, t-Bu is a tert-butyl group, n-Pent is a n-pentyl group, c-Pent is a cyclopentyl group, Ph is a phenyl group, 2-Py is 2-pyridyl, 3-Py is 3-pyridyl, 4-Py is 4-pyridyl, di is two, tri is three, and tetra is four. In columns in Table 1, "-" is a single bond, "=" is a double bond, and "≡" is a triple bond. Further, the number attached to the chemical structure in the column (R⁴)ₘ in Table 1 represents the position to which R⁴ is bonded, and # represents the bonding position.

The position of the substituent indicated in the column (R⁴)ₘ in Table 1 represents the position of the substituent of the chemical structure as shown in Table 1. For example, in the case of the compound of the formula (IAa), the compound indicated with "3-CO₂Me" in the column (R⁴)ₘ, is a compound in which the cyclobutane is substituted by one R⁴ group at the substitution position given in the following chemical structure, that is a compound of "m=1" in which only the 3-position on cyclobutane is substituted with CO₂Me.

The Invention Compounds Aa-1 to Aa-287 are compounds of the formula (IAa): 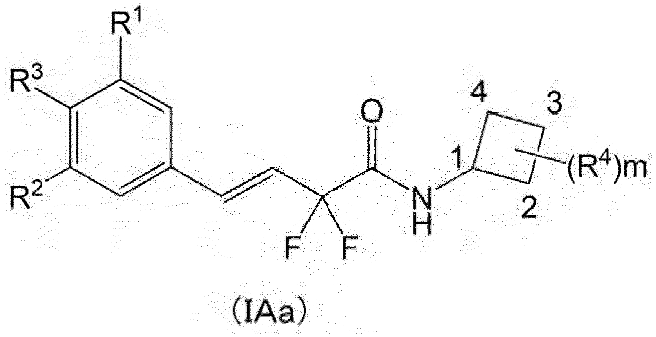 wherein the combinations of R¹, R², R³ and (R⁴)ₘ are combinations (No.1 to 287) as identified in Table 1. For example, the Invention Compound Aa-1 is a compound of the formula (IAa), wherein the combination of R¹, R², R³ and (R⁴)ₘ is a combination (R¹=F, R²=F, R³=H, a (R⁴)m=3-CO₂Me) represented by No.1 in Table 1, that is a compound of the following structure.

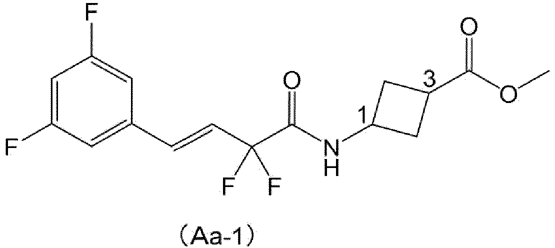

**Table 1**

| No. | R¹ | R² | R³ | (R⁴)ₘ |
|---|---|---|---|---|
| 1 | F | F | H | 3-CO₂Me |
| 2 | F | F | F | 3-CO₂Me |
| 3 | F | H | H | 3-CO₂Me |
| 4 | F | CF₃ | H | 3-CO₂Me |
| 5 | F | CN | H | 3-CO₂Me |
| 6 | F | I | H | 3-CO₂Me |
| 7 | F | Cl | H | 3-CO₂Me |
| 8 | F | Br | H | 3-CO₂Me |
| 9 | F | Me | H | 3-CO₂Me |
| 10 | F | OCF₃ | H | 3-CO₂Me |
| 11 | Cl | Cl | H | 3-CO₂Me |
| 12 | F | OMe | H | 3-CO₂Me |
| 13 | Cl | OMe | H | 3-CO₂Me |
| 14 | Cl | Me | H | 3-CO₂Me |
| 15 | H | H | F | 3-CO₂Me |
| 16 | F | Me | F | 3-CO₂Me |
| 17 | F | i-Pr | H | 3-CO₂Me |
| 18 | F | Et | H | 3-CO₂Me |
| 19 | Cl | H | H | 3-CO₂Me |
| 20 | Br | H | H | 3-CO₂Me |
| 21 | CF₃ | H | H | 3-CO₂Me |
| 22 | Me | H | H | 3-CO₂Me |
| 23 | OMe | H | H | 3-CO₂Me |
| 24 | F | OEt | H | 3-CO₂Me |
| 25 | Me | H | F | 3-CO₂Me |
| 26 | OMe | OMe | H | 3-CO₂Me |
| 27 | Me | Me | H | 3-CO₂Me |
| 28 | Me | Me | F | 3-CO₂Me |
| 29 | F | OCHF₂ | H | 3-CO₂Me |
| 30 | CF₃ | Me | H | 3-CO₂Me |
| 31 | OCF₃ | Me | H | 3-CO₂Me |
| 32 | CN | Me | H | 3-CO₂Me |
| 33 | OMe | Me | H | 3-CO₂Me |
| 34 | F | CHF₂ | H | 3-CO₂Me |
| 35 | F | CH=CH₂ | H | 3-CO₂Me |
| 36 | F | C≡CH | H | 3-CO₂Me |
| 37 | F | Me | H | 3,3-di(CO₂Me) |
| 38 | F | CH₂F | H | 3-CO₂Me |
| 39 | Et | H | H | 3-CO₂Me |
| 40 | i-Pr | H | H | 3-CO₂Me |
| 41 | OEt | H | H | 3-CO₂Me |
| 42 | Oi-Pr | H | H | 3-CO₂Me |
| 43 | F | n-Pr | H | 3-CO₂Me |
| 44 | F | F | H | 3-CH₂OH |
| 45 | F | F | H | 3-CH₂OSO₂Me |
| 46 | F | H | H | 3-CO₂CH₂C≡CH |
| 47 | F | F | H | 3-CN |
| 48 | F | F | H | 3-CO₂H |
| 49 | F | F | H | 3-CONHOMe |
| 50 | F | F | H | 3-CON(Me)OMe |
| 51 | F | F | H | 3-CO₂t-Bu |
| 52 | F | F | H | 3-CHF₂ |
| 53 | F | F | H | 3-CH₂OCOMe |
| 54 | F | F | H | 3-C(=O)H |
| 55 | F | F | H | 3-C(=O)Me |
| 56 | F | F | H | 3-COCH₂CO₂Et |
| 57 | F | F | H | 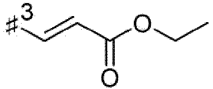 |
| 58 | F | F | H | 3-OH |
| 59 | F | F | H | 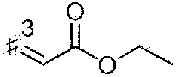 |
| 60 | F | F | H | 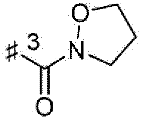 |
| 61 | F | F | H | 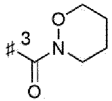 |
| 62 | F | F | H | 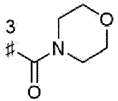 |
| 63 | F | F | H | 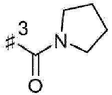 |
| 64 | F | F | H | 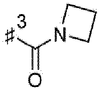 |
| 65 | F | F | H | 3-C(=O)N(Et)₂ |
| 66 | F | F | H | 3-C(=O)NH(CH₂CH₂Cl) |
| 67 | F | F | H | 3-C(=O)N(Et)OMe |
| 68 | F | F | H | 3-C(=O)N(n-Pr)OMe |
| 69 | F | F | H | 3-C(=O)N(i-Pr)OMe |
| 70 | F | F | H | 3-C(=O)N(n-Bu)OMe |
| 71 | F | F | H | 3-C(=O)SEt |
| 72 | F | F | H | 3-CO₂CH₂C≡CH |
| 73 | F | F | H | 3-CO₂CH₂CH₂CN |
| 74 | F | F | H | 3-CO₂CH₂CHF₂ |
| 75 | F | F | H | 3-CO₂CH₂CF₃ |
| 76 | F | F | H | 3-C(=O)N(Me)₂ |
| 77 | F | F | H | 3-CO₂CH₂CH₂SMe |
| 78 | F | F | H | 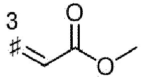 |
| 79 | F | H | H | 3-CO₂CH₂CF₃ |
| 80 | F | F | H | 3-C(=O)NH(n-Pr) |
| 81 | F | F | H | 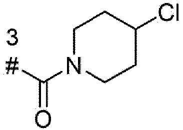 |
| 82 | F | Me | H | 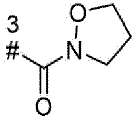 |
| 83 | F | Me | H | 3-CO₂CH₂C≡CH |
| 84 | F | Me | H | 3-CO₂CH₂CH₂CN |
| 85 | F | Me | H | 3-CO₂H |
| 86 | F | Me | H | 3-CONHMe |
| 87 | F | Me | H | 3-COSEt |
| 88 | F | Me | H | 3-CONHCH₂CH₂Cl |
| 89 | F | Me | H | 3-CO₂Et |
| 90 | F | Me | H | 3-CO₂n-Pr |
| 91 | F | Me | H | 3-CO₂CH₂CH=CH₂ |
| 92 | F | Me | H | 3-CO₂c-Pent |
| 93 | F | Me | H | 3-CO₂n-Pent |
| 94 | F | Me | H | 3-C(=O)NH(Et) |
| 95 | F | Me | H | 3-C(=O)NH(n-Pr) |
| 96 | F | Me | H | 3-C(=O)NH(i-Pr) |
| 97 | F | Me | H | 3-C(=O)NH(CH₂CH=CH₂) |
| 98 | F | Me | H | 3-C(=O)NH(n-Pent) |
| 99 | F | Me | H | 3-C(=O)NH(OMe) |
| 100 | F | Me | H | 3-C(=O)NMe(C(=O)Me) |
| 101 | F | Me | H | 3-CO₂c-Pr |
| 102 | F | F | H | 3-CH₂CO₂Me |
| 103 | F | Me | H | 3-COSMe |
| 104 | F | Me | H | 3-CO₂n-Bu |
| 105 | F | Me | H | 3-CO₂CH₂CH=CHMe |
| 106 | F | Me | H | 3-CO₂CH₂CH₂t-Bu |
| 107 | F | Me | H | 3-CO₂CH₂CH₂CH=CH₂ |
| 108 | F | Me | H | 3-CO₂CH₂CH₂C≡CH |
| 109 | F | Me | H | 3-CO₂CH₂CF₂CF₂CF₃ |
| 110 | F | Me | H | 3-CO₂CH₂C≡CMe |
| 111 | F | Me | H | 3-CO₂CH₂CH₂SMe |
| 112 | F | Me | H | 3-CO₂CH₂CH₂SO₂Me |
| 113 | F | OMe | H | 3-CO₂H |
| 114 | F | H | H | 3-CO₂H |
| 115 | F | C≡CH | H | 3-CO₂Et |
| 116 | F | F | H | 3-SMe |
| 117 | F | F | H | 3-SOMe |
| 118 | F | F | H | 3-SO₂Me |
| 119 | F | F | H | 3-CH₂CN |
| 120 | F | F | H | 3-CH₂O(C=O)Et |
| 121 | F | F | H | 3-CH₂O(C=O)i-Pr |
| 122 | F | F | H | 3-CH₂O(C=O)c-Pr |
| 123 | F | H | H | 3-CO₂c-Pent |
| 124 | F | F | H | 3-C(=O)N(Et)Me |
| 125 | F | F | H | 3-C(=O)N(CH₂CH=CH₂)Me |
| 126 | F | H | H | 3-C(=O)NH₂ |
| 127 | F | F | H | 3-CO₂CH₂CN |
| 128 | F | F | H | 3-CH₂Cl |
| 129 | F | F | H | 3-C(=O)NMe₂ |
| 130 | Br | Cl | H | 3-CO₂Me |
| 131 | H | F | F | 3-CO₂Me |
| 132 | Cl | Me | H | 3-CO₂Me |
| 133 | F | F | H | 3-CO₂Et |
| 134 | F | F | H | 3-CO₂n-Pr |
| 135 | F | F | H | 3-CO₂i-Pr |
| 136 | F | F | H | 3-CO₂c-Pent |
| 137 | F | F | H | 3-C(=O)NHOEt |
| 138 | F | F | H | 3-C(=O)NHOi-Pr |
| 139 | F | F | H | 3-C(=O)NHOn-Pr |
| 140 | F | F | H | 3-C(=O)NHCH₂CN |
| 141 | F | F | H | 3-NHCO₂Me |
| 142 | F | F | H | 3-CH₂OMe |
| 143 | F | F | H | 3-CO₂CH₂CH₂SO₂Me |
| 144 | F | F | H | 3-CH₂OSO₂n-Pr |
| 145 | F | F | H | 3-CH₂OSO₂i-Bu |
| 146 | F | F | H | 3-CH₂OSO₂i-Pr |
| 147 | F | F | H | 3-CH₂CH₂O(C=O)Me |
| 148 | F | F | H | 3-CH₂CH₂OSO₂Me |
| 149 | F | F | H | 3-CH₂CH₂OH |
| 150 | F | F | H | 3-CH₂Br |
| 151 | F | F | H | 3-CH₂I |
| 152 | F | F | H | 3-CH₂OSO₂Et |
| 153 | F | F | H | 3-CH(OH)Me |
| 154 | F | F | H | 3-CH(OSO₂Me)Me |
| 155 | F | F | H | 3-C(Me)₂OH |
| 156 | F | F | H | 3-Me, 3-CO₂Me |
| 157 | F | F | H | 3-Me, 3-CH₂OSO₂Me |
| 158 | F | F | H | 3-CH₂F |
| 159 | F | F | H | 4-CO₂Me |
| 160 | F | F | H | 4-CH₂O(C=O)Me |
| 161 | F | F | H | 4-CH₂OH |
| 162 | F | F | H | 3-C(=O)N(Me)CH₂CH=CH₂ |
| 163 | F | H | H | 3-CO₂Et |
| 164 | F | H | H | 3-CO₂n-Pr |
| 165 | F | H | H | 3-CO₂i-Pr |
| 166 | F | F | H | 3-CO₂CH₂CH=CH₂ |
| 167 | F | F | H | 3-CO₂CH₂CH₂Cl |
| 168 | F | F | H | 3-CO₂CH₂CH₂Br |
| 169 | F | F | H | 3-CO₂CH₂CO₂Me |
| 170 | F | F | H | 3-CO₂CH(Me)CO₂Me |
| 171 | F | F | H | 3-CO₂CH₂Ph |
| 172 | F | F | H | 3-CO₂CH₂-(2-Py) |
| 173 | F | F | H | 3-CO₂CH₂-(3-Py) |
| 174 | F | F | H | 3-CO₂CH₂-(4-Py) |
| 175 | F | F | H | 3-CO₂c-Pr |
| 176 | F | F | H | 3-C(=O)NH(CH₂CH=CH₂) |
| 177 | F | F | H | 3-C(=O)NH(CH₂C≡CH) |
| 178 | F | F | H | 3-C(=O)NH(CH₂CH₂OMe) |
| 179 | F | F | H | 3-C(=O)NH(CH₂CH₂SMe) |
| 180 | F | F | H | 3-C(=O)NH(CH₂CN) |
| 181 | F | F | H | 3-C(=O)NH(CH₂CO₂Me) |
| 182 | F | F | H | 3-C(=O)NH(CH(Me)CO₂Me) |
| 183 | F | F | H | 3-C(=O)NH(CH₂Ph) |
| 184 | F | F | H | 3-C(=O)NH(OCH₂CH=CH₂) |
| 185 | F | F | H | 3-C(=O)NH(OPh) |
| 186 | F | OMe | H | 3-C(=O)NH(OMe) |
| 187 | F | OMe | H | 3-C(=O)NH(CH₂CH=CH₂) |
| 188 | F | OMe | H | 3-C(=O)NH(n-Pr) |
| 189 | F | OMe | H | 3-C(=O)NMe(OMe) |
| 190 | F | OMe | H | 3-CO₂Et |
| 191 | F | OMe | H | 3-CO₂n-Pr |
| 192 | F | OMe | H | 3-CO₂i-Pr |
| 193 | F | OMe | H | 3-CO₂c-Pr |
| 194 | F | OMe | H | 3-CO₂CH₂CH=CH₂ |
| 195 | F | OMe | H | 3-CO₂CH₂C≡CH |
| 196 | F | OMe | H | 3-CO₂CH₂CF₃ |
| 197 | F | OMe | H | 3-CO₂CH₂CHF₂ |
| 198 | F | OMe | H | 3-CO₂CH₂CH₂OMe |
| 199 | F | OMe | H | 3-CO₂CH₂CH₂SMe |
| 200 | F | OMe | H | 3-CO₂CH₂CH₂CN |
| 201 | F | OMe | H | 3-CO₂CH₂Ph |
| 202 | F | OMe | H | 3-CO₂CH₂-(2-Py) |
| 203 | F | OMe | H | 3-CO₂CH₂-(3-Py) |
| 204 | F | OMe | H | 3-CO₂CH₂-(4-Py) |
| 205 | F | OMe | H | 3-CO₂CH₂CH₂Cl |
| 206 | F | OMe | H | 3-CO₂CH₂CH₂Br |
| 207 | F | OMe | H | 3-CO₂CH₂CO₂Me |
| 208 | F | OMe | H | 3-CO₂CH(Me)CO₂Me |
| 209 | F | Me | H | 3-CO₂CH₂c-Pr |
| 210 | F | Me | H | 3-C(=O)NH(SO₂Me) |
| 211 | F | Me | H | 3-C(=O)NMe(SO₂Me) |
| 212 | F | Me | H | 3-CO₂CH₂-(4-Py) |
| 213 | F | Me | H | 3-C(=O)NH(OH) |
| 214 | F | Me | H | 3-CO₂CH₂CH₂OSi(Me)₂t-Bu |
| 215 | F | Me | H | 3-CO₂CH₂CH₂OH |
| 216 | F | Me | H | 3-CO₂CH₂CH₂NH₂ |
| 217 | F | F | H | 3-C(=O)SCF₃ |
| 218 | F | F | H | 3-C(=O)NHCH₂CO₂t-Bu |
| 219 | F | Me | H | 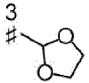 |
| 220 | F | Me | H | 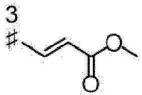 |
| 221 | F | Me | H | 3-C(=O)H |
| 222 | F | Me | H | 3-CH(OMe)₂ |
| 223 | F | F | H | 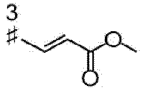 |
| 224 | F | F | H | 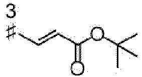 |
| 225 | F | F | H | 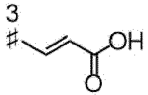 |
| 226 | F | F | H | 3-CH₂CH₂CO₂Me |
| 227 | F | F | H | 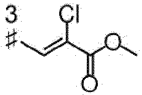 |
| 228 | F | Me | H | 3-CH₂CH₂CO₂Me |
| 229 | F | Me | H | 3-CH₂CH₂CO₂H |
| 230 | F | F | H | 3-C≡CCO₂Me |
| 231 | F | F | H | 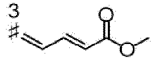 |
| 232 | F | F | H | 3-CH₂CH₂CH₂CO₂Me |
| 233 | F | F | H | 3-C(=O)CO₂Me |
| 234 | F | F | H | 3-C(=N-OMe)CO₂Me |
| 235 | F | F | H | 3-CH(OH)CO₂Me |
| 236 | F | OCF₃ | H | 3-C(=O)CO₂Me |
| 237 | F | F | H | 3-CH₂CH(Me)CO₂Et |
| 238 | F | Me | H | 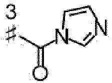 |
| 239 | F | Me | H | 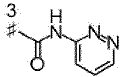 |
| 240 | F | F | H | 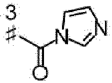 |
| 241 | F | F | H | 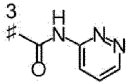 |
| 242 | F | Me | H | 3-C(=O)SCH₂Ph |
| 243 | F | Me | H | 3-C(=O)S(c-Pent) |
| 244 | F | F | H | 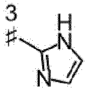 |
| 245 | CH₂CF₃ | H | H | 3-CO₂Me |
| 246 | CF₂CH₃ | H | H | 3-CO₂Me |
| 247 | F | F | H | 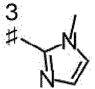 |
| 248 | F | F | H | 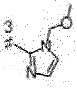 |
| 249 | F | F | H | 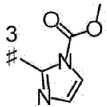 |
| 250 | F | F | H | 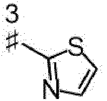 |
| 251 | F | F | H | 3-C(=O)NMe(CH₂CO₂Et) |
| 252 | F | F | H | 3-C(=O)NOMe(CH₂CO₂Et) |
| 253 | F | F | H | 3-C(=O)NH(CH₂C(=O)NH(CH₂CO₂Me)) |
| 254 | F | F | H | 3-C(=O)NOMe(CH₂C=CH) |
| 255 | OMe | H | F | 3-CO₂Me |
| 256 | CN | H | F | 3-CO₂Me |
| 257 | F | OMe | F | 3-CO₂Me |
| 258 | CF₃ | H | F | 3-CO₂Me |
| 259 | CN | CN | H | 3-CO₂Me |
| 260 | F | F | H | 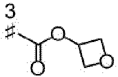 |
| 261 | F | Me | H | 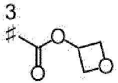 |
| 262 | Cl | CN | H | 3-CO₂Me |
| 263 | CHF₂ | Me | H | 3-CO₂Me |
| 264 | OCHF₂ | Me | H | 3-CO₂Me |
| 265 | CF₃ | CN | H | 3-CO₂Me |
| 266 | CH₂CF₃ | F | H | 3-CO₂Me |
| 267 | CHF₂ | CN | H | 3-CO₂Me |
| 268 | F | CH=CF₂ | H | 3-CO₂Me |
| 269 | CH=CF₂ | H | H | 3-CO₂Me |
| 270 | CH₂CF₃ | H | H | 3-CO₂H |
| 271 | CH₂CHF₂ | H | H | 3-CO₂Me |
| 272 | CH₂CHF₂ | F | H | 3-CO₂Me |
| 273 | CH₂CHF₂ | Cl | H | 3-CO₂Me |
| 274 | F | CH₂OMe | H | 3-CO₂Me |
| 275 | CH₂OMe | H | H | 3-CO₂Me |
| 276 | CH₂CN | H | H | 3-CO₂Me |
| 277 | CH₂CHF₂ | H | F | 3-CO₂Me |
| 278 | F | F | H | 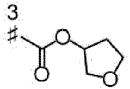 |
| 279 | F | F | H | 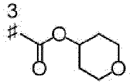 |

**Table 1 (continued)**

| No. | R¹ | R² | R³ | (R⁴)ₘ |
|---|---|---|---|---|
| 280 | F | F | H | 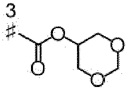 |
| 281 | F | F | H | 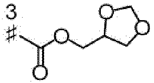 |
| 282 | CH₂CHF₂ | H | H | 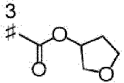 |
| 283 | CH₂CHF₂ | H | H | 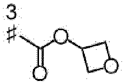 |
| 284 | CH₂CHF₂ | H | H | 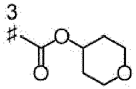 |
| 285 | CH₂CHF₂ | H | H | 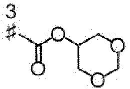 |
| 286 | CH₂CHF₂ | H | H | 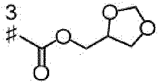 |
| 287 | OCH₂CHF₂ | H | H | 3-CO₂Me |

The Invention Compounds Ab-1 to Ab-36, Ab-38 to Ab-58, Ab-60 to Ab-77, Ab-79 to Ab-155, Ab-158 to Ab-230 and Ab-232 to Ab-287 are compounds of the formula (IAb):

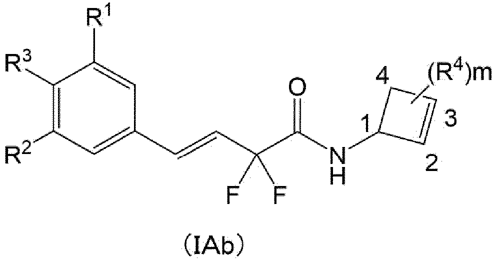

wherein the combinations of R¹, R², R³ and (R⁴)ₘ are combinations (No. 1 to 36, No. 38 to 58, No. 60 to 77, No. 79 to 155, No. 158 to 230 and No. 232 to No. 287) as identified in Table 1.

The Invention Compounds Ba-1 to Ba-287 are compounds of the formula (IBa): 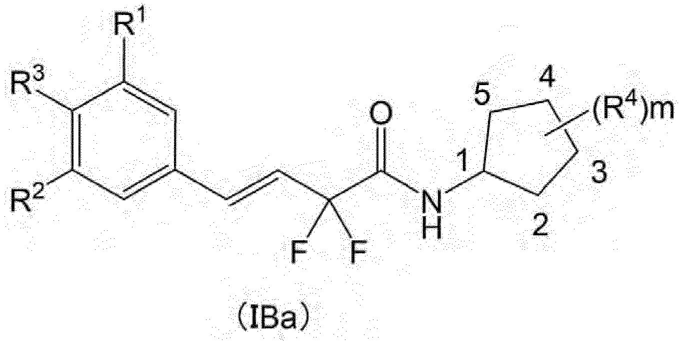 wherein the combinations of R¹, R², R³ and (R⁴)ₘ are combinations (No. 1 to 287) as identified in Table 1.

The Invention Compounds Bb-1 to Ba-287 are compounds of the formula (IBb): 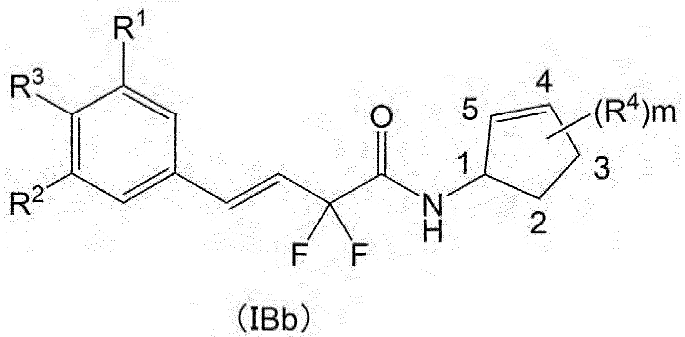 wherein the combinations of R¹, R², R³ and (R⁴)ₘ are combinations (No. 1 to 287) as identified in Table 1.

The Invention Compounds Bc-1 to Bc-36, Bc-38 to Bc-58, Bc-60 to Bc-77, Bc-79 to Bc-155, Bc-158 to Bc-230 and Bc-232 to Bc-287 are compounds of the formula (IBc): 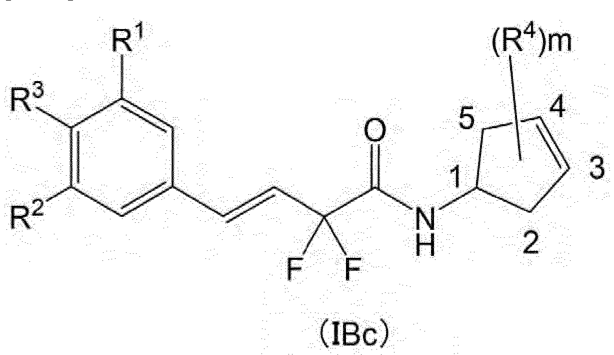 wherein the combinations of R¹, R², R³ and (R⁴)ₘ are combinations (No. 1 to 36, No. 38 to 58, No. 60 to 77, No. 79 to 155, No. 158 to 230 and No. 232 to No. 287) as identified in Table 1.

¹H-NMR spectrum data of the above-described Invention Compounds are shown in Table 2.

In Table 2, Comp No. represents the numbers of the compounds. In the column Remarks, the steric configuration of the difluorobutenoic acid amide group and R⁴ substituting the C₄-C₅ alicyclic hydrocarbon in the Invention Compound is described. The difluorobutenoic acid amide group means a moiety represented by the following chemical structure in the Invention Compound (the symbols in the chemical structure are as defined above).

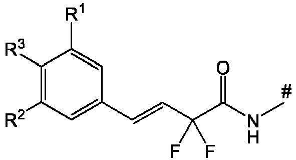

For example, "cis" in the column Remarks in Table 2 means that the difluorobutenoic acid amide group and R⁴ are in cis-configuration. "trans" means that the difluorobutenoic acid amide group and R⁴ are in trans-configuration. "cis-trans" means a mixture of a compound in which the difluorobutenoic acid amide group and R⁴ are in cis-configuration and a compound in which the difluorobutenoic acid amide group and R⁴ are in trans-configuration. "Optically active (1R,4S)" means an optically active substance in which the difluorobutenoic acid amide group is in R-configuration and R⁴ is in S-configuration. "Optically active (1S,4R)" means an optically active substance in which the difluorobutenoic acid amide group is in S-configuration and R⁴ is in R-configuration. A case where the Invention Compound has two identical R⁴ at the same substitution position of the C₄-C₅ alicyclic hydrocarbon, and a case where R⁴ is =CHC(=O)R¹¹ or =CHCH=CHC(=O)R¹¹, are represented by "-" in the column Remarks.

The compound (IAb) which has R⁴ at the 2-position or at the 3-position or at both on cyclobutenyl, is represented by "-" in the column Remarks. However, the compound (IAb) in which the difluorobutenoic acid amide group is in R-configuration, is represented as R-form in the column Remarks, and the compound in which the difluorobutenoic acid amide group is in S-configuration is represented as S-form in the column Remarks.

The compound (IBb) in which the difluorobutenoic acid amide group is in R-configuration is represented as R-form in the column Remarks, and the compound in which the difluorobutenoic acid amide group is in S-configuration is represented as S-form in the column Remarks.

The compound (IBc) which has R⁴ at the 3-position, at the 4-position or at both on cyclopentenyl, is represented by "-" in the column Remarks. The compound (IBc) in which the difluorobutenoic acid amide group is in R-configuration is represented as R-form in the column Remarks, and the compound in which the difluorobutenoic acid amide group is in S-configuration is represented as S-form in the column Remarks.

In Table 2, Comp No. is represented by the above No. of the Invention Compound (for example Aa-1) with branch numbers 1 to 5 to indicate the above steric configuration. The compound with Comp No. and a branch number 1, for example "Aa-1-1", is the Invention Compound Aa-1 in which the difluorobutenoic acid amide group and R⁴ are in cis-configuration.

The compound with Comp No. and a branch number 2, for example "Aa-1-2", is the Invention Compound Aa-1 in which the difluorobutenoic acid amide group and R⁴ are in trans-configuration.

The compound with Comp No. and a branch number 3, for example "Aa-58-3", is the Invention Compound Aa-58 as a mixture of a compound in which the difluorobutenoic acid amide group and R⁴ are in cis-configuration and a compound in which the difluorobutenoic acid amide group and R⁴ are in trans-configuration.

The compound with Comp No. and a branch number 4, for example, "Bb-1-4", is the Invention Compound Bb-1 which is optically active in which the difluorobutenoic acid amide group is in S-configuration and R⁴ is in R-configuration.

The compound with Comp No. and a branch number 5, for example, "Bb-1-5", is the Invention Compound Bb-1 which is optically active in which the difluorobutenoic acid amide group is in R-configuration and R⁴ is in S-configuration.

Further, the compound (IAb), the compound (IBb) and the compound (IBc), in which the difluorobutenoic acid amide group is in R-configuration, are represented by (R) following Comp No. For example, Bc-1(R) means the Invention Compound Bc-1, in which the difluorobutenoic acid amide group is in R-configuration.

The compound (IAb), the compound (IBb) and the compound (IBc), in which the difluorobutenoic acid amide group is in S-configuration, are represented by (S) following Comp No. For example, Bc-5(S) means the Invention Compound Bc-5 in which the difluorobutenoic acid amide group is in S-configuration.

In a case where the Invention Compound is a salt, the type of the salt is described in the column Remarks in Table 2. In a case where the Invention Compound is a salt, the type of the salt is described following Comp No. For example, Aa-48-1 (Na salt) represents a sodium salt of the Invention Compound Aa-48 in which the difluorobutenoic acid amide group and R⁴ are in cis-configuration.

In ¹H-NMR spectrum data in Table 2, s is singlet, brs is broad singlet, br is broad, d is doublet, brd is broad doublet, t is triplet, q is quartet, quin is quintet, sext is sextet, sep is septet and m is multiplet.

**Table 2**

| Comp No. | ¹H-NMR (300 MHz or 500 MHz, measurement solvent) δ | Remarks |
|---|---|---|
| Aa-1-1 | (300 MHz, CDCl₃) 7.04-6.91 (m, 3H), 6.80 (tt, 1H), 6.71 (br, 1H), 6.36 (dt, 1H), 4.44 (sext, 1H), 3.71 (s, 3H), 2.91 (quin, 1H), 2.77-2.65 (m, 2H), 2.30-2.17 (m, 2H). | cis |
| Aa-1-2 | (300 MHz, CDCl₃) 7.05-6.89 (m, 3H), 6.80 (tt, 1H), 6.55 (br, 1H), 6.36 (dt, 1H), 4.62 (sext, 1H), 3.73 (s, 3H), 3.17-3.05 (m, 1H), 2.79-2.66 (m, 2H), 2.40-2.25 (m, 2H). | trans |
| Aa-2-1 | (300 MHz, CDCl₃) 7.07 (t, 2H), 6.93 (dt, 1H), 6.71 (br, 1H), 6.29 (dt, 1H), 4.44 (sext, 1H), 3.71 (s, 3H), 2.91 (quin, 1H), 2.76-2.66 (m, 2H), 2.28-2.18 (m, 2H). | cis |
| Aa-2-2 | (300 MHz, CDCl₃) 7.12-7.02 (m, 2H), 6.93 (dt, 1H), 6.53 (br, 1H), 6.29 (dt, 1H), 4.62 (sext, 1H), 3.73 (s, 3H), 3.16-3.06 (m, 1H), 2.78-2.68 (m, 2H), 2.38-2.27(m, 2H). | trans |
| Aa-3-1 | (300 MHz, CDCl₃) 7.37-7.30 (m, 1H), 7.22 (d, 1H), 7.15 (d, 1H), 7.08-6.99 (m, 2H), 6.68 (br, 1H), 6.35 (dt, 1H), 4.45 (sext, 1H), 3.71 (s, 3H), 2.90 (quin, 1H), 2.77-2.65 (m, 2H), 2.29-2.17 (m, 2H). | cis |
| Aa-4-1 | (300 MHz, CDCl₃) 7.51 (s, 1H), 7.34 (t, 2H), 7.09 (dt, 1H), 6.77 (d, 1H), 6.47 (dt, 1H), 4.47 (sext, 1H), 3.73 (s, 3H), 2.93 (quin, 1H), 2.82-2.65 (m, 2H), 2.35-2.16 (m, 2H). | cis |
| Aa-4-2 | (300 MHz, CDCl₃) 7.49 (s, 1H), 7.32 (dd, 2H), 7.07 (dt, 1H), 6.55 (br, 1H), 6.44 (dt, 1H), 4.63 (sext, 1H), 3.73 (s, 3H), 3.18-3.06 (m, 1H), 2.80-2.66 (m, 2H), 2.40-2.26 (m, 2H). | trans |
| Aa-5-1 | (300 MHz, CDCl₃) 7.55 (s, 1H), 7.42 (d, 1H), 7.36 (d, 1H), 7.05 (dt, 1H), 6.78 (d, 1H), 6.46 (dt, 1H), 4.46 (sext, 1H), 3.73 (s, 3H), 2.93 (quin, 1H), 2.84-2.64 (m, 2H), 2.37-2.16 (m, 2H). | cis |

**Table 2 (continued)**

| Comp No. | ¹H-NMR (300 MHz or 500 MHz, measurement solvent) δ | Remarks |
|---|---|---|
| Aa-6-1 | (300 MHz, CDCl₃) 7.60 (s, 1H), 7.43 (dt, 1H), 7.13 (dt, 1H), 6.96 (dt, 1H), 6.74 (br, 1H), 6.37 (dt, 1H), 4.46 (sext, 1H), 3.73 (s, 3H), 2.93 (quin, 1H), 2.81-2.65 (m, 2H), 2.35-2.17 (m, 2H). | cis |
| Aa-7-1 | (300 MHz, CDCl₃) 7.23 (s, 1H), 7.09-7.03 (m, 2H), 6.97 (dt, 1H), 6.78 (d, 1H), 6.37 (dt, 1H), 4.44 (sext, 1H), 3.71 (s, 3H), 2.90 (quin, 1H), 2.75-2.65 (m, 2H), 2.29-2.17 (m, 2H). | cis |
| Aa-8-1 | (300 MHz, CDCl₃) 7.38 (s, 1H), 7.22 (d, 1H), 7.09 (dt, 1H), 6.96 (dt, 1H), 6.82 (br, 1H), 6.37 (dt, 1H), 4.44 (sext, 1H), 3.71 (s, 3H), 2.90 (quin, 1H), 2.75-2.65 (m, 2H), 2.28-2.19 (m, 2H). | cis |
| Aa-9-1 | (300 MHz, CDCl₃) 7.03-6.92 (m, 3H), 6.86 (d, 1H), 6.79 (d, 1H), 6.33 (dt, 1H), 4.44 (sext, 1H), 3.70 (s, 3H), 2.90 (quin, 1H), 2.74-2.64 (m, 2H), 2.35 (s, 3H), 2.29-2.19 (m, 2H). | cis |
| Aa-9-2 | (300 MHz, CDCl₃) 7.10-6.90 (m, 3H), 6.86 (d, 1H), 6.56 (br, 1H), 6.33 (dt, 1H), 4.62 (sext, 1H), 3.73 (s, 3H), 3.19-3.05 (m, 1H), 2.80-2.66 (m, 2H), 2.42-2.26 (m, 2H), 2.35 (s, 3H). | trans |
| Aa-10-1 | (300 MHz, CDCl₃) 7.12-7.09 (m, 2H), 7.01 (dt, 1H), 6.94 (d, 1H), 6.83 (d, 1H), 6.40 (dt, 1H), 4.45 (sext, 1H), 3.71 (s, 3H), 2.91 (quin, 1H), 2.75-2.65 (m, 2H), 2.30-2.20 (m, 2H). | cis |
| Aa-10-2 | (300 MHz, CDCl₃) 7.13-7.09 (m, 2H), 7.01 (tt, 1H), 6.95 (d, 1H), 6.56 (d, 1H), 6.39 (dt, 1H), 4.63 (sext, 1H), 3.73 (s, 3H), 3.16-3.06 (m, 1H), 2.77-2.69 (m, 2H), 2.38-2.28 (m, 2H). | trans |
| Aa-11-1 | (300 MHz, CDCl₃) 7.32 (s, 3H), 6.95 (dt, 1H), 6.85 (d, 1H), 6.38 (dt, 1H), 4.44 (sext, 1H), 3.71 (s, 3H), 2.90 (quin, 1H), 2.75-2.65 (m, 2H), 2.30-2.20 (m, 2H). | cis |

**Table 2 (continued)**

| Comp No. | ¹H-NMR (300 MHz or 500 MHz, measurement solvent) δ | Remarks |
|---|---|---|
| Aa-12-1 | (300 MHz, CDCl₃) 6.97 (dt, 1H), 6.82 (brd, 1H), 6.77-6.73 (m, 2H), 6.60 (dt, 1H), 6.34 (dt, 1H), 4.44 (sext, 1H), 3.81 (s, 3H), 3.70 (s, 3H), 2.95-2.84 (m, 1H), 2.74-2.64 (m, 2H), 2.30-2.19 (m, 2H). | cis |
| Aa-12-2 | (300 MHz, CDCl₃) 6.98 (dt, 1H), 6.83-6.70 (m, 2H), 6.61 (dt, 1H), 6.58 (br, 1H), 6.34 (dt, 1H), 4.62 (sext, 1H), 3.81 (s, 3H), 3.73 (s, 3H), 3.18-3.04 (m, 1H), 2.79-2.67 (m, 2H), 2.41-2.26 (m, 2H). | trans |
| Aa-13-1 | (300 MHz, CDCl₃) 7.03 (t, 1H), 6.96 (dt, 1H), 6.87 (t, 1H), 6.84 (t, 1H), 6.76 (d, 1H), 6.34 (dt, 1H), 4.44 (sext, 1H), 3.81 (s, 3H), 3.71 (s, 3H), 2.90 (quin, 1H), 2.75-2.65 (m, 2H), 2.29-2.17 (m, 2H). | cis |
| Aa-14-1 | (300 MHz, CDCl₃) 7.23 (s, 1H), 7.18-7.09 (m, 2H), 6.96 (dt, 1H), 6.75 (d, 1H), 6.34 (dt, 1H), 4.44 (sext, 1H), 3.71 (s, 3H), 2.90 (quin, 1H), 2.77-2.62 (m, 2H), 2.33 (s, 3H), 2.30-2.16 (m, 2H). | cis |
| Aa-15-1 | (300 MHz, CDCl₃) 7.46-7.39 (m, 2H), 7.09-6.99 (m, 3H), 6.74 (d, 1H), 6.27 (dt, 1H), 4.44 (sext, 1H), 3.70 (s, 3H), 2.90 (quin, 1H), 2.75-2.65 (m, 2H), 2.29-2.18 (m, 2H). | cis |
| Aa-16-1 | (300 MHz, CDCl₃) 7.14-7.00 (m, 2H), 6.93 (dt, 1H), 6.69 (br, 1H), 6.25 (dt, 1H), 4.44 (sext, 1H), 3.71 (s, 3H), 2.90 (quin, 1H), 2.77-2.64 (m, 2H), 2.31 (d, 3H), 2.29-2.16 (m, 2H). | cis |
| Aa-17-1 | Mixture of Aa-17-1 and Aa-43-1, obtained as Aa-17-1. (Mixture ratio of Aa-17-1/Aa-43-1=4/1). (300 MHz, CDCl₃) 7.11-6.83 (m, 4H), 6.71 (d, 1H), 6.34 (dt, 1H), 4.45 (sext, 1H), 3.71 (s, 3H), 2.99-2.82 (m, 1.8H), 2.78-2.64 (m, 2H), 2.58 (t, 0.4H), 2.32-2.14 (m, 2H), 1.63 (sext, 0.4H), 1.24 (d, 4.8H), 0.93 (t, 0.6H). | cis |
| Aa-18-1 | (300 MHz, CDCl₃) 7.09-6.84 (m, 4H), 6.70 (d, 1H), 6.34 (dt, 1H), 4.44 (sext, 1H), 3.71 (s, 3H), 2.90 (quin, 1H), 2.77-2.58 (m, 4H), 2.31-2.16 (m ,2H), 1.23 (t, 3H). | cis |
| Aa-19-1 | (300 MHz, CDCl₃) 7.43 (s, 1H), 7.35-7.28 (m, 3H), 7.01 (dt, 1H), 6.80 (d, 1H), 6.36 (dt, 1H), 4.44 (sext, 1H), 3.70 (s, 3H), 2.90 (quin, 1H), 2.78-2.62 (m, 2H), 2.33-2.15 (m, 2H). | cis |

**Table 2 (continued)**

| Comp No. | ¹H-NMR (300 MHz or 500 MHz, measurement solvent) δ | Remarks |
|---|---|---|
| Aa-20-1 | (300 MHz, CDCl₃) 7.60 (t, 1H), 7.47 (d, 1H), 7.36 (d, 1H), 7.23 (t, 1H), 7.00 (dt, 1H), 6.77 (d, 1H), 6.35 (dt, 1H), 4.44 (sext, 1H), 3.71 (s, 3H), 2.90 (quin, 1H), 2.78-2.64 (m, 2H), 2.31-2.16 (m, 2H). | cis |
| Aa-21-1 | (300 MHz, CDCl₃) 7.69 (s, 1H), 7.61 (t, 2H), 7.59 (t, 1H), 7.10 (dt, 1H), 6.91 (d, 1H), 6.44 (dt, 1H), 4.45 (sext, 1H), 3.70 (s, 3H), 2.90 (quin, 1H), 2.77-2.62 (m, 2H), 2.33-2.19 (m, 2H). | cis |
| Aa-22-1 | (300 MHz, CDCl₃) 7.30-7.20 (m, 3H), 7.19-7.10 (m, 1H), 7.02 (dt, 1H), 6.85 (d, 1H), 6.33 (dt, 1H), 4.44 (sext, 1H), 3.70 (s, 3H), 2.89 (quin, 1H), 2.77-2.60 (m, 2H), 2.35 (s, 3H), 2.32-2.18 (m, 2H). | cis |
| Aa-22-2 | (300 MHz, CDCl₃) 7.34-7.20 (m, 3H), 7.20-7.10 (m, 1H), 7.02 (dt, 1H), 6.59 (d, 1H), 6.34 (dt, 1H), 4.62 (sext, 1H), 3.72 (s, 3H), 3.16-3.04 (m, 1H), 2.78-2.65 (m, 2H), 2.43-2.25 (m, 2H), 2.35 (s, 3H). | trans |
| Aa-23-1 | (300 MHz, CDCl₃) 7.27 (t, 1H), 7.08-6.98 (m, 2H), 6.98-6.94 (m, 1H), 6.89 (dd, 1H), 6.89-6.78 (m, 1H), 6.34 (dt, 1H), 4.45 (sext, 1H), 3.81 (s, 3H), 3.70 (s, 3H), 2.89 (quin, 1H), 2.77-2.61 (m, 2H), 2.32-2.18 (m, 2H). | cis |
| Aa-23-2 | (300 MHz, CDCl₃) 7.34-7.20 (m, 3H), 7.20-7.10 (m, 1H), 7.02 (dt, 1H), 6.59 (d, 1H), 6.34 (dt, 1H), 4.62 (sext, 1H), 3.72 (s, 3H), 3.16-3.04 (m, 1H), 2.78-2.65 (m, 2H), 2.43-2.25 (m, 2H), 2.35 (s, 3H). | trans |
| Aa-24-1 | (300 MHz, CDCl₃) 6.97 (dt, 1H), 6.82-6.64 (m, 3H), 6.59 (dt, 1H), 6.32 (dt, 1H), 4.44 (sext, 1H), 4.02 (q, 2H), 3.71 (s, 3H), 2.90 (quin, 1H), 2.77-2.64 (m, 2H), 2.31-2.16 (m, 2H), 1.41 (t, 3H). | cis |
| Aa-25-1 | (300 MHz, CDCl₃) 7.35-7.16 (m, 2H), 7.05-6.92 (m, 2H), 6.85 (d, 1H), 6.25 (dt, 1H), 4.44 (sext, 1H), 3.70 (s, 3H), 2.89 (quin, 1H), 2.77-2.61 (m, 2H), 2.33-2.16 (m, 5H). | cis |
| Aa-26-1 | (300 MHz, CDCl₃) 6.98 (dt, 1H), 6.87 (d, 1H), 6.58 (d, 2H), 6.45 (t, 1H), 6.33 (dt, 1H), 4.44 (sext, 1H), 3.79 (s, 6H), 3.70 (s, 3H), 2.89 (quin, 1H), 2.76-2.61 (m, 2H), 2.33-2.15 (m, 2H). | cis |
| Aa-26-2 | (300 MHz, CDCl₃) 6.98 (dt, 1H), 6.59 (brs, 1H), 6.58 (d, 2H), 6.45 (t, 1H), 6.33 (dt, 1H), 4.63 (sext, 1H), 3.80 (s, 6H), 3.72 (s, 3H), 3.15-3.06 (m, 1H), 2.76-2.68 (m, 2H), 2.38-2.28 (m, 2H). | trans |

**Table 2 (continued)**

| Comp No. | ¹H-NMR (300 MHz or 500 MHz, measurement solvent) δ | Remarks |
|---|---|---|
| Aa-27-1 | (300 MHz, CDCl₃) 7.05-6.95 (m, 4H), 6.89 (brd, 1H), 6.32 (dt, 1H), 4.44 (sext, 1H), 3.69 (s, 3H), 2.94-2.82 (m, 1H), 2.72-2.62 (m, 2H), 2.30 (m, 6H), 2.27-2.20 (m, 2H). | cis |
| Aa-28-1 | (300 MHz, CDCl₃) 7.09 (d, 2H), 6.94 (dt, 1H), 6.65 (d, 1H), 6.23 (dt, 1H), 4.44 (sext, 1H), 3.70 (s, 3H), 2.89 (quin, 1H), 2.76-2.64 (m, 2H), 2.31-2.16 (m, 2H), 2.25 (d, 6H). | cis |
| Aa-29-1 | (300 MHz, CDCl₃) 7.07-6.94 (m, 3H), 6.86 (dt, 1H), 6.73 (d, 1H), 6.52 (t, 1H), 6.37 (dt, 1H), 4.44 (sext, 1H), 3.71 (s, 3H), 2.90 (quin, 1H), 2.77-2.65 (m, 2H), 2.30-2.17 (m, 2H). | cis |
| Aa-29-2 | (300 MHz, CDCl₃) 7.08-6.93 (m, 3H), 6.86 (dt, 1H), 6.60 (d, 1H), 6.52 (t, 1H), 6.37 (dt, 1H), 4.62 (sext, 1H), 3.73 (s, 3H), 3.16-3.06 (m, 1H), 2.79-2.65 (m, 2H), 2.41-2.25 (m, 2H). | trans |
| Aa-30-1 | (300 MHz, CDCl₃) 7.48 (s, 1H), 7.43 (s, 1H), 7.40 (s, 1H), 7.06 (dt, 1H), 6.72 (d, 1H), 6.41 (dt, 1H), 4.45 (sext, 1H), 3.71 (s, 3H), 2.90 (quin, 1H), 2.79-2.64 (m, 2H), 2.42 (s, 3H), 2.32-2.15 (m, 2H). | cis |
| Aa-31-1 | (300 MHz, CDCl₃) 7.19 (s, 1H), 7.09 (s, 1H), 7.01 (s, 1H), 7.01 (dt, 1H), 6.71 (d, 1H), 6.36 (dt, 1H), 4.44 (sext, 1H), 3.71 (s, 3H), 2.90 (quin, 1H), 2.79-2.63 (m, 2H), 2.38 (s, 3H), 2.31-2.15 (m, 2H). | cis |
| Aa-32-1 | (300 MHz, CDCl₃) 7.52 (s, 1H), 7.48 (s, 1H), 7.43 (s, 1H), 7.02 (dt, 1H), 6.72 (d, 1H), 6.40 (dt, 1H), 4.44 (sext, 1H), 3.71 (s, 3H), 2.90 (quin, 1H), 2.80-2.63 (m, 2H), 2.41 (s, 3H), 2.32-2.15 (m, 2H). | cis |
| Aa-32-2 | (300 MHz, CDCl₃) 7.51 (s, 1H), 7.48 (s, 1H), 7.43 (s, 1H), 7.02 (dt, 1H), 6.55 (d, 1H), 6.40 (dt, 1H), 4.62 (sext, 1H), 3.73 (s, 3H), 3.18-3.04 (m, 1H), 2.79-2.67 (m, 2H), 2.41 (s, 3H), 2.40-2.26 (m, 2H). | trans |
| Aa-33-1 | (300 MHz, CDCl₃) 6.99 (dt, 1H), 6.86-6.71 (m, 4H), 6.32 (dt, 1H), 4.44 (sext, 1H), 3.80 (s, 3H), 3.70 (s, 3H), 2.95-2.83 (m, 1H), 2.74-2.66 (m, 2H), 2.32 (s, 3H), 2.28-2.18 (m, 2H). | cis |

**Table 2 (continued)**

| Comp No. | ¹H-NMR (300 MHz or 500 MHz, measurement solvent) δ | Remarks |
|---|---|---|
| Aa-34-1 | (300 MHz, CDCl₃) 7.37 (s, 1H), 7.25 (d, 1H), 7.21 (d, 1H), 7.06 (dt, 1H), 6.72 (d, 1H), 6.63 (t, 1H), 6.42 (dt, 1H), 4.45 (sext, 1H), 3.71 (s, 3H), 2.91 (quin, 1H), 2.78-2.65 (m, 2H), 2.31-2.16 (m, 2H). | cis |
| Aa-35-1 | (300 MHz, CDCl₃) 7.23 (s, 1H), 7.15-6.95 (m, 3H), 6.68 (br, 1H), 6.66 (dd, 1H), 6.37 (dt, 1H), 5.78 (d, 1H), 5.35 (d, 1H), 4.45 (sext, 1H), 3.71 (s, 3H), 2.90 (quin, 1H), 2.79-2.63 (m, 2H), 2.31-2.16 (m, 2H). | cis |
| Aa-36-1 | (300 MHz, CDCl₃) 7.35 (s, 1H), 7.22-7.10 (m, 2H), 6.99 (dt, 1H), 6.89 (d, 1H), 6.37 (dt, 1H), 4.44 (sext, 1H), 3.71 (s, 3H), 3.13 (s, 1H), 2.90 (quin, 1H), 2.78-2.65 (m, 2H), 2.30-2.16 (m, 2H). | cis |
| Aa-37 | (300 MHz, CDCl₃) 7.03-6.93 (m, 3H), 6.86 (d, 1H), 6.81 (d, 1H), 6.33 (dt, 1H), 4.56 (sext, 1H), 3.79 (s, 3H), 3.78 (s, 3H), 3.07-3.00 (m, 2H), 2.57-2.51 (m, 2H), 2.35 (s, 3H). | - |
| Aa-38-1 | (300 MHz, CDCl₃) 7.22 (s, 1H), 7.18-6.97 (m, 3H), 6.71 (d, 1H), 6.38 (dt, 1H), 5.38 (d, 2H), 4.44 (sext, 1H), 3.71 (s, 3H), 2.90 (quin, 1H), 2.81-2.62 (m, 2H), 2.32-2.15 (m, 2H). | cis |
| Aa-39-1 | (500 MHz, CDCl₃) 7.29-7.24 (m, 3H), 7.17 (d, 1H), 7.04 (d, 1H), 6,65 (d, 1H), 6.38-6.29 (m, 1H), 4.48-4.39 (m, 1H), 3.70 (s, 3H), 2.92-2.84 (m, 1H), 2.71-2.62 (m, 4H), 2.25-2.18 (m, 2H), 1.23 (t, 3H). | cis |
| Aa-40-1 | (500 MHz, CDCl₃) 7.30-7.24 (m, 3H), 7.20 (d, 1H), 7.05 (d, 1H), 6,65 (d, 1H), 6.38-6.29 (m, 1H), 4.48-4.39 (m, 1H), 3.70 (s, 3H), 2.93-2.85 (m, 2H), 2.73-2.66 (m, 2H), 2.25-2.18 (m, 2H), 1.24 (d, 6H). | cis |
| Aa-41-1 | (500 MHz, CDCl₃) 7.27-7.23(m, 1H), 7.03-6.95 (m, 3H), 6.87 (d, 1H), 6,65 (d, 1H), 6.36-6.28 (m, 1H), 4.47-4.41 (m, 1H), 4.03 (dd, 2H), 3.70 (s, 3H), 2.92-2.84 (m, 1H), 2.73-2.66 (m, 2H), 2.25-2.18 (m, 2H), 1.41 (t, 3H). | cis |

**Table 2 (continued)**

| Comp No. | ¹H-NMR (300 MHz or 500 MHz, measurement solvent) δ | Remarks |
|---|---|---|
| Aa-42-1 | (500 MHz, CDCl₃) 7.26-7.22 (m, 1H), 7.03-6.94 (m, 3H), 6.86 (d, 1H), 6,65 (br, 1H), 6.35-6.27 (m, 1H), 4.58-4.52 (m, 1H), 4.46-4.39 (m, 1H), 3.70 (s, 3H), 2.92-2.84 (m, 1H), 2.73-2.66 (m, 2H), 2.25-2.18 (m, 2H), 1.33 (d, 6H). | cis |
| Aa-43-1 | (300 MHz, CDCl₃) 7.08-6.91 (m, 3H), 6.86 (d, 1H), 6.71 (d, 1H), 6.33 (dt, 1H), 4.44 (sext, 1H), 3.71 (s, 3H), 2.90 (quin, 1H), 2.77-2.64 (m, 2H), 2.58 (t, 2H), 2.30-2.16 (m, 2H), 1.64 (sext, 2H), 0.93 (t, 3H). | cis |
| Aa-44-1 | (300 MHz, CDCl₃) 7.06-6.88 (m, 3H), 6.81 (tt, 1H), 6.72 (br, 1H), 6.39 (dt, 1H), 4.37 (sext, 1H), 3.66 (d, 2H), 2.61-2.48 (m, 2H), 2.41-2.23 (m, 1H), 1.85 (ddd, 2H)). One proton peak detected as overlapped with proton peak of water in measurement solvent. | cis |
| Aa-45-1 | (300 MHz, CDCl₃) 7.06-6.89 (m, 3H), 6.82 (tt, 1H), 6.54 (br, 1H), 6.38 (dt, 1H), 4.38 (sext, 1H), 4.24 (d, 2H), 3.06 (s, 3H), 2.67-2.40 (m, 3H), 1.93 (ddd, 2H). | cis |
| Aa-47-1 | (300 MHz, CDCl₃) 7.06-6.90 (m, 3H), 6.81 (tt, 1H), 6.68 (br, 1H), 6.35 (dt, 1H), 4.58-4.36 (m, 1H), 2.97-2.79 (m, 3H), 2.53-2.34 (m, 2H). | cis |
| Aa-48-1 | (300 MHz, acetone-ds) 10.64 (br, 0.44H), 8.30 (br, 1H), 7.42-7.28 (m, 2H), 7.18-7.00 (m, 2H), 6.69 (dt, 1H), 4.40 (sext, 1H), 2.99-2.85 (m, 1H), 2.65-2.52 (m, 2H), 2.42-2.25 (m, 2H). Proton peak of carboxy group detected at 10.64 ppm. | cis |
| Aa-48-1 (Na salt) | (300 MHz, D₂O) 7.11-6.99 (m, 2H), 6.95 (dt, 1H), 6.86 (tt, 1H), 6.37 (dt, 1H), 4.11 (quin, 1H), 2.68 (quin, 1H), 2.54-2.39 (m, 2H), 2.12-1.95 (m, 2H). Proton peak of amide underwent H-D exchange reaction in measurement solvent and not detected. | cis, Na salt of Aa-48-1 |

**Table 2 (continued)**

| Comp No. | ¹H-NMR (300 MHz or 500 MHz, measurement solvent) δ | Remarks |
|---|---|---|
| Aa-49-1 | 300 MHz, CDCl₃) 8.10 (br, 1H), 7.21 (br, 0.77H), 7.10-6.89 (m, 3.23H), 6.81 (tt, 1H), 6.39 (dt, 1H), 4.67-4.39 (m, 1H), 3.81 (s, 3H), 3.32 (br, 0.27H), 2.85-2.49 (m, 2.73H), 2.45-2.17 (m, 2H). | cis |
| Aa-49-2 | (500 MHz, DMSO-ds) 10.97 (s, 1H), 9.08 (d, 1H), 7.48-7.45 (m, 2H), 7.26 (tt, 1H), 7.04 (dt, 1H), 6.79 (dt, 1H), 4.45 (sext, 1H), 3.57 (s, 3H), 2.74-2.69 (m, 1H), 2.37-2.26 (m, 4H). | trans |
| Aa-50-1 | 300 MHz, CDCl₃) 7.16-6.88 (m, 4H), 6.78 (tt, 1H), 6.36 (dt, 1H), 4.47 (sext, 1H), 3.66 (s, 3H), 3.31 (t, 1H), 3.20 (s, 3H), 2.72-2.58 (m, 2H), 2.32-2.17 (m, 2H). | cis |
| Aa-51-1 | (300 MHz, CDCl₃) 7.07-6.88 (m, 3H), 6.79 (tt, 1H), 6.71 (d, 1H), 6.36 (dt, 1H), 4.41 (sext, 1H), 2.80 (quin, 1H), 2.73-2.59 (m, 2H), 2.23-2.08 (m, 2H), 1.45 (s, 9H). | cis |
| Aa-52-1 | (300 MHz, CDCl₃) 7.06-6.90 (m, 3H), 6.80 (tt, 1H), 6.51 (br, 1H), 6.36 (dt, 1H), 5.78 (td, 1H), 4.42 (sext, 1H), 2.64-2.41 (m, 3H), 2.13-1.95 (m, 2H). | cis |
| Aa-53-1 | (300 MHz, CDCl₃) 7.08-6.91 (m, 3H), 6.82 (tt, 1H), 6.53 (br, 1H), 6.39 (dt, 1H), 4.36 (sext, 1H), 4.08 (d, 2H), 2.66-2.50 (m, 2H), 2.49-2.34 (m, 1H), 2.10 (s, 3H), 1.79 (ddd, 2H). | cis |
| Aa-54-1 | (300 MHz, CDCl₃) 9.78 (d, 1H), 7.08-6.92 (m, 3H), 6.81 (tt, 1H), 6.64 (br, 1H), 6.38 (dt, 1H), 4.51 (sext, 1H), 3.16-3.00 (m, 1H), 2.74-2.59 (m, 2H), 2.32-2.17 (m, 2H). | cis |
| Aa-55-1 | (300 MHz, CDCl₃) 7.04-6.89 (m, 3H), 6.79 (tt, 1H), 6.68 (d, 1H), 6.35 (dt, 1H), 4.43 (sext, 1H), 3.05 (quin, 1H), 2.71-2.57 (m, 2H), 2.22-2.07 (m, 2H), 2.14 (s, 3H). | cis |
| Aa-56-1 | (300 MHz, CDCl₃) 7.07-6.90 (m, 3H), 6.80 (tt, 1H), 6.60 (d, 1H), 6.35 (dt, 1H), 4.44 (sext, 1H), 4.20 (q, 2H), 3.44 (s, 2H), 3.19 (quin, 1H), 2.74-2.58 (m, 2H), 2.30-2.12 (m, 2H), 1.28 (t, 3H). | cis |
| Aa-57-1 | (300 MHz, CDCl₃) 7.18-6.89 (m, 4H), 6.80 (tt, 1H), 6.47 (d, 1H), 6.36 (dt, 1H), 5.78 (dd, 1H), 4.38 (sext, 1H), 4.19 (q, 2H), 2.92-2.76 (m, 1H), 2.76-2.61 (m, 2H), 2.00-1.83 (m, 2H), 1.29 (s, 3H). | cis |
| Aa-58-3 | (300 MHz, CDCl₃) 7.07-6.89 (m, 3H), 6.80 (tt, 1H), 6.55 (br, 1H), 6.37 (dt, 0.5H), 6.37 (dt, 0.5H), 4.65-4.46 (m, 1H), 4.13 (t, 0.5H), 3.99 (sext, 0.5H), 2.95-2.79 (m, 1H), 2.52-2.27 (m, 2H), 2.11 (br, 0.5H), 2.04-1.82 (m, 1.5H). | cis-trans isomer |

**Table 2 (continued)**

| Comp No. | ¹H-NMR (300 MHz or 500 MHz, measurement solvent) δ | Remarks |
|---|---|---|
| Aa-59 | (300 MHz, CDCl₃) 7.07-6.91 (m, 3H), 6.80 (tt, 1H), 6.63 (br, 1H), 6.37 (dt, 1H), 5.74 (quin, 1H), 4.54 (sext, 1H), 4.16 (q, 2H), 3.75-3.61 (m, 1H), 3.38-3.25 (m, 1H), 3.13-3.01 (m, 1H), 2.92-2.80 (m, 1H), 1.28 (s, 3H). | - |
| Aa-60-1 | (300 MHz, CDCl₃) 7.02-6.92 (m, 4H), 6.79 (tt, 1H), 6.37 (dt, 1H), 4.47 (sext, 1H), 3.94 (t, 2H), 3.72 (t, 2H), 3.31 (t, 1H), 2.72-2.62 (m, 2H), 2.32 (quin, 2H), 2.28-2.17 (m, 2H). | cis |
| Aa-61-1 | (300 MHz, CDCl₃) 7.07 (d, 1H), 7.01-6.92 (m, 3H), 6.79 (tt, 1H), 6.37 (dt, 1H), 4.46 (sext, 1H), 3.93 (t, 2H), 3.78 (t, 2H), 3.30 (quin, 1H), 2.69-2.60 (m, 2H), 2.30-2.17 (m, 2H), 1.86-1.68 (m, 4H). | cis |
| Aa-62-1 | (300 MHz, CDCl₃) 7.07 (d, 1H), 7.01-6.92 (m, 3H), 6.79 (tt, 1H), 6.37 (dt, 1H), 4.45 (sext, 1H), 3.69-3.60 (m, 6H), 3.40 (t, 2H), 3.04 (quin, 1H), 2.70-2.61 (m, 2H), 2.37-2.27 (m, 2H). | cis |
| Aa-63-1 | (300 MHz, CDCl₃) 7.48 (d, 1H), 7.02-6.92 (m, 3H), 6.78 (tt, 1H), 6.38 (dt, 1H), 4.47 (sext, 1H), 3.47 (t, 2H), 3.38 (t, 2H), 2.98 (quin, 1H), 2.70-2.61 (m, 2H), 2.35-2.26 (m, 2H), 2.00-1.81 (m, 4H). | cis |
| Aa-64-1 | (300 MHz, CDCl₃) 7.45 (d, 1H), 7.02-6.93 (m, 3H), 6.78 (tt, 1H), 6.37 (dt, 1H), 4.48 (sext, 1H), 4.12 (t, 2H), 4.04 (t, 2H), 2.79 (quin, 1H), 2.66-2.57 (m, 2H), 2.34-2.17 (m, 4H) | cis |
| Aa-65-1 | (300 MHz, CDCl₃) 7.13 (d, 1H), 7.02-6.91 (m, 3H), 6.79 (tt, 1H), 6.36 (dt, 1H), 4.46 (sext, 1H), 3.38 (q, 2H), 3.25 (q, 2H), 3.05 (quin, 1H), 2.70-2.61 (m, 2H), 2.32-2.23 (m, 2H), 1.14 (dt, 6H). | cis |
| Aa-66-1 | (300 MHz, CDCl₃) 7.21 (d, 1H), 7.03-6.91 (m, 3H), 6.79 (tt, 1H), 6.37 (dt, 1H), 5.91 (br, 1H), 4.46 (sext, 1H), 3.65-3.60 (m, 4H), 2.80-2.63 (m, 3H), 2.34-2.22 (m, 2H). | cis |

**Table 2 (continued)**

| Comp No. | ¹H-NMR (300 MHz or 500 MHz, measurement solvent) δ | Remarks |
|---|---|---|
| Aa-67-1 | (300 MHz, CDCl₃) 7.31-7.21 (m, 1H), 7.02-6.89 (m, 3H), 6.79 (tt, 1H), 6.38 (dt, 1H), 4.49 (sext, 1H), 3.69-3.62 (m, 5H), 3.35-3.24 (m, 1H), 2.69-2.60 (m, 2H), 2.34-2.23 (m, 2H), 1.17 (t, 3H). | cis |
| Aa-68-1 | (300 MHz, CDCl₃) 7.08 (d, 1H), 7.02-6.92 (m, 3H), 6.79 (tt, 1H), 6.37 (dt, 1H), 4.48 (sext, 1H), 3.65 (s, 3H), 3.58 (t, 2H), 3.35-3.25 (m, 1H), 2.71-2.62 (m, 2H), 2.30-2.20 (m, 2H), 1.63 (sext, 2H), 0.90 (t, 3H). | cis |
| Aa-69-1 | (300 MHz, CDCl₃) 7.08 (d, 1H), 7.02-6.93 (m, 3H), 6.79 (tt, 1H), 6.37 (dt, 1H), 4.56 (br, 1H), 4.48 (sext, 1H), 3.75 (s, 3H), 3.26-3.21 (m, 1H), 2.70-2.61 (m, 2H), 2.30-2.17 (m, 2H), 1.22 (d, 6H). | cis |
| Aa-70-1 | (300 MHz, CDCl₃) 7.15 (d, 1H), 7.02-6.92 (m, 3H), 6.79 (tt, 1H), 6.37 (dt, 1H), 4.48 (sext, 1H), 3.69-3.54 (m, 5H), 3.34-3.23 (m, 1H), 2.70-2.61 (m, 2H), 2.31-2.21 (m, 2H), 1.58 (quin, 2H), 1.37-1.24 (m, 2H), 0.92 (t, 3H). | cis |
| Aa-71-1 | (300 MHz, CDCl₃) 7.02-6.92 (m, 3H), 6.80 (dt, 1H), 6.69 (d, 1H), 6.36 (dt, 1H), 4.43 (sext, 1H), 3.11 (quin, 1H), 2.91 (q, 2H), 2.75-2.65 (m, 2H), 2.30-2.20 (m, 2H), 1.26 (t, 3H). | cis |
| Aa-72-1 | (300 MHz, CDCl₃) 7.02-6.92 (m, 3H), 6.80 (dt, 1H), 6.73 (d, 1H), 6.36 (dt, 1H), 4.71 (d, 2H), 4.46 (sext, 1H), 3.00-2.89 (m, 1H), 2.78-2.68 (m, 2H), 2.49 (t, 1H), 2.32-2.21 (m, 2H). | cis |
| Aa-73-1 | (300 MHz, CDCl₃) 7.02-6.92 (m, 3H), 6.80 (tt, 1H), 6.74 (brs, 1H), 6.36 (dt, 1H), 4.46 (sext, 1H), 4.32 (t, 2H), 2.96 (quin, 1H), 2.79-2.69 (m, 4H), 2.33-2.22 (m, 2H). | cis |
| Aa-74-1 | (300 MHz, CDCl₃) 7.02-6.93 (m, 3H), 6.80 (tt, 1H), 6.36 (dt, 1H), 6.70 (brs, 1H), 5.95 (tt, 1H), 4.46 (sext, 1H), 4.31 (dt, 2H), 3.03-2.91 (m, 1H), 2.79-2.69 (m, 2H), 2.32-2.21 (m, 2H). | cis |
| Aa-75-1 | (300 MHz, CDCl₃) 7.02-6.93 (m, 3H), 6.80 (tt, 1H), 6.65 (d, 1H), 6.36 (dt, 1H), 4.50 (q, 2H), 4.45 (sext, 1H), 3.01 (quin, 1H), 2.82-2.71 (m, 2H), 2.33-2.22 (m, 2H). | cis |

**Table 2 (continued)**

| Comp No. | ¹H-NMR (300 MHz or 500 MHz, measurement solvent) δ | Remarks |
|---|---|---|
| Aa-76-1 | (300 MHz, CDCl₃) 7.28 (d, 1H), 7.01-6.92 (m, 3H), 6.78 (tt, 1H), 6.37 (dt, 1H), 4.45 (sext, 1H), 3.07 (quin, 1H), 2.97 (s, 3H), 2.96 (s, 3H), 2.70-2.61 (m, 2H), 2.35-2.25 (m, 2H). | cis |
| Aa-77-1 | (300 MHz, CDCl₃) 7.00-6.92 (m, 3H), 6.79 (tt, 1H), 6.73 (brs, 1H), 6.36 (dt, 1H), 4.44 (sext, 1H), 4.28 (t, 2H), 2.92 (quin, 1H), 2.77-2.67 (m, 4H), 2.30-2.20 (m, 2H), 2.15 (s, 3H). | cis |
| Aa-78 | (300 MHz, CDCl₃) 7.03-6.92 (m, 3H), 6.80 (tt, 1H), 6.78 (brs, 1H), 6.37 (dt, 1H), 5.75 (quin, 1H), 4.54 (sext, 1H), 3.73-3.61 (m, 1H), 3.70 (s, 3H), 3.37-3.26 (m, 1H), 3.09 (dquin, 1H), 2.88 (dquin, 1H). | - |
| Aa-81-1 | (300 MHz, CDCl₃) 7.16 (d, 1H), 7.01-6.92 (m, 3H), 6.79 (tt, 1H), 6.37 (dt, 1H), 4.45 (sext, 1H), 4.28 (sep, 1H), 3.81 (ddd, 1H), 3.68-3.56 (m, 2H), 3.32 (ddd, 1H), 3.06 (quin, 1H), 2.71-2.59 (m, 2H), 2.37-2.27 (m, 2H), 2.08-1.98 (m, 2H), 1.88-1.77 (m, 2H). | cis |
| Aa-82-1 | (300 MHz, CDCl₃) 7.12 (d, 1H), 7.01-6.93 (m, 3H), 6.85 (d, 1H) 6.33 (dt , 1H), 4.48 (sext, 1H), 3.94 (t, 2H), 3.71 (t, 2H), 3.30 (t, 1H), 2.70-2.60 (m, 2H), 2.37-2.23 (m, 4H), 2.34 (s, 3H). | cis |
| Aa-83-1 | (300 MHz, CDCl₃) 7.03-6.93 (m, 3H), 6.86 (d, 1H), 6.75 (d, 1H), 6.33 (dt, 1H), 4.70 (d, 2H), 4.46 (sext, 1H), 3.00-2.89 (m, 1H), 2.77-2.68 (m , 2H), 2.49 (t, 1H), 2.35 (s, 3H), 2.32-2.21 (m, 2H). | cis |
| Aa-84-1 | (300 MHz, CDCl₃) 7.03-6.92 (m, 3H), 6.87-6.81 (m, 2H), 6.33 (dt, 1H), 4.46 (sext, 1H), 4.31 (t, 2H), 3.00-2.90 (s, 1H), 2.77-2.67 (m, 4H), 2.34 (s, 3H), 2.33-2.23 (m, 2H). | cis |
| Aa-85-1 | (500 MHz, DMSO-ds) 12.13 (br, 1H), 9.04 (d, 1H), 7.29 (d, 1H), 7.26 (s, 1H), 7.01 (d, 1H), 6.94 (d, 1H), 6.61 (dt, 1H), 4.15 (sext, 1H), 2.72 (quin, 1H), 2.38-2.33 (m, 2H), 2.29 (s, 3H), 2.23-2.17 (m, 2H). | cis |

**Table 2 (continued)**

| Comp No. | ¹H-NMR(300 MHz or 500 MHz, measurement solvent) δ | Remarks |
|---|---|---|
| Aa-85-2 | (500 MHz, DMSO-ds) 12.22 (s, 1H), 9.09 (d, 1H), 7.31-7.26 (m, 2H), 7.02 (d, 1H), 6.95 (d, 1H), 6.67-6.58 (m, 1H), 4.41-4.32 (m, 1H), 2.92-2.88 (m, 1H), 2.38-2.27 (m, 7H). | trans |
| Aa-86-1 | (300 MHz, CDCl₃) 7.46 (brd, 1H), 7.02-6.93 (m, 3H), 6.85 (d, 1H), 6.35 (dt, 1H), 5.75 (brd, 1H), 4.44 (sext, 1H), 2.82 (d, 3H), 2.75-2.55 (m, 3H), 2.34 (s, 3H), 2.32-2.25 (m, 2H). | cis |
| Aa-87-1 | (300 MHz, CDCl₃) 7.03-6.92 (m, 3H), 6.85 (d, 1H), 6.74 (d, 1H), 6.33 (dt, 1H), 4.43 (sext, 1H), 3.10 (quin, 1H), 2.90 (q, 2H), 2.74-2.64 (m, 2H), 2.34 (s, 3H), 2.31-2.20 (m, 2H), 1.26 (t, 3H). | cis |
| Aa-88-1 | (300 MHz, CDCl₃) 7.50 (brd, 1H), 7.01-6.91 (m, 3H), 6.85 (d, 1H), 6.33 (dt, 1H), 6.27 (brs, 1H), 4.44 (sext, 1H), 3.64-3.56 (m, 1H), 3.61 (s, 3H), 2.75 (dd, 1H), 2.68-2.59 (m, 2H), 2.36-2.27 (m, 2H), 2.33 (s, 3H). | cis |
| Aa-89-1 | (300 MHz, CDCl₃) 7.03-6.92 (m, 3H), 6.86 (d, 1H), 6.74 (d, 1H), 6.33 (dt, 1H), 4.44 (sext, 1H), 4.15 (q, 2H), 2.88 (quin, 1H), 2.74-2.64 (m, 2H), 2.35 (s, 3H), 2.27-2.17 (m, 2H), 1.27 (t, 3H). | cis |
| Aa-90-1 | (300 MHz, CDCl₃) 7.03-6.92 (m, 3H), 6.86 (d, 1H), 6.72 (d, 1H), 6.33 (dt, 1H), 4.44 (sext, 1H), 4.06 (t, 2H), 2.89 (quin, 1H), 2.75-2.65 (m, 2H), 2.35 (s, 3H), 2.27-2.17 (m, 2H), 1.66 (sext, 2H), 0.94 (t, 3H). | cis |
| Aa-91-1 | (300 MHz, CDCl₃) 7.03-6.92 (m, 3H), 6.86 (d, 1H), 6.70 (d, 1H), 6.33 (dt, 1H), 5.92 (ddt, 1H), 5.32 (ddd, 1H), 5.25 (ddd, 1H), 4.60 (dt, 2H), 4.45 (sext, 1H), 2.92 (quin, 1H), 2.76-2.66 (m, 2H), 2.35 (s, 3H), 2.29-2.19 (m, 2H). | cis |
| Aa-92-1 | (300 MHz, CDCl₃) 7.03-6.93 (m, 3H), 6.86 (d, 1H), 6.72 (d, 1H), 6.33 (dt, 1H), 5.17 (sep, 1H), 4.43 (sext, 1H), 2.84 (quin, 1H), 2.73-2.63 (m, 2H), 2.35 (s, 3H), 2.24-2.14 (m, 2H), 1.92-1.82 (m, 2H), 1.75-1.55 (m, 6H). | cis |

**Table 2 (continued)**

| Comp No. | ¹H-NMR (300 MHz or 500 MHz, measurement solvent) δ | Remarks |
|---|---|---|
| Aa-93-1 | (300 MHz, CDCl₃) 7.03-6.92 (m, 3H), 6.86 (d, 1H), 6.75 (d, 1H), 6.33 (dt, 1H), 4.44 (sext, 1H), 4.09 (q, 2H), 2.88 (quin, 1H), 2.74-2.65 (m, 2H), 2.35 (s, 3H), 2.27-2.17 (m, 2H), 1.68-1.59 (m, 2H), 1.37-1.26 (m, 4H), 0.91 (t, 3H). | cis |
| Aa-94-1 | (300 MHz, CDCl₃) 7.26 (br, 1H), 7.03-6.92 (m, 3H), 6.85 (d, 1H), 6.33 (dt, 1H), 5.48 (br, 1H), 4.45 (sext, 1H), 3.31 (quin, 1H), 3.31 (q, 1H), 2.69-2.58 (m, 3H), 2.34 (s, 3H), 2.31-2.21 (m, 2H), 1.14 (t, 3H). | cis |
| Aa-95-1 | (300 MHz, CDCl₃) 7.37 (d, 1H), 7.02-6.92 (m, 3H), 6.85 (d, 1H), 6.33 (dt, 1H), 5.60 (br, 1H), 4.44 (sext, 1H), 3.26-3.19 (m, 2H), 2.73-2.58 (m, 3H), 2.34 (s, 3H), 2.31-2.23 (m, 2H), 1.52 (sext, 2H), 0.92 (t, 3H). | cis |
| Aa-96-1 | (300 MHz, CDCl₃) 7.30 (d, 1H), 7.03-6.92 (m, 3H), 6.85 (d, 1H), 6.33 (dt, 1H), 5.34 (d, 1H), 4.44 (sext, 1H), 4.16-4.00 (m, 1H), 2.68-2.55 (m, 3H), 2.34 (s, 3H), 2.30-2.17 (m, 2H), 1.15 (d, 6H). | cis |
| Aa-97-1 | (300 MHz, CDCl₃) 7.28 (d, 1H), 7.02-6.92 (m, 3H), 6.85 (d, 1H), 6.33 (dt, 1H), 5.83 (ddt, 1H), 5.65 (br, 1H), 5.21-5.12 (m, 2H), 4.45 (sext, 1H), 3.89 (tt, 2H), 2.74-2.60 (m, 3H), 2.34 (s, 3H), 2.33-2.24 (m, 2H). | cis |
| Aa-98-1 | (300 MHz, CDCl₃) 7.32 (d, 1H), 7.02-6.92 (m, 3H), 6.85 (d, 1H), 6.33 (dt, 1H), 5.54 (br, 1H), 4.44 (sext, 1H), 3.25 (td, 2H), 2.70-2.58 (m, 3H), 2.34 (s, 3H), 2.32-2.22 (m, 2H), 1.50 (quin, 2H), 1.39-1.23 (m, 4H), 0.89 (t, 3H). | cis |
| Aa-99-1 | (300 MHz, CDCl₃) 8.24 (brs, 1H), 7.10 (brs, 1H), 7.03-6.84 (m, 4H), 6.33 (dt, 1H), 4.54-4.41 (m, 1H), 3.78 (s, 3H), 2.70-2.56 (m, 3H), 2.35 (s, 3H), 2.32-2.29 (m, 2H). | cis |
| Aa-100-1 | (300 MHz, CDCl₃) 7.03-6.92 (m, 3H), 6.86 (d, 1H), 6.77 (d, 1H), 6.33 (dt, 1H), 4.45 (sext, 1H), 3.45 (quin, 1H), 3.19 (s, 3H), 2.78-2.68 (m, 2H), 2.40 (s, 3H), 2.34 (s, 3H), 2.33-2.23 (m, 2H). | cis |

**Table 2 (continued)**

| Comp No. | ¹H-NMR (300 MHz or 500 MHz, measurement solvent) δ | Remarks |
|---|---|---|
| Aa-101-1 | (300 MHz, CDCl₃) 7.09-6.81 (m, 4H), 6.67 (d, 1H), 6.33 (dt, 1H), 4.43 (sext, 1H), 4.15 (m, 1H), 2.84 (quin, 1H), 2.76-2.61 (m, 2H), 2.35 (s, 3H), 2.28-2.14 (m, 2H), 0.80-0.64 (m, 4H). | cis |
| Aa-102-3 | Comp No. Aa-102-3 was obtained at 80% purity. (300 MHz, CDCl₃) 7.00-6.94 (m, 3H), 6.79 (tt, 0.6H), 6.78 (tt, 0.4H), 6.58 (br, 0.4H), 6.53 (br, 0.6H), 6.39 (dt, 0.6H), 6.33 (dt, 0.4H), 4.50 (sext, 0.4H), 4.30 (sext, 0.6H), 3.67 (s, 1.2H), 3.66 (s, 1.8H), 2.76-2.60 (m, 2.4H), 2.47-2.38 (m, 1.8H), 2.25-2.20 (m, 1.6H), 1.77-1.67 (m, 1.2H). | cis-trans isomer |
| Aa-103-1 | (300 MHz, CDCl₃) 7.10-6.82 (m, 4H), 6.66 (d, 1H), 6.33 (dt, 1H), 4.43 (sext, 1H), 3.14 (quin, 1H), 2.78-2.64 (m, 2H), 2.35 (s, 3H), 2.33 (s, 3H), 2.31-2.19 (m, 2H). | cis |
| Aa-103-2 | (500 MHz, CDCl₃) 7.03 (s, 1H), 7.00-6.92 (m, 2H), 6.86 (d, 1H), 6.50 (brs, 1H), 6.37-6.28 (m, 1H), 4.60-4.52 (m, 1H), 3.37-3.31 (m, 1H), 2.78-2.72 (m, 2H), 2.37-2.30 (m, 8H). | trans |
| Aa-104-1 | (300 MHz, CDCl₃) 7.03-6.92 (m, 3H), 6.86 (d, 1H), 6.79 (d, 1H), 6.33 (dt, 1H), 4.44 (sext, 1H), 4.10 (q, 2H), 2.88 (quin, 1H), 2.74-2.65 (m, 2H), 2.35 (s, 3H), 2.28-2.17 (m, 2H), 1.62 (quin, 2H), 1.37 (sext, 2H), 0.93 (t, 3H). | cis |
| Aa-105-1 | (300 MHz, CDCl₃) 7.03-6.92 (m, 3H), 6.86 (d, 1H), 6.76 (d, 1H), 6.33 (dt, 1H), 5.86-5.75 (m, 1H), 5.63-5.52 (m, 1H), 4.52 (dt, 2H), 4.44 (sext, 1H), 2.89 (quin, 1H), 2.74-2.65 (m, 2H), 2.35 (s, 3H), 2.29-2.18 (m, 2H), 1.73 (dd, 3H). | cis |
| Aa-106-1 | (300 MHz, CDCl₃) 7.02-6.92 (m, 3H), 6.91 (br, 1H), 6.85 (d, 1H), 6.34 (dt, 1H), 4.44 (sext, 1H), 4.15 (t, 2H), 2.87 (quin, 1H), 2.73-2.63 (m, 2H), 2.34 (s, 3H), 2.30-2.19 (m, 2H), 1.56 (t, 2H), 0.93 (s, 9H). | cis |
| Aa-107-1 | (300 MHz, CDCl₃) 7.03-6.92 (m, 3H), 6.86 (d, 1H), 6.80 (d, 1H), 6.33 (dt, 1H), 5.84-5.70 (m, 1H), 5.14-5.05 (m, 2H), 4.44 (sext, 1H), 4.16 (t, 2H), 2.89 (quin, 1H), 2.74-2.64 (m, 2H), 2.39 (qt, 2H), 2.34 (s, 3H), 2.28-2.18 (m, 2H). | cis |
| Aa-108-1 | (300 MHz, CDCl₃) 7.03-6.93 (m, 3H), 6.86 (d, 1H), 6.81 (d, 1H), 6.33 (dt, 1H), 4.45 (sext, 1H), 4.21 (t, 2H), 2.92 (quin, 1H), 2.75-2.66 (m, 2H), 2.54 (td, 2H), 2.34 (s, 3H), 2.31-2.20 (m, 2H), 2.01 (t, 1H). | cis |

**Table 2 (continued)**

| Comp No. | ¹H-NMR (300 MHz or 500 MHz, measurement solvent) δ | Remarks |
|---|---|---|
| Aa-109-1 | (300 MHz, CDCl₃) 7.03-6.92 (m, 3H), 6.86 (d, 1H), 6.73 (d, 1H), 6.33 (dt, 1H), 4.61 (t, 2H), 4.47 (sext, 1H), 3.00 (quin, 1H), 2.79-2.70 (m, 2H), 2.34 (s, 3H), 2.33-2.23 (m, 2H). | cis |
| Aa-110-1 | (300 MHz, CDCl₃) 7.03-6.93 (m, 3H), 6.86 (d, 1H), 6.76 (d, 1H), 6.33 (dt, 1H), 4.67 (q, 2H), 4.45 (sext, 1H), 2.93 (quin, 1H), 2.76-2.66 (m, 2H), 2.35 (s, 3H), 2.31-2.20 (m, 2H), 1.86 (t, 3H). | cis |
| Aa-111-1 | (300 MHz, CDCl₃) 7.03-6.93 (m, 3H), 6.86 (d, 1H), 6.76 (d, 1H), 6.33 (dt, 1H), 4.45 (sext, 1H), 4.28 (t, 2H), 2.91 (quin, 1H), 2.75-2.66 (m, 2H), 2.73 (t, 2H), 2.35 (s, 3H), 2.30-2.20 (m, 2H), 2.15 (s, 3H). | cis |
| Aa-112-1 | (300 MHz, CDCl₃) 7.02-6.92 (m, 3H), 6.87 (br, 1H), 6.85 (d, 1H), 6.32 (dt, 1H), 4.56 (t, 2H), 4.44 (sext, 1H), 3.35 (t, 2H), 2.98 (s, 3H), 2.90 (quin, 1H), 2.75-2.65 (m, 2H), 2.34 (s, 3H), 2.34-2.24 (m, 2H). | cis |
| Aa-113-1 | (500 MHz, DMSO-d₆) 12.14 (s, 1H) ,9.04 (d, 1H), 7.08 (d, 1H), 7.03 (d, 1H), 6.95 (d, 1H), 6,80 (d, 1H), 6.71-6.62 (m, 1H), 4.20-4.11 (m, 1H), 3.76 (s, 3H), 2.76-2.68 (m, 1H), 2.39-2.33 (m, 2H), 2.23-2.16 (m, 2H). | cis |
| Aa-115-1 | (300 MHz, CDCl₃) 7.35 (s, 1H), 7.22-7.10 (m, 2H), 6.99 (dt, 1H), 6.72 (d, 1H), 6.37 (dt, 1H), 4.44 (sext, 1H), 4.16 (q, 2H), 3.13 (s, 1H), 2.88 (quin, 1H), 2.78-2.63 (m, 2H), 2.31-2.14 (m, 2H), 1.27 (t, 3H). | cis |
| Aa-131-1 | (500 MHz, CDCl₃) 7.30-7.24 (m, 1H), 7.21-7.10 (m, 2H), 6.98 (dt, 1H), 6.82 (d, 1H), 6.28 (dt, 1H), 4.44 (sext, 1H), 3.71 (s, 3H), 2.90 (quin, 1H), 2.75-2.65 (m, 2H), 2.30-2.17 (m, 2H). | cis |

**Table 2 (continued)**

| Comp No. | ¹H-NMR (300 MHz or 500 MHz, measurement solvent) δ | Remarks |
|---|---|---|
| Aa-133-1 | (300 MHz, CDCl₃) 7.02-6.92 (m, 3H), 6.79 (tt, 1H), 6.73 (d, 1H), 6.37 (dt, 1H), 4.44 (sext, 1H), 4.16 (q, 2H), 2.88 (quin, 1H), 2.76-2.67 (m, 2H), 2.27-2.17 (m, 2H), 1.27 (t, 3H). | cis |
| Aa-134-1 | (300 MHz, CDCl₃) 7.02-6.91 (m, 3H), 6.79 (tt, 1H), 6.83-6.76 (br, 1H), 6.36 (dt, 1H), 4.44 (sext, 1H), 4.06 (t, 2H), 2.90 (quin, 1H), 2.75-2.65 (m, 2H), 2.28-2.17 (m, 2H), 1.66 (sext, 2H), 0.94 (t, 3H). | cis |
| Aa-135-1 | (300 MHz, CDCl₃) 7.02-6.92 (m, 3H), 6.79 (tt, 1H), 6.83-6.73 (br, 1H), 6.36 (dt, 1H), 5.01 (sep, 1H), 4.43 (sext, 1H), 2.85 (quin, 1H), 2.74-2.65 (m, 2H), 2.25-2.14 (m, 2H), 1.24 (d, 6H). | cis |
| Aa-137-1 | (300 MHz, CDCl₃) 8.27 (br, 1H), 7.38-7.22 (br, 1H), 7.05-6.93 (m, 3H), 6.81 (tt, 1H), 6.39 (dt, 1H), 4.61-4.42 (m, 1H), 4.07-3.81 (m, 2H), 2.76-2.56 (m, 3H), 2.37-2.25 (m, 2H), 1.30 (t, 3H). | cis |
| Aa-138-1 | (300 MHz, CDCl₃) 8.20-7.75 (br, 1H), 7.35-7.19 (br, 1H), 7.01-6.91 (m, 3H), 6.79 (tt, 1H), 6.36 (dt, 1H), 4.58-4.39 (m, 1H), 4.22-4.05 (m, 0.7H), 3.92 (br, 0.3H), 3.30 (br, 0.3H), 2.71-2.56 (m, 2.7H), 2.36-2.20 (m, 2H), 1.25 (d, 6H). | cis |
| Aa-156-3 | (300 MHz, CDCl₃) 7.04-6.90 (m, 3H), 6.80 (tt, 1H), 6.65 (d, 1H), 6.35 (dt, 1H), 4.62-4.44 (m, 1H), 3.74 (s, 1.8H), 3.72 (S, 1.2H), 2.96-2.89 (m, 1H), 2.43-2.38 (m, 2H), 2.03-1.95 (m, 1H), 1.45 (s, 1.2H), 1.43 (s, 1.8H). | cis-trans isomer |
| Aa-166-1 | (300 MHz, CDCl₃) 6.95-6.85 (m, 3H), 6.76-6.68 (br, 1H), 6.72 (tt, 1H), 6.29 (dt, 1H), 5.84 (ddt, 1H), 5.25 (ddt, 1H), 5.18 (ddd, 1H), 4.53 (dt, 2H), 4.38 (sext, 1H), 2.86 (quin, 1H), 2.69-2.59 (m, 2H), 2.23-2.13 (m, 2H). | cis |
| Aa-167-1 | (300 MHz, CDCl₃) 6.95-6.85 (m, 3H), 6.73 (tt, 1H), 6.63 (d, 1H), 6.29 (dt, 1H), 4.38 (sext, 1H), 4.30 (t, 2H), 3.63 (t, 2H), 2.88 (quin, 1H), 2.72-2.61 (m, 2H), 2.24-2.13 (m, 2H). | cis |

**Table 2 (continued)**

| Comp No. | ¹H-NMR (300 MHz or 500 MHz, measurement solvent) δ | Remarks |
|---|---|---|
| Aa-168-1 | (300 MHz, CDCl₃) 6.95-6.85 (m, 3H), 6.72 (tt, 1H), 6.66 (d, 1H), 6.29 (dt, 1H), 4.38 (sext, 1H), 4.35 (t, 2H), 3.45 (t, 2H), 2.88 (quin, 1H), 2.71-2.61 (m, 2H), 2.24-2.14 (m, 2H). | cis |
| Aa-169-1 | (300 MHz, CDCl₃) 7.03-6.94 (m, 3H), 6.86 (br, 1H), 6.81 (tt, 1H), 6.38 (dt, 1H), 4.66 (s, 2H), 4.49 (sext, 1H), 3.79 (s, 3H), 3.04 (quin, 1H), 2.82-2.72 (m, 2H), 2.39-2.28 (m, 2H). | cis |
| Aa-170-1 | (300 MHz, CDCl₃) 7.01-6.91 (m, 3H), 6.79 (tt, 1H), 6.72 (d, 1H), 6.36 (dt, 1H), 5.11 (q, 1H), 4.46 (sext, 1H), 3.74 (s, 3H), 2.98 (quin, 1H), 2.81-2.66 (m, 2H), 2.33-2.23 (m, 2H), 1.49 (d, 3H). | cis |
| Aa-171-1 | (300 MHz, CDCl₃) 7.41-7.30 (m, 5H), 7.01-6.91 (m, 3H), 6.72 (tt, 1H), 6.66 (d, 1H), 6.35 (dt, 1H), 5.14 (s, 2H), 4.44 (sext, 1H), 2.95 (quin, 1H), 2.76-2.67 (m, 2H), 2.29-2.17 (m, 2H). | cis |
| Aa-172-1 | (300 MHz, CDCl₃) 8.62 (d, 1H), 7.73 (td, 1H), 7.35 (d, 1H), 7.27 (dd, 1H), 7.04-6.94 (m, 3H), 6.90-6.80 (br, 1H), 6.81 (tt, 1H), 6.38 (dt, 1H), 5.27 (s, 2H), 4.49 (sext, 1H), 3.04 (quin, 1H), 2.81-2.72 (m, 2H), 2.37-2.26 (m, 2H). | cis |
| Aa-173-1 | (300 MHz, CDCl₃) 8.87-8.42 (br, 2H), 7.70 (d, 1H), 7.32 (dd, 1H), 7.03-6.93 (m, 3H), 6.89 (d, 1H), 6.80 (tt, 1H), 6.37 (dt, 1H), 5.17 (s, 2H), 4.46 (sext, 1H), 2.96 (quin, 1H), 2.77-2.65 (m, 2H), 2.32-2.18 (m, 2H). | cis |
| Aa-174-1 | (300 MHz, CDCl₃) 8.63 (d, 2H), 7.26 (d, 2H), 7.04-6.94 (m, 3H), 6.85-6.78 (br, 1H), 6.82 (tt, 1H), 6.38 (dt, 1H), 5.17 (s, 2H), 4.49 (sext, 1H), 3.02 (quin, 1H), 2.82-2.72 (m, 2H), 2.35-2.25 (m, 2H). | cis |
| Aa-175-1 | (300 MHz, CDCl₃) 7.02-6.91 (m, 3H), 6.79 (tt, 1H), 6.72 (d, 1H), 6.36 (dt, 1H), 4.43 (sext, 1H), 4.15 (sep, 1H), 2.84 (quin, 1H), 2.73-2.64 (m, 2H), 2.27-2.16 (m, 2H), 0.75-0.68 (m, 4H). | cis |
| Aa-176-1 | (300 MHz, CDCl₃) 7.32 (d, 1H), 7.04-6.93 (m, 3H), 6.81 (tt, 1H), 6.39 (dt, 1H), 5.85 (ddt, 1H), 5.59 (br, 1H), 5.21 (ddd, 1H), 5.16 (ddd, 1H), 4.48 (sext, 1H), 3.93 (tt, 2H), 2.79-2.63 (m, 3H), 2.37-2.25 (m, 2H). | cis |

**Table 2 (continued)**

| Comp No. | ¹H-NMR (300 MHz or 500 MHz, measurement solvent) δ | Remarks |
|---|---|---|
| Aa-177-1 | (300 MHz, CDCl₃) 7.21 (d, 1H), 7.02-6.92 (m, 3H), 6.78 (tt, 1H), 6.36 (dt, 1H), 5.74 (br, 1H), 4.45 (sext, 1H), 4.07 (dd, 2H), 2.76-2.61 (m, 3H), 2.35-2.23 (m, 3H). | cis |
| Aa-178-1 | (300 MHz, CDCl₃) 7.33 (d, 1H), 7.05-6.93 (m, 3H), 6.81 (tt, 1H), 6.39 (dt, 1H), 5.88 (br, 1H), 4.48 (sext, 1H), 3.48 (d, 4H), 3.38 (s, 3H), 2.78-2.63 (m, 3H), 2.33-2.22 (m, 2H). | cis |
| Aa-179-1 | (300 MHz, CDCl₃) 7.34-7.22 (br, 1H), 7.05-6.95 (m, 3H), 6.81 (tt, 1H), 6.39 (dt, 1H), 5.92 (br, 1H), 4.48 (sext, 1H), 3.51 (q, 2H), 2.79-2.64 (m, 5H), 2.36-2.23 (m, 2H), 2.13 (s, 3H). | cis |
| Aa-180-1 | (300 MHz, CDCl₃) 7.11 (d, 1H), 7.05-6.95 (m, 3H), 6.81 (tt, 1H), 6.38 (dt, 1H), 6.32 (t, 1H), 4.46 (sext, 1H), 4.23 (d, 2H), 2.80 (quin, 1H), 2.77-2.65 (m, 2H), 2.42-2.32 (m, 2H). | cis |
| Aa-181-1 | (300 MHz, CDCl₃) 7.25 (d, 1H), 7.04-6.94 (m, 3H), 6.81 (tt, 1H), 6.39 (dt, 1H), 6.09 (br, 1H), 4.49 (sext, 1H), 4.08 (d, 2H), 3.79 (s, 3H), 2.82 (quin, 1H), 2.74-2.65 (m, 2H), 2.34-2.25 (m, 2H). | cis |
| Aa-182-1 | (300 MHz, CDCl₃) 7.30 (d, 1H), 7.01-6.92 (m, 3H), 6.78 (tt, 1H), 6.36 (dt, 1H), 6.18 (d, 1H), 4.59 (quin, 1H), 4.45 (sext, 1H), 3.75 (s, 3H), 2.75 (quin, 1H), 2.69-2.60 (m, 2H), 2.30-2.21 (m, 2H), 1.40 (d, 3H). | cis |
| Aa-183-1 | (300 MHz, CDCl₃) 7.40-7.27 (m, 6H), 7.04-6.95 (m, 3H), 6.81 (tt, 1H), 6.39 (dt, 1H), 5.82 (br, 1H), 4.55-4.40 (m, 1H), 4.47 (d, 2H), 2.79-2.63 (m, 3H), 2.39-2.28 (m, 2H). | cis |
| Aa-184-1 | (300 MHz, CDCl₃) 8.21-7.90 (br, 1H), 7.33-7.11 (br, 1H), 7.02-6.91 (m, 3H), 6.79 (tt, 1H), 6.36 (dt, 1H), 6.06-5.84 (m, 1H), 5.38-5.32 (m, 2H), 4.57-4.22 (m, 3H), 2.80-2.47 (m, 3H), 2.37-2.20 (m, 2H). | cis |
| Aa-185-1 | (300 MHz, DMSO-ds) 11.79 (br, 1H), 9.20 (d, 1H), 7.49 (d, 2H), 7.39-7.26 (m, 3H), 7.11-6.90 (m, 4H), 6.83 (dt, 1H), 4.30 (sext, 1H), 2.82 (quin, 1H), 2.48-2.30 (m, 4H). | cis |
| Aa-186-1 | (300 MHz, acetone-ds) 10.14 (br, 1H), 8.35 (br, 1H), 7.08-6.98 (m, 3H), 6.73 (tt, 1H), 6.60 (dt, 1H), 4.36 (sext, 1H), 3.86 (s, 3H), 3.65 (s, 3H), 2.68 (quin, 1H), 2.48-2.40 (m, 2H), 2.33-2.23 (m, 2H). | cis |

**Table 2 (continued)**

| Comp No. | ¹H-NMR (300 MHz or 500 MHz, measurement solvent) δ | Remarks |
|---|---|---|
| Aa-187-1 | (300 MHz, CDCl₃) 7.29 (br, 1H), 6.97 (dt, 1H), 6.76-6.73 (m, 2H), 6.59 (dt, 1H), 6.33 (dt, 1H), 5.82 (ddt, 1H), 5.61 (br, 1H), 5.18 (ddd, 1H), 5.13 (ddd, 1H), 4.45 (sext, 1H), 3.89 (tt, 2H), 3.80 (s, 3H), 2.76-2.59 (m, 3H), 2.35-2.24 (m, 2H). | cis |
| Aa-188-1 | (300 MHz, CDCl₃) 7.29 (d, 1H), 6.97 (dt, 1H), 6.76-6.73 (m, 2H), 6.59 (dt, 1H), 6.33 (dt, 1H), 5.52 (br, 1H), 4.44 (sext, 1H), 3.80 (s, 3H), 3.23 (dt, 2H), 2.72-2.59 (m, 3H), 2.31-2.21 (m, 2H), 1.52 (sext, 2H), 0.92 (t, 3H). | cis |
| Aa-189-1 | (300 MHz, CDCl₃) 7.02 (br, 1H), 6.97 (dt, 1H), 6.76-6.73 (m, 2H), 6.59 (dt, 1H), 6.33 (dt, 1H), 4.47 (sext, 1H), 3.80 (s, 3H), 3.66 (s, 3H), 3.30 (quin, 1H), 3.19 (s, 3H), 2.69-2.60 (m, 2H), 2.30-2.20 (m, 2H). | cis |
| Aa-190-1 | (300 MHz, CDCl₃) 6.97 (dt, 1H), 6.76-6.73 (m, 3H), 6.59 (dt, 1H), 6.33 (dt, 1H), 4.44 (sext, 1H), 4.15 (q, 2H), 3.81 (s, 3H), 2.88 (quin, 1H), 2.74-2.65 (m, 2H), 2.27-2.17 (m, 2H), 1.27 (t, 3H). | cis |
| Aa-191-1 | (300 MHz, CDCl₃) 6.97 (dt, 1H), 6.76-6.73 (m, 3H), 6.59 (dt, 1H), 6.33 (dt, 1H), 4.44 (sext, 1H), 4.05 (t, 2H), 3.80 (s, 3H), 2.89 (quin, 1H), 2.74-2.65 (m, 2H), 2.27-2.17 (m, 2H), 1.65 (sext, 2H), 0.94 (t, 3H). | cis |
| Aa-192-1 | (300 MHz, CDCl₃) 6.97 (dt, 1H), 6.76-6.73 (m, 3H), 6.59 (dt, 1H), 6.33 (dt, 1H), 5.01 (sep, 1H), 4.43 (sext, 1H), 3.81 (s, 3H), 2.84 (quin, 1H), 2.73-2.64 (m, 2H), 2.25-2.14 (m, 2H), 1.24 (d, 6H). | cis |
| Aa-193-1 | (300 MHz, CDCl₃) 6.98 (dt, 1H), 6.77-6.74 (m, 2H), 6.70 (d, 1H), 6.60 (dt, 1H), 6.33 (dt, 1H), 4.43 (sext, 1H), 4.15 (sep, 1H), 3.81 (s, 3H), 2.84 (quin, 1H), 2.73-2.64 (m, 2H), 2.26-2.15 (m, 2H), 0.75-0.67 (m, 4H). | cis |
| Aa-194-1 | (300 MHz, CDCl₃) 6.97 (dt, 1H), 6.77-6.73 (m, 3H), 6.59 (dt, 1H), 6.33 (dt, 1H), 5.91 (ddt, 1H), 5.31 (ddd, 1H), 5.25 (ddd, 1H), 4.59 (dt, 2H), 4.44 (sext, 1H), 3.80 (s, 3H), 2.92 (quin, 1H), 2.75-2.65 (m, 2H), 2.30-2.19 (m, 2H). | cis |
| Aa-195-1 | (300 MHz, CDCl₃) 6.97 (dt, 1H), 6.76-6.73 (m, 3H), 6.60 (dt, 1H), 6.33 (dt, 1H), 4.70 (d, 2H), 4.45 (sext, 1H), 3.80 (s, 3H), 2.94 (quin, 1H), 2.77-2.67 (m, 2H), 2.49 (t, 1H), 2.31-2.20 (m, 2H). | cis |

**Table 2 (continued)**

| Comp No. | ¹H-NMR(300 MHz or 500 MHz, measurement solvent) δ | Remarks |
|---|---|---|
| Aa-196-1 | (300 MHz, CDCl₃) 6.98 (dt, 1H), 6.77-6.73 (m, 2H), 6.66 (d, 1H), 6.60 (dt, 1H), 6.33 (dt, 1H), 4.49 (q, 2H), 4.48 (sext, 1H), 3.81 (s, 3H), 3.00 (quin, 1H), 2.81-2.71 (m, 2H), 2.33-2.22 (m, 2H). | cis |
| Aa-197-1 | (300 MHz, CDCl₃) 6.97 (dt, 1H), 6.76-6.73 (m, 2H), 6.70 (br, 1H), 6.60 (dt, 1H), 6.33 (dt, 1H), 5.94 (tt, 1H), 4.46 (sext, 1H), 4.30 (td, 2H), 3.80 (s, 3H), 2.97 (quin, 1H), 2.78-2.68 (m, 2H), 2.32-2.21 (m, 2H). | cis |
| Aa-198-1 | (300 MHz, CDCl₃) 6.97 (dt, 1H), 6.76-6.73 (m, 3H), 6.60 (dt, 1H), 6.33 (dt, 1H), 4.43 (sext, 1H), 4.27-4.24 (m, 2H), 3.80 (s, 3H), 3.61-3.58 (m, 2H), 3.38 (s, 3H), 2.94 (quin, 1H), 2.75-2.66 (m, 2H), 2.28-2.18 (m, 2H). | cis |
| Aa-199-1 | (300 MHz, CDCl₃) 6.97 (dt, 1H), 6.79 (br, 1H), 6.76-6.73 (m, 2H), 6.59 (dt, 1H), 6.33 (dt, 1H), 4.44 (sext, 1H), 4.27 (t, 2H), 3.80 (s, 3H), 2.90 (quin, 1H), 2.75-2.66 (m, 2H), 2.72 (t, 2H), 2.30-2.19 (m, 2H), 2.14 (s, 3H). | cis |
| Aa-200-1 | (300 MHz, CDCl₃) 6.97 (dt, 1H), 6.77-6.73 (m, 2H), 6.70 (br, 1H), 6.60 (dt, 1H), 6.33 (dt, 1H), 4.45 (sext, 1H), 4.31 (t, 2H), 3.81 (s, 3H), 2.95 (quin, 1H), 2.78-2.68 (m, 2H), 2.72 (t, 2H), 2.32-2.21 (m, 2H). | cis |
| Aa-201-1 | (300 MHz, CDCl₃) 7.40-7.31 (m, 5H), 6.97 (dt, 1H), 6.77-6.73 (m, 2H), 6.67 (d, 1H), 6.60 (dt, 1H), 6.32 (dt, 1H), 5.14 (s, 2H), 4.44 (sext, 1H), 3.81 (s, 3H), 2.94 (quin, 1H), 2.76-2.66 (m, 2H), 2.28-2.18 (m, 2H). | cis |
| Aa-202-1 | (300 MHz, CDCl₃) 8.60 (d, 1H), 7.71 (td, 1H), 7.32 (d, 1H), 7.24 (dd, 1H), 6.97 (dt, 1H), 6.80-6.73 (m, 3H), 6.60 (dt, 1H), 6.33 (dt, 1H), 5.25 (s, 2H), 4.46 (sext, 1H), 3.80 (s, 3H), 3.01 (quin, 1H), 2.79-2.69 (m, 2H), 2.34-2.24 (m, 2H). | cis |
| Aa-203-1 | (300 MHz, CDCl₃) 8.61 (d, 1H), 8.58 (dd, 1H), 7.67 (dt, 1H), 7.29 (dd, 1H), 6.95 (dt, 1H), 6.89 (d, 1H), 6.75-6.71 (m, 2H), 6.58 (dt, 1H), 6.32 (dt, 1H), 5.14 (s, 2H), 4.43 (sext, 1H), 3.79 (s, 3H), 2.92 (quin, 1H), 2.74-2.64 (m, 2H), 2.30-2.19 (m, 2H). | cis |
| Aa-204-1 | (300 MHz, CDCl₃) 8.58 (d, 2H), 7.21 (d, 2H), 7.01 (d, 1H), 6.94 (dt, 1H), 6.74-6.71 (m, 2H), 6.58 (dt, 1H), 6.32 (dt, 1H), 5.12 (s, 2H), 4.45 (sext, 1H), 3.78 (s, 3H), 2.98 (quin, 1H), 2.76-2.67 (m, 2H), 2.34-2.23 (m, 2H). | cis |

**Table 2 (continued)**

| Comp No. | ¹H-NMR (300 MHz or 500 MHz, measurement solvent) δ | Remarks |
|---|---|---|
| Aa-205-1 | (300 MHz, CDCl₃) 6.97 (dt, 1H), 6.77-6.73 (m, 2H), 6.70 (br, 1H), 6.60 (dt, 1H), 6.33 (dt, 1H), 4.45 (sext, 1H), 4.36 (t, 2H), 3.81 (s, 3H), 3.69 (t, 2H), 2.95 (quin, 1H), 2.77-2.68 (m, 2H), 2.31-2.20 (m, 2H). | cis |
| Aa-206-1 | (300 MHz, CDCl₃) 6.97 (dt, 1H), 6.76-6.73 (m, 3H), 6.59 (dt, 1H), 6.33 (dt, 1H), 4.45 (sext, 1H), 4.41 (t, 1H), 4.36 (t, 1H), 3.80 (s, 3H), 3.69 (t, 1H), 3.52 (t, 1H), 3.00-2.88 (m, 1H), 2.78-2.68 (m, 2H), 2.31-2.21 (m, 2H). | cis |
| Aa-207-1 | (300 MHz, CDCl₃) 6.96 (dt, 1H), 6.85 (br, 1H), 6.76-6.72 (m, 2H), 6.58 (dt, 1H), 6.32 (dt, 1H), 4.63 (s, 2H), 4.46 (sext, 1H), 3.79 (s, 3H), 3.76 (s, 3H), 3.00 (quin, 1H), 2.78-2.69 (m, 2H), 2.36-2.26 (m, 2H). | cis |
| Aa-208-1 | (300 MHz, CDCl₃) 6.98-6.93 (br, 1H), 6.94 (dt, 1H), 6.74-6.71 (m, 2H), 6.57 (dt, 1H), 6.31 (dt, 1H), 5.08 (q, 1H), 4.44 (sext, 1H), 3.77 (s, 3H), 3.72 (s, 3H), 2.95 (quin, 1H), 2.78-2.61 (m, 2H), 2.33-2.22 (m, 2H), 1.47 (d, 3H). | cis |
| Aa-209-1 | (300 MHz, CDCl₃) 7.03-6.93 (m, 3H), 6.86 (d, 1H), 6.70 (d, 1H), 6.33 (dt, 1H), 4.45 (sext, 1H), 3.93 (d, 2H), 2.91 (quin, 1H), 2.76-2.67 (m, 2H), 2.35 (s, 3H), 2.28-2.17 (m, 2H), 1.19-1.06 (m, 1H), 0.61-0.55 (m, 2H), 0.30-0.25 (m, 2H). | cis |
| Aa-210-1 | (300 MHz, DMSO-ds) 11.69 (s, 1H), 9.11 (d, 1H), 7.34-7.29 (m, 2H), 7.04 (d, 1H), 6.98 (d, 1H), 6.65 (dt, 1H), 4.19 (sext, 1H), 3.23 (s, 3H), 2.84 (quin, 1H), 2.43-2.20 (m, 4H), 2.32 (s, 3H). | cis |
| Aa-211-1 | (300 MHz, CDCl₃) 7.03-6.93 (m, 3H), 6.86 (d, 1H), 6.64 (d, 1H), 6.32 (dt, 1H), 4.48 (sext, 1H), 3.35 (quin, 1H), 3.26 (s, 3H), 3.24 (s, 3H), 2.79-2.70 (m, 2H), 2.40-2.29 (m, 2H), 2.35 (s, 3H). | cis |
| Aa-212-1 | (300 MHz, CDCl₃) 8.61 (dd, 2H), 7.23 (dd, 2H), 7.03-6.92 (m, 3H), 6.86 (d, 1H), 6.69 (d, 1H), 6.32 (dt, 1H), 5.15 (s, 2H), 4.47 (sext, 1H), 3.00 (quin, 1H), 2.80-2.70 (m, 2H), 2.35 (s, 3H), 2.33-2.22 (m, 2H). | cis |
| Aa-213-1 | (300 MHz, DMSO-ds) 10.42 (s, 1H), 9.12 (d, 1H), 8.74 (s, 1H), 7.34-7.29 (m, 2H), 7.12-.6.95 (m, 2H), 6.65 (dt, 1H), 4.16 (sext, 1H), 2.59-2.48 (m, 1H), 2.33 (s, 3H), 2.29-2.20 (m, 4H). | cis |

**Table 2 (continued)**

| Comp No. | ¹H-NMR (300 MHz or 500 MHz, measurement solvent) δ | Remarks |
|---|---|---|
| Aa-214-1 | (300 MHz, CDCl₃) 7.03-6.93 (m, 3H), 6.86 (d, 1H), 6.66 (d, 1H), 6.33 (dt, 1H), 4.44 (sext, 1H), 4.17 (t, 2H), 3.81 (t, 2H), 2.91 (quin, 1H), 2.75-2.66 (m, 2H), 2.35 (s, 3H), 2.28-2.18 (m, 2H), 0.89 (s, 9H), 0.06 (s, 6H). | cis |
| Aa-215-1 | (300 MHz, CDCl₃) 7.03-6.93 (m, 3H), 6.86 (d, 1H), 6.73 (d, 1H), 6.33 (dt, 1H), 4.45 (sext, 1H), 4.26-4.23 (m, 2H), 3.88-3.83 (m, 2H), 2.94 (quin, 1H), 2.77-2.67 (m, 2H), 2.35 (s, 3H), 2.31-2.21 (m, 2H), 1.91 (t, 1H). | cis |
| Aa-216-1 (HCl salt) | (300 MHz, DMSO-d₆) 9.14 (d, 1H), 8.06 (br, 3H), 7.34-7.29 (m, 2H), 7.07-.6.96 (m, 2H), 6.66 (dt, 1H), 4.24 (sext, 1H), 4.21 (t, 2H), 3.08 (t, 2H), 2.91 (quin, 1H), 2.45-2.27 (m, 4H), 2.33 (s, 3H). | cis, hydrochloride of Aa-216-1 |
| Aa-217-1 | (300 MHz, CDCl₃) 7.02-6.93 (m, 3H), 6.80 (tt, 1H), 6.58 (br, 1H), 6.35 (dt, 1H), 4.46 (sext, 1H), 3.12 (quin, 1H), 2.81-2.71 (m, 2H), 2.41-2.30 (m, 2H). | cis |
| Aa-218-1 | (300 MHz, CDCl₃) 7.23 (br, 1H), 7.02-6.93 (m, 3H), 6.79 (tt, 1H), 6.37 (dt, 1H), 6.03 (br, 1H), 4.47 (sext, 1H), 3.95 (d, 2H), 2.80-2.62 (m, 3H), 2.33-2.23 (m, 2H), 1.48 (s, 9H). | cis |
| Aa-219-3 | (300 MHz, CDCl₃) 7.03-6.84 (m, 5H), 6.34 (dt, 1H), 4.97 (d, 0.1H), 4.86 (d, 0.9H), 4.54 (sext, 0.1H), 4.42 (sext, 0.9H), 4.04-3.89 (m, 4H), 2.56-2.41 (m, 3H), 2.35 (s, 3H), 2.13-2.09 (m, 0.2H), 1.95-1.85 (m, 1.8H). | cis-trans isomer |
| Aa-220-3 | (300 MHz, CDCl₃) 7.13 (dd, 0.1H), 7.03-6.93 (m, 3.9H), 6.86 (d, 1H), 6.46 (d, 1H), 6.33 (dt, 1H), 5.86 (dd, 0.1H), 5.78 (dd, 0.9H), 4.51 (sext, 0.1H), 4.39 (sext, 0.9H), 3.75 (s, 0.3H), 3.74 (s, 2.7H), 2.90-2.76 (m, 1H), 2.73-2.63 (m, 2H), 2.35 (s, 3H), 1.96-1.85 (m, 2H). | cis-trans isomer |
| Aa-221-3 | (300 MHz, CDCl₃) 9.85 (d, 0.1H), 9.75 (d, 0.9H), 7.03-6.93 (m, 3H), 6.86 (d, 1H), 6.58 (br, 1H), 6.32 (dt, 1H), 4.49 (sext, 0.9H), 4.48-4.39 (m, 0.1H), 3.21-3.10 (m, 0.1H), 3.10-2.99 (m, 0.9H), 2.82-2.73 (m, 0.2H), 2.69-2.59 (m, 1.8H), 2.35 (s, 3H), 2.28-2.17 (m, 2H). | cis-trans isomer |

**Table 2 (continued)**

| Comp No. | ¹H-NMR (300 MHz or 500 MHz, measurement solvent) δ | Remarks |
|---|---|---|
| Aa-222-3 | (300 MHz, CDCl₃) 7.03-6.92 (m, 3H), 6.86 (d, 1H), 6.71 (d, 1H), 6.33 (dt, 1H), 4.51-4.40 (m, 0.1H), 4.35 (sext, 0.9H), 4.27 (d, 1H), 3.37 (s, 5.4H), 3.36 (s, 0.6H), 2.54-2.37 (m, 3H), 2.35 (s, 3H), 1.93-1.83 (m, 2H). | cis-trans isomer |
| Aa-223-3 | (300 MHz, CDCl₃) 7.13 (dd, 0.2H), 7.01-6.94 (m, 3.8H), 6.79 (tt, 1H), 6.61-6.53 (br, 0.2H), 6.52-6.41 (br, 0.8H), 6.36 (dt, 1H), 5.86 (dd, 0.2H), 5.78 (dd, 0.8H), 4.52 (sext, 0.2H), 4.39 (sext, 0.8H), 3.73 (s, 3H), 3.19-3.04 (m, 0.2H), 2.91-2.77 (m, 0.8H), 2.73-2.63 (m, 1.5H), 2.47-2.28 (m, 1H), 1.90 (qd, 1.5H). | cis-trans isomer |
| Aa-224-3 | (300 MHz, CDCl₃) 7.02-6.94 (m, 3H), 6.85 (dd, 1H), 6.79 (tt, 1H), 6.57 (br, 0.3H), 6.47 (d, 0.7H), 6.37 (dt, 1H), 5.78 (dd, 0.3H), 5.70 (dd, 0.7H), 4.51 (sext, 0.3H), 4.37 (sext, 0.7H), 3.15-3.00 (m, 0.3H), 2.88-2.74 (m, 0.7H), 2.71-2.62 (m, 1.5H), 2.47-2.26 (m, 1H), 1.89 (qd, 1.5H), 1.49 (s, 2.7H), 1.48 (s, 6.3H). | cis-trans isomer |
| Aa-225-3 | (300 MHz, CDCl₃) 7.07 (dd, 1H), 7.02-6.91 (m, 3H), 6.80 (tt, 1H), 6.58 (d, 0.2H), 6.49 (d, 0.8H), 6.36 (dt, 1H), 5.87 (dd, 0.2H), 5.79 (dd, 0.8H), 4.53 (sext, 0.2H), 4.53 (sext, 0.8H), 3.21-3.08 (m, 0.2H), 2.91-2.81 (m, 0.8H), 2.75-2.66 (m, 2H), 2.50-2.31 (m, 1H), 1.93 (qd, 2H). | cis-trans isomer |
| Aa-223-2 | (300 MHz, CDCl₃) 7.14 (dd, 1H), 7.02-6.93 (m, 3H), 6.80 (tt, 1H), 6.58 (d, 1H), 6.37 (dt, 1H), 5.86 (dd, 1H), 4.52 (sext, 1H), 3.75 (s, 3H), 3.19-3.08 (m, 1H), 2.47-2.28 (m, 4H). | trans |
| Aa-220-2 | (300 MHz, CDCl₃) 7.14 (dd, 1H), 7.04-6.93 (m, 3H), 6.87 (d, 1H), 6.57 (br, 1H), 6.34 (dt, 1H), 5.86 (dd, 1H), 4.52 (sext, 1H), 3.77 (s, 3H), 3.18-3.05 (m, 1H), 2.47-2.28 (m, 4H), 2.38 (s, 3H). | trans |
| Aa-226-1 | (300 MHz, CDCl₃) 7.01-6.94 (m, 3H), 6.79 (tt, 1H), 6.46 (d, 1H), 6.35 (dt, 1H), 4.25 (sext, 1H), 3.67 (s, 3H), 2.58-2.52 (m, 2H), 2.25 (t, 2H), 2.08-1.95 (m, 1H), 1.75 (dd, 2H), 1.56 (ddd, 2H). | cis |
| Aa-226-2 | (300 MHz, CDCl₃) 7.01-6.91 (m, 3H), 6.79 (tt, 1H), 6.54 (d, 1H), 6.37 (dt, 1H), 4.47 (sext, 1H), 3.67 (s, 3H), 2.33-2.23 (m, 3H), 2.20-2.06 (m, 4H), 1.84 (q, 2H). | trans |

**Table 2 (continued)**

| Comp No. | ¹H-NMR (300 MHz or 500 MHz, measurement solvent) δ | Remarks |
|---|---|---|
| Aa-227-1 | (300 MHz, CDCl₃) 7.06 (d, 1H), 7.02-6.91 (m, 3H), 6.80 (tt, 1H), 6.49 (d, 1H), 6.36 (dt, 1H), 4.39 (sext, 1H), 3.83 (s, 3H), 3.26-3.12 (m, 1H), 2.84-2.75 (m, 2H), 2.03-1.93 (m, 2H). | cis |
| Aa-228-2 | (300 MHz, CDCl₃) 7.03-6.92 (m, 2H), 6.86 (d, 1H), 7.01 (tt, 1H), 6.54 (d, 1H), 6.33 (dt, 1H), 4.47 (sext, 1H), 3.67 (s, 3H), 2.35 (s, 3H), 2.27 (t, 3H), 2.16-2.08 (m, 4H), 1.84 (q, 2H). | trans |
| Aa-229-2 | (300 MHz, CDCl₃) 7.03-6.91 (m, 2H), 6.86 (d, 1H), 7.01 (tt, 1H), 6.53 (d, 1H), 6.33 (dt, 1H), 4.48 (sext, 1H), 2.35 (s, 3H), 2.35-2.24 (m, 3H), 2.21-2.09 (m, 4H), 1.86 (q, 2H). No proton peak of carboxy group detected. | trans |
| Aa-230-3 | (300 MHz, CDCl₃) 7.01-6.91 (m, 3H), 6.80 (tt, 1H), 6.58 (br, 1H), 6.35 (dt, 1H), 4.69 (sext, 0.1H), 4.40 (sext, 0.9H), 3.78 (s, 0.3H), 3.78 (s, 2.7H), 3.25-3.15 (m, 0.1H), 2.97-2.78 (m, 2.6H), 2.70-2.60 (m, 0.3H), 2.50-2.35 (m, 0.2H), 2.29-2.17 (m, 1.8H). | cis-trans isomer |
| Aa-231 | (300 MHz, CDCl₃) 7.20 (dd, 1H), 7.03-6.91 (m, 3H), 6.81 (tt, 1H), 6.65 (d, 1H), 6.38 (dt, 1H), 6.08 (dt, 1H), 5.79 (d, 1H), 4.52 (sext, 1H), 3.75 (s, 3H), 3.47-3.37 (m, 1H), 3.31-3.22 (m, 1H), 2.90-2.81 (m, 2H). | - |
| Aa-232-3 | (300 MHz, CDCl₃) 7.02-6.92 (m, 3H), 6.79 (tt, 1H), 6.56 (br, 0.5H), 6.45 (br, 0.5H), 6.37 (dt, 0.5H), 6.36 (dt, 0.5H), 4.46 (sext, 0.5H), 4.26 (sext, 0.5H), 3.65 (s, 3H), 2.60-2.51 (m, 1H), 2.34-2.05 (m, 4.5H), 2.02-1.91 (m, 0.5H), 1.64-1.40 (m, 5H). | cis-trans isomer |
| Aa-233-1 | (300 MHz, CDCl₃) 7.00-6.92 (m, 3H), 6.79 (tt, 1H), 6.55 (br, 1H), 6.35 (dt, 1H), 4.53 (sext, 1H), 3.88 (s, 3H), 3.66 (quin, 1H), 2.80-2.70 (m, 2H), 2.29-2.19 (m, 2H). | cis |
| Aa-234-1 | (300 MHz, CDCl₃) 7.02-6.93 (m, 3H), 6.84 (br, 1H), 6.80 (tt, 1H), 6.37 (dt, 1H), 4.45 (sext, 1H), 4.04 (s, 3H), 3.87 (s, 3H), 3.54 (quin, 1H), 2.82-2.72 (m, 2H), 2.40-2.30 (m, 2H). | cis |

**Table 2 (continued)**

| Comp No. | ¹H-NMR (300 MHz or 500 MHz, measurement solvent) δ | Remarks |
|---|---|---|
| Aa-235-1 | (300 MHz, CDCl₃) 7.02-6.94 (m, 3H), 6.80-6.70 (br, 1H), 6.79 (tt, 1H), 6.36 (dt, 1H), 4.36 (sext, 1H), 4.15 (t, 1H), 3.79 (s, 3H), 3.00 (d, 1H), 2.60-2.47 (m, 2H), 2.41-2.30 (m, 1H), 2.13-2.02 (m, 1H), 1.90 (dt, 1H). | cis |
| Aa-236-1 | (300 MHz, CDCl₃) 7.12-7.09 (m, 2H), 7.01 (tt, 1H), 6.95 (d, 1H), 6.57 (d, 1H), 6.38 (dt, 1H), 4.53 (sext, 1H), 3.89 (s, 3H), 3.66 (quin, 1H), 2.79-2.70 (m, 2H), 2.30-2.19 (m, 2H). | cis |
| Aa-237-3 | (300 MHz, CDCl₃) 7.04-6.94 (m, 3H), 6.79 (tt, 1H), 6.48 (d, 1H), 6.39-6.33 (m, 1H), 4.32-4.20 (m, 1H), 4.14 (q, 2H), 2.62-2.49 (m, 2H), 2.40-2.32 (m, 1H), 2.18-1.95 (m, 1H), 1.85-1.73 (m, 1H), 1.60-1.49 (m, 3H), 1.27 (t, 3H), 1.14 (d, 3H). | cis-trans isomer |
| Aa-238-1 | (300 MHz, CDCl₃) 8.11 (s, 1H), 7.44 (dd 1H), 7.13 (dd, 1H), 7.03 (s, 1H), 6.98-6.85 (m, 3H), 6.65 (d, 1H), 6.35 (dt, 1H), 4.58 (sext, 1H), 3.51 (quin, 1H), 2.89-2.82 (m, 2H), 2.54-2.44 (m, 2H), 2.35 (s, 3H). | cis |
| Aa-239-1 | (300 MHz, CDCl₃) 10.03 (s, 1H), 8.96 (dd, 1H), 8.58 (dd, 1H), 7.55 (dd, 1H), 7.04 (s, 1H), 6.98-6.85 (m, 4H), 6.35 (dt, 1H), 4.55 (sext, 1H), 3.37 (quin, 1H), 2.85-2.75 (m, 2H), 2.41-2.35 (m, 2H), 2.38 (s, 3H). | cis |
| Aa-240-1 | (300 MHz, CDCl₃) 8.12 (s, 1H), 7.44 (dd, 1H), 7.13 (dd, 1H), 7.02-6.95 (m, 3H), 6.79 (tt, 1H), 6.78 (d, 1H), 6.38 (dt, 1H), 4.66 (sext, 1H), 3.50 (quin, 1H), 2.89-2.84 (m, 2H), 2.54-2.47 (m, 2H). | cis |
| Aa-241-1 | (300 MHz, CDCl₃) 10.03 (s, 1H), 8.96 (dd, 1H), 8.55 (dd, 1H), 7.55 (dd, 1H), 7.04-6.91 (m, 4H), 6.80 (tt, 1H), 6.35 (dt, 1H), 4.55 (sext, 1H), 3.37 (quin, 1H), 2.82-2.72 (m, 2H), 2.42-2.32 (m, 2H). | cis |
| Aa-242-1 | (500 MHz, CDCl₃) 7.32-7.22 (m, 5H), 7.03 (s, 1H), 7.00-6.93 (m, 2H), 6.86 (d, 1H), 6.62 (brd, 1H), 6.36-6.27 (m, 1H), 4.47-4.38 (m, 1H), 4.14 (s, 2H), 3.16-3.08 (m, 1H), 2.73-2.66 (m, 2H), 2.34 (s, 3H), 2.29-2.22 (m, 2H). | cis |
| Aa-243-1 | (500 MHz, CDCl₃) 7.03 (s, 1H), 7.00-6.92 (m, 2H), 6.85 (d, 1H), 6.65 (brd, 1H), 6.36-6.27 (m, 1H), 4.46-4.37 (m, 1H), 3.76-3.69 (m, 1H), 3.11-3.03 (m, 1H), 2.71-2.64 (m, 2H), 2.34 (s, 3H), 2.26-2.19 (m, 2H), 2.13-2.06 (m, 2H), 1.71-1.48 (m, 6H). | cis |

**Table 2 (continued)**

| Comp No. | ¹H-NMR (300 MHz or 500 MHz, measurement solvent) δ | Remarks |
|---|---|---|
| Aa-244-1 | (500 MHz, DMSO-ds) 11.65 (s, 1H), 9.10 (d, 1H), 7.46-7.42 (m, 2H), 7.26-7.21 (m, 1H), 7.02 (d, 1H), 6.94 (brs 1H), 6.82-6.73 (m, 2H), 4.26-4.17 (m, 1H), 3.18-3.10 (m, 1H), 2.55-2.49 (m, 2H), 2.34-2.27 (m, 2H). | cis |
| Aa-245-1 | (500 MHz, CDCl₃) 7.42 (d, 1H), 7.38-7.34 (m, 2H), 7.28-7.26 (m, 1H), 7.08-7.03 (m, 1H), 6.87 (brd, 1H), 6.41-6.32 (m, 1H), 4.49-4.40 (m, 1H), 3.70 (s, 3H), 3.37 (q, 2H), 2.93-2.85 (m, 1H), 2.73-2.67 (m, 2H), 2.26-2.19 (m, 2H). | cis |
| Aa-245-2 | (500 MHz, CDCl₃) 7.44-7.35 (m, 3H), 7.28 (d, 1H), 7.06 (dt, 1H), 6.52 (d, 1H), 6.38 (dt, 1H), 4.63 (sext, 1H), 3.73 (s, 3H), 3.37 (q, 2H), 3.14-3.08 (m, 1H), 2.73-2.70 (m, 2H), 2.36-2.30 (m, 2H). | trans |
| Aa-246-1 | (500 MHz, CDCl₃) 7.57 (s, 1H), 7.50-7.46 (m, 2H), 7.42 (t, 1H), 7.12-7.06 (m, 1H), 6.68 (brd, 1H), 6.44-6.35 (m, 1H), 4.49-4.40 (m, 1H), 3.70 (s, 3H), 2.93-2.85 (m, 1H), 2.73-2.67 (m, 2H), 2.26-2.19 (m, 2H), 1.91 (t, 3H). | cis |
| Aa-247-1 | (500 MHz, DMSO-ds) 9.14 (d, 1H), 7.42 (d, 2H), 7.25-7.20 (m, 1H), 7.02 (d, 1H), 6.96 (s, 1H), 6.80-6.71 (m, 2H), 4.27-4.21 (m, 1H), 3.46 (s, 3H), 3.26-3.18 (m, 1H), 2.61-2.50 (m, 2H), 2.36-2.29 (m, 2H). | cis |
| Aa-248-1 | (500 MHz, DMSO-ds) 9.13 (d, 1H), 7.45-7.41 (m, 2H), 7.25-7.20 (m, 1H), 7.14 (d, 1H), 7.02 (d, 1H), 6.81-6.72 (m, 2H), 5.15 (s, 2H), 4.27-4.18 (m, 1H), 3.31-3.23 (m, 1H), 3.11 (s, 3H), 2.55-2.46 (m, 2H), 2.40-2.33 (m, 2H). | cis |
| Aa-249-1 | (500 MHz, DMSO-ds) 9.09 (d, 1H), 7.45 (d, 1H), 7.43-7.41 (m, 2H), 7.25-7.20 (m, 1H), 7.01 (d, 1H), 6.89 (d, 1H), 6.80-6.71 (m, 1H), 4.29-4.20 (m, 1H), 3.89 (s, 3H), 3.61-3.53 (m, 1H), 2.61-2.51 (m, 2H), 2.41-2.32 (m, 2H). | cis |
| Aa-250-3 | (500 MHz, CDCl₃) 7.73 (d, 0.5H), 7.72 (d, 0.5H), 7.24 (d, 0.5H), 7.22 (d, 0.5H), 7.18-7.13 (m, 0.5H), 7.01-6.95 (m, 3H), 6.81-6.76 (m, 1H), 6.64-6.59 (m, 0.5H), 6.42-6.33 (m, 1H), 4.74-4.68 (m, 0.5H), 4.57-4.49 (m, 0.5H), 3.93-3.86 (m, 0.5H), 3.66-3.58 (m, 0.5H), 3.01-2.95 (m, 1H), 2.88-2.82 (m, 1H), 2.63-2.56 (m, 1H), 2.41-2.24 (m, 1H). | cis-trans isomer |

**Table 2 (continued)**

| Comp No. | ¹H-NMR (300 MHz or 500 MHz, measurement solvent) δ | Remarks |
|---|---|---|
| Aa-251-1 | (500 MHz, CDCl₃) 6.99-6.94 (m, 3H), 6.84-6.76 (m, 2H), 6.39-6.30 (m, 1H), 4.51-4.39 (m, 1H), 4.34-4.16 (m, 2H), 4.10 (s, 1.6H), 3.97 (s, 0.4H), 3.18-3.10 (m, 0.8H), 3.01 (s, 2.4H), 2.99 (s, 0.6H), 2.94-2.87 (m, 0.2H), 2.74-2.69 (m, 1.6H), 2.64-2.57 (m, 0.4H), 2.32-2.24 (m, 2H), 1.30-1.25 (m, 3H). | cis |
| Aa-252-1 | (500 MHz, CDCl₃) 6.99-6.94 (m, 3H), 6.80-6.76 (m, 2H), 6.39-6.31 (m, 1H), 4.51-4.46 (m, 1H), 4.31 (s, 2H), 4.20 (q, 2H), 3.69 (s, 3H), 3.34-3.30 (m, 1H), 2.75-2.68 (m, 2H), 2.35-2.28 (m, 2H), 1.27 (t, 3H). | cis |
| Aa-253-1 | (500 MHz, CDCl₃) 7.12 (brd, 1H), 7.00-6.93 (m, 3H), 6.80-6.76 (m, 1H), 6.43 (brd, 1H), 6.40-6.31 (m, 1H), 6.23 (brs 1H), 4.49-4.43 (m, 1H), 4.07 (d, 2H), 4.01 (d, 2H), 3.77 (s, 3H), 2.84-2.76 (m, 1H), 2.71-2.65 (m, 2H), 2.31-2.24 (m, 2H). | cis |
| Aa-254-1 | (500 MHz, CDCl₃) 7.00-6.93 (m, 3H), 6.81-6.76 (m, 2H), 6.40-6.31 (m, 1H), 4.52-4.43 (m, 1H), 4.37 (d, 2H), 3.79 (s, 3H), 3.32-3.26 (m, 1H), 2.71-2.65 (m, 2H), 2.29-2.22 (m, 3H). | cis |
| Aa-255-1 | (500 MHz, CDCl₃) 7.07-6.96 (m, 4H), 6.71 (d, 1H), 6.27 (dt, 1H), 4.45 (sext, 1H), 3.91 (s, 3H), 3.70 (s, 3H), 2.90 (quin, 1H), 2.73-2.68 (m, 2H), 2.26-2.20 (m, 2H). | cis |
| Aa-256-1 | (500 MHz, CDCl₃) 7.70-7.67 (m, 2H), 7.23 (d, 1H), 7.02 (dt, 1H), 6.74 (d, 1H), 6.35 (dt, 1H), 4.44 (sext, 1H), 3.71 (s, 3H), 2.91 (quin, 1H), 2.74-2.68 (m, 2H), 2.26-2.20 (m, 2H). | cis |
| Aa-257-1 | (500 MHz, CDCl₃) 6.94 (dt, 1H), 6.88-6.85 (m, 1H), 6.81 (d, 1H), 6.76 (d, 1H), 6.27 (dt, 1H), 4.44 (sext, 1H), 3.92 (s, 3H), 3.70 (s, 3H), 2.90 (quin, 1H), 2.73-2.67 (m, 2H), 2.26-2.20 (m, 2H). | cis |
| Aa-257-2 | (500 MHz, CDCl₃) 6.94 (dt, 1H), 6.88-6.84 (m, 1H), 6.81 (d, 1H), 6.55 (d, 1H), 6.28 (dt, 1H), 4.62 (sext, 1H), 3.92 (s, 3H), 3.73 (s, 3H), 3.13-3.08 (m, 1H), 2.36-2.29 (m, 2H), 2.75-2.70 (m, 2H). | trans |
| Aa-258-1 | (500 MHz, CDCl₃) 7.68 (d, 1H), 7.63-7.61 (m, 1H), 7.21 (t, 1H), 7.05 (dt, 1H), 6.73 (d, 1H), 6.35 (dt, 1H), 4.44 (sext, 1H), 3.71 (s, 3H), 2.90 (quin, 1H), 2.74-2.68 (m, 2H), 2.26-2.20 (m, 2H). | cis |
| Aa-259-1 | (500 MHz, CDCl₃) 7.94 (s, 2H), 7.90 (s, 1H), 7.07 (dt, 1H), 6.83 (d, 1H), 6.52 (dt, 1H), 4.44 (sext, 1H), 3.71 (s, 3H), 2.91 (quin, 1H), 2.74-2.68 (m, 2H), 2.27-2.21 (m, 2H). | cis |

**Table 2 (continued)**

| Comp No. | ¹H-NMR (300 MHz or 500 MHz, measurement solvent) δ | Remarks |
|---|---|---|
| Aa-259-2 | (500 MHz, CDCl₃) 7.93 (s, 2H), 7.90 (s, 1H), 7.07 (dt, 1H), 6.55-6.48 (m, 2H), 4.62 (sext, 1H), 3.73 (s, 3H), 3.15-3.09 (m, 1H), 2.76-2.71 (m, 2H), 2.37-2.30 (m, 2H). | trans |
| Aa-260-1 | (500 MHz, CDCl₃) 7.01-6.94 (m, 3H), 6.80 (tt, 1H), 6.66 (d, 1H), 6.36 (dt, 1H), 5.45 (quin, 1H), 4.90 (t, 2H), 4.63 (dd, 2H), 4.46 (sext, 1H), 2.95 (quin, 1H), 2.77-2.71 (m, 2H), 2.26-2.21 (m, 2H). | cis |
| Aa-261-2 | (500 MHz, CDCl₃) 7.03 (s, 1H), 6.99 (dt, 1H), 6.94 (d, 1H), 6.87 (d, 1H), 6.53 (d, 1H), 6.33 (dt, 1H), 5.47 (quin, 1H), 4.91 (t, 2H), 4.65-4.58 (m, 3H), 3.19-3.13 (m, 1H), 2.76-2.71 (m, 2H), 2.40-2.34 (m, 2H), 2.35 (s, 3H). | trans |
| Aa-262-1 | (500 MHz, CDCl₃) 7.66 (s, 1H), 7.60 (s, 1H), 7.60 (s, 1H), 7.01 (dt, 1H), 6.73 (d, 1H), 6.44 (dt, 1H), 4.44 (sext, 1H), 3.71 (s, 3H), 2.91 (quin, 1H), 2.74-2.68 (m, 2H), 2.26-2.20 (m, 2H). | cis |
| Aa-262-2 | (500 MHz, CDCl₃) 7.66 (s, 1H), 7.61 (s, 1H), 7.60 (s, 1H), 7.01 (dt, 1H), 6.54 (d, 1H), 6.44 (dt, 1H), 4.62 (sext, 1H), 3.73 (s, 3H), 3.14-3.09 (m, 1H), 2.76-2.70 (m, 2H), 2.36-2.30 (m, 2H). | trans |
| Aa-263-1 | (300 MHz, CDCl₃) 7.38 (s, 1H), 7.37 (s, 1H), 7.29 (s, 1H), 7.06 (dt, 1H), 6.70 (d, 1H), 6.60 (t, 1H), 6.39 (dt, 1H), 4.44 (sext, 1H), 3.71 (s, 3H), 2.90 (quin, 1H), 2.75-2.66 (m, 2H), 2.40 (s, 3H), 2.28-2.18 (m, 2H). | cis |
| Aa-263-2 | (500 MHz, CDCl₃) 7.37 (s, 1H), 7.36 (s, 1H), 7.29 (s, 1H), 7.05 (dt, 1H), 6.60 (t, 1H), 6.52 (d, 1H), 6.39 (dt, 1H), 4.62 (sext, 1H), 3.72 (s, 3H), 3.13-3.08 (m, 1H), 2.75-2.70 (m, 2H), 2.40 (s, 3H), 2.35-2.29 (m, 2H). | trans |
| Aa-264-1 | (300 MHz, CDCl₃) 7.11 (s, 1H), 7.00 (dt, 1H), 6.99 (s, 1H), 6.92 (s, 1H), 6.68 (d, 1H), 6.49 (t, 1H), 6.34 (dt, 1H), 4.44 (sext, 1H), 3.71 (s, 3H), 2.90 (quin, 1H), 2.75-2.66 (m, 2H), 2.36 (s, 3H), 2.27-2.18 (m, 2H). | cis |
| Aa-265-1 | (500 MHz, CDCl₃) 7.91 (s, 1H), 7.90 (s, 1H), 7.88 (s, 1H), 7.11 (dt, 1H), 6.81 (d, 1H), 6.52 (dt, 1H), 4.45 (sext, 1H), 3.71 (s, 3H), 2.91 (quin, 1H), 2.74-2.69 (m, 2H), 2.27-2.21 (m, 2H). | cis |
| Aa-265-2 | (500 MHz, CDCl₃) 7.91 (s, 1H), 7.89 (s, 1H), 7.88 (s, 1H), 7.11 (dt, 1H), 6.58 (d, 1H), 6.52 (dt, 1H), 4.63 (sext, 1H), 3.73 (s, 3H), 3.14-3.09 (m, 1H), 2.76-2.70 (m, 2H), 2.37-2.30 (m, 2H). | trans |

**Table 2 (continued)**

| Comp No. | ¹H-NMR (300 MHz or 500 MHz, measurement solvent) δ | Remarks |
|---|---|---|
| Aa-266-1 | (500 MHz, CDCl₃) 7.15-7.13 (m, 2H), 7.05-7.00 (m, 2H), 6.70 (d, 1H), 6.37 (dt, 1H), 4.44 (sext, 1H), 3.71 (s, 3H), 3.37 (q, 2H), 2.90 (quin, 1H), 2.74-2.68 (m, 2H), 2.26-2.20 (m, 2H). | cis |
| Aa-266-2 | (500 MHz, CDCl₃) 7.14-7.13 (m, 2H), 7.05-7.00 (m, 2H), 6.54 (d, 1H), 6.38 (dt, 1H), 4.62 (sext, 1H), 3.73 (s, 3H), 3.37 (q, 2H), 3.14-3.08 (m, 1H), 2.75-2.70 (m, 2H), 2.36-2.30 (m, 2H). | trans |
| Aa-267-1 | (500 MHz, CDCl₃) 7.82 (s, 1H), 7.80 (s, 1H), 7.76 (s, 1H), 7.10 (dt, 1H), 6.76 (d, 1H), 6.68 (t, 1H), 6.49 (dt, 1H), 4.44 (sext, 1H), 3.71 (s, 3H), 2.91 (quin, 1H), 2.74-2.69 (m, 2H), 2.27-2.21 (m, 2H). | cis |
| Aa-267-2 | (500 MHz, CDCl₃) 7.82 (s, 1H), 7.81 (s, 1H), 7.77 (s, 1H), 7.09 (dt, 1H), 6.68 (t, 1H), 6.58-6.48 (m, 2H), 4.63 (sext, 1H), 3.73 (s, 3H), 3.14-3.09 (m, 1H), 2.76-2.71 (m, 2H), 2.36-2.30 (m, 2H). | trans |
| Aa-268-1 | (500 MHz, CDCl₃) 7.13 (s, 1H), 7.06-6.99 (m, 3H), 6.70 (d, 1H), 6.34 (dt, 1H), 5.26 (dd, 1H), 4.44 (sext, 1H), 3.70 (s, 3H), 2.89 (quin, 1H), 2.73-2.67 (m, 2H), 2.25-2.19 (m, 2H). | cis |
| Aa-269-1 | (500 MHz, CDCl₃) 7.38 (s, 1H), 7.34-7.30 (m, 3H), 7.05 (dt, 1H), 6.69 (d, 1H), 6.35 (dt, 1H), 5.27 (dd, 1H), 4.46 (sext, 1H), 3.70 (s, 3H), 2.90 (quin, 1H), 2.73-2.68 (m, 2H), 2.26-2.20 (m, 2H). | cis |
| Aa-270-2 | (500 MHz, DMSO-ds) 12.21 (s, 1H), 9.11 (d, 1H), 7.62-7.60 (m, 2H), 7.44-7.36 (m, 2H), 7.03 (dt, 1H), 6.59 (dt, 1H), 4.40 (sext, 1H), 3.66 (q, 2H), 2.96-2.91 (m, 1H), 2.43-2.31 (m, 4H). | trans |
| Aa-271-1 | (500 MHz, CDCl₃) 7.39-7.33 (m, 3H), 7.24 (d, 1H), 7.05 (dt, 1H), 6.68 (d, 1H), 6.36 (dt, 1H), 5.93 (tt, 1H), 4.44 (sext, 1H), 3.70 (s, 3H), 3.15 (td, 2H), 2.90 (quin, 1H), 2.73-2.68 (m, 2H), 2.26-2.20 (m, 2H). | cis |
| Aa-271-2 | (500 MHz, CDCl₃) 7.39-7.33 (m, 3H), 7.24 (d, 1H), 7.05 (dt, 1H), 6.53 (d, 1H), 6.37 (dt, 1H), 5.93 (tt, 1H), 4.62 (sext, 1H), 3.73 (s, 3H), 3.18-3.08 (m, 3H), 2.75-2.70 (m, 2H), 2.36-2.29 (m, 2H). | trans |

**Table 2 (continued)**

| Comp No. | ¹H-NMR (300 MHz or 500 MHz, measurement solvent) δ | Remarks |
|---|---|---|
| Aa-272-1 | (500 MHz, CDCl₃) 7.11-7.08 (m, 2H), 7.02 (dt, 1H), 6.96 (d, 1H), 6.68 (d, 1H), 6.36 (dt, 1H), 5.93 (tt, 1H), 4.62 (sext, 1H), 3.71 (s, 3H), 3.14 (td, 2H), 2.90 (quin, 1H), 2.74-2.68 (m, 2H), 2.26-2.20 (m, 2H). | cis |
| Aa-272-2 | (500 MHz, CDCl₃) 7.11-7.08 (m, 2H), 7.01 (dt, 1H), 6.97 (d, 1H), 6.53 (d, 1H), 6.37 (dt, 1H), 5.93 (tt, 1H), 4.62 (sext, 1H), 3.73 (s, 3H), 3.16-3.08 (m, 3H), 2.75-2.70 (m, 2H), 2.36-2.30 (m, 2H). | trans |
| Aa-273-2 | (500 MHz, CDCl₃) 7.38 (s, 1H), 7.23 (s, 1H), 7.20 (s, 1H), 6.99 (dt, 1H), 6.53 (d, 1H), 6.37 (dt, 1H), 5.93 (tt, 1H), 4.62 (sext, 1H), 3.73 (s, 3H), 3.16-3.08 (m, 3H), 2.75-2.70 (m, 2H), 2.36-2.29 (m, 2H). | trans |
| Aa-274-1 | (500 MHz, CDCl₃) 7.20 (s, 1H), 7.06-7.00 (m, 3H), 6.68 (d, 1H), 6.36 (dt, 1H), 4.48-4.40 (m, 3H), 3.71 (s, 3H), 3.40 (s, 3H), 2.90 (quin, 1H), 2.73-2.68 (m, 2H), 2.26-2.20 (m, 2H). | cis |
| Aa-274-2 | (500 MHz, CDCl₃) 7.20 (s, 1H), 7.06-7.00 (m, 3H), 6.53 (d, 1H), 6.37 (dt, 1H), 4.62 (quin, 1H), 4.44 (s, 2H), 3.73 (s, 3H), 3.40 (s, 3H), 3.13-3.08 (m, 1H), 2.75-2.70 (m, 2H), 2.36-2.29 (m, 2H). | trans |
| Aa-275-2 | (500 MHz, CDCl₃) 7.44 (s, 1H), 7.37-7.30 (m, 3H), 7.06 (dt, 1H), 6.31 (d, 1H), 6.37 (dt, 1H), 4.62 (sext, 1H), 4.46 (s, 2H), 3.73 (s, 3H), 3.40 (s, 3H), 3.13-3.08 (m, 1H), 2.75-2.70 (m, 2H), 2.35-2.29 (m, 2H). | trans |
| Aa-275-1 | (500 MHz, CDCl₃) 7.44 (s, 1H), 7.37-7.30 (m, 3H), 7.06 (dt, 1H), 6.66 (d, 1H), 6.37 (dt, 1H), 4.49-4.40 (m, 3H), 3.70 (s, 3H), 3.40 (s, 3H), 2.89 (quin, 1H), 2.73-2.67 (m, 2H), 2.25-2.19 (m, 2H). | cis |
| Aa-276-1 | (500 MHz, CDCl₃) 7.43-7.37 (m, 3H), 7.31 (d, 1H), 7.06 (dt, 1H), 6.69 (d, 1H), 6.38 (dt, 1H), 4.45 (sext, 1H), 3.77 (s, 2H), 3.71 (s, 3H), 2.90 (quin, 1H), 2.74-2.68 (m, 2H), 2.26-2.20 (m, 2H). | cis |

**Table 2 (continued)**

| Comp No. | ¹H-NMR (300 MHz or 500 MHz, measurement solvent) δ | Remarks |
|---|---|---|
| Aa-276-2 | (500 MHz, CDCl₃) 7.43-7.38 (m, 3H), 7.31 (d, 1H), 7.06 (dt, 1H), 6.54 (d, 1H), 6.39 (dt, 1H), 4.63 (sext, 1H), 3.77 (s, 2H), 3.73 (s, 3H), 3.14-3.08 (m, 1H), 2.75-2.70 (m, 2H), 2.36-2.30 (m, 2H). | trans |
| Aa-277-1 | (500 MHz, CDCl₃) 7.40-7.35 (m, 2H), 7.08 (t, 1H), 7.01 (dt, 1H), 6.68 (d, 1H), 6.28 (dt, 1H), 5.98 (tt, 1H), 4.44 (sext, 1H), 3.70 (s, 3H), 3.19 (td, 2H), 2.90 (quin, 1H), 2.73-2.68 (m, 2H), 2.26-2.20 (m, 2H). | cis |
| Aa-277-2 | (500 MHz, CDCl₃) 7.40-7.35 (m, 2H), 7.08 (t, 1H), 7.01 (dt, 1H), 6.52 (d, 1H), 6.29 (dt, 1H), 5.99 (tt, 1H), 4.62 (sext, 1H), 3.73 (s, 3H), 3.19 (td, 2H), 3.14-3.08 (m, 1H), 2.75-2.70 (m, 2H), 2.36-2.29 (m, 2H). | trans |
| Aa-278-2 | (500 MHz, CDCl₂) 7.00-6.94 (m, 3H), 6.80 (tt, 1H), 6.53 (d, 1H), 6.36 (dt, 3H), 5.35-5.33 (m, 1H), 4.60 (sext, 1H), 3.94-3.89 (m, 2H), 3.88-3.81 (m, 2H), 3.13-3.07 (m, 1H), 2.36-2.30 (m, 2H), 2.24-2.17 (m, 1H), 2.03-1.98 (m, 1H). | trans |
| Aa-279-2 | (500 MHz, CDCl₂) 7.00-6.94 (m, 3H), 6.80 (tt, 1H), 6.58 (d, 1H), 6.36 (dt, 1H), 4.98 (sep, 1H), 4.62 (quin, 1H), 3.91 (dt, 2H), 3.57-3.52 (m, 2H), 3.12-3.07 (m, 1H), 2.74-2.69 (m, 2H), 2.37-2.30 (m, 2H), 1.95-1.91 (m, 2H), 1.72-1.65 (m, 2H). | trans |
| Aa-280-2 | (500 MHz, CDCl₃) 7.00-6.94 (m, 3H), 6.80 (tt, 1H), 6.54 (d, 1H), 6.36 (dt, 1H), 4.88 (dd, 2H), 4.76 (quin, 1H), 4.64 (sext, 1H), 3.99 (ddd, 4H), 3.25-3.19 (m, 1H), 2.77-2.72 (m, 2H), 2.40-2.34 (m, 2H). | trans |
| Aa-281-2 | (500 MHz, CDCl₃) 7.00-6.94 (m, 3H), 6.80 (tt, 1H), 6.53 (d, 1H), 6.36 (dt, 1H), 5.05 (s, 1H), 4.90 (s, 1H), 4.63 (sext, 1H), 4.32-4.27 (m, 1H), 4.25-4.16 (m, 2H), 3.99 (dd, 1H), 3.70 (dd, 1H), 3.18-3.13 (m, 1H), 2.77-2.71 (m, 2H), 2.38-2.32 (m, 2H). | trans |
| Aa-282-2 | (500 MHz, CDCl₃) 7.39-7.33 (m, 3H), 7.24 (d, 1H), 7.05 (dt, 1H), 6.54 (d, 1H), 6.36 (dt, 1H), 5.93 (tt, 1H), 5.35-5.32 (m, 1H), 4.61 (sext, 1H), 3.94-3.90 (m, 2H), 3.87-3.80 (m, 2H), 3.18-3.07 (m, 3H), 2.74-2.68 (m, 2H), 2.36-2.30 (m, 2H), 2.24-2.17 (m, 1H), 2.03-1.98 (m, 1H). | trans |

**Table 2 (continued)**

| Comp No. | ¹H-NMR (300 MHz or 500 MHz, measurement solvent) δ | Remarks |
|---|---|---|
| Aa-283-2 | (500 MHz, CDCl₃) 7.39-7.33 (m, 3H), 7.24 (d, 1H), 7.05 (dt, 1H), 6.36 (d, 1H), 6.37 (dt, 1H), 5.93 (tt, 1H), 5.50-5.45 (m, 1H), 4.91 (t, 2H), 4.65-4.59 (m, 3H), 3.18-3.11 (m, 3H), 2.76-2.70 (m, 2H), 2.40-2.34 (m, 2H). | trans |
| Aa-284-2 | (500 MHz, CDCl₃) 7.39-7.33 (m, 3H), 7.24 (d, 1H), 7.05 (dt, 1H), 6.35 (d, 1H), 6.37 (dt, 1H), 5.93 (tt, 1H), 4.98 (sep, 1H), 4.62 (quin, 1H), 3.92 (dt, 2H), 3.55 (ddd, 2H), 3.15 (td, 2H), 3.12-3.07 (m, 1H), 2.74-2.69 (m, 2H), 2.36-2.30 (m, 2H), 1.96-1.91 (m, 2H), 1.72-1.65 (m, 2H). | trans |
| Aa-285-2 | (500 MHz, CDCl₃) 7.39-7.33 (m, 3H), 7.24 (d, 1H), 7.05 (dt, 1H), 6.52 (d, 1H), 6.36 (dt, 1H), 5.93 (tt, 1H), 4.96 (d, 1H), 4.80 (d, 1H), 4.76 (quin, 1H), 4.64 (sext, 1H), 3.99 (ddd, 4H), 3.24-3.11 (m, 3H), 2.77-2.72 (m, 2H), 2.40-2.34 (m, 2H). | trans |
| Aa-286-2 | (500 MHz, CDCl₃) 7.40-7.33 (m, 3H), 7.24 (d, 1H), 7.05 (dt, 1H), 6.52 (d, 1H), 6.37 (dt, 1H), 5.93 (tt, 1H), 4.98 (d, 2H), 4.63 (quin, 1H), 4.32-4.27 (m, 1H), 4.24-4.16 (m, 2H), 3.99 (dd, 1H), 3.70 (dd, 1H), 3.18-3.11 (m, 3H), 2.76-2.71 (m, 2H), 2.38-2.32 (m, 2H). | trans |
| Aa-287-1 | (500 MHz, CDCl₃) 7.30 (t, 1H), 7.10 (d, 1H), 7.04 (dt, 1H), 6.99 (s, 1H), 6.91 (dd, 1H), 6.68 (d, 1H), 6.35 (dt, 1H), 5.09 (tt, 1H), 4.44 (sext, 1H), 4.20 (td, 2H), 3.70 (s, 3H), 2.90 (quin, 1H), 2.73-2.68 (m, 2H), 2.25-2.20 (m, 2H). | cis |
| Aa-287-2 | (500 MHz, CDCl₃) 7.30 (t, 1H), 7.10 (d, 1H), 7.02 (dt, 1H), 6.99 (s, 1H), 6.91 (dd, 1H), 6.52 (d, 1H), 6.35 (dt, 1H), 6.09 (tt, 1H), 4.62 (sext, 1H), 4.20 (td, 2H), 3.73 (s, 3H), 3.14-3.08 (m, 1H), 2.75-2.70 (m, 2H), 2.36-2.29 (m, 2H). | trans |

**Table 2 (continued)**

| Comp No. | ¹H-NMR (300 MHz or 500 MHz, measurement solvent) δ | Remarks |
|---|---|---|
| Ba-1-1 | (500 MHz, CDCl₃) 7.48 (br, 1H), 7.01-6.96 (m, 3H), 6.78 (tt, 1H), 6.38 (dt, 1H), 4.42 (s, 1H), 4.11 (dd, 1H), 3.72 (s, 3H), 3.00-2.96 (m, 1H), 2.18-2.12 (m, 1H), 2.11-2.00 (m, 1H), 1.98-1.87 (m, 2H), 1.83-1.77 (m, 1H). | cis |
| Ba-1-2 | (300 MHz, CDCl₃) 7.07-6.89 (m, 3H), 6.80 (tt, 1H), 6.37 (dt, 1H), 6.36-6.22 (m, 1H), 4.37 (sext, 1H), 3.69 (s, 3H), 2.96 (quin, 1H), 2.43-2.30 (m, 1H), 2.29-2.17 (m, 1H), 2.16-2.01 (m, 1H), 1.99-1.76 (m, 2H), 1.64-1.48 (m, 1H). | trans |
| Ba-3-1 | (500 MHz, CDCl₃) 7.46 (br, 1H), 7.32 (dt, 1H), 7.21 (d, 1H), 7.15 (dt, 1H), 7.06-7.04 (m, 1H), 7.03-7.01 (m, 1H), 6.38 (dt, 1H), 4.48-4.39 (m, 1H), 3.71 (s, 3H), 3.00-2.94 (m, 1H), 2.18-2.13 (m, 1H), 2.10-2.03 (m, 1H), 1.99-1.87 (m, 3H), 1.88-1.73 (m, 1H). | cis |
| Ba-9-1 | (300 MHz, CDCl₃) 7.45 (brd, 1H), 7.03-6.94 (m, 3H), 6.85 (d, 1H), 6.36 (dt, 1H), 4.47-4.38 (m, 1H), 3.72 (s, 3H), 3.01-2.92 (m, 1H), 2.35 (s, 3H), 2.22-1.77 (m, 6H). | cis |
| Ba-11-1 | (500 MHz, CDCl₃) 7.48 (d, 1H), 7.32 (s, 2H), 7.25 (s, 1H), 6.96 (tt, 1H), 6.40 (dt, 1H), 4.43 (s, 1H), 3.72 (s, 3H), 3.00-2.96 (m, 1H), 2.18-2.07 (m, 2H), 1.99-1.87 (m, 3H), 1.83-1.77 (m, 1H). | cis |

**Table 2 (continued)**

| Comp No. | ¹H-NMR (300 MHz or 500 MHz, measurement solvent) δ | Remarks |
|---|---|---|
| Ba-19-1 | (500 MHz, CDCl₃) 7.46-7.44 (m, 2H), 7.33-7.29 (m, 3H), 7.02 (dt, 1H), 6.37 (dt, 1H), 4.47-4.40 (m 1H), 3.72 (s, 3H), 3.01-2.95 (m, 1H), 2.19-2.13 (m, 1H), 2.09-2.03 (m, 1H), 1.99-1.87 (m, 3H), 1.83-1.77 (m, 1H). | cis |
| Ba-44-1 | (300 MHz, CDCl₃) 7.75 (d, 1H), 6.99-6.92 (m, 3H), 6.78 (tt, 1H), 6.38 (dt, 1H), 4.35-4.26 (m, 1H), 3.69 (dd, 1H), 3.65 (dd, 1H), 2.40-2.27 (m, 2H), 2.17 (ddd, 1H), 1.88-1.78 (m, 2H), 1.75-1.60 (m, 2H), 1.46 (dt, 1H). | cis |
| Ba-45-1 | (300 MHz, CDCl₃) 7.03-6.94 (m, 3H), 6.79 (tt, 1H), 6.61 (d, 1H), 6.38 (dt, 1H), 4.28 (sext, 1H), 4.20 (dd, 2H), 3.04 (s, 3H), 2.48-2.29 (m, 2H), 2.13-2.04 (m, 1H), 1.93-1.84 (m, 1H), 1.69-1.53 (m, 2H), 1.39-1.23 (m, 1H). | cis |
| Ba-53-1 | (300 MHz, CDCl₃) 7.02-6.93 (m, 3H), 6.80 (tt, 1H), 6.54 (br, 1H), 6.39 (dt, 1H), 4.35-4.22 (m, 1H), 4.13-3.97 (m, 2H), 2.39-2.25 (m, 2H), 2.10-2.03 (m, 1H), 2.08 (s, 3H), 1.91-1.79 (m, 1H), 1.66-1.48 (m, 2H), 1.31-1.21 (m, 1H). | cis |
| Ba-58-1 | (300 MHz, CDCl₃) 7.28 (br, 1H), 7.05-6.90 (m, 3H), 6.79 (tt, 1H), 6.38 (dt, 1H), 4.57-4.52 (m, 2H), 2.18-2.06 (m, 1H), 2.04-1.72 (m, 6H). | cis |
| Ba-63-1 | (300 MHz, CDCl₃) 9.00 (d, 1H), 7.05-6.89 (m, 3H), 6.74 (tt, 1H), 6.39 (dt, 1H), 4.44-4.33 (m, 1H), 3.56-3.38 (m, 4H), 3.18-3.06 (m, 1H), 2.19-1.68 (m, 10H). | cis |
| Ba-64-1 | (300 MHz, CDCl₃) 8.96 (d, 1H), 7.06-6.88 (m, 3H), 6.75 (tt, 1H), 6.39 (dt, 1H), 4.48-4.37 (m, 1H), 4.28-4.13 (m, 2H) 4.04 (t, 2H), 2.96-2.81 (m, 1H), 2.29 (quin, 2H), 2.12-1.69 (m, 6H). | cis |
| Ba-65-1 | (300 MHz, CDCl₃) 8.98 (d, 1H), 7.05-6.93 (m, 3H), 6.77 (tt, 1H), 6.42 (dt, 1H), 4.47-4.38 (m, 1H), 3.51-3.30 (m, 4H), 3.28-3.19 (m, 1H), 2.18-2.05 (m, 1H), 2.01-1.88 (m, 3H), 1.86-1.73 (m, 2H), 1.21 (t, 3H), 1.12 (t, 3H). | cis |
| Ba-76-1 | (300 MHz, CDCl₃) 8.82 (d, 1H), 7.05-6.93 (m, 3H), 6.77 (tt, 1H), 6.41 (dt, 1H), 4.45-4.39 (m, 1H), 3.33-3.25 (m, 1H), 3.10 (s, 3H), 2.99 (s, 3H), 2.19-2.07 (m, 1H), 2.05-1.88 (m, 3H), 1.87-1.69 (m, 2H). | cis |

**Table 2 (continued)**

| Comp No. | ¹H-NMR (300 MHz or 500 MHz, measurement solvent) δ | Remarks |
|---|---|---|
| Ba-116-3 | (300 MHz, CDCl₃) 7.04-6.84 (m, 5H), 6.38 (dt, 1H), 4.50-4.35 (m, 1H), 3.29-3.15 (m, 1H), 2.46-2.04 (m, 3H), 2.13 (s, 2H), 2.11 (s, 1H), 1.84-1.59 (m, 3H). | cis-trans isomer |
| Ba-117-3 | (300 MHz, CDCl₃) 8.20 (br, 1H), 7.06-6.90 (m, 3H), 6.77 (tt, 1H), 6.39 (dt, 1H), 4.59-4.38 (m, 1H), 3.21-3.07 (m, 1H), 2.56-2.23 (m, 2H), 2.52 (s, 1.5H), 2.51 (s, 1.5H), 2.15-1.76 (m, 4H). | cis-trans isomer |
| Ba-118-3 | (300 MHz, CDCl₃) 8.50 (d, 1H), 7.07-6.92 (m, 3H), 6.81 (tt, 1H), 6.39 (dt, 1H), 4.61-4.49 (m, 0.85H), 4.42 (q, 0.15H), 3.70-3.52 (m, 1H), 2.97 (s, 2.55H), 2.90 (s, 0.45H), 2.59 (quin, 0.15H), 2.43 (ddd, 0.85H), 2.34-2.14 (s, 3H), 2.08-1.93 (m, 2H). | cis-trans isomer |
| Ba-119-1 | (300 MHz, CDCl₃) 7.02-6.93 (m, 3H), 6.79 (tt, 1H), 6.63 (d, 1H), 6.38 (dt, 1H), 4.30 (sext, 1H), 4.04 (dd, 2H), 2.47-2.24 (m, 2H), 2.18-1.92 (m, 2H), 1.70-1.52 (m, 2H), 1.38-1.23 (m, 1H). | cis |
| Ba-120-1 | (300 MHz, CDCl₃) 7.02-6.94 (m, 3H), 6.80 (tt, 1H), 6.51 (br, 1H), 6.39 (dt, 1H), 4.85-4.84 (m, 1H), 4.28 (sext, 1H), 4.05 (ddd, 2H), 2.41-2.25 (m, 2H), 2.35 (q, 2H), 2.13-2.03 (m, 1H), 1.89-1.79 (m, 1H), 1.65-1.45 (m, 1H), 1.29-1.20 (m, 1H), 1.15 (t, 3H). | cis |
| Ba-121-1 | (300 MHz, CDCl₃) 7.02-6.93 (m, 3H), 6.80 (tt, 1H), 6.48 (d, 1H), 6.39 (dt, 1H), 4.28 (sext, 1H), 4.04 (ddd, 2H), 2.57 (sep, 1H), 2.41-2.26 (m, 2H), 2.15-2.06 (m, 1H), 1.88-1.79 (m, 1H), 1.64-1.47 (m, 2H), 1.25-1.15 (m, 1H), 1.18 (d, 6H). | cis |
| Ba-122-1 | (300 MHz, CDCl₃) 7.02-6.93 (m, 3H), 6.80 (tt, 1H), 6.53 (d, 1H), 6.38 (dt, 1H), 4.29 (sext, 1H), 4.10-4.00 (m, 2H), 2.44-2.26 (m, 2H), 2.13-2.03 (m, 1H), 1.90-1.80 (m, 1H), 1.74-1.47 (m, 2H), 1.30-1.21 (m, 1H), 1.20-1.15 (m, 1H), 1.07-0.98 (m, 2H), 0.91-0.85 (m, 2H). | cis |
| Ba-124-1 | (300 MHz, CDCl₃) 8.94 (d, 0.5H), 8.80 (d, 0.5H), 7.07-6.88 (m, 3H), 6.75 (tt, 1H), 6.49-6.30 (m, 1H), 4.48-4.34 (br, 1H), 3.55-3.31 (m, 2H), 3.31-3.17 (m, 1H), 3.04 (s, 1.5H), 2.94 (s, 1.5H), 2.19-2.05 (m, 1H), 2.02-1.63 (m, 5H), 1.20 (t, 1.5H), 1.09 (t, 1.5H). | cis |
| Ba-125-1 | (300 MHz, CDCl₃) 8.87 (d, 0.5H), 8.70 (d, 0.5H), 7.06-6.88 (m, 3H), 6.74 (tt, 1H), 6.48-6.29 (m, 1H), 5.86-5.61 (m, 1H), 5.27-5.05 (m, 2H), 4.39 (br, 1H), 4.09-3.83 (m, 2H), 3.33-3.11 (m, 1H), 3.00 (s, 1.5H), 2.94 (s, 1.5H), 2.19-1.65 (m, 6H). | cis |

**Table 2 (continued)**

| Comp No. | ¹H-NMR (300 MHz or 500 MHz, measurement solvent) δ | Remarks |
|---|---|---|
| Ba-223-1 | (300 MHz, CDCl₃) 7.02-6.95 (m, 3H), 6.91 (d, 1H), 6.80 (tt, 1H), 6.38 (dt, 1H), 6.40-6.31 (br, 1H), 5.83 (dd, 1H), 4.32 (sext, 1H), 3.73 (s, 3H), 2.74 (sext, 1H), 2.39 (dt, 1H), 2.21-2.11 (m, 1H), 2.01-1.92 (m, 1H), 1.70-1.60 (m, 2H), 1.41-1.31 (m, 1H). | cis |
| Ba-226-1 | (300 MHz, CDCl₃) 7.01-6.92 (m, 3H), 6.79 (tt, 1H), 6.38 (dt, 1H), 6.39-6.31 (br, 1H), 4.23 (sext, 1H), 3.67 (s, 3H), 2.37-2.28 (m, 3H), 2.15-2.03 (m, 1H), 1.96-1.81 (m, 2H), 1.75-1.68 (m, 2H), 1.59-1.47 (m, 1H), 1.41-1.30 (m, 1H), 1.05 (dt, 1H). | cis |

**Table 2 (continued)**

| Comp No. | ¹H-NMR (300 MHz or 500 MHz, measurement solvent) δ | Remarks |
|---|---|---|
| Bb-1-1 | (500 MHz, CDCl₃) 7.17 (d, 1H), 7.02-6.96 (m, 3H), 6.78 (tt, 1H), 6.38 (dt, 1H), 5.99-5.98 (m, 1H), 5.95-5.94 (m, 1H), 5.08 (t, 1H), 3.72 (s, 3H), 3.57-3.53 (m, 1H), 2.45 (dt, 1H), 2.00 (dt, 1H). | cis |
| Bb-1-2 | (500 MHz, CDCl₃) 7.01-6.94 (m, 3H), 6.80 (tt, 1H), 6.42-6.33 (m, 2H), 6.04 (dt, 1H), 5.87 (dt, 1H), 5.19-515 (m, 1H), 3.80-3.75 (m, 1H), 3.71 (s, 3H), 2.76-2.71 (m, 1H), 1.96-1.91 (m, 1H). | trans |
| Bb-1-4 | (300 MHz, CDCl₃) 7.17 (br, 1H), 7.06-6.90 (m, 3H), 6.79 (tt, 1H), 6.39 (dt, 1H), 6.04-5.91 (m, 2H), 5.09 (t, 1H), 3.75 (s, 3H), 3.62-3.53 (m, 1H), 2.46 (dt, 1H), 2.00 (dt, 1H). | Optically active (1S, 4R) |
| Bb-1-5 | (300 MHz, CDCl₃) 7.18 (br, 1H), 7.06-6.90 (m, 3H), 6.79 (tt, 1H), 6.39 (dt, 1H), 6.04-5.91 (m, 2H), 5.09 (t, 1H), 3.75 (s, 3H), 3.62-3.53 (m, 1H), 2.46 (dt, 1H), 2.00 (dt, 1H). | Optically active (1R, 4S) |
| Bb-2-1 | (300 MHz, CDCl₃) 7.18 (d, 1H), 7.13-7.03 (m, 2H), 6.95 (dt, 1H), 6.32 (dt, 1H), 6.00 (dd, 1H), 5.95 (dt, 1H), 5.09 (t, 1H), 3.75 (s, 3H), 3.57 (dq, 1H), 2.46 (dt, 1H), 2.00 (dt, 1H). | cis |
| Bb-3-1 | (300 MHz, CDCl₃) 7.32 (dt, 1H), 7.22 (d, 1H), 7.16-7.14 (m, 2H), 7.06-7.01 (m, 2H), 6.37 (dt, 1H), 5.99-5.97 (m, 1H), 5.95-5.93 (m, 1H), 5.09 (t, 1H), 3.73 (s, 3H), 3.55 (d, 1H), 2.45 (dt, 1H), 1.98 (dt, 1H). | cis |
| Bb-5-1 | (500 MHz, CDCl₃) 7.53 (s, 1H), 7.40 (dt, 1H), 7.33 (dt, 1H), 7.20 (d, 1H), 7.05 (dt, 1H), 6.46 (dt, 1H), 6.01-5.95 (m, 2H), 5.09 (t, 1H), 3.75 (s, 3H), 3.58-3.56 (m, 1H), 2.46 (dt, 1H), 2.01 (dt, 1H). | cis |
| Bb-5-5 | (500 MHz, CDCl₃) 7.53 (s, 1H), 7.41 (dt, 1H), 7.33 (dt, 1H), 7.20 (d, 1H), 7.05 (dt, 1H), 6.46 (dt, 1H), 6.01-5.94 (m, 2H), 5.09 (t, 1H), 3.75 (s, 3H), 3.59-3.56 (m, 1H), 2.46 (dt, 1H), 2.01 (dt, 1H). | Optically active (1R, 4S) |
| Bb-7-1 | (500 MHz, CDCl₃) 7.23 (s, 1H), 7.17 (br, 1H), 7.05-6.97 (m, 3H), 6.39 (dt, 1H), 5.97-5.93 (m, 2H), 5.08 (t, 1H), 3.73 (s, 3H), 3.57-3.54 (m, 1H), 2.48-2.42 (m, 1H), 2.00-1.97 (m, 1H). | cis |
| Bb-8-1 | (300 MHz, CDCl₃) 7.40 (s, 1H), 7.22 (dt, 1H), 7.18 (br, 1H), 7.10 (dt, 1H), 6.98 (dt, 1H), 6.39 (dt, 1H), 6.03-5.92 (m, 2H), 5.09 (t, 1H), 3.75 (s, 3H), 3.61-3.63 (m, 1H), 2.46 (dt, 1H), 2.00 (dt, 1H). | cis |

**Table 2 (continued)**

| Comp No. | ¹H-NMR (300 MHz or 500 MHz, measurement solvent) δ | Remarks |
|---|---|---|
| Bb-9-1 | (500 MHz, CDCl₃) 7.11 (br, 1H), 7.03-6.92 (m, 4H), 6.86 (d, 1H), 5.97-5.94 (m, 2H), 5.08 (t, 1H), 3.73 (s, 3H), 3.56-3.54 (m, 1H), 2.49-2.43 (m, 1H), 2.34 (s, 3H), 1.99 (dt, 1H). | cis |
| Bb-10-1 | (300 MHz, CDCl₃) 7.24-7.08 (m, 3H), 7.03 (dt, 1H), 6.94 (d, 1H), 6.41 (dt, 1H), 6.05-5.91 (m, 2H), 5.09 (t, 1H), 3.75 (s, 3H), 3.62-3.52 (m, 1H), 2.46 (dt, 1H), 2.00 (dt, 1H). | cis |
| Bb-11-1 | (500 MHz, CDCl₃) 7.32 (s, 1H), 7.25 (s, 2H), 7.15 (d, 1H), 6.96 (dt, 1H), 6.39 (dt, 1H), 5.99-5.97 (m, 1H), 5.96-5.94 (m, 1H), 5.08 (t, 1H), 3.74 (s, 3H), 3.57-3.55 (m, 1H), 2.45 (dt, 1H), 1.99 (dt, 1H). | cis |
| Bb-12-1 | (300 MHz, CDCl₃) 7.15 (d, 1H), 7.00 (dt, 1H), 6.78-6.74 (m, 2H), 6.60 (dt, 1H), 6.36 (dt, 1H), 6.00-5.98 (m, 1H), 5.96-5.93 (m, 1H), 5.10 (t, 1H), 3.81 (s, 3H), 3.74 (s, 3H), 3.56 (ddd, 1H), 2.47 (dt, 1H), 2.00 (dt, 1H). | cis |
| Bb-13-1 | (300 MHz, CDCl₃) 7.15 (d, 1H), 7.04 (t, 1H), 6.98 (dt, 1H), 6.88-6.83 (m, 2H), 6.37 (dt, 1H), 6.00-5.92 (m, 2H), 5.09 (t, 1H), 3.81 (s, 3H), 3.74 (s, 3H), 3.59-3.54 (m, 1H), 2.47 (dt, 1H), 1.99 (dt, 1H). | cis |
| Bb-15-1 | (300 MHz, CDCl₃) 7.47-7.40 (m, 2H), 7.16 (d, 1H), 7.09-7.01 (m, 3H), 6.30 (dt, 1H), 6.00-5.92 (m, 2H), 5.10 (t, 1H), 3.74 (s, 3H), 3.59-3.54 (m, 1H), 2.47 (dt, 1H), 2.00 (dt, 1H). | cis |
| Bb-19-1 | (300 MHz, CDCl₃) 7.44 (s, 1H), 7.33-7.27 (m, 3H), 7.15 (d, 1H), 7.02 (dt, 1H), 6.37 (dt, 1H), 5.98-5.97 (m, 1H), 5.96-5.93 (m, 1H), 5.09 (t, 1H), 3.74 (s, 3H), 3.59-3.55 (m, 1H), 2.45 (dt, 1H), 1.98 (dt, 1H). | cis |
| Bb-20-1 | (500 MHz, CDCl₃) 7.60 (s, 1H), 7.46 (d, 1H), 7.36 (d, 1H), 7.23 (t, 1H), 7.13 (d, 1H), 7.01 (dt, 1H), 6.37 (dt, 1H), 5.99-5.97 (m, 1H), 5.95-5.93 (m, 1H), 5.09 (t, 1H), 3.74 (s, 3H), 3.57-3.55 (m, 1H), 2.46 (dt, 1H), 1.99 (dt, 1H). | cis |
| Bb-26-1 | (300 MHz, CDCl₃) 7.14 (d, 1H), 7.00 (dt, 1H), 6.59 (d, 2H), 6.45 (t, 1H), 6.36 (dt, 1H), 6.00-5.93 (m, 2H), 5.10 (t, 1H), 3.80 (s, 6H), 3.74 (s, 3H), 3.58-3.53 (m, 1H), 2.47 (dt, 1H), 1.99 (dt, 1H). | cis |

**Table 2 (continued)**

| Comp No. | ¹H-NMR (300 MHz or 500 MHz, measurement solvent) δ | Remarks |
|---|---|---|
| Bb-27-1 | (300 MHz, CDCl₃) 7.10 (br, 1H), 7.07 (s, 2H), 7.01 (dt, 1H), 6.98 (s, 1H), 6.34 (dt, 1H), 5.99-5.92 (m, 2H), 5.10 (t, 1H), 3.74 (s, 3H), 3.58-3.53 (m, 1H), 2.48 (dt, 1H), 2.31 (s, 6H), 1.99 (dt, 1H). | cis |
| Bb-44-1 | (300 MHz, CDCl₃) 7.26 (br, 1H), 7.03-6.90 (m, 3H), 6.79 (tt, 1H), 6.38 (dt, 1H), 5.90-5.82 (m, 2H), 5.02 (t, 1H), 3.80-3.61 (m, 2H), 2.93-2.85 (m, 1H), 2.51 (dt, 1H), 1.67-1.52 (m, 2H). | cis |
| Bb-45-1 | (300 MHz, CDCl₃) 7.06-6.90 (m, 3H), 6.79 (tt, 1H), 6.64 (d, 1H), 6.39 (dt, 1H), 5.93-5.83 (m, 2H), 5.06 (br, 1H), 4.26 (ddd, 2H), 3.16-3.04 (m, 1H), 3.03 (s, 3H), 2.66 (dt, 1H), 1.54 (dt, 1H). | cis |
| Bb-46-3 | (300 MHz, CDCl₃) 7.34 (td, 1H), 7.22 (d, 1H), 7.15 (dt, 1H), 7.08-7.00 (m, 2.75H), 6.49-6.41 (br, 0.25H), 6.38 (dt, 1H), 6.05 (ddd, 0.25H), 6.03-5.95 (m, 1.5H), 5.90 (dt, 0.25H), 5.24-5.16 (m, 0.25H), 5.11 (t, 0.75H), 4.73 (dd, 1.5H), 4.70 (d, 0.5H), 3.86-3.79 (m, 0.25H), 3.64-3.59 (m, 0.75H), 2.75 (ddd, 0.25H), 2.57-2.46 (m, 1.75H), 2.02 (dt, 0.75H), 2.02-1.93 (m, 0.25H). | cis-trans isomer |
| Bb-48-1 | (300 MHz, CDCl₃) 7.02-6.94 (m, 4H), 6.79 (tt, 1H), 6.38 (dt, 1H), 6.05 (ddd, 1H), 5.99 (dt, 1H), 5.11 (t, 1H), 3.65-3.61 (m, 1H), 2.53 (dt, 1H), 2.04 (dt, 1H). No proton peak of carboxy group detected. | cis |
| Bb-49-1 | (300 MHz, CDCl₃) 8.56 (br, 0.75H), 8.12 (br, 0.25H), 7.91-7.67 (m, 1H), 7.06-6.90 (m, 3H), 6.78 (tt, 1H), 6.38 (dt, 1H), 6.06-5.80 (m, 2H), 5.10 (t, 1H), 3.97 (br, 0.25H), 3.81 (s, 3H), 3.21 (d, 0.75H), 2.39 (quin, 1H), 2.10-1.90 (m, 1H). | cis |
| Bb-49-2 | (300 MHz, CDCl₃) 8.40-7.48 (m, 1H), 7.05-6.89 (m, 3H), 6.80 (tt, 1H), 6.46-6.29 (m, 2H), 5.97 (br, 2H), 5.24-5.12 (m, 1H), 3.83-3.42 (m, 1H), 3.78 (s, 3H), 2.79-2.60 (m, 1H), 2.07-1.90 (m, 1H). | trans |

**Table 2 (continued)**

| Comp No. | ¹H-NMR (300 MHz or 500 MHz, measurement solvent) δ | Remarks |
|---|---|---|
| Bb-50-1 | (300 MHz, CDCl₃) 7.91 (d, 1H), 7.06-6.90 (m, 3H), 6.78 (tt, 1H), 6.39 (dt, 1H), 6.00-5.89 (m, 2H), 5.07 (t, 1H), 4.02 (d, 1H), 3.77 (s, 3H), 3.23 (s, 3H), 2.35 (dt, 1H), 2.00 (d, 1H). | cis |
| Bb-50-2 | (300 MHz, CDCl₃) 7.06-6.88 (m, 3H), 6.80 (tt, 1H), 6.39 (dt, 1H), 6.37 (br, 1H), 6.01 (ddd, 1H), 5.88 (dt, 1H), 5.24-5.11 (m, 1H), 4.19-4.02 (m, 1H), 3.73 (d, 3H), 3.21 (s, 3H), 2.72 (ddd, 1H), 1.93 (ddd, 1H). | trans |
| Bb-53-1 | (300 MHz, CDCl₃) 7.07-6.92 (m, 3H), 6.80 (tt, 1H), 6.54 (d, 1H), 6.39 (dt, 1H), 5.90 (dt, 1H), 5.81 (dt, 1H), 5.04 (br, 1H), 4.19 (dd, 1H), 4.00 (dd, 1H), 3.13-2.95 (m, 1H), 2.63 (dt, 1H), 2.09 (s, 3H) 1.44 (dt, 1H). | cis |
| Bb-55-1 | (300 MHz, CDCl₃) 7.13 (d, 1H), 7.08-6.92 (m, 3H), 6.81 (tt, 1H), 6.41 (dt, 1H), 6.03 (ddd, 1H), 5.97-5.92 (m, 1H), 5.06 (t, 1H), 3.84-3.76 (m, 1H), 2.44-2.28 (m, 1H), 2.32 (s, 3H), 2.01 (dt, 1H). | cis |
| Bb-60-1 | (300 MHz, CDCl₃) 7.94 (d, 1H), 7.04-6.93 (m, 3H), 6.78 (tt, 1H), 6.39 (dt, 1H), 5.97-5.91 (m, 2H), 5.05 (t, 1H), 4.11-3.95 (m, 1H), 4.06 (t, 2H), 3.83-3.68 (m, 2H), 2.43-2.28 (m, 3H), 2.03 (d, 1H). | cis |
| Bb-60-2 | (300 MHz, CDCl₃) 7.02-6.93 (m, 3H), 6.80 (tt, 1H), 6.44 (br, 1H), 6.39 (dt, 1H), 6.03 (ddd, 1H), 5.87 (dt, 1H), 5.21-5.11 (m, 1H), 4.14-4.09 (m, 1H), 4.06-3.94 (m, 2H), 3.82-3.63 (m, 2H), 2.72 (ddd, 1H), 2.34 (quin, 2H), 1.93 (ddd, 1H). | trans |
| Bb-61-1 | (300 MHz, CDCl₃) 7.96 (d, 1H), 7.04-6.92 (m, 3H), 6.78 (tt, 1H), 6.40 (dt, 1H), 5.96-5.91 (m, 2H), 5.05 (t, 1H), 4.10-3.99 (m, 3H), 3.98-3.89 (m, 1H), 3.71-3.63 (m, 1H), 2.33 (dt, 1H), 2.02 (d, 1H), 1.90-1.71 (m, 4H). | cis |
| Bb-61-2 | (300 MHz, CDCl₃) 7.03-6.92 (m, 3H), 6.80 (tt, 1H), 6.39 (dt, 1H), 6.38 (br, 1H), 6.02 (ddd, 1H), 5.86 (dt, 1H), 5.22-5.11 (m, 1H), 4.16-4.09 (m, 1H), 4.07-3.85 (m, 3H), 3.70-3.62 (m, 1H), 2.72 (ddd, 1H), 1.92 (ddd, 1H), 1.87-1.70 (m, 4H). | trans |

**Table 2 (continued)**

| Comp No. | ¹H-NMR (300 MHz or 500 MHz, measurement solvent) δ | Remarks |
|---|---|---|
| Bb-63-1 | (300 MHz, CDCl₃) 8.38 (d, 1H), 7.09-6.92 (m, 3H), 6.79 (tt, 1H), 6.42 (dt, 1H), 6.03-5.97 (m, 1H), 5.93 (dd, 1H), 5.08 (t, 1H), 3.76-3.68 (m, 1H), 3.67-3.56 (m, 2H), 3.56-3.45 (m, 2H), 2.30 (dt, 1H), 2.12-1.98 (m, 3H), 1.98-1.86 (m, 2H). | cis |
| Bb-64-1 | (300 MHz, CDCl₃) 8.30 (d, 1H), 7.07-6.89 (m, 3H), 6.78 (tt, 1H), 6.40 (dt, 1H), 6.04-5.94 (m, 1H), 5.87 (dd, 1H), 5.07 (t, 1H), 4.40-4.21 (m, 2H), 4.08 (t, 2H), 3.51-3.41 (m, 1H), 2.43-2.18 (m, 3H), 1.94 (d, 1H). | cis |
| Bb-65-1 | (300 MHz, CDCl₃) 8.33 (d, 1H), 7.05-6.93 (m, 3H), 6.78 (tt, 1H), 6.40 (dt, 1H), 5.97 (dt, 1H), 5.88 (dd, 1H), 5.06 (t, 1H), 3.78 (dquin, 1H), 3.59-3.28 (m, 4H), 2.31 (dt, 1H), 2.00 (d, 1H), 1.26 (t, 3H), 1.12 (t, 3H). | cis |
| Bb-71-3 | (300 MHz, CDCl₃) 7.16 (d, 1H), 7.07-6.90 (m, 3H), 6.79 (tt, 1H), 6.39 (dt, 1H), 6.07-6.01 (m, 0.16H), 6.00-5.90 (m, 1.84H), 5.25-5.14 (m, 0.16H), 5.09 (t, 0.84H), 4.00-3.90 (m, 0.16H), 3.82-3.70 (m, 0.84H), 2.92 (q, 2H), 2.71 (ddd, 0.16H), 2.44 (dt, 0.84H), 2.02-1.90 (m, 0.16H), 1.94 (dt, 0.84H), 1.27 (t, 2.52H), 1.25 (t, 0.48H). | cis-trans isomer |
| Bb-72-3 | (300 MHz, CDCl₃)7.03-6.94 (m, 4H), 6.80 (tt, 1H), 6.38 (dt, 1H), 6.05 (ddd, 0.2H), 6.03-6.00 (m, 0.8H), 5.97 (dt, 0.8H), 5.90 (dt, 0.2H), 5.10 (t, 1H), 4.73 (dd, 1.6H), 4.71 (d, 0.4H), 3.882 (dsext, 0.2H), 3.662 (ddd, 0.8H), 2.76 (ddd, 0.2H), 2.56-2.44 (m, 1.8H), 2.02 (dt, 1H). | cis-trans isomer |
| Bb-73-1 | (300 MHz, CDCl₃) 7.03-6.94 (m, 3H), 6.80 (dq, 1H), 6.41 (br, 1H), 6.38 (dt, 1H), 6.04-5.90 (m, 2H), 5.21-5.08 (m, 1H), 4.33 (dt, 2H), 3.87-3.81 (m, 0.6H), 3.63 (ddd, 0.4H), 2.74 (dt, 2H), 2.54 (dt, 0.6H), 2.06-1.93 (m, 1.4H). | cis |
| Bb-75-3 | (300 MHz, CDCl₃) 7.03-6.91 (m, 4H), 6.80 (tt, 1H), 6.38 (dt, 1H), 6.06-5.97 (m, 1.6H), 5.93 (dt, 0.4H), 5.12 (t, 1H), 4.51 (quin, 2H), 3.89 (dsext, 0.2H), 3.67 (dd, 0.8H), 2.76 (ddd, 0.2H), 2.57 (dt, 0.8H), 2.06-1.96(m, 1H). | cis-trans isomer |
| Bb-76-1 | (300 MHz, CDCl₃) 8.22 (d, 1H), 7.09-6.89 (m, 3H), 6.77 (tt, 1H), 6.40 (dt, 1H), 6.00-5.86 (m, 2H), 5.05 (t, 1H), 3.94-3.80 (m, 1H), 3.18 (s, 3H), 2.99 (s, 3H), 2.31 (dt, 1H), 2.06 (d, 1H). | cis |

**Table 2 (continued)**

| Comp No. | ¹H-NMR (300 MHz or 500 MHz, measurement solvent) δ | Remarks |
|---|---|---|
| Bb-77-3 | (300 MHz, CDCl₃) 7.13 (d, 1H), 7.06-6.90 (m, 3H), 6.79 (tt, 1H), 6.39 (dt, 1H), 6.09-5.85 (m, 2H), 5.17 (br, 0.2H), 5.10 (t, 0.8H), 4.31 (t, 1.6H), 4.28 (t, 0.4H), 3.85-3.76 (m, 0.2H), 3.65-3.53 (m, 0.8H), 2.81-2.69 (m, 0.2H), 2.76 (t, 1.6H), 2.73 (t, 0.4H), 2.49 (dt, 0.8H), 2.16 (s, 2.4H), 2.15 (s, 0.6H), 2.02 (dt, 0.8H), 1.99-1.90 (m, 0.2H). | cis-trans isomer |
| Bb-79-3 | (300 MHz, CDCl₃) 7.40-7.29 (m, 1H), 7.22 (d, 1H), 7.15 (d, 1H), 7.11-6.97 (m, 2H), 6.90 (d, 0.7H), 6.37 (dt, 1H), 6.49-6.32 (m, 0.3H), 6.11-5.87 (m, 2H), 5.25-5.07 (m, 0.3H), 5.13 (t, 0.7H), 5.59-5.43 (m, 2H), 3.94-3.83 (m, 0.3H), 3.74-3.60 (m, 0.7H), 2.76 (ddd, 0.3H), 2.58 (dt, 0.7H), 2.03 (dt, 0.7H), 2.07-1.95 (m, 0.3H). | cis-trans isomer |
| Bb-80-2 | (300 MHz, CDCl₃) 7.08-6.89 (m, 3H), 6.80 (tt, 1H), 6.49-6.28 (m, 2H), 6.06-5.93 (m, 2H), 5.51 (br, 1H), 5.17 (br, 1H), 3.67-3.57 (m, 1H), 3.33-3.16 (m, 2H), 2.63 (ddd, 1H), 2.00 (ddd, 1H), 1.52 (sext, 2H), 0.92 (t, 3H). | trans |
| Bb-80-1 | (300 MHz, CDCl₃) 8.14 (d, 1H), 7.04-6.93 (m, 3H), 6.78 (tt, 1H), 6.39 (dt, 1H), 5.99-5.96 (m, 1H), 5.90 (dd, 1H), 5.78 (br, 1H), 5.07 (t, 1H), 3.31-3.21 (m, 3H), 2.32 (dt, 1H), 1.95 (d, 1H), 1.55 (sext, 2H), 0.94 (m, 3H). | cis |
| Bb-102-1 | (300 MHz, CDCl₃) 7.02-6.93 (m, 3H), 6.84 (brd, 1H), 6.79 (tt, 1H), 6.39 (dt, 1H), 5.90 (dt, 1H), 5.72 (dt, 1H), 5.04 (dd, 1H), 3.68 (s, 3H), 2.73 (dt, 1H), 2.78-2.68 (m, 1H), 2.53 (dd, 1H), 2.45 (dd, 1H), 1.41 (dt, 1H). | cis |
| Bb-114-3 | (300 MHz, CDCl₃) 7.33 (td, 1H), 7.21 (d, 1H), 7.14 (dt, 1H), 7.07-7.00 (m, 2H), 6.97 (br, 1H), 6.37 (dt, 1H), 6.06-5.97 (m, 2H), 5.12 (t, 1H), 3.64-3.59 (m, 1H), 2.53 (dt, 1H), 2.03 (dt, 1H). No proton peak of carboxy group detected. | cis-trans isomer |
| Bb-119-1 | (300 MHz, CDCl₃) 7.04-6.93 (m, 3H), 6.80 (tt, 1H), 6.78 (br, 1H), 6.38 (dt, 1H), 5.92-5.87 (m, 2H), 5.12 (sext, 1H), 3.14-3.06 (m, 1H), 2.78 (dt, 1H), 2.61 (dd, 1H), 2.53 (dd, 1H), 1.44 (dt, 1H). | cis |
| Bb-123-3 | (300 MHz, CDCl₃) 7.33 (td, 1H), 7.22 (d, 1.75H), 7.15 (dt, 1H), 7.08-7.00 (m, 2H), 6.49 (d, 0.25H), 6.38 (dt, 0.75H), 6.38 (dt, 0.25H), 6.02 (ddd, 0.25H), 5.99-5.92 (m, 1.5H), 5.86 (dt, 0.25H), 5.21-5.14 (m, 1H), 5.09 (t, 1H), 3.75-3.68 (m, 0.25H), 3.53-3.48 (m, 0.75H), 2.70 (ddd, 0.25H), 2.43 (dt, 0.75H), 1.98 (dt, 1H), 1.93-1.79 (m, 2H), 1.78-1.53 (m, 6H). | cis-trans isomer |

**Table 2 (continued)**

| Comp No. | ¹H-NMR (300 MHz or 500 MHz, measurement solvent) δ | Remarks |
|---|---|---|
| Bb-124-1 | (300 MHz, CDCl₃) 8.32 (d, 0.5H), 8.23 (d, 0.5H), 7.11-6.90 (m, 3H), 6.79 (tt, 1H), 6.41 (dt, 1H), 6.07-5.81 (m, 2H), 5.06 (dd, 1H), 3.84 (d, 1H), 3.68-3.30 (m, 2H), 3.14 (s, 1.5H), 2.97 (s, 1.5H), 2.41-2.22 (m, 1H), 2.08 (d, 0.5H), 2.03 (d, 0.5H), 1.27 (t, 1.5H), 1.13 (t, 1.5H). | cis |
| Bb-126-2 | (300 MHz, CDCl₃) 7.85 (br, 1H), 7.33 (dt, 1H), 7.21 (d, 1H), 7.14 (d, 1H), 7.09-6.98 (m, 2H), 6.38 (dt, 1H), 6.04-5.91 (m, 2H), 5.71 (br, 1H), 5.51 (br, 1H), 5.08 (t, 1H), 3.44-3.36 (m, 1H), 2.38 (dt, 1H), 1.99 (d, 1H). | trans |
| Bb-127-1 | (300 MHz, CDCl₃) 7.04-6.93 (m, 3H), 6.90 (d, 1H), 6.80 (tt, 1H), 6.38 (dt, 1H), 6.03-5.98 (m, 2H), 5.12 (dt, 1H), 4.79 (s, 2H), 3.70-3.62 (m, 1H), 2.59 (dt, 1H), 2.03 (dt, 1H). | cis |
| Bb-128-1 | (300 MHz, CDCl₃) 7.02-6.92 (m, 3H), 6.79 (tt, 1H), 6.62 (brd, 1H), 6.39 (dt, 1H), 5.89 (dt, 1H), 5.82 (dt, 1H), 5.10-5.03 (m, 1H), 3.66 (dd, 1H), 3.59 (dd, 1H), 3.23-3.13 (m, 1H), 2.67 (dt, 1H), 1.48 (dt, 1H). | cis |
| Bb-130-1 | (500 MHz, CDCl₃) 7.47(s, 1H), 7.46(s, 1H), 7.36(s, 1H), 7.13(br, 1H), 6.96(dt. 1H), 6.38 (dt, 1H), 5.98-5.93 (m, 2H), 5.07 (t, 1H), 3.73 (s, 3H), 3.56-3.54 (m, 1H), 2.45 (dt, 1H), 1.99 (dt, 1H). | cis |
| Bb-131-1 | (300 MHz, CDCl₃) 7.31-7.25 (m, 1H), 7.21-7.09 (m, 3H), 7.00 (dt, 1H), 6.31 (dt, 1H), 6.01-5.93 (m, 2H), 5.09 (t, 1H), 3.74 (s, 3H), 3.60-3.55 (m, 1H), 2.47 (dt, 1H), 2.00 (dt, 1H). | cis |
| Bb-132-1 | (500 MHz, CDCl₃) 7.24 (s, 1H), 7.13-7.11 (m, 3H), 6.98 (dt, 1H), 6.36 (dt, 1H), 6.00-5.93 (m, 2H), 5.09 (t, 1H), 3.72 (s, 3H), 3.58-3.55 (m, 1H), 2.49-2.44 (m, 1H), 2.41 (s, 3H), 2.02-1.96 (m, 1H). | cis |
| Bb-133-3 | (300 MHz, CDCl₃) 7.22 (d, 1H), 7.04-6.94 (m, 3H), 6.79 (dt, 1H), 6.39 (dt, 1H), 6.05 (ddd, 0.2H), 6.01-5.93 (m, 1.6H), 5.87 (dt, 0.2H), 5.21-5.15 (m, 0.2H), 5.09 (t, 0.8H), 4.19 (q, 1.6H), 4.16 (q, 0.4H), 3.76 (dsext, 0.2H), 3.55 (ddd, 0.8H), 2.73 (ddd, 0.2H), 2.45 (dt, 0.8H), 2.00 (dt, 1H), 1.30 (t, 3H). | cis-trans isomer |
| Bb-134-3 | (300 MHz, CDCl₃) 7.23 (d, 1H), 7.03 (t, 1H), 7.00-6.94 (m, 2H), 6.79 (dt, 1H), 6.39 (dt, 1H), 6.07-5.86 (m, 2H), 5.20-5.13 (m, 0.2H), 5.09 (t, 0.8H), 4.09 (t, 1.6H), 4.06 (t, 0.4H), 3.77 (dsext, 0.2H), 3.57 (ddd, 0.8H), 2.74 (ddd, 0.2H), 2.45 (dt, 0.8H), 2.02-1.90 (m, 1H), 1.69 (q, 2H), 0.96 (t, 3H). | cis-trans isomer |

**Table 2 (continued)**

| Comp No. | ¹H-NMR (300 MHz or 500 MHz, measurement solvent) δ | Remarks |
|---|---|---|
| Bb-135-3 | (300 MHz, CDCl₃) 7.24 (d, 1H), 7.03-6.93 (m, 3H), 6.79 (tt, 1H), 6.39 (dt, 1H), 6.04 (ddd, 0.2H), 6.02-5.93 (m, 1.6H), 5.87 (dt, 0.2H), 5.09 (t, 1H), 5.03 (sep, 1H), 3.72 (dsext, 0.2H), 3.51 (ddd, 0.8H), 2.71 (ddd, 0.2H), 2.43 (dt, 0.8H), 1.99 (dt, 1H), 1.27 (d, 3H), 1.26 (d, 3H). | cis-trans isomer |
| Bb-136-3 | (300 MHz, CDCl₃) 7.22 (d, 1H), 7.04-6.94 (m, 3H), 6.79 (dt, 1H), 6.39 (dt, 1H), 6.04-6.01 (m, 0.2H), 5.99-5.92 (m, 1.6H), 5.88-5.84 (m, 0.2H), 5.21-5.15 (m, 1H), 5.08 (t, 1H), 3.72 (dsext, 0.2H), 3.51 (ddd, 0.8H), 2.75 (ddd, 0.2H), 2.43 (dt, 0.8H), 1.98 (dt, 1H), 1.93-1.82 (m, 2H), 1,76-1.59 (m, 6H). | cis-trans isomer |
| Bb-137-1 | (300 MHz, CDCl₃) 9.64 (s, 1H), 8.07 (d, 1H), 7.03-6.96 (m, 3H), 6.78 (tt, 1H), 6.38 (dt, 1H), 5.97-5.85 (m, 2H), 5.07 (t, 1H), 4.98 (q, 2H), 3.31 (d, 1H), 2.436 (dt, 1H), 1.93 (d, 1H), 1.26 (t, 3H). | cis |
| Bb-137-2 | (300 MHz, CDCl₃) 8.16 (br, 1H), 7.02-6.91 (m, 3H), 6.80 (tt, 1H), 6.44-6.31 (m, 1H), 6.38 (dt, 1H), 5.97 (br, 2H), 5.23-5.04 (m, 1H), 3.97 (q, 2H), 3.56 (br, 1H), 2.68 (br, 1H), 1.99 (br, 1H), 1.33-1.25 (m, 3H). | trans |
| Bb-138-1 | (300 MHz, CDCl₃) 9.52 (s, 1H), 8.08 (d, 1H), 7.02-6.93 (m, 3H), 6.78 (tt, 1H), 6.37 (dt, 1H), 5.99-5.81 (m, 2H), 5.07 (m, 1H), 4.15 (quin, 1H), 3.35 (d, 1H), 2.35 (m, 1H), 1.93 (d, 1H), 1.24 (m, 6H). | cis |
| Bb-138-2 | (300 MHz, CDCl₃) 7.99 (br, 1H), 7.03-6.91 (m, 3H), 6.80 (tt, 1H), 6.44-6.31 (m, 1H), 6.38 (dt, 1H), 5.97 (br, 2H), 5.18 (br, 1H), 4.13 (br, 1H), 3.56 (br, 1H), 2.68 (br, 1H), 2.00 (br, 1H), 1.28-1.22 (m, 6H). | trans |
| Bb-139-1 | (300 MHz, CDCl₃) 9.36 (s, 0.8H), 8.76 (s, 0.2H), 8.02 (d, 0.8H), 7.90 (d, 0.2H), 7.03-6.95 (m, 3H), 6.78 (tt, 1H), 6.38 (dt, 1H), 6.02-5.82 (m, 2H), 5.07 (t, 1H), 3.99 (d, 0.2H), 3.88 (t, 2H), 3.29 (d, 0.8H), 2.35 (dt, 1H), 1.93 (d, 1H), 1.68 (sext, 2H), 1.02-0.92 (m, 0.6H), 0.95 (t, 2.4H). | cis |

**Table 2 (continued)**

| Comp No. | ¹H-NMR (300 MHz or 500 MHz, measurement solvent) δ | Remarks |
|---|---|---|
| Bb-139-2 | (300 MHz, CDCl₃) 8.16 (br, 1H), 7.01-6.91 (m, 3H), 6.80 (tt, 1H), 6.44-6.31 (m, 1H), 6.38 (dt, 1H), 5.96 (br, 2H), 5.23-5.10 (m, 1H), 3.86 (t, 2H), 3.54 (br, 1H), 2.68 (br, 1H), 1.98 (br, 1H), 1.68 (sext, 2H), 0.97 (t, 3H). | trans |
| Bb-141-1 | (300 MHz, CDCl₃) 7.46 (br, 1H), 7.06-6.90 (m, 3H), 6.79 (tt, 1H), 6.39 (dt, 1H), 6.07-5.82 (m, 2H), 5.22-5.04 (m, 1H), 5.02-4.88 (m, 1H), 4.60-4.38 (m, 1H), 3.66 (s, 3H), 2.88 (dt, 1H), 1.65 (d, 1H). | cis |
| Bb-142-1 | (300 MHz, CDCl₃) 7.43 (br, 1H), 7.04-6.89 (m, 3H), 6.79 (tt, 1H), 6.38 (dt, 1H), 5.90-5.76 (m, 2H), 4.98 (t, 1H), 3.42 (ddd, 2H), 3.40 (s, 3H), 2.95-2.85 (m, 1H), 2.45 (dt, 1H), 1.55 (dt, 1H). | cis |
| Bb-143-3 | (300 MHz, CDCl₃) 7.10 (d, 1H), 7.07-6.94 (m, 3H), 6.89-6.76 (m, 1H), 6.47-6.32 (m, 0.25H), 6.41 (dt, 0.75H), 6.09-5.90 (m, 2H), 5.18 (br, 0.25H), 5.12 (t, 0.75H), 4.63 (t, 1.5H), 4.60 (t, 0.5H), 3.88-3.79 (m, 0.25H), 3.66-3.55 (m, 0.75H), 3.39 (t, 1H), 3.37 (t, 1H), 3.03 (s, 2.25H), 3.01 (s, 0.75H), 2.76 (ddd, 0.25H), 2.55 (dt, 0.75H), 2.07 (dt, 0.75H), 2.00 (ddd, 0.25H). | cis-trans isomer |
| Bb-144-1 | (300 MHz, CDCl₃) 7.04-6.93 (m, 3H), 6.79 (tt, 1H), 6.71 (d, 1H), 6.39 (dt, 1H), 5.90 (dt, 1H), 5.86 (dt, 1H), 5.06 (t, 1H), 4.25 (qd, 2H), 3.12-3.06 (m, 3H), 2.65 (dt, 1H), 1.96-1.83 (m, 2H), 1.55 (dt, 1H), 1.08 (t, 3H). | cis |
| Bb-145-1 | (300 MHz, CDCl₃) 7.04-6.93 (m, 3H), 6.79 (tt, 1H), 6.71 (d, 1H), 6.39 (dt, 1H), 5.90 (dt, 1H), 5.86 (dt, 1H), 5.06 (t, 1H), 4.25 (qd, 2H), 3.14-3.08 (m, 2H), 3.06 (br, 1H), 2.65 (dt, 1H), 1.85-1.80 (m, 1H), 1.83 (quin, 1H), 1.55 (dt, 1H), 1.47 (sep, 2H), 0.96 (t, 3H). | cis |
| Bb-146-1 | (300 MHz, CDCl₃) 7.03-6.93 (m, 3H), 6.81 (br, 1H), 6.79 (tt, 1H), 6.39 (dt, 1H), 5.91 (dt, 1H), 5.86 (dt, 1H), 5.06 (t, 1H), 4.27 (qd, 2H), 3.31 (sep, 1H), 3.08 (br, 1H), 2.65 (dt, 1H), 1.56 (dt, 1H), 1.43 (d, 6H). | cis |

**Table 2 (continued)**

| Comp No. | ¹H-NMR (300 MHz or 500 MHz, measurement solvent) δ | Remarks |
|---|---|---|
| Bb-147-1 | (300 MHz, CDCl₃) 7.04-6.93 (m, 3H), 6.80 (tt, 1H), 6.43 (br, 1H), 6.39 (dt, 1H), 5.94 (dt, 1H), 5.69 (dt, 1H), 5.05-4.97 (m, 1H), 4.25-4.17 (m, 1H), 4.12-4.04 (m, 1H), 2.84-2.73 (m, 1H), 2.70 (dt, 1H), 2.06 (s, 3H), 1.89-1.78 (m, 1H), 1.77-1.65 (m, 1H), 1.29-1.21 (m, 1H). | cis |
| Bb-148-1 | (300 MHz, CDCl₃) 7.03-6.93 (m, 3H), 6.80 (tt, 1H), 6.48 (d, 1H), 6.39 (dt, 1H), 5.94 (dt, 1H), 5.73 (dt, 1H), 5.06-4.98 (m, 1H), 4.36-4.24 (m, 2H), 3.02 (s, 3H), 2.91-2.81 (m, 1H), 2.73 (dt, 1H), 2.04-1.92 (m, 1H), 1.88-1.77 (m, 1H), 1.33-1.24 (m, 1H). | cis |
| Bb-149-1 | (300 MHz, CDCl₃) 7.00-6.91 (m, 4H), 6.78 (dt, 1H), 6.38 (dt, 1H), 5.93 (dt, 1H), 5.70 (dt, 1H), 5.00-4.94 (m, 1H), 3.76-3.65 (m, 2H), 2.89-2.77 (m, 1H), 2.64 (dt, 1H), 2.41 (br, 1H), 1.85-1.74 (m, 1H), 1.67-1.56 (m, 1H), 1.36 (dt, 1H). | cis |
| Bb-150-1 | (300 MHz, CDCl₃) 7.26-6.92 (m, 3H), 6.80 (tt, 1H), 6.60 (d, 1H), 6.39 (dt, 1H), 5.87 (dt, 1H), 5.82 (dt, 1H), 5.10-5.03 (m, 1H), 3.54 (dd, 1H), 3.47 (dd, 1H), 3.26-3.16 (m, 1H), 2.69 (dt, 1H), 1.44 (dt, 1H). | cis |
| Bb-151-1 | (300 MHz, CDCl₃) 7.04-6.94 (m, 3H), 6.80 (tt, 1H), 6.60 (d, 1H), 6.39 (dt, 1H), 5.87-5.82 (m, 2H), 5.09-5.02 (m, 1H), 3.36 (dd, 1H), 3.28 (dd, 1H), 3.01-2.92 (m, 1H), 2.69 (dt, 1H), 1.30 (dt, 1H). | cis |
| Bb-152-1 | (300 MHz, CDCl₃) 7.10-6.92 (m, 3H), 6.81 (tt, 1H), 6.72 (d, 1H), 6.41 (dt, 1H), 5.93 (dt, 1H), 5.88 (dt, 1H), 5.15-5.02 (m, 1H), 4.28 (ddd, 2H), 3.17 (q, 2H), 3.10 (br, 1H), 2.67 (dt, 1H), 1.57 (dt, 1H), 1.45 (t, 3H). | cis |
| Bb-153-1 | Diastereomeric mixture. (300 MHz, CDCl₃) 7.40 (d, 0.66H), 7.24 (d, 0.34H), 7.08-6.90 (m, 3H), 6.81 (tt, 1H), 6.40 (dt, 1H), 6.06-5.80 (m, 2H), 5.09-4.84 (m, 1H), 4.03-3.93 (m, 0.34H), 3.93-3.81 (m, 0.66H), 2.84-2.67 (m, 1H), 2.50 (dt, 0.66H), 2.44-2.31 (m, 0.34H), 1.69 (dt, 0.34H), 1.65-1.49 (m, 1.66H), 1.31 (d, 1.98H), 1.27 (d, 1.02H). | cis |
| Bb-154-1 | Diastereomeric mixture. (300 MHz, CDCl₃) 7.09-6.92 (m, 3H), 6.81 (tt, 1H), 6.74 (br, 1H), 6.41 (dt, 1H), 6.01 (dt, 0.66H), 5.95-5.85 (m, 1.34H), 5.16-4.98 (m, 1H), 4.97-4.75 (m, 1H), 3.07 (s, 1.98H), 3.03 (s, 1.02H), 3.01-2.86 (m, 1H), 2.67 (dt, 0.66H), 2.55 (dt, 0.34H), 1.69 (dt, 0.34H), 1.54 (dt, 0.66H), 1.49 (d, 1.98H), 1.48 (d, 1.02H). | cis |

**Table 2 (continued)**

| Comp No. | ¹H-NMR (300 MHz or 500 MHz, measurement solvent) δ | Remarks |
|---|---|---|
| Bb-155-1 | (300 MHz, CDCl₃) 7.25 (br, 1H), 7.00-6.92 (m, 3H), 6.79 (tt, 1H), 6.38 (dt, 1H), 6.01 (ddd, 1H), 5.87 (ddd, 1H), 4.97 (t, 1H), 2.71-2.66 (m, 1H), 2.44-2.30 (m, 1H), 1.69 (dt, 1H), 1.68 (br, 1H), 1.30 (d, 6H). | cis |
| Bb-156-1 | Compound in which difluorobutenoic acid amide group and R⁴ (3-CO₂Me) are in cis-configuration. (300 MHz, CDCl₃) 7.24 (d, 1H), 7.07-6.90 (m, 3H), 6.79 (tt, 1H), 6.39 (dt, 1H), 5.90-5.80 (m, 2H), 5.12 (t, 1H), 3.74 (s, 3H), 2.27 (dd, 1H), 2.11 (dd, 1H), 1.39 (s, 3H). | cⁱs |
| Bb-157-1 | Compound in which difluorobutenoic acid amide group and R⁴ (3-CH₂OSO₂Me) are in cis-configuration. (300 MHz, CDCl₃) 7.27-6.95 (m, 3H), 6.79 (tt, 1H), 6.76 (br, 1H), 6.39 (dt, 1H), 5.77 (dd, 1H), 5.72 (dd, 1H), 5.15-5.09 (m, 1H), 4.13-4.02 (m, 2H), 3.03 (s, 3H), 2.21 (dd, 1H), 1.77 (dd, 1H), 1.16-1.15 (m, 3H). | cis |
| Bb-158-1 | (300 MHz, CDCl₃) 7.02-6.93 (m, 3H), 6.76 (tt, 1H), 6.53 (br, 1H), 6.37 (dt, 1H), 5.93 (dt, 1H), 5.83 (dt, 1H), 5.09-5.03 (m, 1H), 4.53 (ddd, 1H), 4.37 (ddd, 1H), 3.07-2.91 (m, 1H), 2.61 (dt, 1H), 1.55 (dt, 1H). | cis |
| Bb-159-1 | (300 MHz, CDCl₃) 7.03-6.93 (m, 3H), 6.80 (tt, 1H), 6.61 (q, 1H), 6.49 (br, 1H), 6.37 (dt, 1H), 5.21-5.13 (m, 1H), 3.77 (s, 3H), 2.81-2.69 (m, 1H), 2.66-2.56 (m, 2H), 1.84-1.71 (m, 1H). | cis |
| Bb-160-1 | (300 MHz, CDCl₃) 7.05-6.93 (m, 3H), 6.80 (tt, 1H), 6.46 (brd, 1H), 6.38 (dt, 1H), 5.63-5.61 (m, 1H), 5.04 (brs, 1H), 4.66 (s, 2H), 2.58-2.46 (m, 2H), 2.40-2.29 (m, 1H), 2.11 (s, 3H), 1.79-1.68 (m, 1H). | cis |
| Bb-161-1 | (300 MHz, CDCl₃) 7.02-6.92 (m, 3H), 6.80 (tt, 1H), 6.42 (brs, 1H), 6.38 (dt, 1H), 5.65-5.62 (m, 1H), 5.03 (brs, 0.7H), 4.70-4.59 (m, 0.3H), 4.24-4.21 (m, 2H), 2.92-2.79 (m, 0.5H), 2.57-2.45 (m, 1.5H), 2.39-2.25 (m, 1H), 1.79-1.69 (m, 2H). | cis |

**Table 2 (continued)**

| Comp No. | ¹H-NMR (300 MHz or 500 MHz, measurement solvent) δ | Remarks |
|---|---|---|
| Bb-162-1 | (300 MHz, CDCl₃) 8.27 (d, 0.5H), 8.15 (d, 0.5H), 7.10-6.91 (m, 3H), 6.79 (tt, 1H), 6.51-6.32 (m, 1H), 6.04-5.66 (m, 3H), 5.36-5.13 (m, 2H), 5.08 (t, 1H), 4.22-3.92 (m, 2H), 3.92-3.73 (m, 1H), 3.12 (s, 1.5H), 2.99 (s, 1.5H), 2.42-2.23 (m, 1H), 2.09 (d, 0.5H), 2.04 (d, 0.5H). | cis |
| Bb-163-3 | (300 MHz, CDCl₃) 7.37-7.29 (m, 1H), 7.22-7.13 (m, 2.75H), 7.06-7.01 (m, 2H), 6.45-6.31 (m, 1.25H), 6.06-6.02 (m, 0.25H), 6.01-5.93 (m, 1.5H), 5.89-5.86 (m, 0.25H), 5.17 (br, 0.25H), 5.09 (t, 0.75H), 4.22-4.12 (m, 2H), 3.79-3.70 (m, 0.25H), 3.57-3.49 (m, 0.75H), 2.77-2.67 (m, 0.25H), 2.51-2.39 (m, 0.75H), 2.03-1.90 (m, 1H), 1.32-1.23 (m, 3H). | cis-trans isomer |
| Bb-164-3 | (300 MHz, CDCl₃) 7.33 (td, 1H), 7.22 (d, 1.75H), 7.15 (d, 1H), 7.08-7.01 (m, 2H), 6.45-6.39 (m, 0.25H), 6.38 (dt, 0.75H), 6.38 (dt, 0.25H), 6.04 (ddd, 0.25H), 6.01-5.93 (m, 1.5H), 5.87 (dt, 0.25H), 5.23-5.13 (m, 0.25H), 5.10 (t, 0.75H), 4.09 (t, 1.5H), 4.06 (t, 0.5H), 3.80-3.73 (m, 0.25H), 3.58-3.53 (m, 0.75H), 2.73 (ddd, 0.25H), 2.46 (dt, 0.75H), 2.00 (dt, 0.75H), 1.99-1.90 (m, 0.25H), 1.68 (sext, 2H), 0.96 (t, 2.25H), 0.94 (t, 0.75H). | cis-trans isomer |
| Bb-165-3 | (300 MHz, CDCl₃) 7.37-7.30 (m, 1H), 7.22 (d, 1.75H), 7.15 (d, 1H), 7.08-7.01 (m, 2H), 6.45-6.39 (m, 0.25H), 6.38 (dt, 0.75H), 6.38 (dt, 0.25H), 6.03 (ddd, 0.25H), 6.00-5.93 (m, 1.5H), 5.87 (dt, 0.25H), 5.22-5.12 (m, 0.25H), 5.09 (t, 0.75H), 5.03 (sep, 1H), 3.75-3.69 (m, 0.25H), 3.53-3.48 (m, 0.75H), 2.71 (ddd, 0.25H), 2.44 (dt, 0.75H), 1.98 (dt, 0.75H), 1.98-1.89 (m, 0.25H), 1.26 (dd, 4.5H), 1.24 (dd, 1.5H). | cis-trans isomer |
| Bb-262-1 | (500 MHz, CDCl₃) 7.67 (s, 1H), 7.61 (s, 1H), 7.60 (s, 1H), 7.20 (d, 1H), 7.03 (dt, 1H), 6.46 (dt, 1H), 6.01-5.95 (m, 2H), 5.08 (t, 1H), 3.75 (s, 3H), 3.58-3.56 (m, 1H), 2.45 (dt, 1H), 2.02 (dt, 1H). | cis |
| Bb-265-1 | (500 MHz, CDCl₃) 7.92 (s, 1H), 7.91 (s, 1H), 7.87 (s, 1H), 7.23 (d, 1H), 7.13 (dt, 1H), 6.54 (dt, 1H), 6.01-5.95 (m, 2H), 5.09 (t, 1H), 3.75 (s, 3H), 3.59-3.56 (m, 1H), 2.46 (dt, 1H), 2.01 (dt, 1H). | cis |
| Bb-267-1 | (500 MHz, CDCl₃) 7.83 (s, 1H), 7.81 (s, 1H), 7.76 (s, 1H), 7.22 (d, 1H), 7.11 (dt, 1H), 6.68 (t, 1H), 6.52 (dt, 1H), 6.01-5.94 (m, 2H), 5.09 (t, 1H), 3.75 (s, 3H), 3.59-3.56 (m, 1H), 2.46 (dt, 1H), 2.01 (dt, 1H). | cis |

**Table 2 (continued)**

| Comp No. | ¹H-NMR (300 MHz or 500 MHz, measurement solvent) δ | Remarks |
|---|---|---|
| Bc-1 | (300 MHz, CDCl₃) 7.02-6.93 (m, 3H), 6.80 (tt, 1H), 6.78-6.74 (m, 1H), 6.65 (d, 1H), 6.37 (dt, 1H), 4.73-4.62 (m, 1H), 3.76 (s, 3H), 3.12-2.97 (m, 2H), 2.59-2.43 (m, 2H). | - |
| Bc-1 (R) | (500 MHz, CDCl₃) 7.00-6.96 (m, 3H), 7.80 (tt, 1H), 6.76 (t, 1H), 6.56 (d, 1H), 6.37 (dt, 1H), 4.70-4.64 (m, 1H), 3.76 (s, 3H), 3.10-2.99 (m, 2H), 2.57-2.46 (m, 2H). | R-form |
| Bc-1(S) | (500 MHz, CDCl₃) 7.00-6.94 (m, 3H), 7.80 (tt, 1H), 6.75 (t, 1H), 6.57 (d, 1H), 6.37 (dt, 1H), 4.70-4.64 (m, 1H), 3.79 (s, 3H), 3.10-2.99 (m, 2H), 2.57-2.44 (m, 2H). | S-form |
| Bc-3 | (300 MHz, CDCl₃) 7.34 (dt, 1H), 7.22 (d, 1H), 7.15 (d, 1H), 7.10-6.98 (m, 2H), 6.80-6.71 (m, 1H). 6.55 (br, 1H), 6.36 (dt, 1H), 4.76-4.61 (m, 1H), 3.76 (s, 3H), 3.15-2.94 (m, 2H), 2.61-2.40 (m, 2H). | - |
| Bc-5 | (300 MHz, CDCl₃) 7.53 (s, 1H), 7.41-7.34 (m, 2H), 7.03 (dt, 1H), 6.76 (t, 1H), 6.56 (d, 1H), 6.44 (dt, 1H), 4.70-4.62 (m, 1H), 3.76 (s, 3H), 3.10-3.00 (m, 2H), 2.57-2.46 (m, 2H). | - |
| Bc-5(S) | (300 MHz, CDCl₃) 7.53 (s, 1H), 7.42-7.32 (m, 2H), 7.03 (dt, 1H), 6.76 (t, 1H), 6.56 (d, 1H), 6.45 (dt, 1H), 4.73-4.62 (m, 1H), 3.77 (s, 3H), 3.13-2.98 (m, 2H), 2.59-2.44 (m, 2H). | S-form |
| Bc-44 | (300 MHz, CDCl₃) 7.07-6.89 (m, 3H), 6.80 (tt, 1H), 6.63 (d, 1H), 6.38 (dt, 1H), 5.68-5.60 (m, 1H), 4.72-4.56 (m, 1H), 4.22 (s, 2H), 2.96-2.75 (m, 2H), 2.37-2.21 (m, 2H), 1.69-1.54 (m, 1H). | - |

**Table 2 (continued)**

| Comp No. | ¹H-NMR (300 MHz or 500 MHz, measurement solvent) δ | Remarks |
|---|---|---|
| Bc-48 | (300 MHz, acetone-ds) 8.18 (br, 1H), 7.26-6.88 (m, 5H), 6.63-6.50 (m, 2H), 4.56-4.45 (m, 1H), 2.89-2.77 (m, 2H), 2.50-2.39 (m, 2H). | - |
| Bc-49 | (300 MHz, acetone-ds) 10.08 (br, 1H), 8.14 (br,1H), 7.22-7.17 (m, 2H), 6.99-6.88 (m, 2H), 6.56 (dt, 1H), 6.35-6.31 (m, 1H), 4.53-4.42 (m, 1H), 3.55 (s, 3H), 2.86-2.71 (m, 2H), 2.49-2.34 (m, 2H). | - |
| Bc-53 | (300 MHz, CDCl₃) 7.02-6.93 (m, 3H), 6.80 (tt, 1H), 6.56 (d, 1H), 6.38 (dt, 1H), 5.68 (t, 1H), 4.70-4.61 (m, 3H), 2.93-2.81 (m, 2H), 2.33-2.22 (m, 2H), 2.09 (s, 3H). | - |
| Bc-72 | (300 MHz, CDCl₃) 7.02-6.93 (m, 3H), 6.85-6.76 (m, 2H), 6.58 (d, 1H), 6.37 (dt, 1H), 4.77 (d, 2H), 4.74-4.63 (m, 1H), 3.15-2.99 (m, 2H), 2.60-2.46 (m, 3H). | - |
| Bc-73 | (300 MHz, CDCl₃) 7.03-6.94 (m, 3H), 6.88-6.86 (m, 1H), 6.80 (tt, 1H), 6.61 (d, 1H), 6.38 (dt, 1H), 4.74-4.64 (m, 1H), 4.38 (t, 2H), 3.14-3.01 (m, 2H), 2.77 (t, 2H), 2.61-2.46 (m, 2H). | - |
| Bc-60 | (300 MHz, CDCl₃) 7.05-6.90 (m, 3H), 6.79 (tt, 1H), 6.73 (d, 1H), 6.69-6.62 (m, 1H), 6.38 (dt, 1H), 4.72-4.54 (m, 1H), 4.00 (t, 2H), 3.81 (dd, 2H), 3.23-3.09 (m, 1H), 3.08-2.92 (m, 1H), 2.73-2.61 (m, 1H), 2.54-2.42 (m, 1H), 2.33 (quin, 2H). | - |
| Bc-126 | (300 MHz, CDCl₃) 7.34 (dt, 1H), 7.22 (d, 1H),7.15 (d, 1H), 7.10-6.98 (m, 2H), 6.65 (br, 1H), 6.52 (t, 1H), 6.36 (dt, 1H), 5.54 (br, 2H), 4.76-4.63 (m, 1H), 3.15-2.96 (m, 2H), 2.53 (t, 2H). | - |
| Bc-127 | (300 MHz, CDCl₃) 7.03-6.91 (m, 4H), 6.80 (tt, 1H), 6.67 (d, 1H), 6.37 (dt, 1H), 4.81 (s, 2H), 4.75-4.65 (m, 1H), 3.12-3.02 (m, 2H), 2.64-2.50 (m, 2H). | - |
| Bc-128 | (300 MHz, CDCl₃) 7.05-6.90 (m, 3H), 6.80 (tt, 1H), 6.57 (d, 1H), 6.38 (dt, 1H), 5.79-5.72 (m, 1H), 4.74-4.60 (m, 1H), 4.22-4.07 (m, 2H), 2.98-2.81 (m, 2H), 2.43-2.25 (m, 2H). | - |
| Bc-129 | (300 MHz, CDCl₃) 7.08-6.90 (m, 4H), 6.79 (tt, 1H), 6.38 (dt, 1H), 5.95-5.85 (m, 1H), 4.73-4.58 (m, 1H), 3.13-2.91 (m, 8H), 2.71-2.59 (m, 1H), 2.52-2.39 (m, 1H). | - |

**Table 2 (continued)**

| Comp No. | ¹H-NMR (300 MHz or 500 MHz, measurement solvent) δ | Remarks |
|---|---|---|
| Bc-223 | (300 MHz, CDCl₃) 7.46 (d, 1H), 7.02-6.93 (m, 3H), 6.80 (tt, 1H), 6.54 (d, 1H), 6.38 (dt, 1H), 6.14 (s, 1H), 5.77 (d, 1H), 4.75-4.65 (m, 1H), 3.77 (s, 3H), 3.00 (td, 2H), 2.43 (t, 2H). | - |
| Bc-262 | (500 MHz, CDCl₃) 7.66 (s, 1H), 7.60 (s, 2H), 7.01 (d, 1H), 6.75 (d, 1H), 6.57 (d, 1H), 6.45 (dt, 1H), 4.69-4.65 (m, 1H), 3.76 (s, 3H), 3.10-3.00 (m, 2H), 2.57-2.46 (m, 2H). | - |
| Bc-265 | (500 MHz, CDCl₃) 7.91 (s, 1H), 7.90 (s, 1H), 7.88 (s, 1H), 7.11 (dt, 1H), 6.76 (t, 1H), 6.59 (d, 1H), 6.53 (dt, 1H), 4.71-4.64 (m, 1H), 3.77 (s, 3H), 3.11-3.00 (m, 2H), 2.58-2.45 (m, 2H). | - |
| Bc-267 | (500 MHz, CDCl₃) 7.82 (s, 1H), 7.81 (s, 1H), 7.77 (s, 1H), 7.10 (dt, 1H), 6.76 (t, 1H), 6.68 (t, 1H), 6.56 (d, 1H), 6.50 (dt, 1H), 4.71-4.65 (m, 1H), 3.77 (s, 3H), 3.10-3.01 (m, 2H), 2.58-2.45 (m, 2H). | - |

### Test Example 1: (1-1) Herbicidal effect on barnyardgrass (foliar treatment)

Upland soil was put into a 1/300,000 hectare (ha) pot, seeds of barnyardgrass (Echinochloa crus-galli) were sown, and about 1.5 cm of cover soil was put. The barnyardgrass was grown for 10 days in a greenhouse until it reached a predetermined leaf stage. The Invention Compound was weighed to achieve a predetermined active ingredient amount, dissolved in acetone (containing 0.5 vol% of surfactant (TritonX-100: manufactured by Kishida Chemical Co., Ltd.)) or dimethyl sulfoxide and diluted with water (containing 0.1 vol% of agricultural spreader (Surfactant WK: manufactured by MARUWA Biochemical Co., Ltd.)) in an amount corresponding to 1,000 liter per hectare, to prepare a spray solution. The spray solution was sprayed over the grown barnyardgrass with a small sprayer for foliar treatment. On the 14th day after the foliar treatment, the state of growth of each plant was visually observed. As a result, compositions containing the following compounds (active ingredient dose: 125, 250, 500 or 1,000 g/ha) showed a growth inhibition rate of 70% or higher on barnyardgrass.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-62-1, Aa-63-1, Aa-81-1, Ba-11-1, Ba-44-1, Ba-45-1, Ba-53-1, Ba-58-1, Bb-1-4, Bb-9-1, Bb-11-1, Bb-12-1, Bb-13-1, Bb-15-1, Bb-26-1, Bb-27-1, Bb-44-1, Bb-50-1, Bb-50-2, Bb-55-1, Bb-61-2, Bb-80-2, Bb-126-2, Bb-142-1, Bb-144-1, Bb-146-1, Bb-149-1, Bb-150-1, Bb-151-1, Bb-153-1, Bb-154-1, Bb-158-1, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-18-1, Aa-19-1, Aa-20-1, Aa-21-1, Aa-23-1, Aa-25-1, Aa-26-1, Aa-26-2, Aa-27-1, Aa-28-1, Aa-29-1, Aa-30-1, Aa-31-1, Aa-32-1, Aa-33-1, Aa-34-1, Aa-35-1, Aa-36-1, Aa-38-1, Aa-1-2, Aa-2-2, Aa-4-2, Aa-29-2, Aa-32-2, Aa-44-1, Aa-48-1, Aa-48-1 (Na salt), Aa-49-1, Aa-49-2, Aa-50-1, Aa-54-1, Aa-57-1, Aa-58-3, Aa-60-1, Aa-61-1, Aa-64-1, Aa-66-1, Aa-67-1, Aa-68-1, Aa-70-1, Aa-71-1, Aa-72-1, Aa-73-1, Aa-74-1, Aa-75-1, Aa-76-1, Aa-77-1, Aa-82-1, Aa-83-1, Aa-84-1, Aa-85-1, Aa-85-2, Aa-86-1, Aa-87-1, Aa-88-1, Aa-89-1, Aa-90-1, Aa-91-1, Aa-92-1, Aa-93-1, Aa-94-1, Aa-97-1, Aa-99-1, Aa-101-1, Aa-102-3, Aa-103-1, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-171-1, Aa-172-1, Aa-173-1, Aa-174-1, Aa-175-1, Aa-176-1, Aa-177-1, Aa-178-1, Aa-180-1, Aa-181-1, Aa-182-1, Aa-184-1, Aa-185-1, Aa-191-1, Aa-194-1, Aa-195-1, Aa-196-1, Aa-197-1, Aa-198-1, Aa-199-1, Aa-201-1, Aa-202-1, Aa-203-1, Aa-204-1, Aa-205-1, Aa-206-1, Aa-207-1, Aa-208-1, Aa-209-1, Aa-210-1, Aa-212-1, Aa-213-1, Aa-214-1, Aa-215-1, Aa-216-1 (HCl salt), Aa-217-1, Aa-218-1, Aa-219-3, Aa-220-2, Aa-220-3, Aa-221-3, Aa-222-3, Aa-223-2, Aa-223-3, Aa-224-3, Aa-225-3, Aa-226-1, Aa-226-2, Aa-227-1, Aa-228-2, Aa-229-2, Aa-230-3, Aa-232-3, Aa-233-1, Aa-234-1, Aa-235-1, Aa-236-1, Aa-238-1, Aa-240-1, Aa-241-1, Aa-242-1, Aa-243-1, Aa-244-1, Aa-246-1, Aa-247-1, Aa-248-1, Aa-249-1, Aa-250-3, Aa-253-1, Aa-254-1, Aa-255-1, Aa-256-1, Aa-257-1, Aa-257-2, Aa-258-1, Aa-259-1, Aa-259-2, Aa-260-1, Aa-261-2, Aa-262-1, Aa-262-2, Aa-263-1, Aa-264-1, Aa-265-1, Aa-265-2, Aa-263-2, Aa-267-1, Aa-272-1, Aa-272-2, Aa-273-2, Aa-277-2, Aa-278-2, Aa-279-2, Aa-280-2, Aa-281-2, Ba-1-2, Ba-64-1, Ba-76-1, Ba-119-1, Ba-223-1, Ba-226-1, Bb-1-2, Bb-1-5, Bb-2-1, Bb-5-1, Bb-5-5, Bb-7-1, Bb-45-1, Bb-49-1, Bb-49-2, Bb-60-1, Bb-60-2, Bb-64-1, Bb-71-3, Bb-73-1, Bb-76-1, Bb-77-3, Bb-102-1, Bb-119-1, Bb-124-1, Bb-127-1, Bb-128-1, Bb-130-1, Bb-131-1, Bb-132-1, Bb-137-2, Bb-139-1, Bb-139-2, Bb-141-1, Bb-143-3, Bb-147-1, Bb-152-1, Bb-156-1, Bb-157-1, Bb-159-1, Bb-262-1, Bb-265-1, Bb-267-1, Bc-1, Bc-1(R), Bc-1(S), Bc-3, Bc-5(R), Bc-44, Bc-48, Bc-49, Bc-53, Bc-60, Bc-72, Bc-73, Bc-127, Bc-129, Bc-223-1, Bc-262, Bc-265

### (1-2) Herbicidal effect on crabgrass (foliar treatment)

To confirm herbicidal effect on crabgrass (Digitaria sanguinalis), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on crabgrass.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-62-1, Ba-11-1, Ba-44-1, Ba-45-1, Ba-53-1, Ba-58-1, Bb-10-1, Bb-11-1, Bb-13-1, Bb-27-1, Bb-50-1, Bb-55-1, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-50-2
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-14-1, Aa-15-1, Aa-16-1, Aa-18-1, Aa-19-1, Aa-20-1, Aa-21-1, Aa-25-1, Aa-26-2, Aa-27-1, Aa-28-1, Aa-29-1, Aa-30-1, Aa-31-1, Aa-32-1, Aa-33-1, Aa-34-1, Aa-36-1, Aa-38-1, Aa-1-2, Aa-2-2, Aa-4-2, Aa-29-2, Aa-32-2, Aa-22-2, Aa-23-2, Aa-44-1, Aa-45-1, Aa-47-1, Aa-48-1, Aa-49-1, Aa-49-2, Aa-50-1, Aa-53-1, Aa-54-1, Aa-57-1, Aa-66-1, Aa-67-1, Aa-71-1, Aa-72-1, Aa-73-1, Aa-75-1, Aa-76-1, Aa-82-1, Aa-83-1, Aa-84-1, Aa-85-1, Aa-85-2, Aa-86-1, Aa-87-1, Aa-88-1, Aa-89-1, Aa-90-1, Aa-91-1, Aa-92-1, Aa-93-1, Aa-94-1, Aa-95-1, Aa-96-1, Aa-97-1, Aa-99-1, Aa-100-1, Aa-102-3, Aa-103-1, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-172-1, Aa-173-1, Aa-174-1, Aa-175-1, Aa-177-1, Aa-178-1, Aa-179-1, Aa-180-1, Aa-181-1, Aa-182-1, Aa-184-1, Aa-185-1, Aa-209-1, Aa-210-1, Aa-211-1, Aa-212-1, Aa-213-1, Aa-214-1, Aa-215-1, Aa-216-1 (HCl salt), Aa-217-1, Aa-218-1, Aa-219-3, Aa-220-3, Aa-221-3, Aa-223-3, Aa-224-3, Aa-225-3, Aa-223-2, Aa-226-1, Aa-226-2, Aa-227-1, Aa-228-2, Aa-229-2, Aa-230-3, Aa-231, Aa-232-3, Aa-233-1, Aa-234-1, Aa-235-1, Aa-236-1, Aa-238-1, Aa-240-1, Aa-241-1, Aa-244-1, Aa-247-1, Aa-248-1, Aa-249-1, Aa-250-3, Aa-252-1, Aa-253-1, Aa-254-1, Aa-255-1, Aa-256-1, Aa-257-1, Aa-257-2, Aa-258-1, Aa-259-1, Aa-259-2, Aa-260-1, Aa-261-2, Aa-262-1, Aa-262-2, Aa-263-1, Aa-263-2, Aa-264-1, Aa-265-1, Aa-272-1, Aa-272-2, Aa-273-2, Aa-274-2, Aa-277-2, Aa-278-2, Aa-279-2, Aa-280-2, Aa-281-2, Ba-1-2, Ba-48-1, Ba-63-1, Ba-64-1, Ba-65-1, Ba-76-1, Ba-119-1, Ba-120-1, Ba-121-1, Ba-122-1, Ba-124-1, Ba-125-1, Ba-223-1, Ba-226-1, Bb-1-2, Bb-2-1, Bb-5-1, Bb-5-5, Bb-7-1, Bb-45-1, Bb-49-1, Bb-60-1, Bb-60-2, Bb-63-1, Bb-64-1, Bb-65-1, Bb-71-3, Bb-73-1, Bb-76-1, Bb-77-3, Bb-102-1, Bb-119-1, Bb-124-1, Bb-127-1, Bb-128-1, Bb-130-1, Bb-1-5, Bb-137-1, Bb-138-1, Bb-139-1, Bb-143-3, Bb-147-1, Bb-152-1, Bb-155-1, Bb-156-1, Bb-157-1, Bb-159-1, Bb-160-1, Bb-161-1, Bb-162-1, Bb-49-2, Bb-137-2, Bb-138-2, Bb-262-1, Bb-265-1, Bb-267-1, Bc-1, Bc-1(S), Bc-44, Bc-5(R), Bc-5, Bc-48, Bc-49, Bc-53, Bc-72, Bc-73, Bc-60, Bc-127, Bc-128, Bc-129, Bc-262, Bc-265

### (1-3) Herbicidal effect on green foxtail (foliar treatment)

To confirm herbicidal effect on green foxtail (Setaria viridis), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on green foxtail.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-52-1, Aa-63-1, Ba-11-1, Ba-44-1, Ba-45-1, Ba-53-1, Ba-58-1, Bb-10-1, Bb-13-1, Bb-44-1, Bb-50-1, Bb-55-1, Bb-80-2, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-17-1, Aa-18-1, Aa-19-1, Aa-20-1, Aa-21-1, Aa-22-1, Aa-23-1, Aa-25-1, Aa-26-1, Aa-26-2, Aa-27-1, Aa-28-1, Aa-29-1, Aa-30-1, Aa-31-1, Aa-32-1, Aa-33-1, Aa-34-1, Aa-36-1, Aa-1-2, Aa-12-2, Aa-2-2, Aa-4-2, Aa-29-2, Aa-32-2, Aa-22-2, Aa-23-2, Aa-44-1, Aa-45-1, Aa-48-1, Aa-49-1, Aa-49-2, Aa-50-1, Aa-53-1, Aa-54-1, Aa-57-1, Aa-60-1, Aa-61-1, Aa-64-1, Aa-67-1, Aa-68-1, Aa-70-1, Aa-71-1, Aa-72-1, Aa-73-1, Aa-74-1, Aa-75-1, Aa-76-1, Aa-77-1, Aa-78, Aa-82-1, Aa-83-1, Aa-84-1, Aa-85-1, Aa-85-2, Aa-86-1, Aa-87-1, Aa-88-1, Aa-89-1, Aa-90-1, Aa-91-1, Aa-92-1, Aa-93-1, Aa-94-1, Aa-95-1, Aa-96-1, Aa-97-1, Aa-98-1, Aa-99-1, Aa-100-1, Aa-101-1, Aa-103-1, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-172-1, Aa-173-1, Aa-174-1, Aa-175-1, Aa-176-1, Aa-177-1, Aa-178-1, Aa-180-1, Aa-181-1, Aa-182-1, Aa-184-1, Aa-191-1, Aa-194-1, Aa-195-1, Aa-196-1, Aa-197-1, Aa-198-1, Aa-199-1, Aa-201-1, Aa-203-1, Aa-204-1, Aa-205-1, Aa-206-1, Aa-207-1, Aa-208-1, Aa-209-1, Aa-210-1, Aa-212-1, Aa-213-1, Aa-214-1, Aa-215-1, Aa-216-1 (HCl salt), Aa-217-1, Aa-220-3, Aa-221-3, Aa-223-3, Aa-224-3, Aa-225-3, Aa-223-2, Aa-220-2, Aa-226-1, Aa-226-2, Aa-227-1, Aa-228-2, Aa-229-2, Aa-230-3, Aa-231, Aa-232-3, Aa-233-1, Aa-234-1, Aa-235-1, Aa-236-1, Aa-238-1, Aa-240-1, Aa-241-1, Aa-242-1, Aa-243-1, Aa-244-1, Aa-247-1, Aa-248-1, Aa-249-1, Aa-250-3, Aa-253-1, Aa-254-1, Aa-255-1, Aa-257-1, Aa-257-2, Aa-258-1, Aa-259-1, Aa-259-2, Aa-260-1, Aa-261-2, Aa-262-1, Aa-262-2, Aa-263-1, Aa-263-2, Aa-264-1, Aa-265-1, Aa-265-2, Aa-267-1, Aa-267-2, Aa-272-1, Aa-272-2, Aa-273-2, Aa-277-2, Aa-278-2, Aa-279-2, Aa-280-2, Aa-281-2, Ba-1-2, Ba-63-1, Ba-64-1, Ba-76-1, Ba-119-1, Ba-120-1, Ba-121-1, Ba-122-1, Ba-223-1, Ba-226-1, Bb-1-2, Bb-2-1, Bb-5-5, Bb-7-1, Bb-45-1, Bb-49-1, Bb-60-1, Bb-60-2, Bb-63-1, Bb-64-1, Bb-71-3, Bb-73-1, Bb-76-1, Bb-77-3, Bb-102-1, Bb-119-1, Bb-124-1, Bb-127-1, Bb-128-1, Bb-1-5, Bb-137-1, Bb-143-3, Bb-147-1, Bb-152-1, Bb-156-1, Bb-157-1, Bb-159-1, Bb-160-1, Bb-161-1, Bb-162-1, Bb-49-2, Bb-137-2, Bb-139-2, Bb-262-1, Bb-265-1, Bc-1, Bc-1(R), Bc-1(S), Bc-5(R), Bc-44, Bc-48, Bc-49, Bc-53, Bc-72, Bc-73, Bc-60, Bc-127, Bc-128, Bc-129, Bc-223-1, Bc-265

### (1-4) Herbicidal effect on wild oat (foliar treatment)

To confirm herbicidal effect on wild oat (Avena fatua), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on wild oat.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-42-1, Aa-43-1, Aa-52-1, Aa-55-1, Aa-62-1, Aa-63-1, Aa-81-1, Ba-11-1, Ba-44-1, Ba-53-1, Ba-58-1, Ba-116-3, Ba-117-3, Ba-118-3, Bb-9-1, Bb-10-1, Bb-11-1, Bb-12-1, Bb-13-1, Bb-15-1, Bb-26-1, Bb-27-1, Bb-44-1, Bb-50-1, Bb-55-1, Bb-61-1, Bb-80-1, Bb-80-2, Bb-126-2, Bb-1-4, Bb-142-1, Bb-144-1, Bb-145-1, Bb-146-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-17-1, Aa-18-1, Aa-19-1, Aa-20-1, Aa-21-1, Aa-22-1, Aa-23-1, Aa-24-1, Aa-25-1, Aa-26-1, Aa-26-2, Aa-27-1, Aa-28-1, Aa-29-1, Aa-30-1, Aa-31-1, Aa-32-1, Aa-33-1, Aa-34-1, Aa-35-1, Aa-36-1, Aa-37, Aa-38-1, Aa-39-1, Aa-40-1, Aa-41-1, Aa-1-2, Aa-12-2, Aa-2-2, Aa-4-2, Aa-29-2, Aa-32-2, Aa-22-2, Aa-23-2, Aa-44-1, Aa-45-1, Aa-47-1, Aa-48-1, Aa-49-1, Aa-49-2, Aa-50-1, Aa-53-1, Aa-54-1, Aa-56-1, Aa-57-1, Aa-58-3, Aa-59, Aa-60-1, Aa-61-1, Aa-64-1, Aa-65-1, Aa-66-1, Aa-67-1, Aa-68-1, Aa-69-1, Aa-70-1, Aa-71-1, Aa-72-1, Aa-73-1, Aa-74-1, Aa-75-1, Aa-76-1, Aa-77-1, Aa-78, Aa-82-1, Aa-83-1, Aa-84-1, Aa-85-1, Aa-85-2, Aa-86-1, Aa-87-1, Aa-88-1, Aa-89-1, Aa-90-1, Aa-91-1, Aa-92-1, Aa-93-1, Aa-94-1, Aa-95-1, Aa-96-1, Aa-97-1, Aa-99-1, Aa-100-1, Aa-101-1, Aa-102-3, Aa-103-1, Aa-103-2, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-171-1, Aa-172-1, Aa-173-1, Aa-174-1, Aa-175-1, Aa-176-1, Aa-177-1, Aa-178-1, Aa-179-1, Aa-180-1, Aa-181-1, Aa-182-1, Aa-183-1, Aa-184-1, Aa-185-1, Aa-186-1, Aa-187-1, Aa-188-1, Aa-189-1, Aa-190-1, Aa-191-1, Aa-192-1, Aa-193-1, Aa-194-1, Aa-195-1, Aa-196-1, Aa-197-1, Aa-198-1, Aa-199-1, Aa-200-1, Aa-201-1, Aa-202-1, Aa-203-1, Aa-204-1, Aa-205-1, Aa-206-1, Aa-207-1, Aa-208-1, Aa-209-1, Aa-210-1, Aa-211-1, Aa-212-1, Aa-213-1, Aa-214-1, Aa-215-1, Aa-216-1 (HCl salt), Aa-217-1, Aa-218-1, Aa-219-3, Aa-220-3, Aa-221-3, Aa-222-3, Aa-223-3, Aa-224-3, Aa-225-3, Aa-223-2, Aa-220-2, Aa-226-1, Aa-226-2, Aa-227-1, Aa-228-2, Aa-229-2, Aa-230-3, Aa-231, Aa-232-3, Aa-233-1, Aa-234-1, Aa-235-1, Aa-236-1, Aa-237-3, Aa-238-1, Aa-239-1, Aa-240-1, Aa-241-1, Aa-242-1, Aa-243-1, Aa-244-1, Aa-245-1, Aa-245-2, Aa-246-1, Aa-247-1, Aa-248-1, Aa-249-1, Aa-250-3, Aa-251-1, Aa-252-1, Aa-253-1, Aa-254-1, Aa-255-1, Aa-256-1, Aa-257-1, Aa-257-2, Aa-258-1, Aa-259-1, Aa-259-2, Aa-260-1, Aa-261-2, Aa-262-1, Aa-262-2, Aa-263-1, Aa-263-2, Aa-264-1, Aa-265-1, Aa-265-2, Aa-266-1, Aa-267-1, Aa-267-2, Aa-268-1, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-277-1, Aa-277-2, Aa-278-2, Aa-279-2, Aa-280-2, Aa-281-2, Aa-282-2, Aa-283-2, Aa-284-2, Aa-285-2, Aa-286-2, Aa-287-1, Aa-287-2, Ba-9-1, Ba-1-2, Ba-63-1, Ba-64-1, Ba-65-1, Ba-76-1, Ba-119-1, Ba-120-1, Ba-121-1, Ba-122-1, Ba-125-1, Ba-223-1, Ba-226-1, Bb-1-2, Bb-2-1, Bb-5-1, Bb-5-5, Bb-7-1, Bb-45-1, Bb-49-1, Bb-53-1, Bb-60-1, Bb-60-2, Bb-71-3, Bb-73-1, Bb-76-1, Bb-77-3, Bb-102-1, Bb-119-1, Bb-127-1, Bb-128-1, Bb-130-1, Bb-131-1, Bb-132-1, Bb-1-5, Bb-137-1, Bb-138-1, Bb-139-1, Bb-141-1, Bb-143-3, Bb-147-1, Bb-155-1, Bb-156-1, Bb-157-1, Bb-159-1, Bb-160-1, Bb-161-1, Bb-49-2, Bb-137-2, Bb-138-2, Bb-139-2, Bb-262-1, Bb-265-1, Bb-267-1, Bc-1, Bc-1(R), Bc-1(S), Bc-3, Bc-5(R), Bc-5, Bc-44, Bc-48, Bc-49, Bc-53, Bc-72, Bc-73, Bc-60, Bc-127, Bc-128, Bc-129, Bc-223-1, Bc-262, Bc-265, Bc-267

### (1-5) Herbicidal effect on Italian ryegrass (foliar treatment)

To confirm herbicidal effect on Italian ryegrass (Lolium multiflorum), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on Italian ryegrass.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-42-1, Aa-55-1, Aa-62-1, Aa-63-1, Ba-11-1, Ba-44-1, Ba-45-1, Ba-53-1, Ba-58-1, Ba-116-3, Ba-117-3, Ba-118-3, Bb-9-1, Bb-10-1, Bb-11-1, Bb-12-1, Bb-13-1, Bb-15-1, Bb-26-1, Bb-27-1, Bb-44-1, Bb-50-1, Bb-55-1, Bb-61-1, Bb-80-1, Bb-80-2, Bb-126-2, Bb-1-4, Bb-142-1, Bb-144-1, Bb-145-1, Bb-146-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-17-1, Aa-18-1, Aa-19-1, Aa-20-1, Aa-21-1, Aa-22-1, Aa-23-1, Aa-25-1, Aa-26-1, Aa-26-2, Aa-27-1, Aa-28-1, Aa-29-1, Aa-30-1, Aa-31-1, Aa-32-1, Aa-33-1, Aa-34-1, Aa-35-1, Aa-36-1, Aa-38-1, Aa-39-1, Aa-40-1, Aa-41-1, Aa-1-2, Aa-12-2, Aa-2-2, Aa-4-2, Aa-29-2, Aa-32-2, Aa-22-2, Aa-23-2, Aa-44-1, Aa-45-1, Aa-48-1, Aa-49-1, Aa-49-2, Aa-50-1, Aa-51-1, Aa-53-1, Aa-54-1, Aa-56-1, Aa-57-1, Aa-58-3, Aa-59, Aa-60-1, Aa-61-1, Aa-64-1, Aa-65-1, Aa-66-1, Aa-67-1, Aa-68-1, Aa-70-1, Aa-71-1, Aa-72-1, Aa-73-1, Aa-74-1, Aa-75-1, Aa-76-1, Aa-77-1, Aa-78, Aa-82-1, Aa-83-1, Aa-84-1, Aa-85-1, Aa-85-2, Aa-86-1, Aa-87-1, Aa-88-1, Aa-89-1, Aa-90-1, Aa-91-1, Aa-92-1, Aa-93-1, Aa-94-1, Aa-95-1, Aa-96-1, Aa-97-1, Aa-99-1, Aa-100-1, Aa-101-1, Aa-102-3, Aa-103-1, Aa-103-2, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-172-1, Aa-173-1, Aa-174-1, Aa-175-1, Aa-176-1, Aa-177-1, Aa-178-1, Aa-179-1, Aa-180-1, Aa-181-1, Aa-182-1, Aa-184-1, Aa-185-1, Aa-186-1, Aa-190-1, Aa-191-1, Aa-192-1, Aa-193-1, Aa-194-1, Aa-195-1, Aa-196-1, Aa-197-1, Aa-198-1, Aa-199-1, Aa-200-1, Aa-201-1, Aa-202-1, Aa-203-1, Aa-204-1, Aa-205-1, Aa-206-1, Aa-207-1, Aa-208-1, Aa-209-1, Aa-210-1, Aa-211-1, Aa-212-1, Aa-213-1, Aa-214-1, Aa-215-1, Aa-216-1 (HCl salt), Aa-217-1, Aa-218-1, Aa-219-3, Aa-220-3, Aa-221-3, Aa-223-3, Aa-224-3, Aa-225-3, Aa-223-2, Aa-220-2, Aa-226-1, Aa-226-2, Aa-227-1, Aa-228-2, Aa-229-2, Aa-230-3, Aa-231, Aa-232-3, Aa-233-1, Aa-234-1, Aa-235-1, Aa-236-1, Aa-237-3, Aa-238-1, Aa-240-1, Aa-241-1, Aa-242-1, Aa-243-1, Aa-244-1, Aa-245-1, Aa-245-2, Aa-246-1, Aa-247-1, Aa-248-1, Aa-249-1, Aa-250-3, Aa-252-1, Aa-253-1, Aa-254-1, Aa-255-1, Aa-256-1, Aa-257-1, Aa-257-2, Aa-258-1, Aa-259-1, Aa-259-2, Aa-260-1, Aa-261-2, Aa-262-1, Aa-262-2, Aa-263-1, Aa-263-2, Aa-264-1, Aa-265-1, Aa-265-2, Aa-267-1, Aa-267-2, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-276-2, Aa-277-1, Aa-277-2, Aa-278-2, Aa-279-2, Aa-280-2, Aa-281-2, Aa-282-2, Aa-283-2, Aa-284-2, Aa-285-2, Aa-286-2, Ba-1-2, Ba-119-1, Ba-120-1, Ba-122-1, Ba-223-1, Ba-226-1, Bb-1-2, Bb-2-1, Bb-5-1, Bb-5-5, Bb-7-1, Bb-45-1, Bb-49-1, Bb-53-1, Bb-60-1, Bb-60-2, Bb-71-3, Bb-73-1, Bb-76-1, Bb-77-3, Bb-102-1, Bb-119-1, Bb-127-1, Bb-128-1, Bb-130-1, Bb-131-1, Bb-132-1, Bb-1-5, Bb-137-1, Bb-138-1, Bb-139-1, Bb-141-1, Bb-143-3, Bb-147-1, Bb-152-1, Bb-156-1, Bb-157-1, Bb-159-1, Bb-160-1, Bb-161-1, Bb-49-2, Bb-137-2, Bb-138-2, Bb-139-2, Bb-262-1, Bb-265-1, Bb-267-1, Bc-1, Bc-1(R), Bc-1(S), Bc-3, Bc-5(R), Bc-5, Bc-44, Bc-48, Bc-49, Bc-53, Bc-72, Bc-73, Bc-60, Bc-127, Bc-128, Bc-129, Bc-223-1, Bc-262, Bc-265, Bc-267

### (1-6) Herbicidal effect on foxtail shortawn (foliar treatment)

To confirm herbicidal effect on foxtail shortawn (Alopecurus aequalis), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on foxtail shortawn.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-42-1, Aa-52-1, Aa-55-1, Aa-62-1, Aa-63-1, Aa-81-1, Ba-11-1, Ba-44-1, Ba-45-1, Ba-53-1, Ba-58-1, Ba-116-3, Ba-117-3, Ba-118-3, Bb-9-1, Bb-10-1, Bb-11-1, Bb-12-1, Bb-13-1, Bb-15-1, Bb-26-1, Bb-27-1, Bb-44-1, Bb-50-1, Bb-55-1, Bb-61-1, Bb-80-1, Bb-80-2, Bb-126-2, Bb-1-4, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-17-1, Aa-18-1, Aa-19-1, Aa-20-1, Aa-21-1, Aa-22-1, Aa-23-1, Aa-24-1, Aa-25-1, Aa-26-1, Aa-26-2, Aa-27-1, Aa-28-1, Aa-29-1, Aa-30-1, Aa-31-1, Aa-32-1, Aa-33-1, Aa-34-1, Aa-35-1, Aa-36-1, Aa-38-1, Aa-39-1, Aa-40-1, Aa-41-1, Aa-1-2, Aa-12-2, Aa-2-2, Aa-4-2, Aa-29-2, Aa-32-2, Aa-22-2, Aa-23-2, Aa-44-1, Aa-45-1, Aa-47-1, Aa-48-1, Aa-49-1, Aa-49-2, Aa-50-1, Aa-51-1, Aa-53-1, Aa-54-1, Aa-56-1, Aa-57-1, Aa-58-3, Aa-59, Aa-60-1, Aa-61-1, Aa-64-1, Aa-65-1, Aa-66-1, Aa-67-1, Aa-68-1, Aa-69-1, Aa-70-1, Aa-71-1, Aa-72-1, Aa-73-1, Aa-74-1, Aa-75-1, Aa-77-1, Aa-78, Aa-82-1, Aa-83-1, Aa-84-1, Aa-85-1, Aa-85-2, Aa-86-1, Aa-87-1, Aa-88-1, Aa-100-1, Aa-101-1, Aa-102-3, Aa-103-1, Aa-103-2, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-171-1, Aa-172-1, Aa-173-1, Aa-174-1, Aa-175-1, Aa-176-1, Aa-177-1, Aa-178-1, Aa-179-1, Aa-180-1, Aa-181-1, Aa-182-1, Aa-183-1, Aa-184-1, Aa-185-1, Aa-186-1, Aa-187-1, Aa-188-1, Aa-189-1, Aa-190-1, Aa-191-1, Aa-192-1, Aa-193-1, Aa-194-1, Aa-195-1, Aa-196-1, Aa-197-1, Aa-198-1, Aa-199-1, Aa-200-1, Aa-201-1, Aa-202-1, Aa-203-1, Aa-204-1, Aa-205-1, Aa-206-1, Aa-207-1, Aa-208-1, Aa-209-1, Aa-210-1, Aa-211-1, Aa-212-1, Aa-213-1, Aa-214-1, Aa-215-1, Aa-216-1 (HCl salt), Aa-217-1, Aa-218-1, Aa-219-3, Aa-220-3, Aa-221-3, Aa-222-3, Aa-223-3, Aa-224-3, Aa-225-3, Aa-223-2, Aa-220-2, Aa-226-1, Aa-226-2, Aa-227-1, Aa-228-2, Aa-229-2, Aa-230-3, Aa-231, Aa-232-3, Aa-233-1, Aa-234-1, Aa-235-1, Aa-236-1, Aa-237-3, Aa-238-1, Aa-239-1, Aa-240-1, Aa-241-1, Aa-242-1, Aa-243-1, Aa-244-1, Aa-245-1, Aa-245-2, Aa-246-1, Aa-247-1, Aa-248-1, Aa-249-1, Aa-250-3, Aa-251-1, Aa-252-1, Aa-253-1, Aa-254-1, Aa-255-1, Aa-256-1, Aa-257-1, Aa-257-2, Aa-258-1, Aa-259-1, Aa-259-2, Aa-260-1, Aa-261-2, Aa-262-1, Aa-262-2, Aa-263-1, Aa-263-2, Aa-264-1, Aa-265-1, Aa-265-2, Aa-266-1, Aa-266-2, Aa-267-1, Aa-267-2, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-276-2, Aa-277-1, Aa-277-2, Aa-278-2, Aa-279-2, Aa-280-2, Aa-281-2, Aa-282-2, Aa-283-2, Aa-284-2, Aa-285-2, Aa-286-2, Aa-287-1, Aa-287-2, Ba-9-1, Ba-1-2, Ba-63-1, Ba-64-1, Ba-65-1, Ba-76-1, Ba-119-1, Ba-120-1, Ba-121-1, Ba-122-1, Ba-223-1, Ba-226-1, Bb-1-2, Bb-2-1, Bb-5-1, Bb-5-5, Bb-7-1, Bb-45-1, Bb-49-1, Bb-53-1, Bb-60-1, Bb-60-2, Bb-63-1, Bb-64-1, Bb-65-1, Bb-71-3, Bb-73-1, Bb-76-1, Bb-77-3, Bb-102-1, Bb-119-1, Bb-124-1, Bb-127-1, Bb-128-1, Bb-130-1, Bb-131-1, Bb-132-1, Bb-1-5, Bb-137-1, Bb-138-1, Bb-139-1, Bb-141-1, Bb-143-3, Bb-147-1, Bb-152-1, Bb-155-1, Bb-156-1, Bb-157-1, Bb-159-1, Bb-160-1, Bb-161-1, Bb-162-1, Bb-49-2, Bb-137-2, Bb-138-2, Bb-139-2, Bb-262-1, Bb-265-1, Bb-267-1, Bc-1, Bc-1(R), Bc-1(S), Bc-3, Bc-5(R), Bc-5, Bc-44, Bc-48, Bc-49, Bc-53, Bc-72, Bc-73, Bc-60, Bc-127, Bc-128, Bc-223-1, Bc-262, Bc-265, Bc-267

### (1-7) Herbicidal effect on annual bluegrass (foliar treatment)

To confirm herbicidal effect on annual bluegrass (Poa annua), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on annual bluegrass.
Compounds (active ingredient dose: 1,000 g/ha): Comp No. Bb-3-1, Bb-20-1
Compounds (active ingredient dose: 500 g/ha): Comp No. Aa-55-1, Aa-62-1, Aa-63-1, Ba-44-1, Ba-53-1, Ba-58-1, Ba-116-3, Ba-117-3, Bb-10-1, Bb-15-1, Bb-44-1, Bb-50-1, Bb-55-1, Bb-61-1, Bb-80-2, Bb-126-2, Bb-1-4, Bb-142-1, Bb-145-1, Bb-146-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-19-1, Aa-20-1, Aa-21-1, Aa-23-1, Aa-25-1, Aa-26-1, Aa-26-2, Aa-27-1, Aa-28-1, Aa-29-1, Aa-30-1, Aa-31-1, Aa-32-1, Aa-33-1, Aa-34-1, Aa-36-1, Aa-38-1, Aa-1-2, Aa-12-2, Aa-2-2, Aa-4-2, Aa-29-2, Aa-32-2, Aa-22-2, Aa-23-2, Aa-44-1, Aa-48-1, Aa-49-1, Aa-49-2, Aa-50-1, Aa-53-1, Aa-54-1, Aa-56-1, Aa-57-1, Aa-59, Aa-60-1, Aa-61-1, Aa-64-1, Aa-65-1, Aa-66-1, Aa-67-1, Aa-68-1, Aa-69-1, Aa-71-1, Aa-72-1, Aa-73-1, Aa-74-1, Aa-75-1, Aa-76-1, Aa-77-1, Aa-78, Aa-82-1, Aa-83-1, Aa-85-1, Aa-85-2, Aa-86-1, Aa-88-1, Aa-90-1, Aa-91-1, Aa-93-1, Aa-94-1, Aa-97-1, Aa-99-1, Aa-102-3, Aa-103-2, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 Na(salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-171-1, Aa-172-1, Aa-173-1, Aa-174-1, Aa-175-1, Aa-176-1, Aa-177-1, Aa-178-1, Aa-179-1, Aa-180-1, Aa-181-1, Aa-182-1, Aa-183-1, Aa-184-1, Aa-185-1, Aa-186-1, Aa-187-1, Aa-188-1, Aa-189-1, Aa-190-1, Aa-191-1, Aa-192-1, Aa-193-1, Aa-194-1, Aa-195-1, Aa-196-1, Aa-197-1, Aa-198-1, Aa-199-1, Aa-200-1, Aa-201-1, Aa-202-1, Aa-203-1, Aa-204-1, Aa-205-1, Aa-206-1, Aa-207-1, Aa-208-1, Aa-215-1, Aa-217-1, Aa-218-1, Aa-219-3, Aa-220-3, Aa-221-3, Aa-222-3, Aa-223-3, Aa-224-3, Aa-225-3, Aa-223-2, Aa-220-2, Aa-226-1, Aa-226-2, Aa-227-1, Aa-228-2, Aa-229-2, Aa-230-3, Aa-231, Aa-232-3, Aa-233-1, Aa-234-1, Aa-235-1, Aa-236-1, Aa-237-3, Aa-238-1, Aa-239-1, Aa-240-1, Aa-241-1, Aa-242-1, Aa-243-1, Aa-244-1, Aa-245-1, Aa-245-2, Aa-246-1, Aa-247-1, Aa-248-1, Aa-249-1, Aa-250-3, Aa-251-1, Aa-252-1, Aa-253-1, Aa-254-1, Aa-255-1, Aa-256-1, Aa-257-1, Aa-257-2, Aa-258-1, Aa-259-1, Aa-259-2, Aa-260-1, Aa-261-2, Aa-262-1, Aa-262-2, Aa-263-1, Aa-263-2, Aa-264-1, Aa-265-1, Aa-265-2, Aa-266-1, Aa-266-2, Aa-267-1, Aa-267-2, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-276-2, Aa-277-1, Aa-277-2, Aa-278-2, Aa-279-2, Aa-280-2, Aa-281-2, Aa-282-2, Aa-283-2, Aa-284-2, Aa-285-2, Aa-286-2, Ba-1-2, Ba-64-1, Ba-119-1, Ba-120-1, Ba-121-1, Ba-122-1, Ba-223-1, Ba-226-1, Bb-1-2, Bb-2-1, Bb-5-1, Bb-5-5, Bb-7-1, Bb-45-1, Bb-49-1, Bb-53-1, Bb-60-1, Bb-60-2, Bb-71-3, Bb-73-1, Bb-76-1, Bb-77-3, Bb-102-1, Bb-119-1, Bb-127-1, Bb-130-1, Bb-131-1, Bb-1-5, Bb-137-1, Bb-138-1, Bb-139-1, Bb-143-3, Bb-152-1, Bb-156-1, Bb-159-1, Bb-161-1, Bb-49-2, Bb-137-2, Bb-138-2, Bb-139-2, Bb-262-1, Bb-265-1, Bb-267-1, Bc-1, Bc-1(R), Bc-1(S), Bc-3, Bc-5(R), Bc-5, Bc-48, Bc-72, Bc-73, Bc-60, Bc-127, Bc-129, Bc-223-1, Bc-262, Bc-265, Bc-267

### (1-8) Herbicidal effect on redroot pigweed (foliar treatment)

To confirm herbicidal effect on redroot pigweed (Amaranthus retroflexus), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on redroot pigweed.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-62-1, Aa-63-1, Ba-11-1, Bb-10-1, Bb-11-1, Bb-13-1, Bb-44-1, Bb-50-1, Bb-80-2, Bb-142-1, Bb-144-1, Bb-145-1, Bb-146-1, Bb-149-1, Bb-150-1, Bb-151-1, Bb-158-1, Bb-61-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-7-1, Aa-10-1, Aa-10-2, Aa-19-1, Aa-21-1, Aa-26-1, Aa-28-1, Aa-29-1, Aa-34-1, Aa-29-2, Aa-44-1, Aa-49-2, Aa-50-1, Aa-57-1, Aa-61-1, Aa-72-1, Aa-78, Aa-102-3, Aa-131-1, Aa-48-1 (Na salt), Aa-133-1, Aa-134-1, Aa-135-1, Aa-137-1, Aa-138-1, Aa-166-1, Aa-167-1, Aa-168-1, Aa-172-1, Aa-173-1, Aa-174-1, Aa-176-1, Aa-177-1, Aa-178-1, Aa-181-1, Aa-182-1, Aa-207-1, Aa-217-1, Aa-220-3, Aa-223-3, Aa-225-3, Aa-223-2, Aa-226-1, Aa-226-2, Aa-230-3, Aa-233-1, Aa-234-1, Aa-235-1, Aa-236-1, Aa-240-1, Aa-246-1, Aa-247-1, Aa-254-1, Aa-263-1, Aa-264-1, Aa-271-2, Ba-1-2, Ba-223-1, Bb-1-2, Bb-2-1, Bb-5-1, Bb-5-5, Bb-7-1, Bb-49-1, Bb-60-1, Bb-60-2, Bb-71-3, Bb-73-1, Bb-77-3, Bb-102-1, Bb-127-1, Bb-128-1, Bb-131-1, Bb-1-5, Bb-137-1, Bb-139-1, Bb-143-3, Bb-152-1, Bb-156-1, Bb-157-1, Bb-161-1, Bb-49-2, Bb-137-2, Bb-138-2, Bb-139-2, Bb-262-1, Bb-265-1, Bc-1(S), Bc-5(R), Bc-49, Bc-53, Bc-72, Bc-60, Bc-127, Bc-128, Bc-262, Bc-265

### (1-9) Herbicidal effect on common chickweed (foliar treatment)

To confirm herbicidal effect on common chickweed (Stellaria media), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on common chickweed.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-52-1, Aa-55-1, Aa-62-1, Aa-63-1, Aa-81-1, Ba-11-1, Ba-44-1, Ba-45-1, Ba-53-1, Ba-58-1, Ba-116-3, Ba-117-3, Ba-118-3, Bb-9-1, Bb-10-1, Bb-11-1, Bb-12-1, Bb-13-1, Bb-15-1, Bb-26-1, Bb-27-1, Bb-44-1, Bb-50-1, Bb-55-1, Bb-61-1, Bb-80-1, Bb-80-2, Bb-126-2, Bb-1-4, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-18-1, Aa-19-1, Aa-20-1, Aa-21-1, Aa-22-1, Aa-23-1, Aa-25-1, Aa-26-1, Aa-27-1, Aa-28-1, Aa-29-1, Aa-30-1, Aa-31-1, Aa-32-1, Aa-33-1, Aa-34-1, Aa-35-1, Aa-36-1, Aa-38-1, Aa-39-1, Aa-1-2, Aa-2-2, Aa-32-2, Aa-22-2, Aa-23-2, Aa-44-1, Aa-45-1, Aa-48-1, Aa-49-1, Aa-49-2, Aa-50-1, Aa-51-1, Aa-53-1, Aa-54-1, Aa-56-1, Aa-57-1, Aa-58-3, Aa-59, Aa-60-1, Aa-61-1, Aa-64-1, Aa-65-1, Aa-66-1, Aa-67-1, Aa-68-1, Aa-70-1, Aa-71-1, Aa-72-1, Aa-73-1, Aa-74-1, Aa-75-1, Aa-76-1, Aa-77-1, Aa-78, Aa-82-1, Aa-83-1, Aa-84-1, Aa-85-1, Aa-85-2, Aa-86-1, Aa-87-1, Aa-88-1, Aa-89-1, Aa-90-1, Aa-91-1, Aa-92-1, Aa-93-1, Aa-94-1, Aa-95-1, Aa-96-1, Aa-97-1, Aa-98-1, Aa-99-1, Aa-101-1, Aa-102-3, Aa-103-1, Aa-104-1, Aa-105-1, Aa-106-1, Aa-107-1, Aa-108-1, Aa-109-1, Aa-110-1, Aa-111-1, Aa-112-1, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-172-1, Aa-173-1, Aa-174-1, Aa-175-1, Aa-176-1, Aa-177-1, Aa-178-1, Aa-180-1, Aa-181-1, Aa-182-1, Aa-183-1, Aa-184-1, Aa-185-1, Aa-187-1, Aa-188-1, Aa-190-1, Aa-191-1, Aa-192-1, Aa-193-1, Aa-194-1, Aa-195-1, Aa-196-1, Aa-197-1, Aa-198-1, Aa-199-1, Aa-200-1, Aa-201-1, Aa-202-1, Aa-203-1, Aa-204-1, Aa-205-1, Aa-206-1, Aa-207-1, Aa-208-1, Aa-209-1, Aa-210-1, Aa-212-1, Aa-213-1, Aa-214-1, Aa-215-1, Aa-216-1 (HCl salt), Aa-217-1, Aa-219-3, Aa-220-3, Aa-221-3, Aa-222-3, Aa-223-3, Aa-224-3, Aa-225-3, Aa-223-2, Aa-220-2, Aa-226-1, Aa-226-2, Aa-227-1, Aa-228-2, Aa-229-2, Aa-231, Aa-232-3, Aa-233-1, Aa-234-1, Aa-235-1, Aa-236-1, Aa-238-1, Aa-239-1, Aa-240-1, Aa-241-1, Aa-242-1, Aa-243-1, Aa-244-1, Aa-247-1, Aa-248-1, Aa-249-1, Aa-250-3, Aa-254-1, Aa-255-1, Aa-257-1, Aa-257-2, Aa-258-1, Aa-259-1, Aa-260-1, Aa-261-2, Aa-262-1, Aa-263-1, Aa-263-2, Aa-264-1, Aa-267-1, Aa-272-1, Aa-272-2, Aa-273-2, Aa-278-2, Aa-279-2, Aa-281-2, Ba-9-1, Ba-1-2, Ba-64-1, Ba-65-1, Ba-76-1, Ba-119-1, Ba-120-1, Ba-121-1, Ba-122-1, Ba-124-1, Ba-125-1, Ba-223-1, Ba-226-1, Bb-1-2, Bb-2-1, Bb-5-1, Bb-5-5, Bb-7-1, Bb-45-1, Bb-49-1, Bb-53-1, Bb-60-1, Bb-60-2, Bb-63-1, Bb-64-1, Bb-65-1, Bb-73-1, Bb-76-1, Bb-77-3, Bb-102-1, Bb-119-1, Bb-124-1, Bb-127-1, Bb-128-1, Bb-130-1, Bb-131-1, Bb-132-1, Bb-1-5, Bb-137-1, Bb-138-1, Bb-139-1, Bb-141-1, Bb-143-3, Bb-147-1, Bb-152-1, Bb-155-1, Bb-156-1, Bb-157-1, Bb-159-1, Bb-160-1, Bb-161-1, Bb-162-1, Bb-49-2, Bb-137-2, Bb-138-2, Bb-139-2, Bb-262-1, Bb-265-1, Bb-267-1, Bc-1, Bc-1(R), Bc-1(S), Bc-3, Bc-5(R), Bc-5, Bc-44, Bc-48, Bc-49, Bc-53, Bc-72, Bc-73, Bc-60, Bc-127, Bc-128, Bc-129, Bc-262, Bc-265, Bc-267

### (1-10) Herbicidal effect on velvetleaf (foliar treatment)

To confirm herbicidal effect on velvetleaf (Abutilon theophrasti), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on velvetleaf.
Compounds (active ingredient dose: 1,000 g/ha): Comp No. Ba-19-1, Bb-3-1, Bb-8-1, Bb-19-1, Bb-20-1
Compounds (active ingredient dose: 500 g/ha): Comp No. Aa-62-1, Aa-63-1, Aa-81-1, Ba-11-1, Ba-45-1, Bb-9-1, Bb-10-1, Bb-11-1, Bb-12-1, Bb-13-1, Bb-15-1, Bb-27-1, Bb-44-1, Bb-50-1, Bb-55-1, Bb-142-1, Bb-144-1, Bb-145-1, Bb-146-1, Bb-150-1, Bb-151-1, Bb-153-1, Bb-154-1, Bb-158-1
Compounds (active ingredient dose: 250 g/ha): Comp No. Bb-1-1, Bb-46-3, Bb-48-1, Bb-79-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-4-1, Aa-8-1, Aa-9-1, Aa-10-1, Aa-14-1, Aa-27-1, Aa-28-1, Aa-34-1, Aa-67-1, Aa-86-1, Aa-88-1, Aa-91-1, Aa-93-1, Aa-94-1, Aa-96-1, Aa-97-1, Aa-99-1, Aa-178-1, Aa-180-1, Aa-219-3, Aa-254-1, Ba-64-1, Ba-65-1, Ba-119-1, Ba-124-1, Bb-2-1, Bb-5-1, Bb-7-1, Bb-45-1, Bb-49-1, Bb-60-1, Bb-63-1, Bb-64-1, Bb-65-1, Bb-71-3, Bb-73-1, Bb-76-1, Bb-77-3, Bb-119-1, Bb-124-1, Bb-127-1, Bb-128-1, Bb-130-1, Bb-131-1, Bb-132-1, Bb-1-5, Bb-138-1, Bb-143-3, Bb-152-1, Bb-156-1, Bb-157-1, Bb-162-1, Bb-262-1, Bb-265-1, Bb-267-1, Bc-1(S), Bc-129, Bc-265

### (1-11) Herbicidal effect on common lambsquarters (foliar treatment)

To confirm herbicidal effect on common lambsquarters (Chenopodium album), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on common lambsquarters.
Compounds (active ingredient dose: 1,000 g/ha): Comp No. Ba-19-1, Bb-3-1, Bb-8-1, Bb-19-1, Bb-20-1
Compounds (active ingredient dose: 500 g/ha): Comp No. Aa-52-1, Aa-62-1, Aa-63-1, Aa-81-1, Ba-11-1, Ba-58-1, Ba-116-3, Ba-117-3, Bb-9-1, Bb-10-1, Bb-11-1, Bb-12-1, Bb-13-1, Bb-15-1, Bb-26-1, Bb-27-1, Bb-44-1, Bb-50-1, Bb-61-1, Bb-80-1, Bb-80-2, Bb-1-4, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-61-2, Bb-50-2, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-4-1, Aa-5-1, Aa-6-1, Aa-7-1, Aa-8-1, Aa-9-1, Aa-9-2, Aa-10-1, Aa-10-2, Aa-11-1, Aa-12-1, Aa-13-1, Aa-14-1, Aa-15-1, Aa-16-1, Aa-18-1, Aa-19-1, Aa-20-1, Aa-21-1, Aa-25-1, Aa-27-1, Aa-28-1, Aa-29-1, Aa-32-1, Aa-33-1, Aa-34-1, Aa-35-1, Aa-12-2, Aa-22-2, Aa-44-1, Aa-48-1, Aa-49-1, Aa-49-2, Aa-50-1, Aa-56-1, Aa-57-1, Aa-59, Aa-60-1, Aa-61-1, Aa-64-1, Aa-65-1, Aa-66-1, Aa-67-1, Aa-68-1, Aa-71-1, Aa-72-1, Aa-73-1, Aa-76-1, Aa-78, Aa-82-1, Aa-85-2, Aa-88-1, Aa-89-1, Aa-102-3, Aa-103-1, Aa-104-1, Aa-107-1, Aa-108-1, Aa-109-1, Aa-110-1, Aa-115-1, Aa-131-1, Aa-48-1 (Na salt), Aa-134-1, Aa-135-1, Aa-137-1, Aa-138-1, Aa-166-1, Aa-170-1, Aa-172-1, Aa-174-1, Aa-176-1, Aa-177-1, Aa-178-1, Aa-180-1, Aa-182-1, Aa-183-1, Aa-184-1, Aa-185-1, Aa-191-1, Aa-192-1, Aa-194-1, Aa-195-1, Aa-196-1, Aa-197-1, Aa-198-1, Aa-199-1, Aa-201-1, Aa-203-1, Aa-205-1, Aa-206-1, Aa-207-1, Aa-208-1, Aa-210-1, Aa-212-1, Aa-214-1, Aa-215-1, Aa-219-3, Aa-220-3, Aa-221-3, Aa-222-3, Aa-223-3, Aa-225-3, Aa-223-2, Aa-220-2, Aa-226-1, Aa-226-2, Aa-227-1, Aa-228-2, Aa-229-2, Aa-231, Aa-232-3, Aa-233-1, Aa-234-1, Aa-240-1, Aa-242-1, Aa-243-1, Aa-244-1, Aa-249-1, Aa-250-3, Aa-254-1, Aa-255-1, Aa-257-1, Aa-257-2, Aa-258-1, Aa-260-1, Aa-261-2, Aa-263-2, Aa-264-1, Aa-265-1, Aa-272-2, Aa-273-2, Aa-277-2, Aa-281-2, Ba-9-1, Ba-1-2, Ba-64-1, Ba-76-1, Ba-124-1, Ba-125-1, Bb-1-2, Bb-2-1, Bb-5-1, Bb-5-5, Bb-7-1, Bb-45-1, Bb-49-1, Bb-60-1, Bb-60-2, Bb-71-3, Bb-73-1, Bb-76-1, Bb-77-3, Bb-102-1, Bb-124-1, Bb-127-1, Bb-128-1, Bb-130-1, Bb-131-1, Bb-132-1, Bb-1-5, Bb-137-1, Bb-138-1, Bb-139-1, Bb-143-3, Bb-147-1, Bb-152-1, Bb-156-1, Bb-157-1, Bb-159-1, Bb-162-1, Bb-49-2, Bb-137-2, Bb-138-2, Bb-139-2, Bb-262-1, Bb-265-1, Bc-1(R), Bc-1(S), Bc-48, Bc-49, Bc-72, Bc-73, Bc-60

### (1-12) Herbicidal effect on black nightshade (foliar treatment)

To confirm herbicidal effect on black nightshade (Solanum nigrum), the same test as in Test Example 1 (1-1) (foliar treatment) was conducted using the following compounds. As a result, compositions containing the following compounds (active ingredient dose: 125, 250, 500 or 1,000 g/ha) showed a growth inhibition rate of 70% or higher on black nightshade.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-52-1, Aa-55-1, Aa-62-1, Aa-63-1, Aa-81-1, Ba-11-1, Ba-44-1, Ba-45-1, Ba-53-1, Ba-58-1, Ba-116-3, Ba-117-3, Ba-118-3, Bb-10-1, Bb-11-1, Bb-12-1, Bb-13-1, Bb-15-1, Bb-26-1, Bb-27-1, Bb-44-1, Bb-50-1, Bb-55-1, Bb-61-1, Bb-80-1, Bb-80-2, Bb-126-2, Bb-1-4, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-50-2, Bc-126
Compounds (active ingredient dose: 250 g/ha): Comp No. Aa-1-1, Bb-1-1, Bb-46-3, Bb-48-1, Bb-79-3, Bb-114-3, Bb-123-3, Bb-135-3, Bb-163-3, Bb-164-3, Bb-165-3
Compounds (active ingredient dose: 125 g/ha): Comp No. Aa-5-1, Aa-7-1, Aa-8-1, Aa-10-1, Aa-10-2, Aa-11-1, Aa-13-1, Aa-14-1, Aa-26-1, Aa-27-1, Aa-28-1, Aa-29-1, Aa-30-1, Aa-31-1, Aa-32-1, Aa-33-1, Aa-34-1, Aa-1-2, Aa-44-1, Aa-45-1, Aa-48-1, Aa-49-1, Aa-50-1, Aa-51-1, Aa-53-1, Aa-58-3, Aa-60-1, Aa-61-1, Aa-64-1, Aa-66-1, Aa-67-1, Aa-76-1, Aa-78, Aa-82-1, Aa-86-1, Aa-88-1, Aa-94-1, Aa-95-1, Aa-96-1, Aa-131-1, Aa-170-1, Aa-176-1, Aa-177-1, Aa-178-1, Aa-180-1, Aa-182-1, Aa-183-1, Aa-211-1, Aa-216-1 (HCl salt), Aa-219-3, Aa-227-1, Aa-230-3, Aa-232-3, Aa-233-1, Aa-234-1, Aa-236-1, Aa-239-1, Aa-240-1, Aa-244-1, Aa-247-1, Aa-248-1, Aa-249-1, Aa-250-3, Aa-254-1, Aa-259-1, Aa-263-1, Aa-263-2, Aa-264-1, Aa-265-1, Aa-269-1, Aa-273-2, Ba-1-2, Ba-63-1, Ba-64-1, Ba-65-1, Ba-76-1, Ba-119-1, Ba-120-1, Ba-121-1, Ba-122-1, Ba-124-1, Ba-125-1, Bb-1-2, Bb-2-1, Bb-5-1, Bb-5-5, Bb-7-1, Bb-45-1, Bb-49-1, Bb-63-1, Bb-64-1, Bb-65-1, Bb-71-3, Bb-73-1, Bb-76-1, Bb-77-3, Bb-102-1, Bb-119-1, Bb-124-1, Bb-127-1, Bb-128-1, Bb-130-1, Bb-131-1, Bb-132-1, Bb-1-5, Bb-137-1, Bb-141-1, Bb-143-3, Bb-147-1, Bb-152-1, Bb-155-1, Bb-156-1, Bb-157-1, Bb-159-1, Bb-160-1, Bb-161-1, Bb-162-1, Bb-49-2, Bb-262-1, Bb-265-1, Bb-267-1, Bc-1, Bc-5(R), Bc-5, Bc-44, Bc-48, Bc-49, Bc-53, Bc-72, Bc-73, Bc-60, Bc-127, Bc-128, Bc-129, Bc-265, Bc-267

### Test Example 2: (2-1) Herbicidal effect on barnyardgrass (soil treatment)

Upland soil was put into a 1/300,000 hectare (ha) pot, seeds of barnyardgrass (Echinochloa crus-galli) were sown, and about 1.5 cm of cover soil was put. The Invention Compound was weighed to achieve a predetermined active ingredient amount, dissolved in acetone (containing 0.5 vol% of surfactant (TritonX-100: manufactured by Kishida Chemical Co., Ltd.)) or dimethyl sulfoxide and diluted with water (containing 0.1 vol% of agricultural spreader (Surfactant WK: manufactured by MARUWA Biochemical Co., Ltd.)) in an amount corresponding to 1,000 liter per hectare, to prepare a spray solution. On the next day after sowing, the spray solution was sprayed with a small sprayer for soil treatment.

On the 14th day after the soil treatment, the state of growth of each plant was visually observed. As a result, compositions containing the following compounds (active ingredient dose: 125, 250, 500 or 1,000 g/ha) showed a growth inhibition rate of 70% or higher on barnyardgrass.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-52-1, Aa-55-1, Aa-62-1, Aa-63-1, Aa-81-1, Ba-11-1, Ba-44-1, Ba-45-1, Ba-53-1, Ba-58-1, Bb-2-1, Bb-9-1, Bb-10-1, Bb-11-1, Bb-15-1, Bb-44-1, Bb-50-1, Bb-55-1, Bb-60-1, Bb-61-1, Bb-80-1, Bb-80-2, Bb-102-1, Bb-126-2, Bb-128-1, Bb-130-1, Bb-131-1, Bb-132-1, Bb-138-1, Bb-139-1, Bb-142-1, Bb-144-1, Bb-146-1, Bb-147-1, Bb-148-1, Bb-149-1, Bb-150-1, Bb-153-1, Bb-154-1, Bb-158-1, Bb-49-2, Bb-61-2, Bb-50-2, Bc-3, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-7-1, Aa-8-1, Aa-10-1, Aa-10-2, Aa-11-1, Aa-16-1, Aa-21-1, Aa-28-1, Aa-29-1, Aa-1-2, Aa-2-2, Aa-4-2, Aa-29-2, Aa-32-2, Aa-44-1, Aa-45-1, Aa-48-1, Aa-49-2, Aa-50-1, Aa-51-1, Aa-53-1, Aa-54-1, Aa-56-1, Aa-57-1, Aa-60-1, Aa-61-1, Aa-67-1, Aa-68-1, Aa-70-1, Aa-71-1, Aa-72-1, Aa-73-1, Aa-74-1, Aa-75-1, Aa-76-1, Aa-77-1, Aa-92-1, Aa-96-1, Aa-131-1, Aa-48-1 (Na salt), Aa-133-1, Aa-134-1, Aa-135-1, Aa-137-1, Aa-138-1, Aa-166-1, Aa-167-1, Aa-168-1, Aa-169-1, Aa-170-1, Aa-172-1, Aa-173-1, Aa-174-1, Aa-175-1, Aa-177-1, Aa-178-1, Aa-181-1, Aa-182-1, Aa-184-1, Aa-185-1, Aa-217-1, Aa-223-2, Aa-230-3, Aa-233-1, Aa-240-1, Aa-250-3, Aa-253-1, Aa-260-1, Aa-278-2, Aa-279-2, Aa-280-2, Aa-281-2, Ba-1-2, Ba-64-1, Ba-76-1, Ba-121-1, Ba-122-1, Bb-64-1, Bb-65-1, Bb-71-3, Bb-76-1, Bb-119-1, Bb-124-1, Bb-127-1, Bb-1-5, Bb-137-1, Bb-141-1, Bb-143-3, Bb-152-1, Bb-156-1, Bb-157-1, Bb-159-1, Bb-162-1, Bb-137-2, Bb-138-2, Bc-1, Bc-44, Bc-53, Bc-73, Bc-127, Bc-129

### (2-2) Herbicidal effect on crabgrass (soil treatment)

To confirm herbicidal effect on crabgrass (Digitaria sanguinalis), the same test as in Test Example 2 (2-1) (soil treatment) was conducted using the following compounds. As a result, compositions containing the following compounds (active ingredient dose: 125, 250, 500 or 1,000 g/ha) showed a growth inhibition rate of 70% or higher on crabgrass.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-9-2, Aa-52-1, Aa-55-1, Aa-62-1, Aa-63-1, Ba-11-1, Ba-44-1, Ba-45-1, Ba-53-1, Ba-58-1, Ba-116-3, Bb-2-1, Bb-10-1, Bb-11-1, Bb-12-1, Bb-13-1, Bb-15-1, Bb-44-1, Bb-50-1, Bb-55-1, Bb-60-1, Bb-60-2, Bb-61-1, Bb-80-1, Bb-80-2, Bb-102-1, Bb-126-2, Bb-128-1, Bb-130-1, Bb-131-1, Bb-1-4, Bb-138-1, Bb-139-1, Bb-142-1, Bb-144-1, Bb-145-1, Bb-146-1, Bb-147-1, Bb-148-1, Bb-149-1, Bb-150-1, Bb-153-1, Bb-154-1, Bb-158-1, Bb-49-2, Bb-61-2, Bb-50-2, Bc-3, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-6-1, Aa-7-1, Aa-8-1, Aa-10-1, Aa-10-2, Aa-11-1, Aa-14-1, Aa-15-1, Aa-16-1, Aa-19-1, Aa-25-1, Aa-32-1, Aa-36-1, Aa-1-2, Aa-2-2, Aa-4-2, Aa-32-2, Aa-44-1, Aa-45-1, Aa-47-1, Aa-48-1, Aa-49-1, Aa-49-2, Aa-50-1, Aa-51-1, Aa-53-1, Aa-54-1, Aa-56-1, Aa-57-1, Aa-60-1, Aa-61-1, Aa-66-1, Aa-67-1, Aa-68-1, Aa-69-1, Aa-70-1, Aa-71-1, Aa-72-1, Aa-73-1, Aa-74-1, Aa-75-1, Aa-76-1, Aa-77-1, Aa-83-1, Aa-84-1, Aa-86-1, Aa-96-1, Aa-131-1, Aa-133-1, Aa-134-1, Aa-135-1, Aa-137-1, Aa-138-1, Aa-166-1, Aa-167-1, Aa-168-1, Aa-169-1, Aa-170-1, Aa-171-1, Aa-172-1, Aa-173-1, Aa-174-1, Aa-175-1, Aa-176-1, Aa-177-1, Aa-178-1, Aa-181-1, Aa-182-1, Aa-184-1, Aa-217-1, Aa-223-2, Aa-226-2, Aa-230-3, Aa-240-1, Aa-250-3, Aa-253-1, Aa-260-1, Aa-261-2, Aa-262-1, Aa-262-2, Aa-278-2, Aa-279-2, Aa-280-2, Aa-281-2, Ba-1-2, Ba-64-1, Ba-76-1, Ba-120-1, Ba-121-1, Ba-122-1, Bb-1-2, Bb-7-1, Bb-45-1, Bb-49-1, Bb-53-1, Bb-63-1, Bb-64-1, Bb-65-1, Bb-71-3, Bb-73-1, Bb-76-1, Bb-77-3, Bb-119-1, Bb-124-1, Bb-127-1, Bb-1-5, Bb-137-1, Bb-141-1, Bb-143-3, Bb-152-1, Bb-156-1, Bb-157-1, Bb-159-1, Bb-160-1, Bb-161-1, Bb-162-1, Bb-137-2, Bb-138-2, Bb-139-2, Bc-1, Bc-48, Bc-73, Bc-127, Bc-129, Bc-262

### (2-3) Herbicidal effect on green foxtail (soil treatment)

To confirm herbicidal effect on green foxtail (Setaria viridis), the same test as in Test Example 2 (2-1) (soil treatment) was conducted using the following compounds. As a result, compositions containing the following compounds (active ingredient dose: 125, 250, 500 or 1,000 g/ha) showed a growth inhibition rate of 70% or higher on green foxtail.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-52-1, Aa-55-1, Aa-63-1, Aa-81-1, Ba-11-1, Ba-44-1, Ba-45-1, Ba-53-1, Ba-58-1, Bb-2-1, Bb-10-1, Bb-11-1, Bb-12-1, Bb-15-1, Bb-44-1, Bb-50-1, Bb-55-1, Bb-60-1, Bb-60-2, Bb-61-1, Bb-80-1, Bb-80-2, Bb-102-1, Bb-126-2, Bb-128-1, Bb-131-1, Bb-138-1, Bb-139-1, Bb-142-1, Bb-144-1, Bb-145-1, Bb-146-1, Bb-147-1, Bb-148-1, Bb-149-1, Bb-150-1, Bb-151-1, Bb-153-1, Bb-154-1, Bb-158-1, Bb-49-2, Bb-61-2, Bb-50-2, Bc-3, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-7-1, Aa-8-1, Aa-9-1, Aa-10-1, Aa-10-2, Aa-11-1, Aa-16-1, Aa-19-1, Aa-28-1, Aa-1-2, Aa-2-2, Aa-4-2, Aa-29-2, Aa-32-2, Aa-44-1, Aa-45-1, Aa-47-1, Aa-48-1, Aa-49-1, Aa-49-2, Aa-50-1, Aa-51-1, Aa-53-1, Aa-54-1, Aa-56-1, Aa-57-1, Aa-60-1, Aa-67-1, Aa-68-1, Aa-69-1, Aa-71-1, Aa-72-1, Aa-73-1, Aa-74-1, Aa-75-1, Aa-77-1, Aa-87-1, Aa-90-1, Aa-92-1, Aa-131-1, Aa-48-1 (Na salt), Aa-133-1, Aa-134-1, Aa-135-1, Aa-166-1, Aa-167-1, Aa-168-1, Aa-169-1, Aa-170-1, Aa-172-1, Aa-173-1, Aa-174-1, Aa-175-1, Aa-176-1, Aa-177-1, Aa-178-1, Aa-181-1, Aa-182-1, Aa-184-1, Aa-203-1, Aa-217-1, Aa-224-3, Aa-225-3, Aa-226-2, Aa-230-3, Aa-233-1, Aa-237-3, Aa-240-1, Aa-253-1, Aa-257-2, Aa-258-1, Aa-260-1, Aa-262-1, Aa-262-2, Aa-278-2, Aa-279-2, Aa-280-2, Aa-281-2, Ba-1-2, Ba-121-1, Ba-122-1, Bb-1-2, Bb-45-1, Bb-53-1, Bb-64-1, Bb-65-1, Bb-71-3, Bb-76-1, Bb-77-3, Bb-119-1, Bb-124-1, Bb-127-1, Bb-1-5, Bb-137-1, Bb-141-1, Bb-143-3, Bb-152-1, Bb-156-1, Bb-157-1, Bb-159-1, Bb-160-1, Bb-161-1, Bb-162-1, Bb-137-2, Bb-138-2, Bb-139-2, Bb-262-1, Bb-265-1, Bc-1, Bc-1(S), Bc-73, Bc-127, Bc-128, Bc-129, Bc-265

### (2-4) Herbicidal effect on Italian ryegrass (soil treatment)

To confirm herbicidal effect on Italian ryegrass (Lolium multiflorum), the same test as in Test Example 2 (2-1) (soil treatment) was conducted using the following compounds. As a result, compositions containing the following compounds (active ingredient dose: 125 or 500 g/ha) showed a growth inhibition rate of 70% or higher on Italian ryegrass.
Compounds (active ingredient dose: 500 g/ha): Comp No. Aa-9-2, Aa-52-1, Aa-55-1, Aa-62-1, Aa-63-1, Ba-44-1, Ba-45-1, Ba-53-1, Bb-2-1, Bb-60-1, Bb-80-1, Bb-80-2, Bb-102-1, Bb-158-1
Compounds (active ingredient dose: 125 g/ha): Comp No. Aa-2-1, Aa-4-1, Aa-6-1, Aa-7-1, Aa-8-1, Aa-9-1, Aa-10-1, Aa-10-2, Aa-11-1, Aa-12-1, Aa-13-1, Aa-14-1, Aa-15-1, Aa-16-1, Aa-17-1, Aa-19-1, Aa-20-1, Aa-21-1, Aa-25-1, Aa-27-1, Aa-28-1, Aa-29-1, Aa-33-1, Aa-34-1, Aa-36-1, Aa-1-2, Aa-12-2, Aa-2-2, Aa-4-2, Aa-29-2, Aa-44-1, Aa-45-1, Aa-47-1, Aa-48-1, Aa-49-1, Aa-49-2, Aa-50-1, Aa-51-1, Aa-53-1, Aa-54-1, Aa-56-1, Aa-57-1, Aa-58-3, Aa-60-1, Aa-61-1, Aa-64-1, Aa-66-1, Aa-67-1, Aa-68-1, Aa-71-1, Aa-72-1, Aa-73-1, Aa-74-1, Aa-75-1, Aa-76-1, Aa-77-1, Aa-85-2, Aa-86-1, Aa-87-1, Aa-89-1, Aa-90-1, Aa-92-1, Aa-101-1, Aa-102-3, Aa-103-2, Aa-113-1, Aa-115-1, Aa-131-1, Aa-48-1 (Na salt), Aa-133-1, Aa-134-1, Aa-135-1, Aa-137-1, Aa-138-1, Aa-156-3, Aa-166-1, Aa-167-1, Aa-168-1, Aa-169-1, Aa-170-1, Aa-171-1, Aa-172-1, Aa-173-1, Aa-174-1, Aa-175-1, Aa-176-1, Aa-177-1, Aa-178-1, Aa-180-1, Aa-181-1, Aa-182-1, Aa-184-1, Aa-185-1, Aa-203-1, Aa-210-1, Aa-217-1, Aa-218-1, Aa-223-3, Aa-224-3, Aa-225-3, Aa-223-2, Aa-226-1, Aa-226-2, Aa-230-3, Aa-233-1, Aa-235-1, Aa-237-3, Aa-240-1, Aa-244-1, Aa-250-3, Aa-253-1, Aa-255-1, Aa-257-1, Aa-257-2, Aa-258-1, Aa-260-1, Aa-261-2, Aa-262-1, Aa-262-2, Aa-263-1, Aa-277-2, Aa-278-2, Aa-279-2, Aa-280-2, Aa-281-2, Ba-1-2, Ba-76-1, Ba-119-1, Ba-121-1, Ba-122-1, Ba-124-1, Bb-1-2, Bb-64-1, Bb-71-3, Bb-76-1, Bb-77-3, Bb-124-1, Bb-127-1, Bb-156-1, Bb-159-1, Bb-160-1, Bb-161-1, Bb-262-1, Bb-265-1, Bc-1, Bc-1(S), Bc-44, Bc-53, Bc-73, Bc-127, Bc-129, Bc-262

### (2-5) Herbicidal effect on redroot pigweed (soil treatment)

To confirm herbicidal effect on redroot pigweed (Amaranthus retroflexus), the same test as in Test Example 2 (2-1) (soil treatment) was conducted using the following compounds. As a result, compositions containing the following compounds (active ingredient dose: 125, 250, 500 or 1,000 g/ha) showed a growth inhibition rate of 70% or higher on redroot pigweed.
Compounds (active ingredient dose: 1,000 g/ha): Comp No. Ba-3-1, Ba-19-1, Bb-3-1, Bb-8-1, Bb-19-1, Bb-20-1
Compounds (active ingredient dose: 500 g/ha): Comp No. Aa-9-2, Aa-52-1, Aa-62-1, Aa-63-1, Ba-11-1, Ba-44-1, Ba-45-1, Ba-53-1, Ba-58-1, Ba-118-3, Bb-2-1, Bb-9-1, Bb-10-1, Bb-11-1, Bb-12-1, Bb-15-1, Bb-44-1, Bb-50-1, Bb-55-1, Bb-60-1, Bb-61-1, Bb-80-1, Bb-80-2, Bb-102-1, Bb-126-2, Bb-128-1, Bb-130-1, Bb-131-1, Bb-132-1, Bb-138-1, Bb-139-1, Bb-142-1, Bb-144-1, Bb-146-1, Bb-147-1, Bb-149-1, Bb-150-1, Bb-153-1, Bb-154-1, Bb-158-1, Bb-49-2, Bb-61-2, Bb-50-2, Bc-3, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-2-1, Aa-4-1, Aa-7-1, Aa-8-1, Aa-10-1, Aa-10-2, Aa-11-1, Aa-16-1, Aa-19-1, Aa-27-1, Aa-29-1, Aa-30-1, Aa-31-1, Aa-32-1, Aa-33-1, Aa-1-2, Aa-12-2, Aa-2-2, Aa-4-2, Aa-32-2, Aa-44-1, Aa-45-1, Aa-48-1, Aa-49-2, Aa-51-1, Aa-53-1, Aa-54-1, Aa-56-1, Aa-57-1, Aa-60-1, Aa-67-1, Aa-68-1, Aa-71-1, Aa-72-1, Aa-73-1, Aa-74-1, Aa-75-1, Aa-77-1, Aa-83-1, Aa-84-1, Aa-92-1, Aa-133-1, Aa-134-1, Aa-135-1, Aa-166-1, Aa-167-1, Aa-168-1, Aa-169-1, Aa-170-1, Aa-172-1, Aa-173-1, Aa-174-1, Aa-175-1, Aa-217-1, Aa-224-3, Aa-230-3, Aa-240-1, Aa-253-1, Aa-254-1, Aa-260-1, Aa-262-2, Aa-278-2, Aa-280-2, Aa-281-2, Ba-1-2, Ba-120-1, Ba-121-1, Ba-122-1, Bb-1-2, Bb-7-1, Bb-45-1, Bb-49-1, Bb-53-1, Bb-64-1, Bb-73-1, Bb-76-1, Bb-77-3, Bb-119-1, Bb-124-1, Bb-127-1, Bb-1-5, Bb-137-1, Bb-141-1, Bb-143-3, Bb-152-1, Bb-156-1, Bb-157-1, Bb-159-1, Bb-160-1, Bb-161-1, Bb-162-1, Bb-137-2, Bb-138-2, Bb-139-2, Bb-262-1, Bc-1, Bc-48, Bc-49, Bc-53, Bc-60, Bc-72, Bc-73, Bc-127, Bc-129, Bc-262

### (2-6) Herbicidal effect on mexican burningbush (soil treatment)

To confirm herbicidal effect on mexican burningbush (Kochia scoparia), the same test as in Test Example 2 (2-1) (soil treatment) was conducted using the following compounds. As a result, compositions containing the following compounds (active ingredient dose: 125, 250, 500 or 1,000 g/ha) showed a growth inhibition rate of 70% or higher on mexican burningbush.
Compounds (active ingredient dose: 1,000 g/ha): Comp No. Ba-19-1, Bb-3-1, Bb-8-1, Bb-20-1
Compounds (active ingredient dose: 500 g/ha): Comp No. Ba-11-1, Ba-58-1, Ba-117-3, Ba-118-3, Bb-9-1, Bb-10-1, Bb-11-1, Bb-12-1, Bb-13-1, Bb-15-1, Bb-27-1, Bb-44-1, Bb-50-1, Bb-55-1, Bb-61-1, Bb-126-2, Bb-128-1, Bb-131-1, Bb-132-1, Bb-138-1, Bb-139-1, Bb-142-1, Bb-144-1, Bb-146-1, Bb-147-1, Bb-148-1, Bb-149-1, Bb-150-1, Bb-153-1, Bb-154-1, Bb-49-2, Bb-50-2, Bc-3, Bc-126
Compounds (active ingredient dose: 250 g/ha): Comp No. Aa-1-1, Aa-3-1, Ba-1-1, Bb-1-1, Bb-46-3, Bb-48-1, Bb-79-3, Bb-114-3, Bb-123-3, Bb-133-3, Bb-134-3, Bb-135-3, Bb-75-3, Bb-136-3, Bb-72-3, Bb-163-3, Bb-164-3, Bb-165-3
Compounds (active ingredient dose: 125 g/ha): Comp No. Aa-50-1, Bb-7-1, Bb-45-1, Bb-49-1, Bb-119-1, Bb-1-5, Bb-137-1, Bb-141-1, Bb-143-3, Bb-152-1, Bb-137-2, Bb-139-2

### (2-7) Herbicidal effect on velvetleaf (soil treatment)

To confirm herbicidal effect on velvetleaf (Abutilon theophrasti), the same test as in Test Example 2 (2-1) (soil treatment) was conducted using the following compounds. As a result, compositions containing the following compounds (active ingredient dose: 125, 500 or 1,000 g/ha) showed a growth inhibition rate of 70% or higher on velvetleaf.
Compounds (active ingredient dose: 1,000 g/ha): Comp No. Bb-3-1, Bb-8-1, Bb-19-1
Compounds (active ingredient dose: 500 g/ha): Comp No. Ba-45-1, Bb-2-1, Bb-10-1, Bb-12-1, Bb-50-1, Bb-131-1, Bb-132-1, Bb-138-1, Bb-146-1, Bb-153-1, Bb-154-1, Bb-158-1
Compounds (active ingredient dose: 125 g/ha): Comp No. Aa-45-1, Aa-224-3, Bb-76-1, Bb-119-1, Bb-157-1, Bc-129

### Test Example 3: (3-1) Safety for rice (foliar treatment)

To confirm safety for rice (Oryza sativa), the same test as in Test Example 1 (foliar treatment) was conducted using the following compounds. As a result, compositions containing the following compounds (active ingredient dose: 125 or 500 g/ha) showed a growth inhibition rate of less than 30% on rice, and thus safety for rice was confirmed.
Compounds (active ingredient dose: 500 g/ha): Comp No. Aa-43-1, Aa-52-1, Aa-55-1, Aa-62-1, Aa-63-1, Aa-81-1, Ba-45-1, Ba-58-1, Ba-116-3, Ba-117-3, Ba-118-3, Bb-26-1, Bb-55-1, Bb-61-1, Bb-80-1, Bb-144-1, Bb-145-1, Bb-146-1, Bb-148-1, Bb-150-1, Bb-151-1, Bb-153-1, Bb-154-1, Bb-61-2
Compounds (active ingredient dose: 125 g/ha): Comp No. Aa-12-1, Aa-13-1, Aa-17-1, Aa-23-1, Aa-24-1, Aa-26-1, Aa-26-2, Aa-35-1, Aa-37, Aa-39-1, Aa-40-1, Aa-41-1, Aa-12-2, Aa-4-2, Aa-29-2, Aa-32-2, Aa-42-1, Aa-45-1, Aa-47-1, Aa-58-3, Aa-59, Aa-65-1, Aa-68-1, Aa-69-1, Aa-70-1, Aa-78, Aa-84-1, Aa-87-1, Aa-88-1, Aa-95-1, Aa-96-1, Aa-98-1, Aa-100-1, Aa-101-1, Aa-102-3, Aa-103-2, Aa-106-1, Aa-113-1, Aa-115-1, Aa-171-1, Aa-179-1, Aa-183-1, Aa-186-1, Aa-187-1, Aa-188-1, Aa-189-1, Aa-190-1, Aa-191-1, Aa-192-1, Aa-193-1, Aa-194-1, Aa-195-1, Aa-196-1, Aa-197-1, Aa-198-1, Aa-199-1, Aa-200-1, Aa-201-1, Aa-202-1, Aa-203-1, Aa-204-1, Aa-205-1, Aa-206-1, Aa-207-1, Aa-208-1, Aa-209-1, Aa-210-1, Aa-211-1, Aa-213-1, Aa-214-1, Aa-216-1 (HCl salt), Aa-222-3, Aa-234-1, Aa-237-3, Aa-239-1, Aa-245-1, Aa-245-2, Aa-246-1, Aa-251-1, Aa-252-1, Aa-255-1, Aa-256-1, Aa-257-2, Aa-259-1, Aa-259-2, Aa-262-2, Aa-264-1, Aa-265-1, Aa-265-2, Aa-266-1, Aa-266-2, Aa-267-1, Aa-267-2, Aa-268-1, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-276-2, Aa-277-1, Aa-282-2, Aa-283-2, Aa-284-2, Aa-286-2, Aa-287-1, Ba-9-1, Ba-63-1, Ba-64-1, Ba-65-1, Ba-76-1, Ba-119-1, Ba-124-1, Ba-125-1, Ba-226-1, Bb-45-1, Bb-60-1, Bb-63-1, Bb-64-1, Bb-65-1, Bb-76-1, Bb-119-1, Bb-124-1, Bb-128-1, Bb-130-1, Bb-141-1, Bb-152-1, Bb-155-1, Bb-157-1, Bb-162-1, Bb-265-1, Bb-267-1, Bc-1(R), Bc-3, Bc-5, Bc-129, Bc-223-1, Bc-262, Bc-265, Bc-267

### (3-2) Safety for corn (foliar treatment)

To confirm safety for corn (Zea mays), the same test as in Test Example 1 (foliar treatment) was conducted using the following compounds. As a result, compositions containing the following compounds (active ingredient dose: 125 or 500 g/ha) showed a growth inhibition rate of less than 30% on corn, and thus safety for corn was confirmed.
Compounds (active ingredient dose: 500 g/ha): Comp No. Aa-42-1, Aa-43-1, Aa-52-1, Ba-116-3, Ba-118-3, Bb-61-1, Bb-148-1
Compounds (active ingredient dose: 125 g/ha): Comp No. Aa-24-1, Aa-37, Aa-40-1, Aa-47-1, Aa-58-3, Aa-59, Ba-9-1, Ba-63-1, Ba-65-1, Ba-76-1, Ba-124-1, Ba-125-1, Bb-63-1, Bb-65-1, Bb-119-1, Bb-124-1, Bb-155-1, Bb-162-1Aa-183-1, Aa-187-1, Aa-188-1, Aa-216-1 (HCl salt), Aa-231, Aa-239-1, Aa-251-1, Aa-266-2, Aa-275-2, Aa-275-1, Aa-276-2, Aa-287-1, Aa-287-2

### (3-3) Safety for wheat (foliar treatment)

To confirm safety for wheat (Triticum aestivum), the same test as in Test Example 1 (foliar treatment) was conducted using the following compounds. As a result, compositions containing the following compounds (active ingredient dose: 125, 250 or 500 g/ha) showed a growth inhibition rate of less than 30% on wheat, and thus safety for wheat was confirmed.
Compounds (active ingredient dose: 500 g/ha): Comp No. Aa-43-1, Aa-52-1, Aa-55-1, Ba-58-1, Ba-116-3, Ba-117-3, Ba-118-3, Bb-144-1, Bb-146-1, Bb-148-1, Bb-150-1, Bb-151-1, Bb-154-1, Bb-158-1
Compounds (active ingredient dose: 125 g/ha): Comp No. Aa-24-1, Aa-35-1, Aa-37, Aa-40-1, Aa-45-1, Aa-51-1, Aa-58-3, Aa-95-1, Aa-96-1, Aa-98-1, Aa-100-1, Ba-9-1, Ba-63-1, Ba-65-1, Ba-76-1, Ba-121-1, Ba-122-1, Ba-124-1, Bb-45-1, Bb-63-1, Bb-65-1, Bb-124-1, Bb-128-1, Bb-152-1, Bb-155-1, Bb-157-1, Bb-162-1, Bc-129, Aa-187-1, Aa-188-1, Aa-189-1, Aa-200-1, Aa-210-1, Aa-211-1, Aa-216-1 (HCl salt), Aa-239-1, Aa-245-1, Aa-245-2, Aa-251-1, Aa-266-1, Aa-266-2, Aa-268-1, Aa-270-2, Aa-272-1, Aa-274-2, Aa-275-2, Aa-276-2, Aa-285-2, Aa-286-2, Aa-287-1, Aa-287-2, Bc-262, Bc-267

### (3-4) Safety for soybean (foliar treatment)

To confirm safety for soybean (Glycine max), the same test as in Test Example 1 (foliar treatment) was conducted using the following compounds. As a result, compositions containing the following compounds (active ingredient dose: 125 or 500 g/ha) showed a growth inhibition rate of less than 30% on soybean, and thus safety for soybean was confirmed.
Compounds (active ingredient dose: 500 g/ha): Comp No. Aa-42-1, Aa-43-1, Aa-52-1, Aa-55-1, Ba-53-1, Ba-117-3, Ba-118-3, Bb-12-1, Bb-26-1, Bb-27-1, Bb-44-1, Bb-55-1, Bb-80-1, Bb-80-2, Bb-149-1, Bb-150-1, Bb-61-2, Bb-50-2
Compounds (active ingredient dose: 125 g/ha): Comp No. Aa-9-2, Aa-10-2, Aa-13-1, Aa-17-1, Aa-18-1, Aa-22-1, Aa-23-1, Aa-24-1, Aa-26-1, Aa-26-2, Aa-30-1, Aa-31-1, Aa-33-1, Aa-35-1, Aa-37, Aa-39-1, Aa-40-1, Aa-41-1, Aa-12-2, Aa-2-2, Aa-4-2, Aa-29-2, Aa-32-2, Aa-23-2, Aa-45-1, Aa-47-1, Aa-51-1, Aa-59, Aa-68-1, Aa-69-1, Aa-84-1, Aa-85-1, Aa-85-2, Aa-87-1, Aa-88-1, Aa-89-1, Aa-95-1, Aa-96-1, Aa-98-1, Aa-100-1, Aa-101-1, Aa-103-1, Aa-103-2, Aa-171-1, Aa-177-1, Aa-179-1, Aa-183-1, Aa-185-1, Aa-186-1, Aa-187-1, Aa-188-1, Aa-189-1, Aa-190-1, Aa-191-1, Aa-192-1, Aa-193-1, Aa-197-1, Aa-198-1, Aa-200-1, Aa-202-1, Aa-203-1, Aa-207-1, Aa-209-1, Aa-211-1, Aa-213-1, Aa-214-1, Aa-216-1 (HCl salt), Aa-218-1, Aa-219-3, Aa-220-3, Aa-224-3, Aa-223-2, Aa-220-2, Aa-226-2, Aa-229-2, Aa-231, Aa-237-3, Aa-239-1, Aa-241-1, Aa-242-1, Aa-245-1, Aa-245-2, Aa-246-1, Aa-251-1, Aa-252-1, Aa-253-1, Aa-255-1, Aa-256-1, Aa-257-2, Aa-258-1, Aa-259-2, Aa-261-2, Aa-265-1, Aa-265-2, Aa-266-1, Aa-266-2, Aa-267-2, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-276-2, Aa-277-1, Aa-277-2, Aa-278-2, Aa-281-2, Aa-282-2, Aa-283-2, Aa-284-2, Aa-285-2, Aa-286-2, Aa-287-1, Aa-287-2, Ba-9-1, Ba-63-1, Ba-119-1, Ba-120-1, Ba-121-1, Ba-124-1, Ba-125-1, Ba-223-1, Bb-53-1, Bb-132-1, Bb-147-1, Bb-155-1, Bb-138-2, Bb-139-2, Bc-1, Bc-1(R), Bc-1(S), Bc-3, Bc-44, Bc-49, Bc-53, Bc-128, Bc-223-1, Bc-262, Bc-267

### (3-5) Safety for rape (foliar treatment)

To confirm safety for rape (Brassica napus), the same test as in Test Example 1 (foliar treatment) was conducted using the following compounds. As a result, compositions containing the following compounds (active ingredient dose: 125 or 500 g/ha) showed a growth inhibition rate of less than 30% on rape, and thus safety for rape was confirmed.
Compounds (active ingredient dose: 500 g/ha): Comp Aa-42-1, Aa-43-1, Aa-52-1, Aa-55-1, Ba-44-1, Ba-53-1, Bb-12-1, Bb-27-1
Compounds (active ingredient dose: 125 g/ha): Comp No. Aa-9-2, Aa-16-1, Aa-18-1, Aa-22-1, Aa-25-1, Aa-35-1, Aa-37, Aa-39-1, Aa-40-1, Aa-41-1, Aa-12-2, Aa-2-2, Aa-4-2, Aa-29-2, Aa-32-2, Aa-22-2, Aa-23-2, Aa-50-1, Aa-51-1, Aa-53-1, Aa-69-1, Aa-70-1, Aa-84-1, Aa-85-1, Aa-85-2, Aa-87-1, Aa-101-1, Aa-103-1, Aa-103-2, Aa-104-1, Aa-106-1, Aa-108-1, Aa-109-1, Aa-110-1, Aa-111-1, Aa-112-1, Aa-156-3, Aa-186-1, Aa-187-1, Aa-189-1, Aa-190-1, Aa-191-1, Aa-192-1, Aa-195-1, Aa-200-1, Aa-201-1, Aa-202-1, Aa-203-1, Aa-205-1, Aa-206-1, Aa-209-1, Aa-211-1, Aa-212-1, Aa-213-1, Aa-214-1, Aa-215-1, Aa-216-1 (HCl salt), Aa-218-1, Aa-220-3, Aa-221-3, Aa-222-3, Aa-220-2, Aa-228-2, Aa-229-2, Aa-231, Aa-237-3, Aa-238-1, Aa-239-1, Aa-242-1, Aa-243-1, Aa-245-1, Aa-245-2, Aa-251-1, Aa-252-1, Aa-253-1, Aa-255-1, Aa-256-1, Aa-257-2, Aa-259-1, Aa-259-2, Aa-261-2, Aa-262-2, Aa-263-1, Aa-265-2, Aa-266-1, Aa-266-2, Aa-267-1, Aa-267-2, Aa-268-1, Aa-269-1, Aa-270-2, Aa-271-1, Aa-271-2, Aa-272-1, Aa-272-2, Aa-273-2, Aa-274-1, Aa-274-2, Aa-275-2, Aa-275-1, Aa-276-1, Aa-276-2, Aa-277-1, Aa-277-2, Aa-282-2, Aa-283-2, Aa-284-2, Aa-285-2, Aa-286-2, Aa-287-1, Aa-287-2, Ba-9-1, Ba-119-1, Ba-120-1, Ba-121-1, Bb-155-1, Bc-267

### Test Example 4: (4-1) Safety for rice (soil treatment)

To confirm safety for rice (Oryza sativa), the same test as in Test Example 2 (soil treatment) was conducted using the following compounds. As a result, compositions containing the following compounds (active ingredient dose: 125, 250 or 500 g/ha) showed a growth inhibition rate of less than 30% on rice, and thus safety for rice was confirmed.
Compounds (active ingredient dose: 500 g/ha): Comp No. Aa-52-1, Aa-55-1, Aa-62-1, Aa-63-1, Aa-81-1, Ba-116-3, Ba-117-3, Ba-118-3, Bb-12-1, Bb-27-1, Bb-61-1, Bb-128-1, Bb-130-1, Bb-132-1, Bb-144-1, Bb-145-1, Bb-146-1, Bb-148-1, Bb-150-1, Bb-151-1, Bb-154-1
Compounds (active ingredient dose: 250 g/ha): Comp No. Ba-1-1, Bb-1-1
Compounds (active ingredient dose: 125 g/ha): Comp No. Aa-6-1, Aa-9-1, Aa-11-1, Aa-12-1, Aa-13-1, Aa-14-1, Aa-16-1, Aa-17-1, Aa-19-1, Aa-20-1, Aa-21-1, Aa-25-1, Aa-27-1, Aa-28-1, Aa-29-1, Aa-30-1, Aa-31-1, Aa-32-1, Aa-33-1, Aa-34-1, Aa-36-1, Aa-12-2, Aa-29-2, Aa-32-2, Aa-45-1, Aa-47-1, Aa-58-3, Aa-64-1, Aa-68-1, Aa-69-1, Aa-70-1, Aa-76-1, Aa-83-1, Aa-84-1, Aa-85-2, Aa-86-1, Aa-87-1, Aa-89-1, Aa-90-1, Aa-96-1, Aa-101-1, Aa-102-3, Aa-103-2, Aa-113-1, Aa-115-1, Aa-156-3, Aa-171-1, Aa-180-1, Aa-182-1, Aa-185-1, Aa-203-1, Aa-210-1, Aa-218-1, Aa-223-3, Aa-224-3, Aa-225-3, Aa-226-1, Aa-226-2, Aa-235-1, Aa-237-3, Aa-244-1, Aa-250-3, Aa-254-1, Aa-255-1, Aa-257-1, Aa-257-2, Aa-258-1, Aa-261-2, Aa-262-1, Aa-262-2, Aa-263-1, Aa-277-2, Ba-64-1, Ba-76-1, Ba-119-1, Ba-124-1, Bb-7-1, Bb-45-1, Bb-63-1, Bb-64-1, Bb-65-1, Bb-73-1, Bb-76-1, Bb-119-1, Bb-124-1, Bb-141-1, Bb-152-1, Bb-156-1, Bb-157-1, Bb-162-1, Bb-262-1, Bb-265-1, Bc-44, Bc-48, Bc-49, Bc-53, Bc-60, Bc-72, Bc-128, Bc-262, Bc-265

### (4-2) Safety for corn (soil treatment)

To confirm safety for corn (Zea mays), the same test as in Test Example 2 (soil treatment) was conducted using the following compounds. As a result, compositions containing the following compounds (active ingredient dose: 125, 250 or 500 g/ha) showed a growth inhibition rate of less than 30% on corn, and thus safety for corn was confirmed.
Compounds (active ingredient dose: 500 g/ha): Comp No. Aa-81-1, Ba-11-1, Ba-117-3, Bb-10-1, Bb-11-1, Bb-12-1, Bb-13-1, Bb-27-1, Bb-60-1, Bb-60-2, Bb-61-1, Bb-80-1, Bb-128-1, Bb-130-1, Bb-1-4, Bb-142-1, Bb-144-1, Bb-145-1, Bb-148-1, Bb-150-1, Bb-151-1, Bb-61-2
Compounds (active ingredient dose: 250 g/ha): Comp No. Ba-1-1, Bb-114-3
Compounds (active ingredient dose: 125 g/ha): Comp No. Aa-12-1, Aa-13-1, Aa-14-1, Aa-17-1, Aa-20-1, Aa-28-1, Aa-29-1, Aa-30-1, Aa-31-1, Aa-32-1, Aa-33-1, Aa-12-2, Aa-45-1, Aa-47-1, Aa-70-1, Aa-76-1, Aa-83-1, Aa-85-2, Aa-89-1, Aa-96-1, Aa-101-1, Aa-102-3, Aa-103-2, Aa-156-3, Aa-180-1, Aa-185-1, Aa-203-1, Aa-210-1, Aa-218-1, Aa-235-1, Aa-244-1, Aa-250-3, Aa-254-1, Aa-255-1, Aa-260-1, Aa-261-2, Aa-262-1, Aa-262-2, Aa-263-1, Ba-64-1, Ba-124-1, Bb-7-1, Bb-45-1, Bb-63-1, Bb-65-1, Bb-119-1, Bb-1-5, Bb-137-1, Bb-141-1, Bb-152-1, Bb-157-1, Bb-162-1, Bb-137-2, Bb-139-2, Bb-262-1, Bc-53, Bc-60, Bc-72, Bc-262, Bc-265

### (4-3) Safety for wheat (soil treatment)

To confirm safety for wheat (Triticum aestivum), the same test as in Test Example 2 (soil treatment) was conducted using the following compounds. As a result, compositions containing the following compounds (active ingredient dose: 125 or 500 g/ha) showed a growth inhibition rate of less than 30% on wheat, and thus safety for wheat was confirmed.
Compounds (active ingredient dose: 500 g/ha): Comp No. Aa-52-1, Aa-81-1, Ba-45-1, Ba-116-3, Ba-117-3, Bb-12-1, Bb-13-1, Bb-27-1, Bb-61-1, Bb-128-1, Bb-130-1, Bb-144-1, Bb-145-1, Bb-146-1, Bb-148-1, Bb-150-1, Bb-151-1, Bb-154-1
Compounds (active ingredient dose: 125 g/ha): Comp No. Aa-12-1, Aa-13-1, Aa-14-1, Aa-28-1, Aa-29-1, Aa-30-1, Aa-31-1, Aa-32-1, Aa-33-1, Aa-45-1, Aa-69-1, Aa-70-1, Aa-76-1, Aa-84-1, Aa-86-1, Aa-96-1, Aa-101-1, Aa-102-3, Aa-103-2, Aa-156-3, Aa-203-1, Aa-235-1, Aa-250-3, Aa-254-1, Aa-261-2, Aa-262-1, Aa-262-2, Ba-64-1, Ba-76-1, Ba-119-1, Ba-124-1, Bb-7-1, Bb-45-1, Bb-63-1, Bb-64-1, Bb-65-1, Bb-76-1, Bb-119-1, Bb-124-1, Bb-152-1, Bb-157-1, Bb-162-1, Bc-1, Bc-1(S), Bc-44, Bc-53, Bc-60

### (4-4) Safety for soybean (soil treatment)

To confirm safety for soybean (Glycine max), the same test as in Test Example 2 (soil treatment) was conducted using the following compounds. As a result, compositions containing the following compounds (active ingredient dose: 125, 250, 500 or 1,000 g/ha) showed a growth inhibition rate of less than 30% on soybean, and thus safety for soybean was confirmed.
Compounds (active ingredient dose: 1,000 g/ha): Comp No. Ba-3-1, Bb-19-1, Bb-20-1
Compounds (active ingredient dose: 500 g/ha): Comp No. Aa-9-2, Aa-52-1, Aa-55-1, Aa-62-1, Aa-63-1, Aa-81-1, Ba-11-1, Ba-53-1, Ba-58-1, Ba-116-3, Ba-117-3, Ba-118-3, Bb-9-1, Bb-10-1, Bb-11-1, Bb-12-1, Bb-13-1, Bb-27-1, Bb-55-1, Bb-60-1, Bb-60-2, Bb-61-1, Bb-80-1, Bb-102-1, Bb-128-1, Bb-130-1, Bb-132-1, Bb-1-4, Bb-142-1, Bb-144-1, Bb-145-1, Bb-146-1, Bb-148-1, Bb-149-1, Bb-150-1, Bb-151-1, Bb-153-1, Bb-154-1, Bb-158-1, Bb-61-2, Bb-50-2, Bc-3, Bc-126
Compounds (active ingredient dose: 250 g/ha): Comp No. Aa-3-1, Ba-1-1, Bb-133-3, Bb-134-3, Bb-135-3, Bb-136-3
Compounds (active ingredient dose: 125 g/ha): Comp No. Aa-2-1, Aa-4-1, Aa-6-1, Aa-7-1, Aa-8-1, Aa-9-1, Aa-10-1, Aa-11-1, Aa-12-1, Aa-13-1, Aa-14-1, Aa-15-1, Aa-16-1, Aa-17-1, Aa-19-1, Aa-20-1, Aa-21-1, Aa-25-1, Aa-27-1, Aa-28-1, Aa-29-1, Aa-30-1, Aa-31-1, Aa-32-1, Aa-33-1, Aa-34-1, Aa-36-1, Aa-1-2, Aa-12-2, Aa-2-2, Aa-4-2, Aa-29-2, Aa-32-2, Aa-44-1, Aa-45-1, Aa-47-1, Aa-49-2, Aa-50-1, Aa-51-1, Aa-54-1, Aa-56-1, Aa-58-3, Aa-60-1, Aa-61-1, Aa-64-1, Aa-66-1, Aa-67-1, Aa-68-1, Aa-69-1, Aa-70-1, Aa-71-1, Aa-72-1, Aa-73-1, Aa-75-1, Aa-76-1, Aa-83-1, Aa-84-1, Aa-85-2, Aa-86-1, Aa-87-1, Aa-89-1, Aa-90-1, Aa-92-1, Aa-96-1, Aa-101-1, Aa-102-3, Aa-103-2, Aa-113-1, Aa-115-1, Aa-131-1, Aa-48-1 (Na salt), Aa-137-1, Aa-138-1, Aa-156-3, Aa-166-1, Aa-167-1, Aa-168-1, Aa-169-1, Aa-171-1, Aa-172-1, Aa-173-1, Aa-174-1, Aa-175-1, Aa-176-1, Aa-177-1, Aa-178-1, Aa-180-1, Aa-181-1, Aa-182-1, Aa-184-1, Aa-185-1, Aa-203-1, Aa-210-1, Aa-217-1, Aa-218-1, Aa-223-3, Aa-223-2, Aa-226-1, Aa-226-2, Aa-230-3, Aa-233-1, Aa-235-1, Aa-237-3, Aa-244-1, Aa-250-3, Aa-254-1, Aa-255-1, Aa-257-1, Aa-257-2, Aa-258-1, Aa-261-2, Aa-262-1, Aa-262-2, Aa-277-2, Aa-278-2, Aa-280-2, Aa-281-2, Ba-1-2, Ba-64-1, Ba-76-1, Ba-119-1, Ba-120-1, Ba-121-1, Ba-122-1, Ba-124-1, Bb-1-2, Bb-7-1, Bb-45-1, Bb-63-1, Bb-64-1, Bb-65-1, Bb-73-1, Bb-76-1, Bb-119-1, Bb-124-1, Bb-127-1, Bb-1-5, Bb-141-1, Bb-156-1, Bb-157-1, Bb-159-1, Bb-160-1, Bb-161-1, Bb-162-1, Bb-138-2, Bb-139-2, Bb-262-1, Bb-265-1, Bc-1, Bc-1(S), Bc-44, Bc-48, Bc-53, Bc-60, Bc-72, Bc-127, Bc-128, Bc-265

Now, the herbicidal effect of a composition containing compound 4b described in Patent Document 1 (Chinese Patent Publication No. 109896973) and the herbicidal effect of the present composition, on undesired plants, are shown in the following Comparative Test. The structures of the compounds used in the Comparative Test are shown in Table 3.

**[Table 3]**

| Compound No. | Test Compound |
|---|---|
| Comp No. Aa-1-1 | 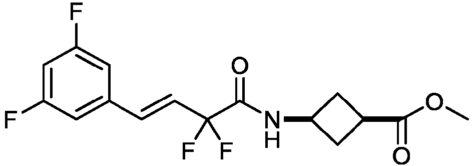 |
| Comp No. Aa-9-2 | 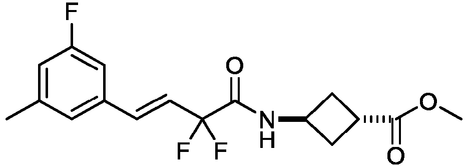 |
| Compound 4b (Chinese Patent Publication No. 109896973) | 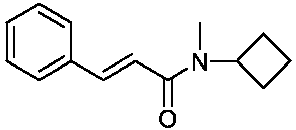 |

### Comparative Test 1: Herbicidal effect on undesired plants (foliar treatment)

Upland soil was put into a 1/300,000 hectare (ha) pot, seeds of barnyardgrass (Echinochloa crus-galli), crabgrass (Digitaria sanguinalis), green foxtail (Setaria viridis), wild oat (Avena fatua), Italian ryegrass (Lolium multiflorum), foxtail shortawn (Alopecurus aequalis), annual bluegrass (Poa annua), redroot pigweed (Amaranthus retroflexus), common chickweed (Stellaria media) common lambsquarters (Chenopodium album) and black nightshade (Solanum nigrum) were sown, and about 1.5 cm of cover soil was put. The plants were grown for 10 days in a greenhouse until they reached a predetermined leaf stage. The Invention Compound (Aa-1-1, Aa-9-2) or the compound 4b described in Patent Document 1 was weighed to achieve a predetermined active ingredient dose, dissolved in acetone (containing 0.5 vol% of surfactant (TritonX-100: manufactured by Kishida Chemical Co., Ltd.)) or dimethyl sulfoxide and diluted with water (containing 0.1 vol% of agricultural spreader (Surfactant WK: manufactured by MARUWA Biochemical Co., Ltd.) in an amount corresponding to 1,000 liter per hectare, to prepare each spray solution. Each spray solution was sprayed over the grown plants with a small sprayer for foliar treatment. On the 14th day after the foliar treatment, the state of growth of each plant was visually observed, and the herbicidal effect was evaluated in accordance with the growth inhibition rate (%) of from 0 (equivalent to the non-treated area) to 100 (complete kill). The results are shown in Table 4. A: growth inhibition rate of 90% or more, B: growth inhibition rate of 70% or more and less than 90%, C: growth inhibition rate of 50% or more and less than 70%, D: growth inhibition rate of 30% or more and less than 50%, E: growth inhibition rate of less than 30%

**[Table 4]**

| Compound No. | | Comp No. Aa-1-1 | | Comp No. Aa-9-2 | | Compound 4b | |
|---|---|---|---|---|---|---|---|
| Active ingredient amount (g/ha) | | 1000 | 500 | 1000 | 500 | 1000 | 500 |
| Growth inhibition rate | barnyardgrass | A | A | A | A | E | E |
| | crabgrass | A | A | A | A | E | E |
| | green foxtail | A | A | A | A | E | E |
| | wild oat | A | A | A | A | E | E |
| | Italian ryegrass | A | A | A | A | E | E |
| | foxtail shortawn | A | A | A | A | E | E |
| | annual bluegrass | A | A | A | A | E | E |
| | redroot pigweed | B | B | B | B | D | E |
| | common chickweed | A | A | A | A | D | E |
| | common lambsquarters | B | B | B | B | E | E |
| | black nightshade | A | B | B | C | A | E |

### Comparative Test 2: Herbicidal effect on undesired plants (soil treatment)

Upland soil was put into a 1/300,000 hectare (ha) pot, seeds of barnyardgrass (Echinochloa crus-galli), crabgrass (Digitaria sanguinalis), green foxtail (Setaria viridis), Italian ryegrass (Lolium multiflorum), redroot pigweed (Amaranthus retroflexus) and common chickweed (Stellaria media) were sown, and about 1.5 cm of cover soil was put. The Invention Compound (Aa-1-1, Aa-9-2) or the compound 4b described in Patent Document 1 was weighed to achieve a predetermined active ingredient dose, dissolved in acetone (containing 0.5 vol% of surfactant (TritonX-100: manufactured by Kishida Chemical Co., Ltd.)) or dimethyl sulfoxide and diluted with water (containing 0.1 vol% of agricultural spreader (Surfactant WK: manufactured by MARUWA Biochemical Co., Ltd.) in an amount corresponding to 1,000 liter per hectare, to prepare each spray solution. On the next day after sowing, each spray solution was sprayed with a small sprayer for soil treatment. On the 14th day after the soil treatment, the state of growth of each plant was visually observed, and the herbicidal effect was evaluated in accordance with the growth inhibition rate (%) of from 0 (equivalent to the non-treated area) to 100 (complete kill). The results are shown in Table 5. A: growth inhibition rate of 90% or more, B: growth inhibition rate of 70% or more and less than 90%, C: growth inhibition rate of 50% or more and less than 70%, D: growth inhibition rate of 30% or more and less than 50%, E: growth inhibition rate of less than 30%

**[Table 5]**

| Compound No. | | Comp No. Aa-1-1 | | Comp No. Aa-9-2 | | Compound 4b | |
|---|---|---|---|---|---|---|---|
| Active ingredient amount (g/ha) | | 1000 | 500 | 1000 | 500 | 1000 | 500 |
| Growth inhibition rate | barnyardgrass | A | A | A | A | E | E |
| | crabgrass | A | A | A | A | E | E |
| | green foxtail | A | A | A | A | E | E |
| | Italian ryegrass | A | A | A | A | E | E |
| | redroot pigweed | A | A | A | A | E | E |
| | common chickweed | A | A | A | A | E | E |

Now, formulation of the present composition will be specifically described. However, the mix ratio, the dosage form, etc. are not limited to the following Formulation Examples.

### Formulation Example 1

| | |
|---|---|
| (1) The Invention Compound | 50 parts by weight |
| (2) Salt of alkylnaphthalene sulfonate condensed with formaldehyde | 2 parts by weight |
| (3) Silicone oil | 0.2 parts by weight |
| (4) Water | 47.8 parts by weight |

The above components are uniformly mixed and pulverized to obtain a base liquid, and

| | |
|---|---|
| (5) Sodium polycarboxylate | 5 parts by weight |
| (6) Anhydrous sodium sulfate | 42.8 parts by weight |

are added, and the mixture is uniformly mixed, granulated and dried to obtain a water-dispersible granule.

### Formulation Example 2

| | |
|---|---|
| (1) Clay | 68 parts by weight |
| (2) Sodium lignin sulfonate | 2 parts by weight |
| (3) Polyoxyethylene styrylphenyl ether sulfate ammonium salt | 5 parts by weight |
| (4) White carbon | 25 parts by weight |

The mixture of the above components is mixed with the Invention Compound in a weight ratio of 4:1 to obtain a wettable powder.

### Formulation Example 3

| | |
|---|---|
| (1) The Invention Compound | 20 parts by weight |
| (2) Clay | 70 parts by weight |
| (3) White carbon | 5 parts by weight |
| (4) Polycarboxylate | 3 parts by weight |
| (5) Sodium alkylnaphthalene sulfonate | 2 parts by weight |

The above components are uniformly mixed to obtain a wettable powder.

### Formulation Example 4

| | |
|---|---|
| (1) The Invention Compound | 5 parts by weight |
| (2) Polyoxyethylene styrylphenyl ether | 1 part by weight |
| (3) Polyoxyethylene alkyl ether phosphate ester | 0.1 parts by weight |
| (4) Granular calcium carbonate | 93.9 parts by weight |

The above components (1) to (3) are preliminarily uniformly mixed and diluted with a proper amount of acetone, and then the mixture is sprayed onto the component (4), and acetone is removed to obtain a granule.

### Formulation Example 5

| | |
|---|---|
| (1) The Invention Compound | 20 parts by weight |
| (2) N,N'-dimethylacetamide | 20 parts by weight |
| (3) Polyoxyethylene styrylphenyl ether | 10 parts by weight |
| (4) Calcium dodecylbenzene sulfonate | 2 parts by weight |
| (5) Xylene | 48 parts by weight |

The above components are uniformly mixed and dissolved to obtain an emulsifiable concentrate.

### Formulation Example 6

| | |
|---|---|
| (1) The Invention Compound | 10 parts by weight |
| (2) Diethylene glycol monoethyl ether | 80 parts by weight |
| (3) Polyoxythylene alkyl ether | 10 parts by weight |

The above components are uniformly mixed to obtain a liquid.

### Formulation Example 7

| | |
|---|---|
| (1) The Invention Compound | 20 parts by weight |
| (2) Polyoxyethylene styrylphenyl ether phosphate calcium salt | 2 parts by weight |
| (3) Silicone oil | 0.2 parts by weight |
| (4) Xanthan gum | 0.1 parts by weight |
| (5) Ethylene glycol | 5 parts by weight |
| (6) Water | 72.7 parts by weight |

The above components are uniformly mixed and pulverized to obtain a water-based suspension.

The entire disclosure of Japanese Patent Application No. 2022-094501 filed on June 10, 2022 including specification, claims and abstract is incorporated herein by reference in its entirety.

## Claims

1. A herbicidal composition comprising as an active ingredient a difluorobutenoic acid amide compound represented by the formula (I) or a salt thereof: 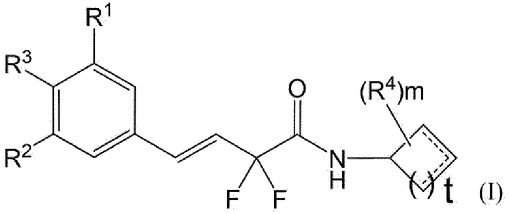
wherein R¹ and R² are each a halogen, a (C₁-C₆)-chain hydrocarbon which may be substituted by at least one Y¹, a (C₁-C₆)-alkoxy which may be substituted by at least one halogen, cyano or H,
R³ is H or a fluorine atom (excluding a case where all of R¹, R² and R³ are H),
Y' is a halogen, a (C₁-C₆)-alkoxy or cyano,
t is 1 or 2,
R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, -C(=O)H, -N(R¹⁰)C(=O)R¹¹, - SR⁶, -S(=O)R⁶, -S(=O)₂R⁶, =CHC(=O)R¹¹, cyano, OH, 1,3-dioxolan-2-yl, 2-thiazolyl, 2-imidazolyl which may be substituted by at least one Z⁴, =CHCH=CHC(=O)R¹¹, - (C=O)₂R¹¹, -C(=NR¹¹)-C(=O)R¹¹ or -CH(OH)-C(=O)R¹¹,
R⁵ is a (C₁-C₆)-alkyl which may be substituted by at least one Z¹, H, a (C₃-C₆)-cycloalkyl, a (C₂-C₆)-alkenyl, a (C₂-C₆)-alkynyl or a 4- to 6-membered oxygen-containing saturated heterocyclic ring,
Z¹ is cyano, a halogen, a (C₁-C₆)-alkylthio, a (C₁-C₆)-alkylsulfonyl, a (C₁-C₆)-alkoxy, phenyl, pyridyl, a (C₁-C₆)-alkoxycarbonyl, a (C₃-C₆)-cycloalkyl, tert-butyldimethylsilyloxy, OH, amino or 1,3-dioxolan-4-yl,
R⁶ is a (C₁-C₆)-alkyl which may be substituted by at least one halogen, a (C₃-C₆)-cycloalkyl or benzyl,
R⁷ is a (C₁-C₆)-alkyl which may be substituted by at least one Y², H, a (C₁-C₆)-alkoxy, a (C₂-C₆)-alkenyl, a (C₂-C₆)-alkynyl, -C(=O)R⁶, 3-pyridazinyl, a (C₂-C₆)-alkenyloxy, phenoxy, benzyl, a (C₁-C₆)-alkylsulfonyl or OH,
R⁸ is H, a (C₁-C₆)-alkyl or a (C₁-C₆)-alkoxy,
R⁷ and R⁸ together may form a completely saturated 4- to 6-membered ring with N to which they are bonded, the ring may be substituted by at least one halogen, and the carbon atom constituting the ring may be replaced with a hetero atom,
Y² is a (C₁-C₆)-alkoxy, a (C₁-C₆)-alkylthio, a (C₁-C₆)-alkoxycarbonyl, -(C=O)NH-(CH₂)-C(=O)R¹¹, a halogen or cyano,
R⁹ is a (C₁-C₆)-alkyl which may be substituted by at least one Z², or 1-imidazolyl, Z² is a (C₁-C₆)-alkoxycarbonyl,
Z³ is a halogen, a (C₁-C₆)-alkoxy, cyano, OH, a (C₁-C₆)-alkoxycarbonyl, a (C₁-C₆)-alkylcarbonyloxy, a (C₁-C₆)-alkylsulfonyloxy or -C(=O)OH,
Z⁴ is a (C₁-C₆)-alkyl, a (C₁-C₆)-alkoxymethyl or a (C₁-C₆)-alkoxycarbonyl,
R¹⁰ is H,
R¹¹ is a (C₁-C₆)-alkoxy,
m is 1, 2, 3 or 4, and
the broken line moiety of carbon-carbon bond is a single bond or a double bond (provided that two broken line moieties of carbon-carbon bond are not simultaneously double bonds).

2. A method for controlling undesired plants or inhibiting their growth, which comprises applying an effective amount of a difluorobutenoic acid amide compound represented by the formula (I) or a salt thereof to harmful weeds, useful plants or soil: 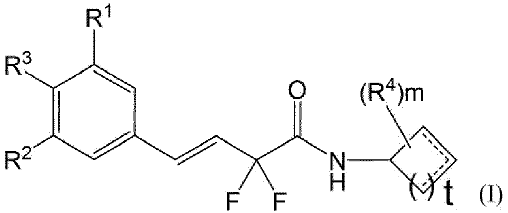
wherein R¹ and R² are each a halogen, a (C₁-C₆)-chain hydrocarbon which may be substituted by at least one Y¹, a (C₁-C₆)-alkoxy which may be substituted by at least one halogen, cyano or H,
R³ is H or a fluorine atom (excluding a case where all of R¹, R² and R³ are H),
Y¹ is a halogen, a (C₁-C₆)-alkoxy or cyano,
t is 1 or 2,
R⁴ is -C(=O)OR⁵, -C(=O)SR⁶, -C(=O)NR⁷R⁶, -C(=O)R⁹, a (C₁-C₆)-chain hydrocarbon which may be substituted by at least one Z³, -C(=O)H, -N(R¹⁰)C(=O)R¹¹, - SR⁶, -S(=O)R⁶, -S(=O)₂R⁶, =CHC(=O)R¹¹, cyano, OH, 1,3-dioxolan-2-yl, 2-thiazolyl, 2-imidazolyl which may be substituted by at least one Z⁴, =CHCH=CHC(=O)R¹¹, - (C=O)₂R¹¹, -C(=NR¹¹)-C(=O)R¹¹ or -CH(OH)-C(=O)R¹¹,
R⁵ is a (C₁-C₆)-alkyl which may be substituted by at least one Z¹, H, a (C₃-C₆)-cycloalkyl, a (C₂-C₆)-alkenyl, a (C₂-C₆)-alkynyl or a 4- to 6-membered oxygen-containing saturated heterocyclic ring,
Z¹ is cyano, a halogen, a (C₁-C₆)-alkylthio, a (C₁-C₆)-alkylsulfonyl, a (C₁-C₆)-alkoxy, phenyl, pyridyl, a (C₁-C₆)-alkoxycarbonyl, a (C₃-C₆)-cycloalkyl, tert-butyldimethylsilyloxy, OH, amino or 1,3-dioxolan-4-yl,
R⁶ is a (C₁-C₆)-alkyl which may be substituted by at least one halogen, a (C₃-C₆)-cycloalkyl or benzyl,
R⁷ is a (C₁-C₆)-alkyl which may be substituted by at least one Y², H, a (C₁-C₆)-alkoxy, a (C₂-C₆)-alkenyl, a (C₂-C₆)-alkynyl, -C(=O)R⁶, 3-pyridazinyl, a (C₂-C₆)-alkenyloxy, phenoxy, benzyl, a (C₁-C₆)-alkylsulfonyl or OH,
R⁸ is H, a (C₁-C₆)-alkyl or a (C₁-C₆)-alkoxy,
R⁷ and R⁸ together may form a completely saturated 4- to 6-membered ring with N to which they are bonded, the ring may be substituted by at least one halogen, and the carbon atom constituting the ring may be replaced with a hetero atom,
Y² is a (C₁-C₆)-alkoxy, a (C₁-C₆)-alkylthio, a (C₁-C₆)-alkoxycarbonyl, -(C=O)NH-(CH₂)-C(=O)R¹¹, a halogen or cyano,
R⁹ is a (C₁-C₆)-alkyl which may be substituted by at least one Z², or 1-imidazolyl,
Z² is a (C₁-C₆)-alkoxycarbonyl,
Z³ is a halogen, a (C₁-C₆)-alkoxy, cyano, OH, a (C₁-C₆)-alkoxycarbonyl, a (C₁-C₆)-alkylcarbonyloxy, a (C₁-C₆)-alkylsulfonyloxy or -C(=O)OH,
Z⁴ is a (C₁-C₆)-alkyl, a (C₁-C₆)-alkoxymethyl or a (C₁-C₆)-alkoxycarbonyl,
R¹⁰ is H,
R¹¹ is a (C₁-C₆)-alkoxy,
m is 1, 2, 3 or 4, and
the broken line moiety of carbon-carbon bond is a single bond or a double bond (provided that two broken line moieties of carbon-carbon bond are not simultaneously double bonds).

3. A difluorobutenoic acid amide compound represented by the formula (I) or a salt thereof: 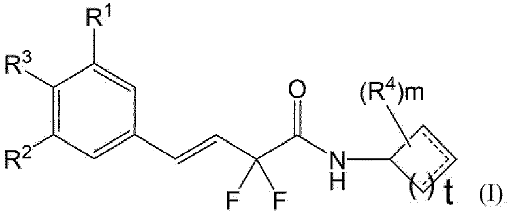
wherein R¹ and R² are each a halogen, a (C₁-C₆)-chain hydrocarbon which may be substituted by at least one Y¹, a (C₁-C₆)-alkoxy which may be substituted by at least one halogen, cyano or H,
R³ is H or a fluorine atom (excluding a case where all of R¹, R² and R³ are H),
Y¹ is a halogen, a (C₁-C₆)-alkoxy or cyano,
t is 1 or 2,
R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, -C(=O)H, -N(R¹⁰)C(=O)R¹¹, - SR⁶, -S(=O)R⁶, -S(=O)₂R⁶, =CHC(=O)R¹¹, cyano, OH, 1,3-dioxolan-2-yl, 2-thiazolyl, 2-imidazolyl which may be substituted by at least one Z⁴, =CHCH=CHC(=O)R¹¹, - (C=O)₂R¹¹, -C(=NR¹¹)-C(=O)R¹¹ or -CH(OH)-C(=O)R¹¹,
R⁵ is a (C₁-C₆)-alkyl which may be substituted by at least one Z¹, H, a (C₃-C₆)-cycloalkyl, a (C₂-C₆)-alkenyl, a (C₂-C₆)-alkynyl or a 4- to 6-membered oxygen-containing saturated heterocyclic ring,
Z¹ is cyano, a halogen, a (C₁-C₆)-alkylthio, a (C₁-C₆)-alkylsulfonyl, a (C₁-C₆)-alkoxy, phenyl, pyridyl, a (C₁-C₆)-alkoxycarbonyl, a (C₃-C₆)-cycloalkyl, tert-butyldimethylsilyloxy, OH, amino or 1,3-dioxolan-4-yl,
R⁶ is a (C₁-C₆)-alkyl which may be substituted by at least one halogen, a (C₃-C₆)-cycloalkyl or benzyl,
R⁷ is a (C₁-C₆)-alkyl which may be substituted by at least one Y², H, a (C₁-C₆)-alkoxy, a (C₂-C₆)-alkenyl, a (C₂-C₆)-alkynyl, -C(=O)R⁶, 3-pyridazinyl, a (C₂-C₆)-alkenyloxy, phenoxy, benzyl, a (C₁-C₆)-alkylsulfonyl or OH,
R⁸ is H, a (C₁-C₆)-alkyl or a (C₁-C₆)-alkoxy,
R⁷ and R⁸ together may form a completely saturated 4- to 6-membered ring with N to which they are bonded, the ring may be substituted by at least one halogen, and the carbon atom constituting the ring may be replaced with a hetero atom,
Y² is a (C₁-C₆)-alkoxy, a (C₁-C₆)-alkylthio, a (C₁-C₆)-alkoxycarbonyl, -(C=O)NH-(CH₂)-C(=O)R¹¹, a halogen or cyano,
R⁹ is a (C₁-C₆)-alkyl which may be substituted by at least one Z², or 1-imidazolyl,
Z² is a (C₁-C₆)-alkoxycarbonyl,
Z³ is a halogen, a (C₁-C₆)-alkoxy, cyano, OH, a (C₁-C₆)-alkoxycarbonyl, a (C₁-C₆)-alkylcarbonyloxy, a (C₁-C₆)-alkylsulfonyloxy or -C(=O)OH,
Z⁴ is a (C₁-C₆)-alkyl, a (C₁-C₆)-alkoxymethyl or a (C₁-C₆)-alkoxycarbonyl,
R¹⁰ is H,
R¹¹ is a (C₁-C₆)-alkoxy,
m is 1, 2, 3 or 4, and
the broken line moiety of carbon-carbon bond is a single bond or a double bond (provided that two broken line moieties of carbon-carbon bond are not simultaneously double bonds).

4. The difluorobutenoic acid amide compound or a salt thereof according to Claim 3, wherein R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, or -CH(OH)-C(=O)R¹¹, R⁷ is a (C₁-C₆)-alkyl which may be substituted by at least one Y², H or a (C₁-C₆)-alkoxy, and Z³ is a (C₁-C₆)-alkoxycarbonyl or -C(=O)OH.

5. The difluorobutenoic acid amide compound or a salt thereof according to Claim 3, wherein R¹ and R² are each a halogen, a (C₁-C₆)-alkyl which may be substituted by at least one Y¹, a (C₁-C₆)-alkoxy which may be substituted by at least one halogen, cyano or H, Y¹ is a halogen, R⁴ is -C(=O)OR⁵, -C(=O)SR⁶, -C(=O)NR⁷R⁸, -C(=O)R⁹, a (C₁-C₆)-chain hydrocarbon which may be substituted by at least one Z³, or -CH(OH)-C(=O)R¹¹, R⁷ is a (C₁-C₆)-alkyl which may be substituted by at least one Y², H or a (C₁-C₆)-alkoxy, and Z³ is a (C₁-C₆)-alkoxycarbonyl or -C(=O)OH.

6. The difluorobutenoic acid amide compound or a salt thereof according to Claim 3, wherein R¹ and R² are each a halogen, a (C₁-C₆)-alkyl which may be substituted by at least one Y¹, a (C₁-C₆)-alkoxy which may be substituted by at least one halogen, cyano or H, Y¹ is a halogen, R⁴ is -C(=O)OR⁵, -C(=O)SR⁶, -C(=O)NR⁷R⁸ or -CH(OH)-C(=O)R¹¹, R⁷ is a (C₁-C₆)-alkyl which may be substituted by at least one Y², H or a (C₁-C₆)-alkoxy, Z¹ is cyano, a halogen, a (C₁-C₆)-alkylthio, a (C₁-C₆)-alkylsulfonyl, a (C₁-C₆)-alkoxy or 1,3-dioxolan-4-yl, R⁶ is a (C₁-C₆)-alkyl which may be substituted by at least one halogen, R⁷ is a (C₁-C₆)-alkyl which may be substituted by at least one Y², H or a (C₁-C₆)-alkoxy, R⁸ is H or a (C₁-C₆)-alkyl, and Y² is a (C₁-C₆)-alkoxy, a halogen or cyano.

7. The difluorobutenoic acid amide compound or a salt thereof according to any one of Claims 3 to 6, wherein m is 1 or 2.

8. A herbicidal composition comprising as an active ingredient the difluorobutenoic acid amide compound or a salt thereof as defined in any one of Claims 4 to 6.

9. A method for controlling undesired plants or inhibiting their growth, which comprises applying an effective amount of the difluorobutenoic acid amide compound or a salt thereof as defined in any one of Claims 4 to 6, to harmful weeds, useful plants or soil.
